# EUROPEAN PATENT APPLICATION

(11) **EP 4 286 393 A1**
(43) Date of publication of application: **06.12.2023**
(21) Application number: 22746065.6
(22) Date of filing: 31.01.2022
(51) Int. Cl.: C07H 5/02, C07H 13/08, C07H 19/067, C07H 19/073, C07H 19/167, C07H 19/207, C07H 19/213, C07H 19/23, C07H 21/02, C07K 7/06, C07K 16/00, C07K 16/18, C07D 487/04, A61K 47/68

(54) **NOVEL METHOD FOR PRODUCING ANTIBODY-IMMUNOSTIMULATOR CONJUGATE**

(30) Priority: 01.02.2021 JP 2021014624; 09.04.2021 JP 2021066316
(71) Applicant: Daiichi Sankyo Company, Limited, Tokyo 103-8426 (JP)
(72) Inventor: OGURA, Tomokazu, Tokyo 103-8426 (JP); NAKAYA, Takeshi, Tokyo 103-8426 (JP); INOUE, Hidekazu, Tokyo 103-8426 (JP); ABE, Narumi, Tokyo 103-8426 (JP); ABE, Yuzo, Tokyo 103-8426 (JP); NAKAMURA, Tatsuya, Tokyo 103-8426 (JP); YAMAMOTO, Yuko, Tokyo 103-8426 (JP); SAKANISHI, Kohei, Tokyo 103-8426 (JP); SAKAMOTO, Tatsuhiro, Tokyo 103-8426 (JP); NAKANE, Satoshi, Tokyo 103-8426 (JP)
(74) Representative: Marks & Clerk LLP
(86) International application number: PCT/JP2022/003512
(87) International publication number: WO 2022/163846

(57) **Abstract**

The purpose of the present invention is to provide a novel stereoselective method for preparing a cyclic dinucleotide derivative and a production intermediate therefor, which can be used for an antibody-immunostimulant conjugate. Also provided is a method for producing a cyclic dinucleotide-linker and an antibody-immunostimulant conjugate while using the above production method. Further provided is a method for preparing a cyclic dinucleotide derivative, the method including the step of subjecting a compound (I) and a compound (IV) to stereoselective condensation using an optically active phosphitylating agent (Rc-II) or (Sc-II).

## Description

### Technical Field

The present invention relates to a novel stereoselective method for preparing a cyclic dinucleotide derivative and to a production intermediate therefor, which can be used for an antibody-immunostimulant conjugate. The present invention also relates to a novel method for preparing a cyclic dinucleotide-linker and an antibody-immunostimulant conjugate, the method including the above preparation method. The present invention further relates to a novel method for preparing a raw material compound used in the preparation of cyclic dinucleotide derivative, and a production intermediate therefor.

### Background Art

Cyclic dinucleotides (hereinafter, also referred to as CDN(s)) can activate STING (Stimulator of Interferon Genes) (Non-Patent Literature 1). This activation has been shown to enhance a STING-mediated anti-tumor immune response, markedly inhibit tumor growth, and improve the survival rate of mice when a CDN is administered to tumor-bearing mice (Non-Patent Literature 2). In recent studies, synthetic small molecule compounds with *in vivo* STING agonist activity without degradation by nucleases have been developed (e.g., Patent Literatures 1 to 5). An Antibody Drug Conjugate (ADC), in which a CDN exhibiting such activity is conjugated to an antibody via a linker, has also been studied; and antitumor effects have been exerted in antigen-expressing tumors (Patent Literatures 6 and 7).

The CDNs studied so far have two sulfur-modified pentavalent phosphate bonds, called phosphorothioate bonds, in the cyclic linkage of the two nucleotides. When asymmetric nucleotides are cyclized with each other, a chiral point is generated on the phosphorus atom of each phosphorothioate moiety. In the previously reported CDN synthesis methods, a CDN is synthesized without controlling the chirality on each phosphorus. The resulting four diastereomers are then purified. In this way, a CDN with the desired absolute stereo configuration is obtained (e.g., Non-Patent Literature 3, Patent Literatures 1 to 7). In the case of such a synthesis method, the formation of four diastereomers results in low yield; because the diastereomers have similar physical properties, their separation and purification require strict preparative purification by HPLC with low loading. Thus, a significant burden has been imposed on the synthesis of a large amount of the target compound by such a synthesis method. There is a report on a stereoselective CDN synthesis method using a compound in which pentavalent phosphorus and a chiral auxiliary group are combined. Unfortunately, the yield upon cyclization remains low (Non-Patent Literature 4). There have also been several research examples of stereoselective phosphorothioate synthesis method involving stereocontrolled oligonucleotides in which trivalent phosphorus and a chiral auxiliary group are combined (e.g., Non-Patent Literatures 5 to 9, Patent Literatures 8 to 9). On the other hand, there are no examples of dinucleotide bond formation using a compound applicable to cyclic dinucleotide synthesis. In fact, there are also no examples of stereoselective synthesis of a cyclic dinucleotide.

### Citation List

### Patent Literature

Patent Literature 1: WO2014/189805
Patent Literature 2: WO2014/189806
Patent Literature 3: WO2016/145102
Patent Literature 4: WO2017/093933
Patent Literature 5: WO2018/060323
Patent Literature 6: WO2020/050406
Patent Literature 7: WO2021/177438
Patent Literature 8: WO2005/092909
Patent Literature 9: WO2016/012305

### Non-Patent Literature

Non Patent Literature 1: Mol. Cell, 2013, 51, 226-235
Non Patent Literature 2: Sci. Rep. 2016, 6, 19049
Non Patent Literature 3: J. Med. Chem. 2016. 59. 10253-10267
Non Patent Literature 4: Science 2018. 361. 1234-1238
Non Patent Literature 5: Tetrahedron Lett. 1998. 39. 2491-2494
Non Patent Literature 6: Bioorg. Med. Chem. Lett. 1998. 8. 2539-2544
Non Patent Literature 7: J. Am. Chem. Soc. 2002. 124. 4962-4963
Non Patent Literature 8: J. Am. Chem. Soc. 2003. 125. 8307-8317
Non Patent Literature 9: J. Am. Chem. Soc. 2008. 130. 16031-16037

### Summary of Invention

### Technical Problem

An aspect of the present invention provides a novel method for preparing a cyclic dinucleotide derivative useful as an intermediate for an antibody-drug conjugate, especially an antibody-immunostimulant conjugate that has STING agonist activity and activates immune cells.

Stereoselective synthesis of a cyclic dinucleotide requires a method in which a nucleotide with a strong acidic functional group, phosphite, and a nucleotide with an optically active amidite moiety, which is significantly unstable to acidity, are bonded in practical yield and high stereoselectivity. However, because the conventional preparation method is not a stereoselective synthesis method, the total yield is remarkably low due to the low production ratio of a material of interest and the associated difficulty in purification.

Therefore, the present invention addresses one of the problems of providing an industrially superior novel method for preparing a CDN such that stereoselective synthesis is used to increase the production ratio of a material of interest, thereby reducing the purification load and increasing the total yield. Also, provided is a novel method for preparing a CDN-linker using the above method.

In addition, the present invention addresses one of the problems of providing a novel preparation method including preparing a raw material compound used for synthesizing a cyclic dinucleotide such that the method has a decreased number of steps and a higher yield.

### Solution to Problem

In order to solve the above problems, the present inventors, after careful investigation, have discovered a stereoselective method of synthesizing a cyclic dinucleotide derivative by using an optically active phosphitylating agent. This reduces the diastereomer purification load and a novel preparation method having an improved total yield has been completed. In addition, a construction of a CDN-linker using the CDN obtained by the above preparation method and a construction of an antibody-immunostimulant conjugate using the CDN-linker are established. Further, the present inventors have found a novel preparation method including preparing a raw material compound used for preparing a cyclic dinucleotide such that the method has a less number of steps and a higher yield than conventional preparation methods. Thus, the present invention has then been completed.

Specifically, the present invention pertains to the following.
[1] A method for preparing a compound represented by formula (Rp-VII): 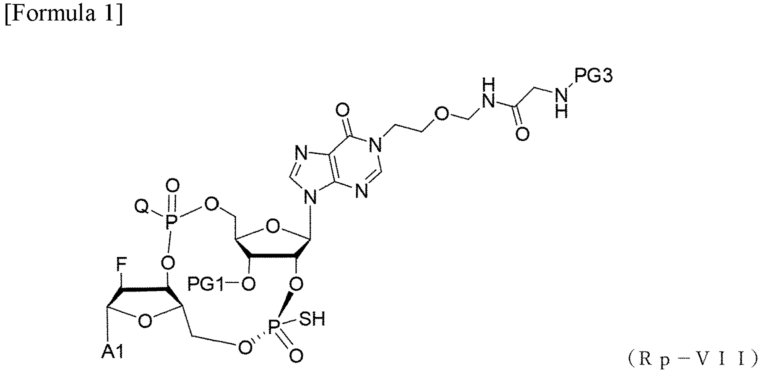 or a salt thereof, wherein
   A1 is 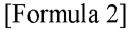 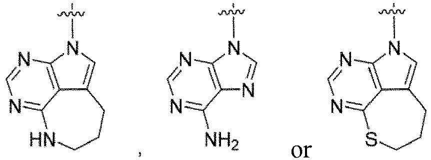
   Q is a thiol group or a hydroxy group,
   PG1 is a protecting group for a hydroxy group, and
   PG3 is a protecting group for an amino group, the method comprising the steps of:
      (step a1) reacting a compound represented by formula (I): 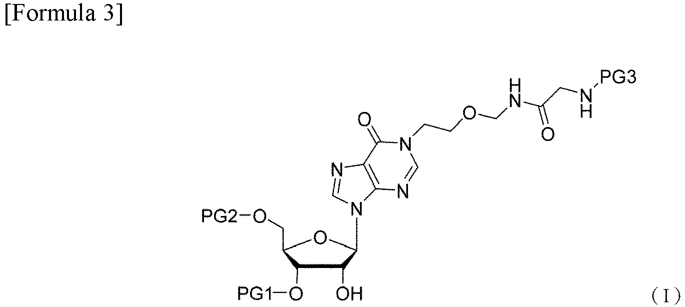 wherein
         PG1 and PG3 are as defined above, and
         PG2 is a protecting group for a hydroxy group,
         with an optically active phosphitylating agent (Rc-II) selected from the group consisting of the following formulas (Rc-II-1) and (Rc-II-2): 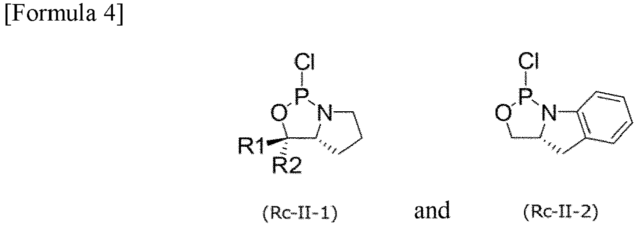 wherein
         R1 is hydrogen or methyl, and
         R2 is hydrogen, C₁₋₃ alkyl, or phenyl,
            wherein the alkyl is unsubstituted or substituted with one or more phenyl, tosyl, or diphenylmethylsilyl, and
            the phenyl is unsubstituted or substituted with nitro or methoxy to obtain a compound represented by formula (Rc-III): 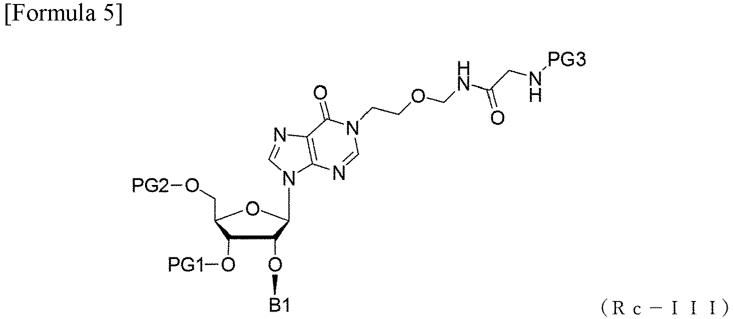 wherein
               PG1, PG2, and PG3 are as defined above, and
               B1 is 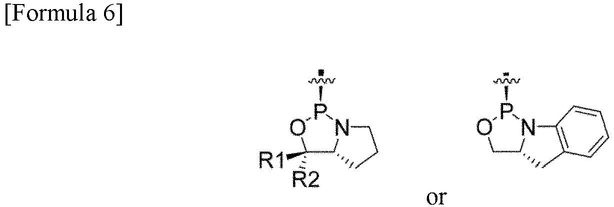 wherein R1 and R2 are as defined above;
      (step a2) reacting, in the presence of an activator, the resulting compound of formula (Rc-III) with a compound represented by formula (IV): 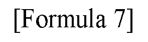 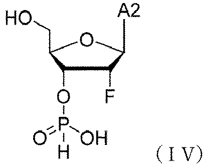 or a salt thereof, wherein
         A2 is 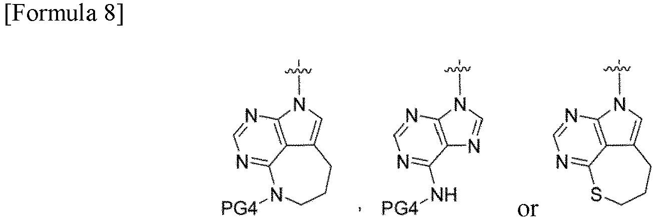 wherein PG4 is a protecting group for an amino group,
            followed by treatment with an acylating agent or alkoxycarbonylating agent, a further reaction with a sulfurizing agent, and then removal of PG2 by deprotection to obtain a compound represented by (Rc-V): 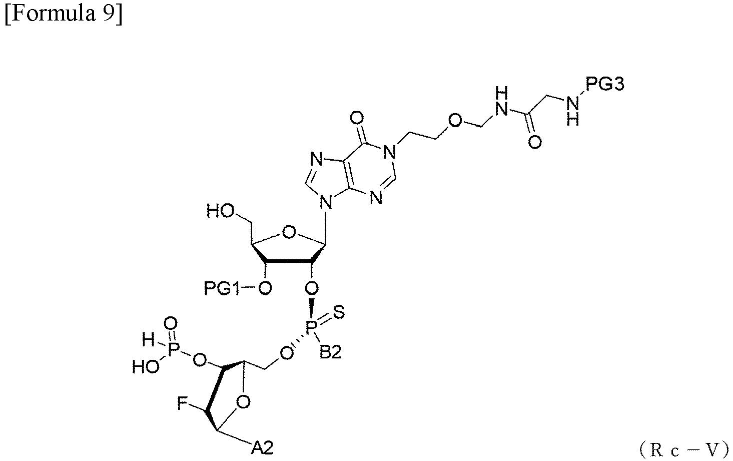
         or a salt thereof, wherein
         A2, PG1, and PG3 are as defined above,
         B2 is 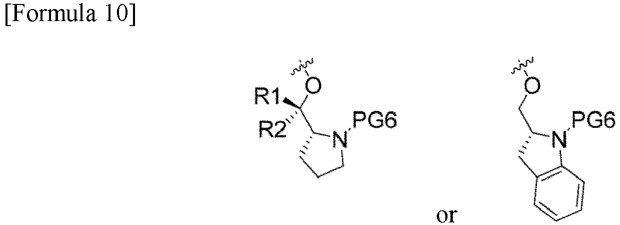 wherein PG6 is a protecting group for an amino group;
      (step a3) cyclizing the resulting compound of formula (Rc-V) or a salt thereof in the presence of a condensing agent, followed by a reaction with a sulfurizing or oxidizing agent to obtain a compound represented by formula (Rc-VI): 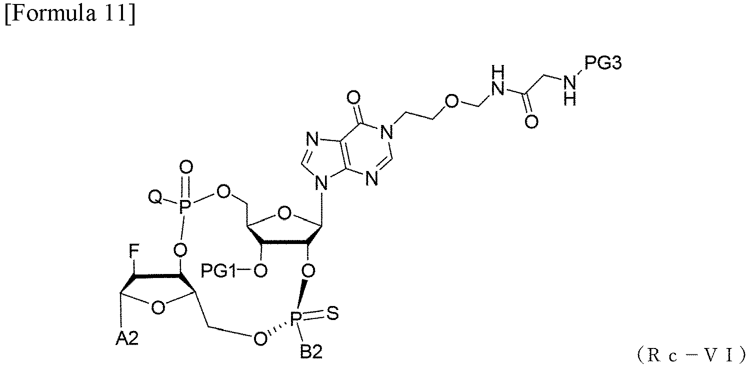
         or a salt thereof, wherein
         A2, B2, Q, PG1, and PG3 are as defined above; and
      (step a4) removing B2, which is a protecting group for a thiophosphoric acid moiety in the resulting compound of formula (Rc-VI), and PG4, which is a protecting group in A2, by deprotection to obtain a compound of formula (Rp-VII) or a salt thereof.
[2] The method according to [1], wherein in step a2, an intermediate obtained after the reaction with the sulfurizing agent but before the removal of PG2 by deprotection is a compound represented by formula (Rc-V₀): 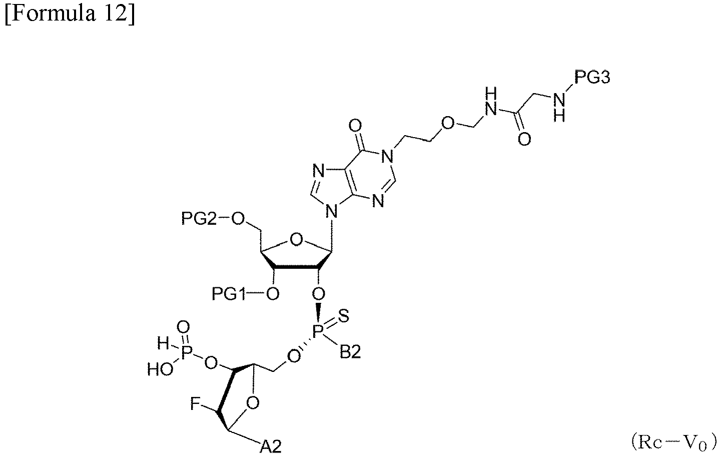
   or a salt thereof, wherein
   A2, B2, PG1, PG2, and PG3 are as defined in [1].
[3] A method for preparing a compound represented by formula (Sp-VII): 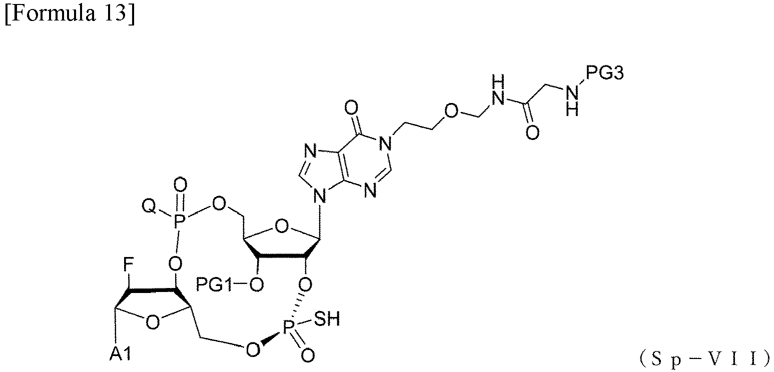
   or a salt thereof, wherein
   A1 is 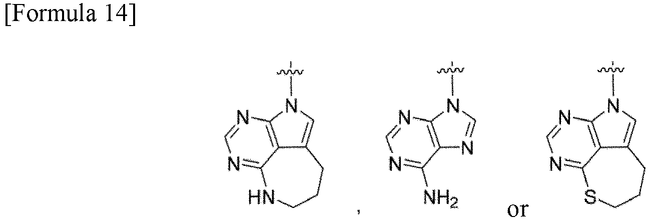
   Q is a thiol group or a hydroxy group,
   PG1 is a protecting group for a hydroxy group, and
   PG3 is a protecting group for an amino group, the method comprising the steps of:
      (step a1) reacting a compound represented by formula (I): 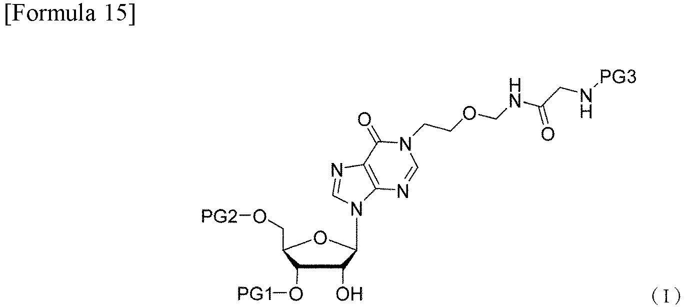 wherein
         PG1 and PG3 are as defined above, and
         PG2 is a protecting group for a hydroxy group,
         with an optically active phosphitylating agent (Sc-II) selected from the group consisting of the following formulas (Sc-II-1) and (Sc-II-2): 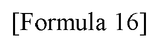 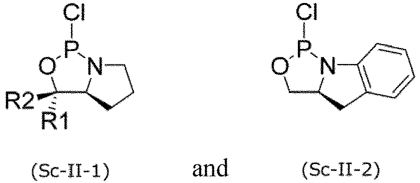 wherein
         R1 is hydrogen or methyl, and
         R2 is hydrogen, C₁₋₃ alkyl, or phenyl,
            wherein the alkyl is unsubstituted or substituted with one or more phenyl, tosyl, or diphenylmethylsilyl, and
            the phenyl is unsubstituted or substituted with nitro or methoxy, to obtain a compound represented by formula (Sc-III): 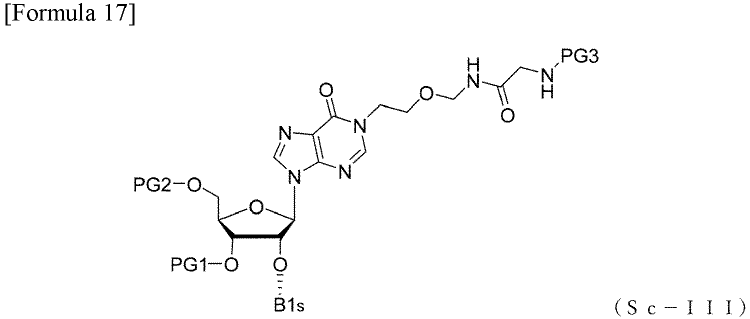
            wherein
         PG1, PG2, and PG3 are as defined above, and
         B1_{S} is 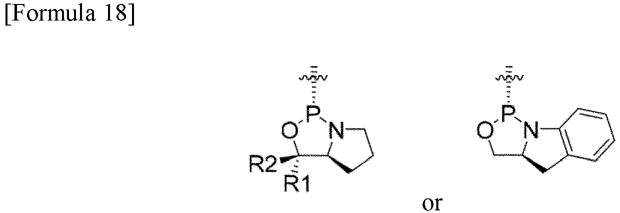 wherein R1 and R2 are as defined above;
      (step a2) reacting, in the presence of an activator, the resulting compound of formula (Sc-III) with a compound represented by formula (IV): 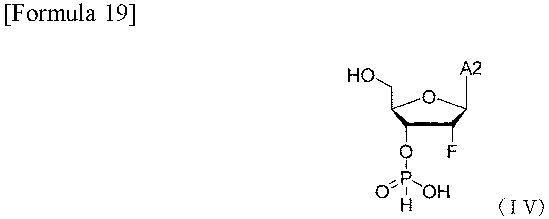
         or a salt thereof, wherein
         A2 is 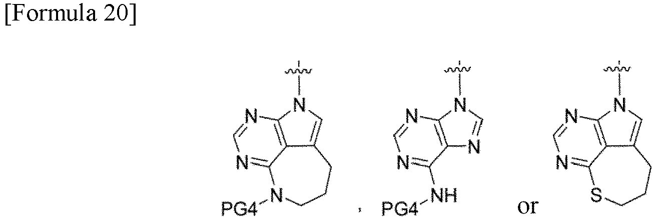 wherein PG4 is a protecting group for an amino group,
         followed by treatment with an acylating agent or alkoxycarbonylating agent, a further reaction with a sulfurizing agent, and then removal of PG2 by deprotection to obtain a compound represented by (Sc-V): 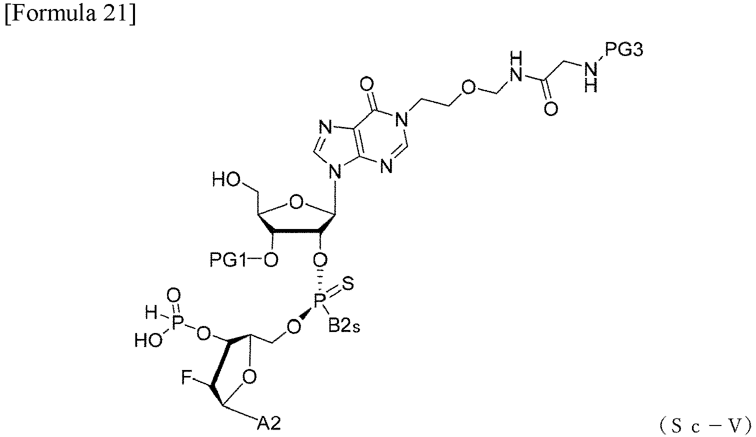
         or a salt thereof, wherein
         A2, PG1, and PG3 are as defined above, and
         B2s is 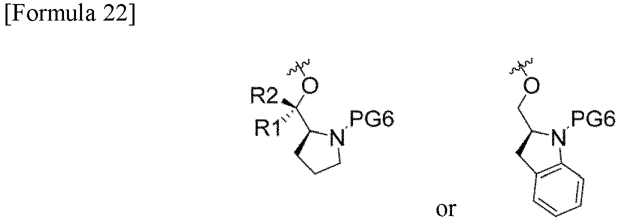 wherein PG6 is a protecting group for an amino group;
      (step a3) cyclizing the resulting compound of formula (Sc-V) or a salt thereof in the presence of a condensing agent, followed by a reaction with a sulfurizing or oxidizing agent to obtain a compound represented by formula (Sc-VI): 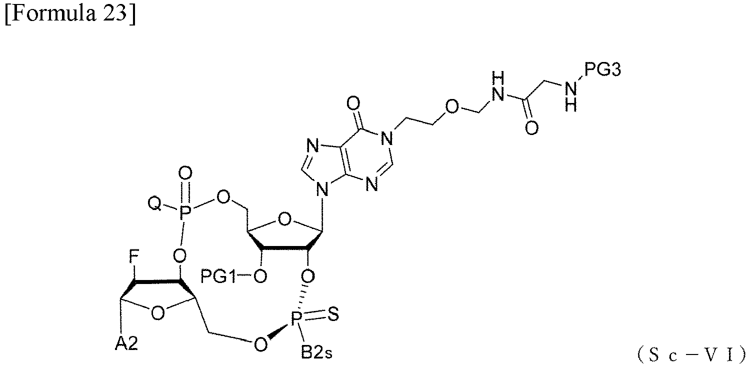 or a salt thereof, wherein A2, B2ₛ, Q, PG1, and PG3 are as defined above; and
      (step a4) removing B2ₛ, which is a protecting group for a thiophosphoric acid moiety in the resulting compound of formula (Sc-VI), and PG4, which is a protecting group in A2, by deprotection to obtain a compound of formula (Sp-VII) or a salt thereof.
[4] The method according to [2], wherein in step a2, the intermediate obtained after the reaction with the sulfurizing agent but before the removal of PG2 by deprotection is a compound represented by formula (Sc-V₀): 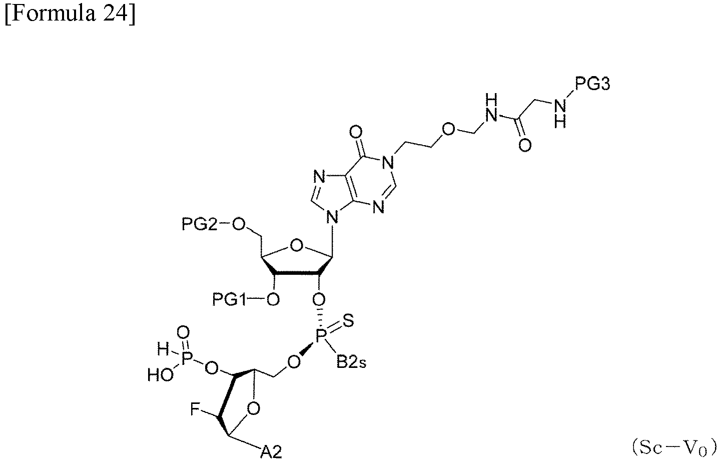 or a salt thereof, wherein A2, B2ₛ, PG1, PG2, and PG3 are as defined in [2].
[5] The method according to any one of [1] to [4], wherein the activator in step a2 is at least one selected from the group consisting of 1-phenylimidazole, benzimidazole, 1-methylbenzimidazole, 1-cyanomethylpiperidine, 1-pyrrolidineacetonitrile, 1-(cyanomethyl)imidazole, and salts thereof.
[6] The method according to any one of [1] to [5], wherein the acylating or alkoxycarbonylating agent in step a2 is at least one selected from the group consisting of acetic anhydride, N-succinimidyl acetate, pentafluorophenyl acetate, ethyl trifluoroacetate, methyl trifluoroacetate, pentafluorophenyl trifluoroacetate, trifluoroacetyl benzotriazole, 1-trifluoroacetylimidazole, benzoic anhydride, pentafluorophenylbenzoate, pentafluorophenylbenzoate, 1-*tert*-butoxycarbonyl-1,2,4-triazole, N-*tert*-butoxycarbonyl imidazole, di-*tert*-butyl dicarbonate, 9-fluorenyl methyl pentafluorophenyl carbonate, 1-[(9H-fluoren-9-ylmethoxy)carbonyloxy]benzotriazole, N-[(9H-fluoren-9-ylmethoxy)carbonyloxy]succinimide, N-(2,2,2-(trichloroethoxycarbonyloxy)succinimide, N-carbobenzyloxysuccinimide, dibenzyl dicarbonate, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, and methylimidazole-1-carboxylate.
[7] The method according to any one of [1] to [6], wherein the sulfurizing agent in step a2 is at least one selected from the group consisting of xanthane hydride, bis(phenylacetyl)disulfide, 3H-1,2-benzodithiol-3-one-1,1-dioxide, 5-phenyl-3H-1,2,4-dithiazol-3-one, and [(N,N-dimethylaminomethylidene)amino]-3H-1,2,4-dithiazolin-3-thione.
[8] The method according to any one of [1] to [7], wherein the condensing agent in step a3 is at least one selected from the group consisting of 2-chloro-5,5-dimethyl-1,3,2-dioxaphosphorinane 2-oxide, dimethylchlorophosphate, diethylchlorophosphate, 2-chloro-2-oxo-1,3,2-dioxaphosphorane, 1H-benzotriazol-1-yloxytripyrrolidino phosphonium hexafluorophosphate, chlorotripyrrolidino hexafluorophosphate, bromotripyrrolidino hexafluorophosphate, and propylphosphonic anhydride.
[9] The method according to any one of [1] to [8], wherein the sulfurizing agent in step a3 is at least one selected from the group consisting of xanthane hydride, bis(phenylacetyl)disulfide, 3H-1,2-benzodithiol-3-one-1,1-dioxide, 5-phenyl-3H-1,2,4-dithiazol-3-one, and [(N,N-dimethylaminomethylidene)amino]-3H-1,2,4-dithiazolin-3-thione.
[10] The method according to any one of [1] to [8], wherein the oxidizing agent in step a3 is at least one selected from the group consisting of iodine, *tert-*butylhydroperoxide, 3-chloroperbenzoic acid, hydrogen peroxide, periodic acid, potassium permanganate, and oxygen.
[11] The method according to any one of [1] to [10], wherein PG4 is benzyl, benzoyl, *tert*-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, or ethoxycarbonyl.
[12] The method according to any one of [1] to [11], wherein PG6 is acetyl, trifluoroacetyl, benzoyl, *tert*-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, methoxycarbonyl, or ethoxycarbonyl.
[13] The method according to any one of [1] to [12], wherein the compound of formula (IV) or a salt thereof is prepared by the following steps of:
   (step a5) reacting a compound represented by formula (VIII): 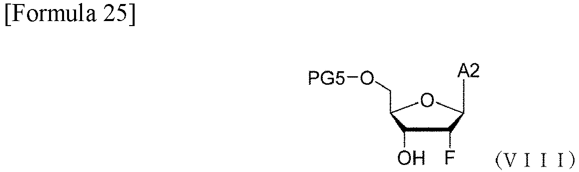 or a salt thereof, wherein
      A2 is as defined in [1], and
      PG5 is a protecting group for a hydroxy group,
      with a phosphite ester to obtain a compound represented by formula (IX): 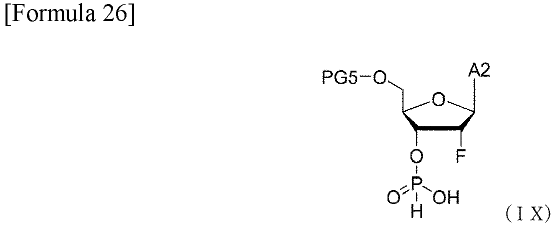 or a salt thereof, wherein A2 and PG5 are as defined above, or
   (step a5') reacting a compound represented by formula (VIII'): 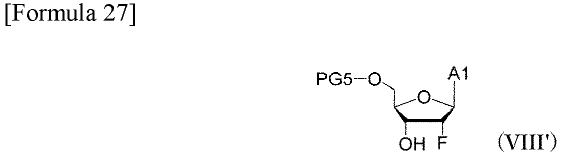
      or a salt thereof, wherein
      A1 is as defined in [1], and
      PG5 is as defined above,
      with a phosphite ester to obtain a compound represented by formula (IX'): 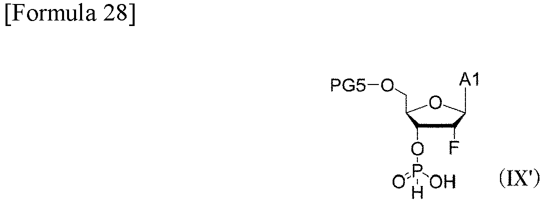 or a salt thereof, wherein A1 and PG5 are as defined above, and
   (step a5") reacting the resulting compound of formula (IX') or a salt thereof with an acylating or alkoxycarbonylating agent to obtain a compound represented by formula (IX): 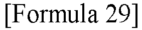 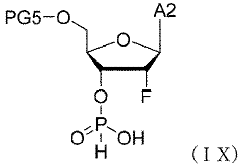 or a salt thereof, wherein A2 and PG5 are as defined above; and
   (step a6) removing PG5, which is a protecting group for a 5' hydroxyl group in the resulting compound of formula (IX) obtained in step a5 or a5", by deprotection to obtain a compound of formula (IV) or a salt thereof.
[14] The method according to [13], wherein the phosphite ester in steps a5 and a5' is at least one selected from the group consisting of diphenyl phosphite, methyl phosphite, diethyl phosphite, and dibutyl phosphite.
[15] The method according to [13] or [14], wherein the acylating or alkoxycarbonylating agent in step a5" is at least one selected from the group consisting of acetic anhydride, acetyl chloride, N-succinimidyl acetate, pentafluorophenyl acetate, 1-acetyl-1H-1,2,3-triazolo[4,5-b]pyridine, N-methoxydiacetamide, N-acetylimidazole, trifluoroacetic anhydride, bistrifluoroacetamide, ethyl trifluoroacetate, methyl trifluoroacetate, pentafluorophenyl trifluoroacetate, trifluoroacetyl benzotriazole, S-ethyl trifluorothioacetate, N-methylbistrifluoroacetamide, trifluoroacetyltriflate, 1-trifluoroacetylimidazole, benzoic anhydride, benzoyl chloride, pentafluorophenylbenzoate, pentafluorophenylbenzoate, benzoyl trifluoromethanesulfonate, 3-benzoylthiazolidine-2-thione, *tert-*butylphenylcarbonate, N-(*tert*-butoxycarbonyloxy)phthalimide, 2-(*tert-*butoxycarbonylthio)-4,6-dimethylpyridine, N-*tert*-butoxycarbonyl imidazole, *tert-*butylcarbazate, 2-(tert-butoxycarbonyloxyimino)-2-phenylacetonitrile, 1*-tert-*butoxycarbonyl-1,2,4-triazole, N-*tert*-butoxycarbonyl imidazole, di*-tert-*butyldicarbonate, 9-fluorenylmethylpentafluorophenylcarbonate, 9-fluorenylmethyl chloroformate, 9-fluorenylmethylcarbazate, 19-fluorenylmethylcarbamate, 1-[(9H-fluoren-9-ylmethoxy)carbonyloxy]benzotriazole, N-[(9H-fluoren-9-ylmethoxy)carbonyloxy]succinimide, allyl chloroformate, diallyl dicarbonate, N-(allyloxycarbonyloxy)succinimide, allyl phenyl carbonate, N-(2,2,2-trichloroethoxycarbonyloxy)succinimide, 2,2,2-trichloroethyl chloroformate, benzyl chloroformate, benzyl carbazate, benzyl phenyl carbonate, N-carbobenzyloxysuccinimide, dibenzyl dicarbonate, 4-[2-(trimethylsilyl)ethoxycarbonyloxy]nitrobenzene, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, ethyl chloroformate, diethyldicarbonate, ethyl imidazole-1-carboxylate, 2-ethoxy-1-(ethoxycarbonyl)-1,2-dihydroquinoline, methyl chloroformate, dimethyl carbonate, dimethyldicarbonate, and methyl imidazole-1-carboxylate.
[16] The method according to any one of [1], [2], [5] to [9], and [11] to [12], wherein in step a3 described in [1], a compound of formula (Rp-VII), where Q is a thiol group, or a salt thereof is obtained by using the sulfurizing agent.
[17] The method according to [16], wherein in step a4 described in [1], the resulting compound of formula (Rp-VII), where Q is a thiol group, is subjected to silica gel column chromatography prior to conversion to a salt thereof and/or crystallization after conversion to a salt thereof to obtain a compound of formula (Rp,Rp-VII'): 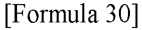 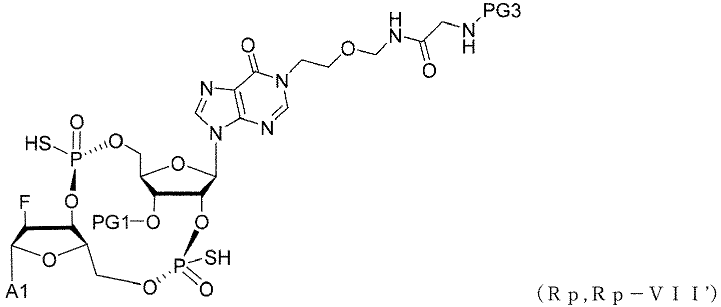 or a salt thereof.
[18] The method according to [17], further comprising the steps of:
   (step a7) removing PG1 and PG3, which are protecting groups in the resulting compound of formula (Rp,Rp-VII'), by deprotection to obtain a compound represented by formula (Rp,Rp-X): 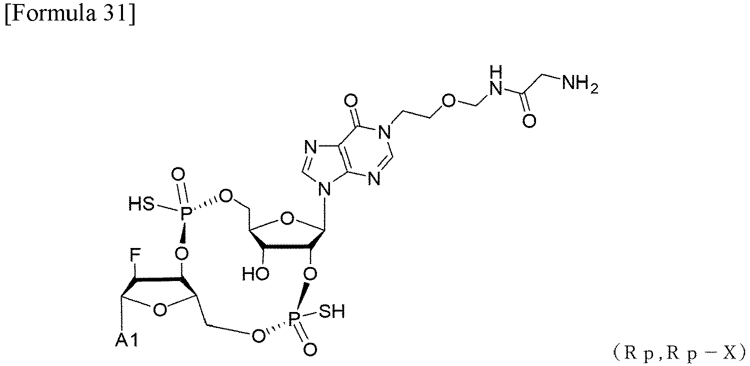 or a salt thereof, wherein
      A1 is as defined in [1]; and
   (step a8) condensing the resulting compound of formula (Rp,Rp-X) or a salt thereof with a compound represented by formula (XI): 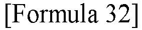 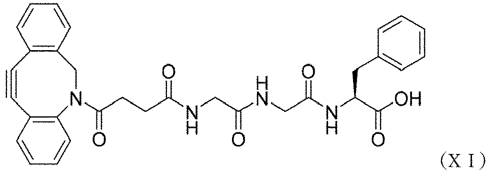 or an active ester thereof to obtain a compound represented by formula (Rp,Rp-XII): 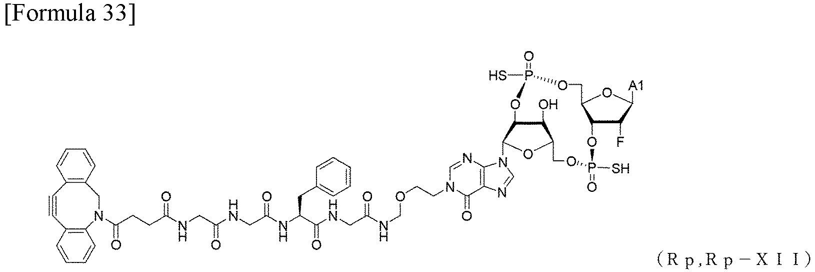 or a salt thereof, wherein A1 is as defined above.
[19] The method according to [18], further comprising the step of:
   (step a9) conjugating the resulting compound of formula (Rp,Rp-XII) or a salt thereof with an antibody or a functional fragment of the antibody (hereinafter, referred to as "Ab") to obtain an antibody-immunostimulant conjugate represented by formula (Rp,Rp-XIII): 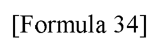 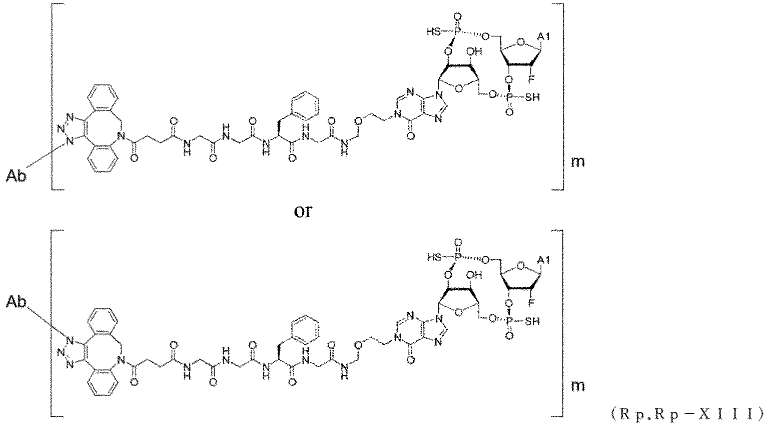
      or a mixture thereof, wherein
      m ranges from 1 to 10,
      a glycan of Ab is optionally remodeled,
      Ab is conjugated to a compound of formula (Rp,Rp-XII) either directly from a side chain of an optionally modified amino acid residue or from a glycan or a remodeled glycan of Ab, and
      A1 is 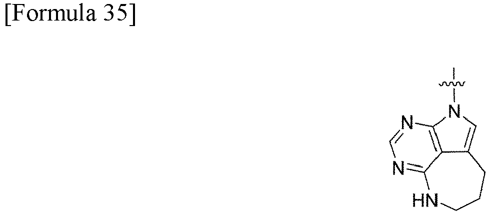
[20] The method according to [19], wherein in step a9, the compound of formula (Rp,Rp-XII) or a salt thereof is conjugated to Ab by a strain-promoted azide-alkyne cycloaddition reaction.
[21] The method according to [19] or [20], wherein the antibody is an antibody selected from the group consisting of anti-HER2 antibody, anti-HER3 antibody, anti-DLL3 antibody, anti-FAP antibody, anti-CDH11 antibody, anti-CDH6 antibody, anti-A33 antibody, anti-CanAg antibody, anti-CD 19 antibody, anti-CD20 antibody, anti-CD22 antibody, anti-CD30 antibody, anti-CD33 antibody, anti-CD56 antibody, anti-CD70 antibody, anti-CD98 antibody, anti-TROP2 antibody, anti-CEA antibody, anti-Cripto antibody, anti-EphA2 antibody, anti-G250 antibody, anti-MUC1 antibody, anti-GPNMB antibody, anti-Integrin antibody, anti-PSMA antibody, anti-Tenascin-C antibody, anti-SLC44A4 antibody, anti-Mesothelin antibody, anti-ENPP3 antibody, anti-CD47 antibody, anti-EGFR antibody, anti-GPR20 antibody, and anti-DR5 antibody.
[22] A compound represented by formula (Rc-III): 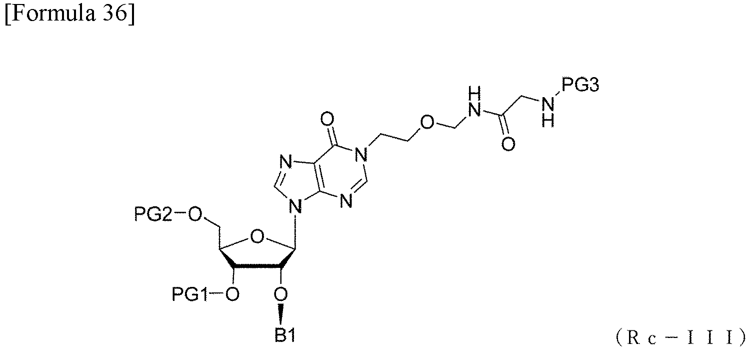 wherein
   B1 is as defined in [1],
   PG1 is *tert*-butyldimethylsilyl, trimethylsilyl, triethylsilyl, triisopropylsilyl, or *tert*-butyldiphenylsilyl,
   PG2 is 4,4'-dimethoxytrityl, 4-methoxytrityl, 2-chlorotrityl, or trityl, and
   PG3 is 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl.
[23] A compound represented by formula (Rc-V₀): 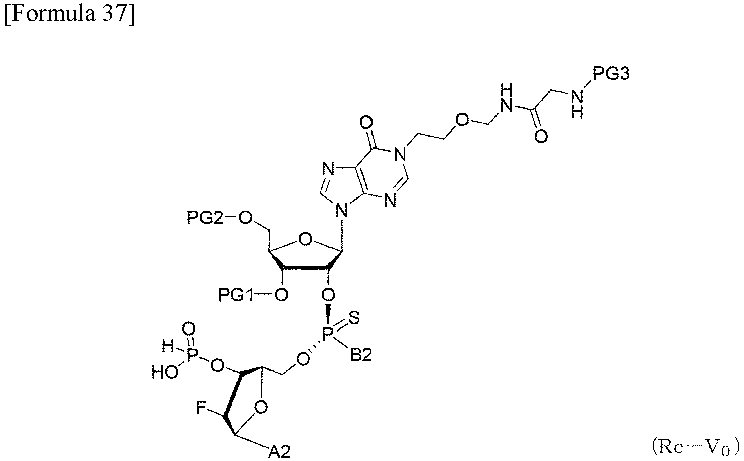
   or a salt thereof, wherein
   A2 and B2 are as defined in [1],
   PG1 is *tert*-butyldimethylsilyl, trimethylsilyl, triethylsilyl, triisopropylsilyl, or *tert*-butyldiphenylsilyl,
   PG2 is 4,4'-dimethoxytrityl, 4-methoxytrityl, 2-chlorotrityl, or trityl, and
   PG3 is 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl.
[24] A compound represented by formula (Rc-V): 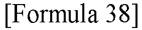 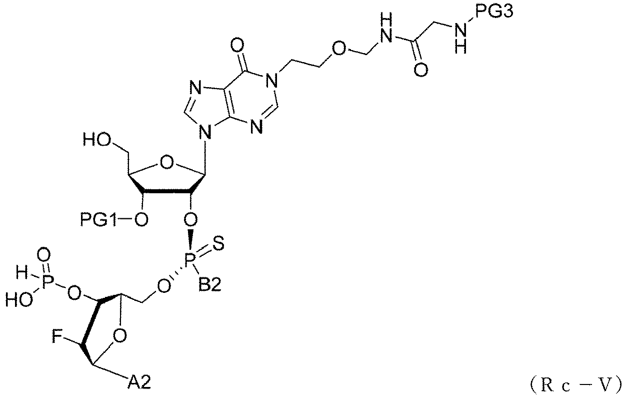
   or a salt thereof, wherein
   A2 and B2 are as defined in [1],
   PG1 is *tert*-butyldimethylsilyl, trimethylsilyl, triethylsilyl, triisopropylsilyl, or *tert*-butyldiphenylsilyl, and
   PG3 is 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl.
[25] The method according to any one of [1] to [21], wherein the compound of formula (I) where PG1 is *tert*-butyldimethylsilyl is prepared through the following steps of:
   (step b1) reacting a compound represented by formula (XIV): 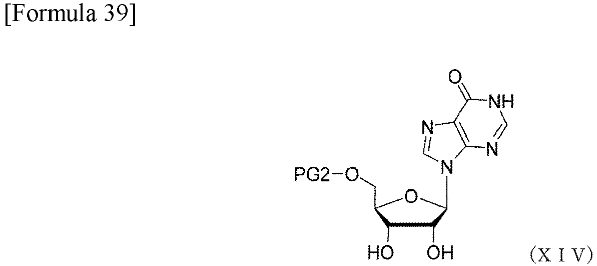
      wherein PG2 is as defined in [1],
         with a compound represented by formula (XV): 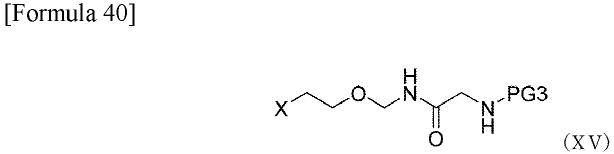
      wherein
      PG3 is as defined in [1], and
      X is Cl, Br, or I,
      to obtain a compound represented by formula (XVI): 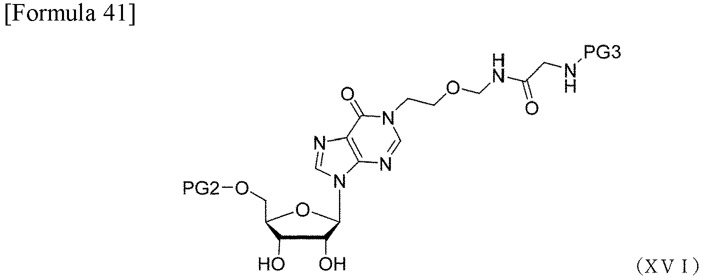 wherein PG2 and PG3 are as defined above; and
   (step b2) reacting the resulting compound of formula (XVI) with a silylating agent to obtain a mixture of the following compounds of formula (I') and formula (XVII): 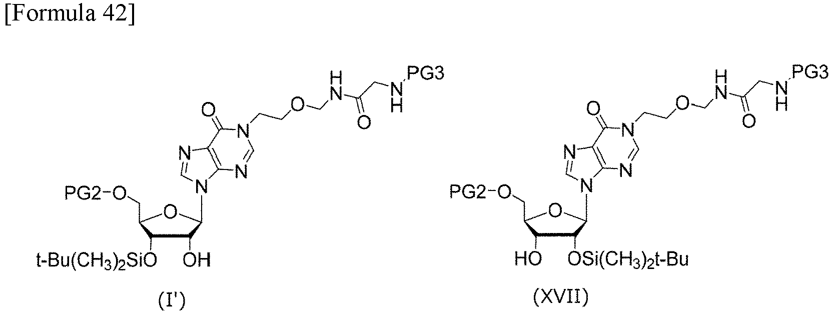 wherein PG2 and PG3 are as defined above;
   and then converting the compound of formula (XVII) in the mixture to the compound of formula (I') in the presence of a base to obtain the compound of formula (I').
[26] The method according to [25], wherein the reaction in step b1 is carried out in the presence of a base and the base is 1,1,3,3-tetramethylguanidine, triethylamine, diisopropylethylamine, or 1,8-diazabicyclo[5.4.0]-7-undecene.
[27] The method according to [25] or [26], wherein in step b2, the reaction of the compound of formula (XVI) with the silylating agent is carried out in the presence of a first base to obtain the mixture of the compound of formula (I') and the compound of formula (XVII), and then the compound of formula (XVII) in the mixture is converted in the presence of a second base to the compound of formula (I') to obtain the compound of formula (I').
[28] The method according to [27], wherein the first base is at least one selected from the group consisting of 1,1,3,3-tetramethylguanidine, triethylamine, diisopropylethylamine, and 1,8-diazabicyclo[5.4.0]-7-undecene.
[29] The method according to [27] or [28], wherein the second base is at least one selected from the group consisting of 1,1,3,3-tetramethylguanidine, triethylamine, diisopropylethylamine, and 1,8-diazabicyclo[5.4.0]-7-undecene.
[30] The method according to any one of [25] to [29], wherein the silylating agent in step b2 is *tert*-butyldimethylchlorosilane or *tert-*butyldimethylsilyl triflate.
[31] The method according to any one of [25] to [30], wherein the compound of formula (XV) is prepared by the following step of:
   (step b3) reacting a compound represented by formula (XVIII): 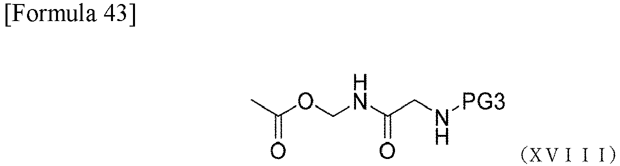 wherein PG3 is as defined in [1]
      with 2-haloethanol in the presence of an acid or a base to obtain a compound of formula (XV).
[32] The method according to [31], wherein the base in step b3 is at least one selected from the group consisting of sodium hydroxide, potassium hydroxide, lithium hydroxide, triethylamine, diisopropylethylamine, and 1,8-diazabicyclo[5.4.0]-7-undecene.
[33] A compound represented by formula (XV): 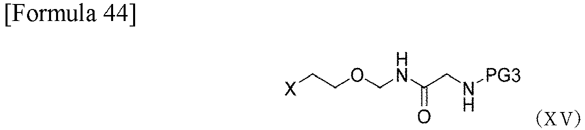 wherein
   PG3 is 2-(trimethylsilyl)ethoxycarbonyl, and
   X is Cl, Br, or I.
[34] The method according to any one of [13] to [21] and [25] to [32], wherein the compound of formula (VIII')
   wherein
   A1 is 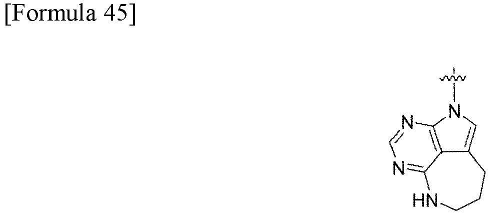 and
   PG5 is 4,4'-dimethoxytrityl, 4-methoxytrityl, 2-chlorotrityl, or trityl, is prepared by the following steps of:
      (step c1) reacting a compound represented by formula (XIX): 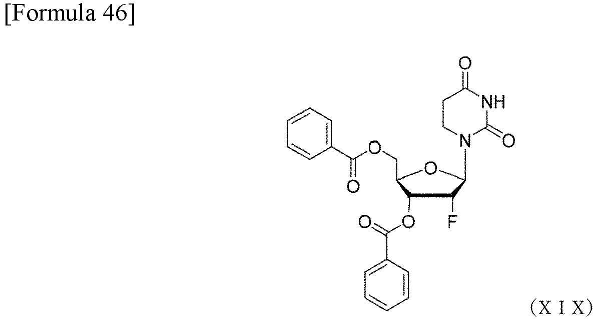 with a benzoylating agent to obtain a compound represented by formula (XX): 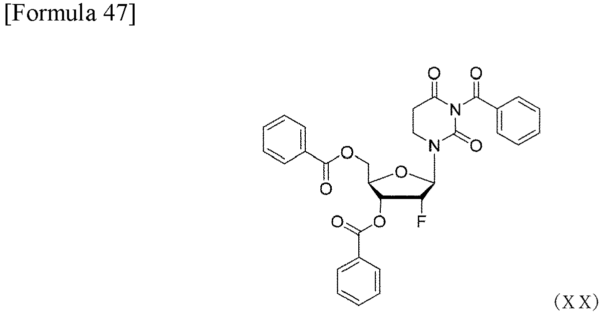
      (step c2) hydrolyzing the resulting compound of formula (XX) to obtain a compound represented by formula (XXI): 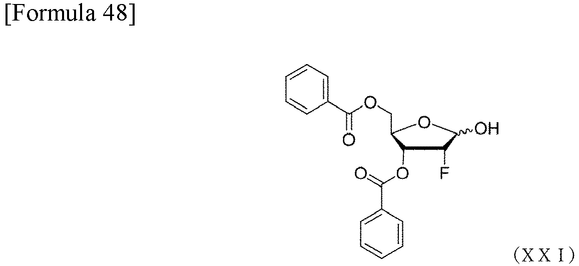
      (step c3) reacting the resulting compound of formula (XXI) with a chlorinating agent to obtain a compound represented by formula (XXII): 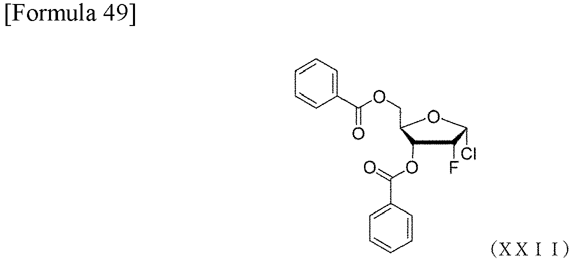
      (step c4) reacting the resulting compound of formula (XXII) with a compound represented by formula (XXIII): 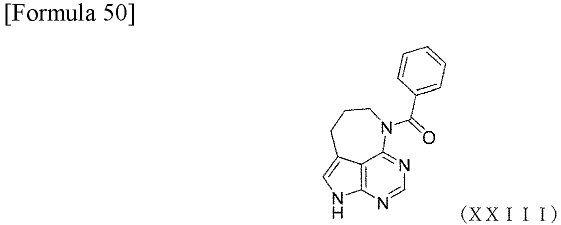 to obtain a compound represented by formula (XVIV): 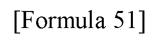 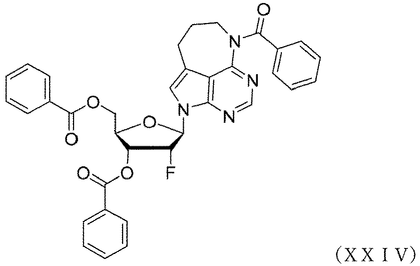
      (step c5) deprotecting the resulting compound of formula (XXIV) to remove a benzoyl group to obtain a compound represented by formula (XXV): 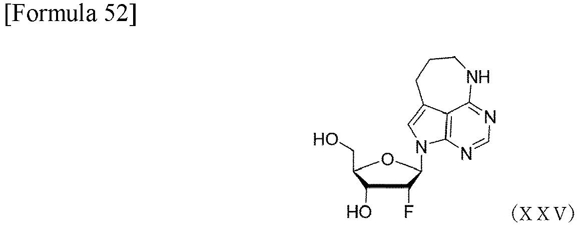 or a salt thereof; and
      (step c6) reacting the resulting compound of formula (XXV) or a salt thereof with a tritylating agent to obtain the compound of formula (VIII'), wherein A1 and PG5 are as defined above, or a salt thereof.
[35] The method according to [34], further comprising the step of:
   (step c7) reacting the resulting compound of formula (VIII'), wherein A1 and PG5 are as defined in [34], or a salt thereof with a silylating agent, followed by a reaction with an acylating or alkoxycarbonylating agent to obtain a compound represented by formula (XXVII): 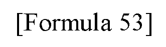 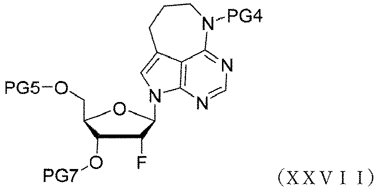 wherein
      PG4 is benzoyl, 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl,
      PG5 is as defined above, and
      PG7 is a protecting group for a hydroxy group; and
   (step c8) removing PG7, which is a protecting group in the resulting compound of formula (XXVII), by deprotection to obtain a compound represented by formula (VIII): 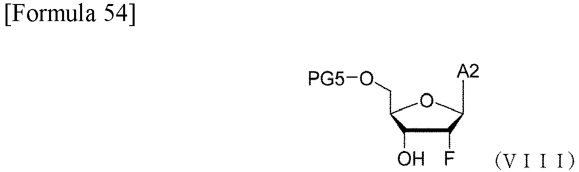 or a salt thereof, wherein
      A2 is 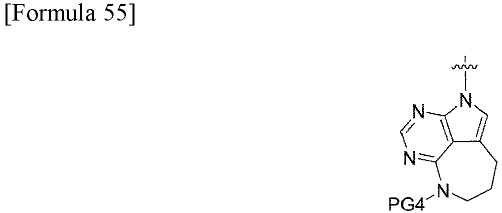 and
      PG4 and PG5 are as defined above.
[36] The method according to [34] or [35], wherein the chlorinating agent in step c3 is at least one selected from the group consisting of trichloroisocyanuric acid, chloroisocyanuric acid, dichloroisocyanuric acid, N-chlorosuccinimide, 1,3-dichloro-5,5-dimethylhydantoin, and carbon tetrachloride, and
   the reaction in step c3 is carried out in the presence of at least one selected from the group consisting of tris(2,4-di-*tert*-butylphenyl)phosphite, tri-o-tolyl phosphite, triphenyl phosphite, triethylphosphite, and triphenylphosphine.
[37] The method according to any one of [34] to [36], wherein step c4 is carried out in the presence of at least one selected from the group consisting of cesium carbonate, potassium carbonate, sodium carbonate, lithium carbonate, sodium hydroxide, potassium hydroxide, lithium hydroxide, and 1,8-diazabicyclo[5.4.0]-7-undecene.
[38] The method according to any one of [34] to [37], wherein the tritylating agent in step c6 is at least one selected from the group consisting of 4,4-dimethoxy trityl chloride, 4-methoxy trityl chloride, 2-chlorotrityl chloride, and trityl chloride.
[39] The method according to any one of [35] to [38], wherein the acylating or alkoxycarbonylating agent in step c7 is at least one selected from the group consisting of benzoylating agents, 2-(trimethylsilyl)ethoxycarbonylating agents, *tert-*butoxycarbonylating agents, 9-fluorenylmethyloxycarbonylating agents, allyloxycarbonylating agents, 2,2,2-trichloroethoxycarbonylating agents, and benzyloxycarbonylating agents.
[40] The method according to any one of [34] to [39], wherein the compound of formula (XXIII) is prepared by the following steps of:
   (step c9) deprotecting a compound represented by formula (XXVIII): 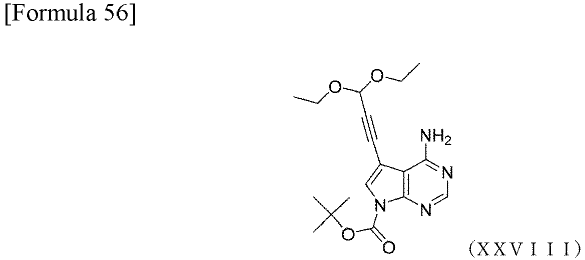 to remove a tert-butoxycarbonyl group, to obtain a compound represented by formula (XXIX): 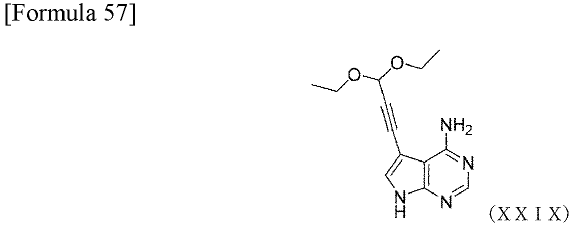
   (step c10) subjecting the resulting compound of formula (XXIX) to alkyne reduction reaction in the presence of a catalyst, followed by reductive amination reaction to obtain a compound represented by formula (XXX): 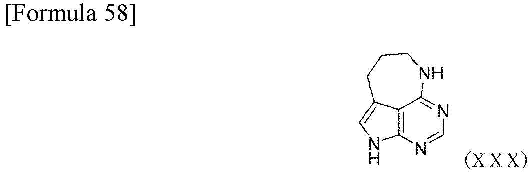 ; and
   (step c 11) reacting the resulting compound of formula (XXX) with a benzoylating agent to obtain a compound of formula (XXIII).
[41] The method according to [40], wherein the compound of formula (XXVIII) is prepared by the following steps of:
   (step c12) reacting a compound represented by formula (XXXI): 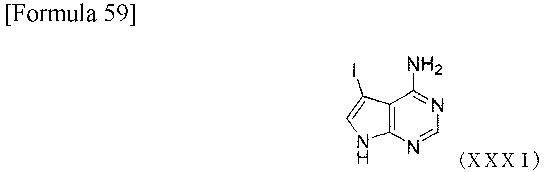 with a tert-butoxycarbonylating agent in the presence of 1-methylimidazole to obtain a compound represented by formula (XXXII): 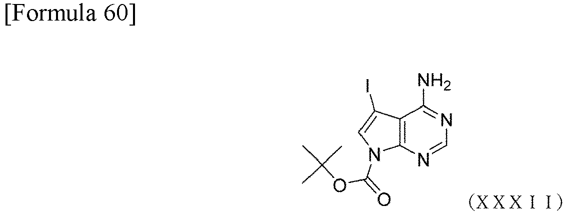 ; and
   (step c13) reacting the resulting compound of formula (XXXII) with propargylaldehyde diethyl acetal to obtain a compound of formula (XXVIII).
[42] The method according to any one of [34] to [39] and [41], wherein the compound of formula (XXIV) is prepared by, instead of step c4, the following steps:
   (step c14) reacting the compound of formula (XXII) with the compound of formula (XXIX) to obtain a compound represented by formula (XXXIII): 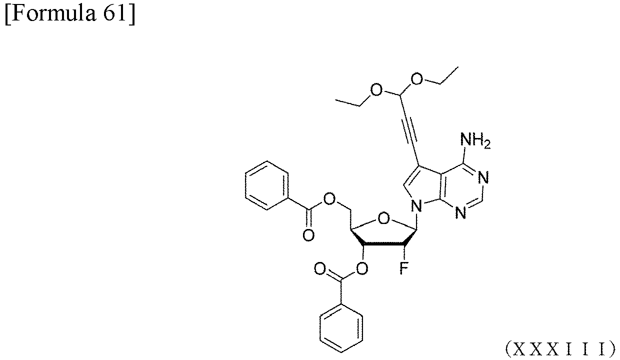 ; and
   (step c 15) subjecting the resulting compound of formula (XXXIII) to alkyne reduction reaction in the presence of a catalyst, followed by reductive amination reaction and a further reaction with a benzoylating agent to obtain a compound of formula (XXIV).
[43] A compound represented by formula (XX): 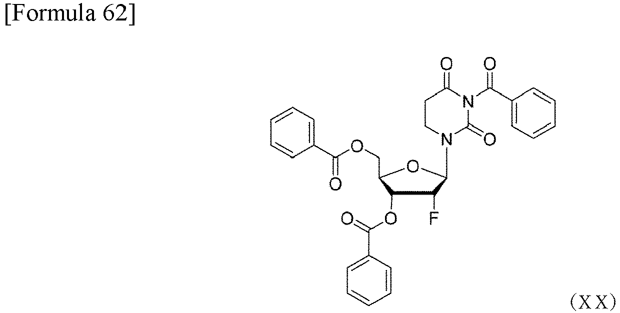
[44] A compound represented by formula (XXII): 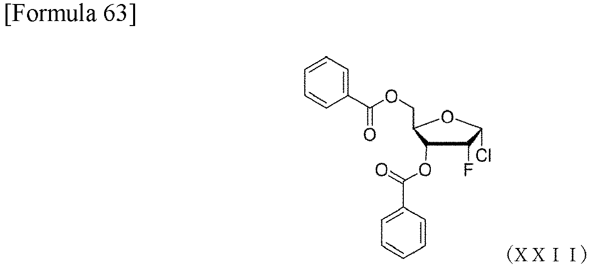
[45] A compound represented by formula (XXIV): 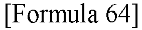 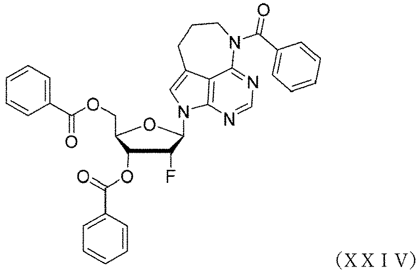
[46] A compound represented by formula (XXV): 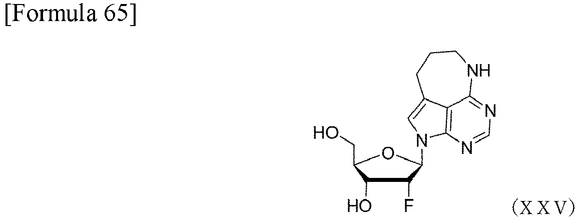 or a salt thereof.
[47] A compound represented by formula (VIII'-1): 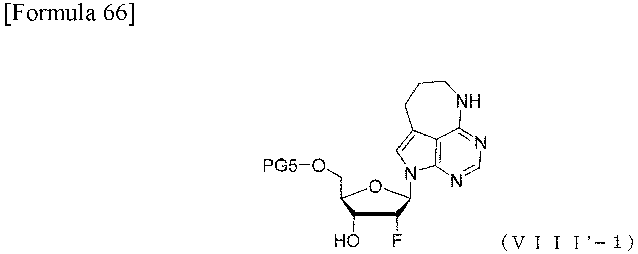 or a salt thereof, wherein PG5 is 4,4'-dimethoxytrityl, 4-methoxytrityl, 2-chlorotrityl, or trityl.
[48] A compound represented by formula (XXVII): 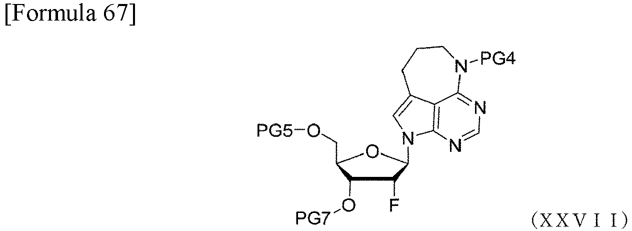 wherein
   PG4 is benzoyl, 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl,
   PG5 is 4,4'-dimethoxytrityl, 4-methoxytrityl, 2-chlorotrityl, or trityl, and
   PG7 is trimethylsilyl, triethylsilyl, triisopropylsilyl, *tert*-butyldimethylsilyl, *tert-*butyldiphenylsilyl, or triphenylsilyl.
[49] A compound represented by formula (XXVIII): 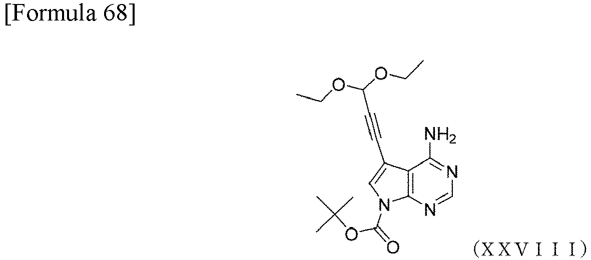
[50] A method for preparing a compound represented by formula (I): 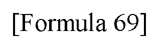 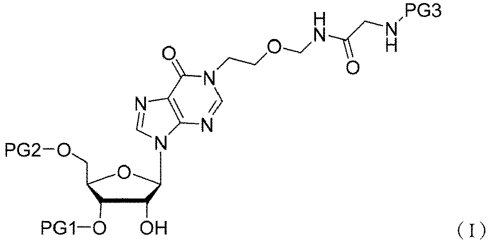 wherein
   PG1 is *tert*-butyldimethylsilyl,
   PG2 is a protecting group for a hydroxy group, and
   PG3 is a protecting group for an amino group,
   the method comprising the steps of:
   (step b1) reacting a compound represented by formula (XIV) 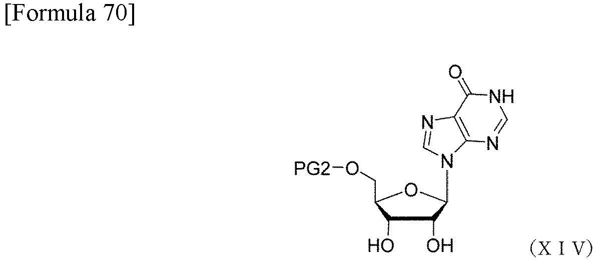 wherein PG2 is as defined above,
      with a compound represented by formula (XV): 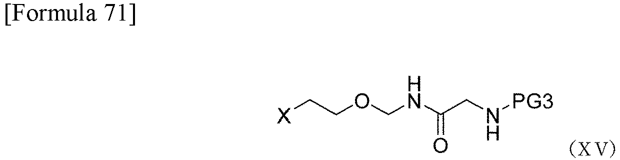 wherein
      PG3 is as defined above, and
      X is Cl, Br, or I,
      to obtain a compound represented by formula (XVI): 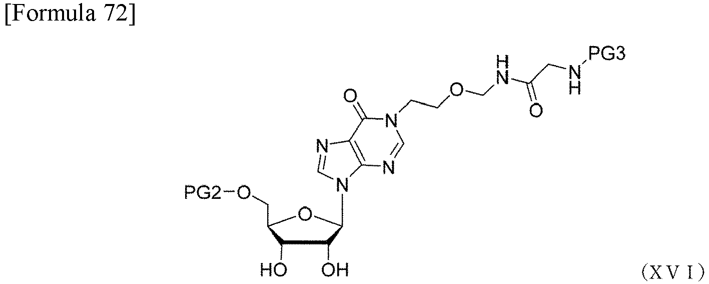 wherein PG2 and PG3 are as defined above; and
   (step b2) reacting the resulting compound of formula (XVI) with a silylating agent to obtain a mixture of the following compounds of formula (I') and formula (XVII): 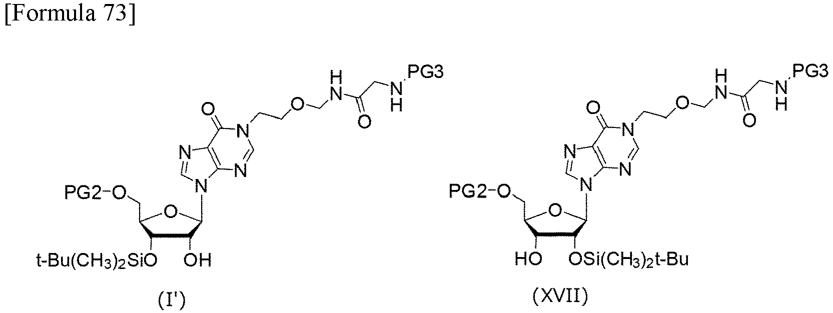 wherein PG2 and PG3 are as defined above;
   and then converting the compound of formula (XVII) in the mixture to the compound of formula (I') in the presence of a base to obtain the compound of formula (I').
[51] The method according to [50], wherein the reaction in step b1 is carried out in the presence of a base and the base is 1,1,3,3-tetramethylguanidine, triethylamine, diisopropylethylamine, or 1,8-diazabicyclo[5.4.0]-7-undecene.
[52] The method according to [50] or [51], wherein in step b2, the reaction of the compound of formula (XVI) with the silylating agent is carried out in the presence of a first base to obtain the mixture of the compound of formula (I') and the compound of formula (XVII), and then the compound of formula (XVII) in the mixture is converted in the presence of a second base to the compound of formula (I') to obtain the compound of formula (I').
[53] The method according to [52], wherein the first base is at least one selected from the group consisting of 1,1,3,3-tetramethylguanidine, triethylamine, diisopropylethylamine, and 1,8-diazabicyclo[5.4.0]-7-undecene.
[54] The method according to [52] or [53], wherein the second base is at least one selected from the group consisting of 1,1,3,3-tetramethylguanidine, triethylamine, diisopropylethylamine, and 1,8-diazabicyclo[5.4.0]-7-undecene.
[55] The method according to any one of [50] to [54], wherein the silylating agent in step b2 is *tert-*butyldimethylchlorosilane or *tert*-butyldimethylsilyl triflate.
[56] A method for preparing a compound represented by formula (XV): 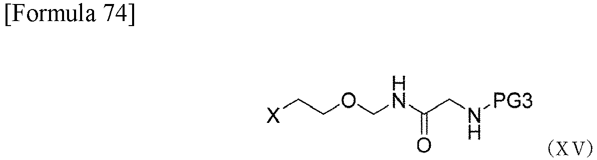 wherein
   PG3 is a protecting group for an amino group, and
   X is Cl, Br, or I,
   the method comprising the steps of:
   (step b3) reacting a compound represented by formula (XVIII): 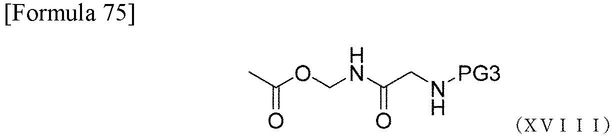 wherein PG3 is as defined above,
      with 2-haloethanol in the presence of an acid or a base to obtain a compound of formula (XV).
[57] The method according to [56], wherein the base in step b3 is at least one selected from the group consisting of sodium hydroxide, potassium hydroxide, lithium hydroxide, triethylamine, diisopropylethylamine, and 1,8-diazabicyclo[5.4.0]-7-undecene.
[58] A method for preparing a compound represented by formula (VIII'): 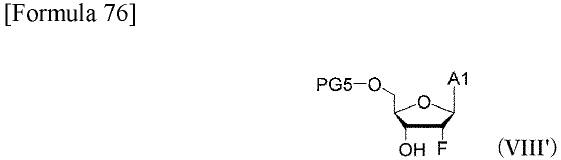 wherein
   A1 is 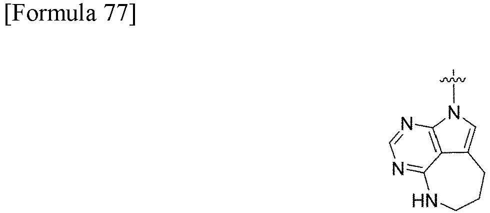 and
   PG5 is 4,4'-dimethoxytrityl, 4-methoxytrityl, 2-chlorotrityl, or trityl,
   the method comprising the steps of:
   (step c1) reacting a compound represented by formula (XIX): 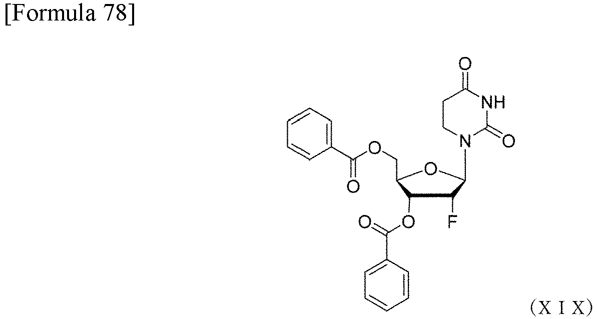 with a benzoylating agent to obtain a compound represented by formula (XX): 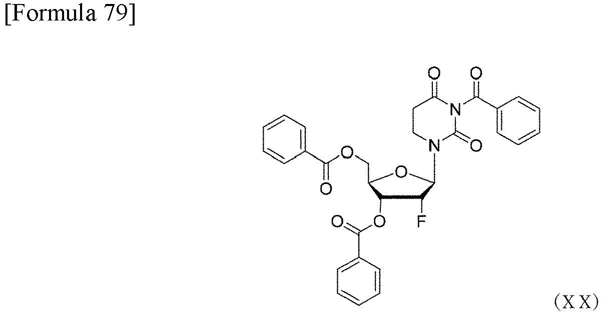
   (step c2) hydrolyzing the resulting compound of formula (XX) to obtain a compound represented by formula (XXI): 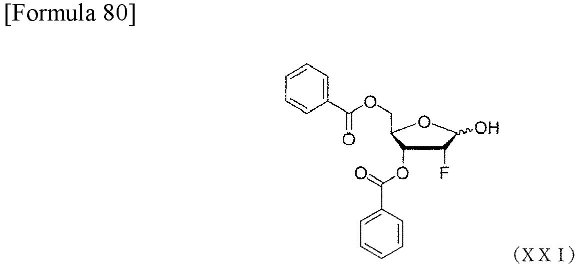
   (step c3) reacting the resulting compound of formula (XXI) with a chlorinating agent to obtain a compound represented by formula (XXII): 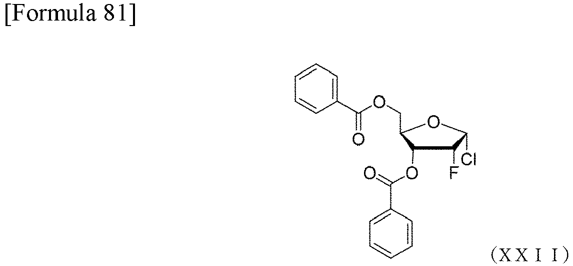
   (step c4) reacting the resulting compound of formula (XXII) with a compound represented by formula (XXIII): 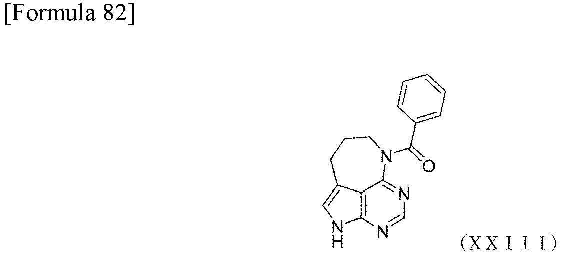 to obtain a compound represented by formula (XXIV): 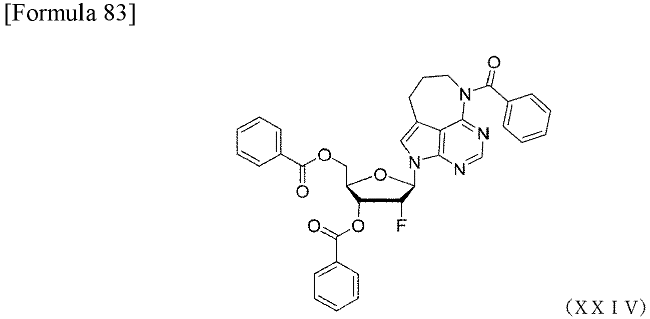
   (step c5) deprotecting the resulting compound of formula (XXIV) to remove a benzoyl group, to obtain a compound represented by formula (XXV): 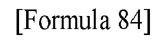 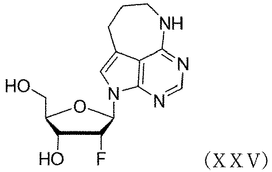 or a salt thereof; and
   (step c6) reacting the resulting compound of formula (XXV) or a salt thereof with a tritylating agent to obtain the compound of formula (VIII'), wherein A1 and PG5 are as defined above, or a salt thereof.
[59] The method according to [58], further comprising the step of:
   (step c7) reacting the resulting compound of formula (VIII'), wherein A1 and PG5 are as defined in [55], or a salt thereof with a silylating agent, followed by a reaction with an acylating or alkoxycarbonylating agent to obtain a compound represented by formula (XXVII): 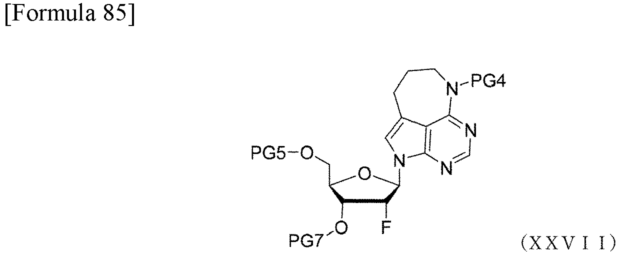 wherein
      PG4 is benzoyl, 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl,
      PG5 is as defined above, and
      PG7 is a protecting group for a hydroxy group; and
   (step c8) removing PG7, which is a protecting group in the resulting compound of formula (XXVII) by deprotection, to obtain a compound represented by formula (VIII): 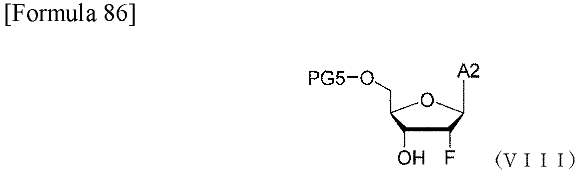 or a salt thereof, wherein
      A2 is 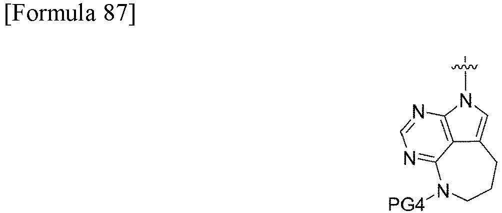 and
      PG4 and PG5 are as defined above.
[60] The method according to [59], wherein the chlorinating agent in step c3 is at least one selected from the group consisting of trichloroisocyanuric acid, chloroisocyanuric acid, dichloroisocyanuric acid, N-chlorosuccinimide, 1,3-dichloro-5,5-dimethylhydantoin, and carbon tetrachloride, and
   the reaction in step c3 is carried out in the presence of at least one selected from the group consisting of tris(2,4-di-*tert*-butylphenyl)phosphite, tri-o-tolyl phosphite, triphenyl phosphite, triethylphosphite, and triphenylphosphine.
[61] The method according to [59] or [60], wherein step c4 is carried out in the presence of at least one selected from the group consisting of cesium carbonate, potassium carbonate, sodium carbonate, lithium carbonate, sodium hydroxide, potassium hydroxide, lithium hydroxide, and 1,8-diazabicyclo[5.4.0]-7-undecene.
[62] The method according to any one of [59] to [61], wherein the tritylating agent in step c6 is at least one selected from the group consisting of 4,4-dimethoxy trityl chloride, 4-methoxy trityl chloride, 2-chlorotrityl chloride, and trityl chloride.
[63] The method according to any one of [59] to [62], wherein the acylating or alkoxycarbonylating agent in step c7 is at least one selected from the group consisting of benzoylating agents, 2-(trimethylsilyl)ethoxycarbonylating agents, *tert-*butoxycarbonylating agents, 9-fluorenylmethyloxycarbonylating agents, allyloxycarbonylating agents, 2,2,2-trichloroethoxycarbonylating agents, and benzyloxycarbonylating agents.
[64] A method for preparing a compound represented by formula (XXIII): 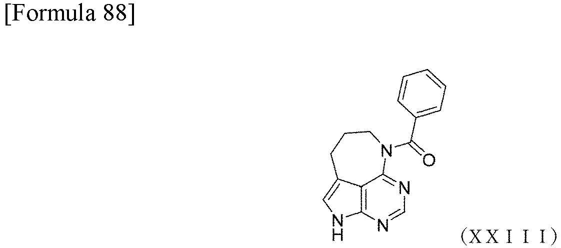 the method comprising the steps of:
   (step c9) deprotecting a compound represented by formula (XXVIII): 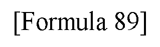 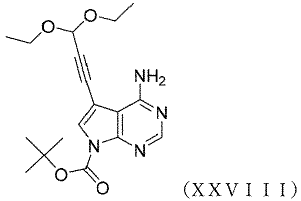 to remove a tert-butoxycarbonyl group, to obtain a compound represented by formula (XXIX): 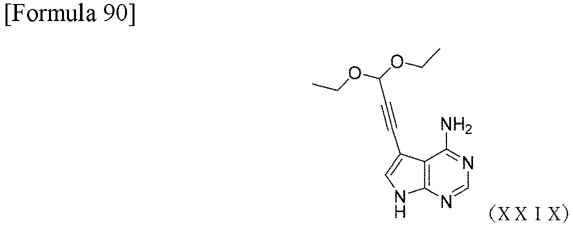
   (step c 10) subjecting the resulting compound of formula (XXIX) to alkyne reduction reaction in the presence of a catalyst, followed by reductive amination reaction to obtain a compound represented by formula (XXX): 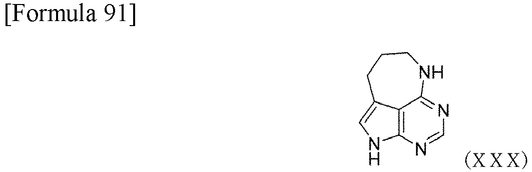 ; and
   (step c 11) reacting the resulting compound of formula (XXX) with a benzoylating agent to obtain a compound of formula (XXIII).
[65] A method for preparing a compound represented by formula (XXVIII): 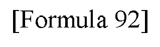 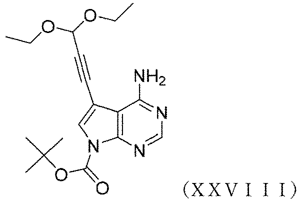 the method comprising the steps of:
   (step c12) reacting a compound represented by formula (XXXI): 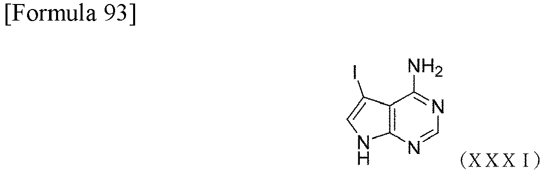 with a *tert*-butoxycarbonylating agent in the presence of 1-methylimidazole to obtain a compound represented by formula (XXXII): 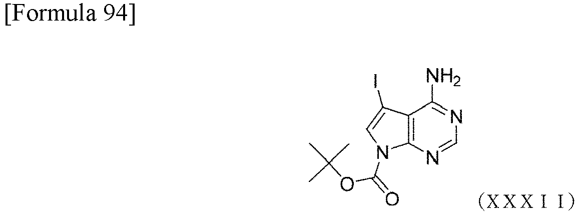 ; and
   (step c13) reacting the resulting compound of formula (XXXII) with propargylaldehyde diethyl acetal to obtain a compound of formula (XXVIII).
[66] A method for preparing a compound represented by formula (XXIV): 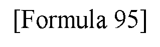 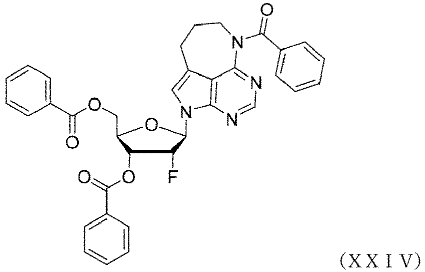 the method comprising the steps of:
   (step c14) reacting a compound represented by formula (XXII): 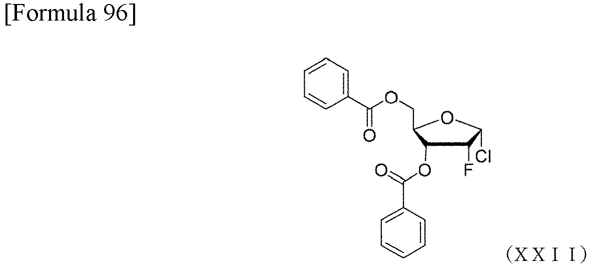 with a compound represented by formula (XXIX): 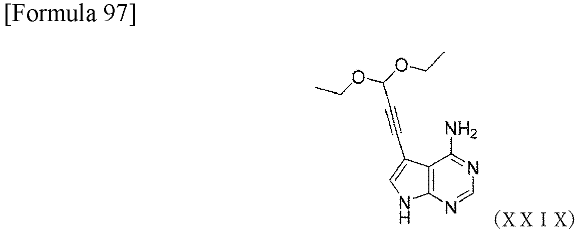 to obtain a compound represented by formula (XXXIII): 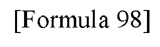 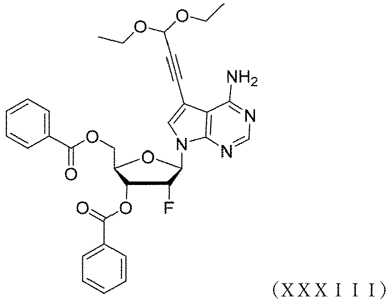 ; and
   (step c15) subjecting the resulting compound of formula (XXXIII) to alkyne reduction reaction in the presence of a catalyst, followed by reductive amination reaction and a further reaction with a benzoylating agent to obtain a compound of formula (XXIV).

### Advantageous Effects of Invention

The present invention can provide a stereoselective method for preparing a cyclic dinucleotide. This preparation method makes it possible to reduce the burden of diastereomer purification, so that a CDN with the desired stereo configuration can be prepared in higher yield and in larger quantity. As a result, a CDN-linker can be prepared in high yield by the CDN-linker preparation method including this stereoselective CDN preparation method. Further, the present invention enables the preparation of a raw material compound used for preparing a cyclic dinucleotide with a less number of steps in higher yield.

### Brief Description of Drawings

[Figure 1] Figure 1 illustrates a step of using an optically active phosphitylating agent (Rc-II) to stereoselectively link two nucleotides (compound (I) and compound (IV)) through a phosphorothioate bond to obtain a production intermediate (Rc-V) in the method for preparing a cyclic dinucleotide.
[Figure 2] Figure 2 shows the light chain amino acid sequence (SEQ ID NO: 1) and the heavy chain amino acid sequence (SEQ ID NO: 2) of anti-CD70 antibody 1.
[Figure 3] Figure 3 shows the light chain amino acid sequence (SEQ ID NO: 3) and the heavy chain amino acid sequence (SEQ ID NO: 4) of anti-CD70 antibody 2.
[Figure 4] Figure 4 shows the light chain amino acid sequence (SEQ ID NO: 5) and the heavy chain amino acid sequence (SEQ ID NO: 6) of anti-TROP2 antibody 1.
[Figure 5] Figure 5 shows the light chain amino acid sequence (SEQ ID NO: 7) and the heavy chain amino acid sequence (SEQ ID NO: 8) of anti-TROP2 antibody 2.
[Figure 6] Figure 6 shows the light chain amino acid sequence (SEQ ID NO: 9) and the heavy chain amino acid sequence (SEQ ID NO: 10) of anti-EGFR antibody 1.
[Figure 7] Figure 7 shows the light chain amino acid sequence (SEQ ID NO: 11) and the heavy chain amino acid sequence (SEQ ID NO: 12) of anti-EGFR antibody 2.
[Figure 8] Figure 8 shows the amino acid sequence of CDRL1 (SEQ ID NO: 13), the amino acid sequence of CDRL2 (SEQ ID NO: 14), the amino acid sequence of CDRL3 (SEQ ID NO: 15), the amino acid sequence of CDRH1 (SEQ ID NO: 16), the amino acid sequence of CDRH2 (SEQ ID NO: 17), and the amino acid sequence of CDRH3 (SEQ ID NO: 18) of anti-CD70 antibody 1. CDR sequences were determined by Kabat's definition.
[Figure 9] Figure 9 shows the amino acid sequence of CDRL1 (SEQ ID NO: 19), the amino acid sequence of CDRL2 (SEQ ID NO: 20), the amino acid sequence of CDRL3 (SEQ ID NO: 21), the amino acid sequence of CDRH1 (SEQ ID NO: 22), the amino acid sequence of CDRH2 (SEQ ID NO: 23), and the amino acid sequence of CDRH3 (SEQ ID NO: 24) of anti-CD70 antibody 2. CDR sequences were determined by Kabat's definition.
[Figure 10] Figure 10 shows the amino acid sequence of CDRL1 (SEQ ID NO: 25), the amino acid sequence of CDRL2 (SEQ ID NO: 26), the amino acid sequence of CDRL3 (SEQ ID NO: 27), the amino acid sequence of CDRH1 (SEQ ID NO: 28), the amino acid sequence of CDRH2 (SEQ ID NO: 29), and the amino acid sequence of CDRH3 (SEQ ID NO: 30) of anti-TROP2 antibody 1. CDR sequences were determined by Kabat's definition.
[Figure 11] Figure 11 shows the amino acid sequence of CDRL1 (SEQ ID NO: 31), the amino acid sequence of CDRL2 (SEQ ID NO: 32), the amino acid sequence of CDRL3 (SEQ ID NO: 33), the amino acid sequence of CDRH1 (SEQ ID NO: 34), the amino acid sequence of CDRH2 (SEQ ID NO: 35), and the amino acid sequence of CDRH3 (SEQ ID NO: 36) of anti-TROP2 antibody 2. CDR sequences were determined by Kabat's definition.
[Figure 12] Figure 12 shows the amino acid sequence of CDRL1 (SEQ ID NO: 37), the amino acid sequence of CDRL2 (SEQ ID NO: 38), the amino acid sequence of CDRL3 (SEQ ID NO: 39), the amino acid sequence of CDRH1 (SEQ ID NO: 40), the amino acid sequence of CDRH2 (SEQ ID NO: 41), and the amino acid sequence of CDRH3 (SEQ ID NO: 42) of anti-EGFR antibody 1. CDR sequences were determined by Kabat's definition.
[Figure 13] Figure 13 shows the amino acid sequence of CDRL1 (SEQ ID NO: 43), the amino acid sequence of CDRL2 (SEQ ID NO: 44), the amino acid sequence of CDRL3 (SEQ ID NO: 45), the amino acid sequence of CDRH1 (SEQ ID NO: 46), the amino acid sequence of CDRH2 (SEQ ID NO: 47), and the amino acid sequence of CDRH3 (SEQ ID NO: 48) of anti-EGFR antibody 2. CDR sequences were determined by Kabat's definition.
[Figure 14] Figure 14 shows the light chain amino acid sequence (SEQ ID NO: 49) and the heavy chain amino acid sequence (SEQ ID NO: 50) of trastuzumab.
[Figure 15] Figure 15 shows the light chain amino acid sequence (SEQ ID NO: 49) and the heavy chain amino acid sequence (SEQ ID NO: 51) of modified HER2 antibody.
[Figure 16] Figure 16 shows the light chain amino acid sequence (SEQ ID NO: 52) and the heavy chain amino acid sequence (SEQ ID NO: 53) of pertuzumab.
[Figure 17] Figure 17 shows the light chain amino acid sequence (SEQ ID NO: 52) and the heavy chain amino acid sequence (SEQ ID NO: 54) of modified HER2 antibody 2.
[Figure 18] Figure 18 shows the light chain amino acid sequence (SEQ ID NO: 55) of anti-CDH6 antibody and the heavy chain amino acid sequence (SEQ ID NO: 56) of anti-CD33 antibody.

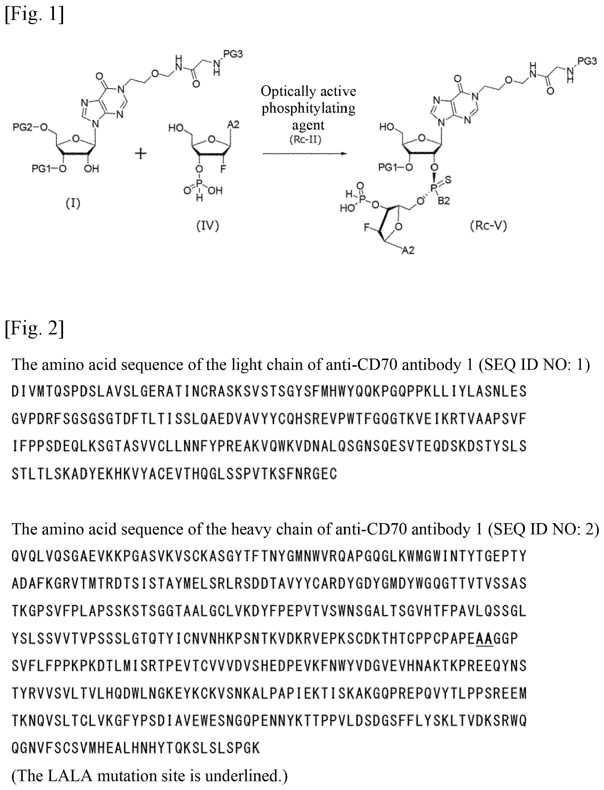

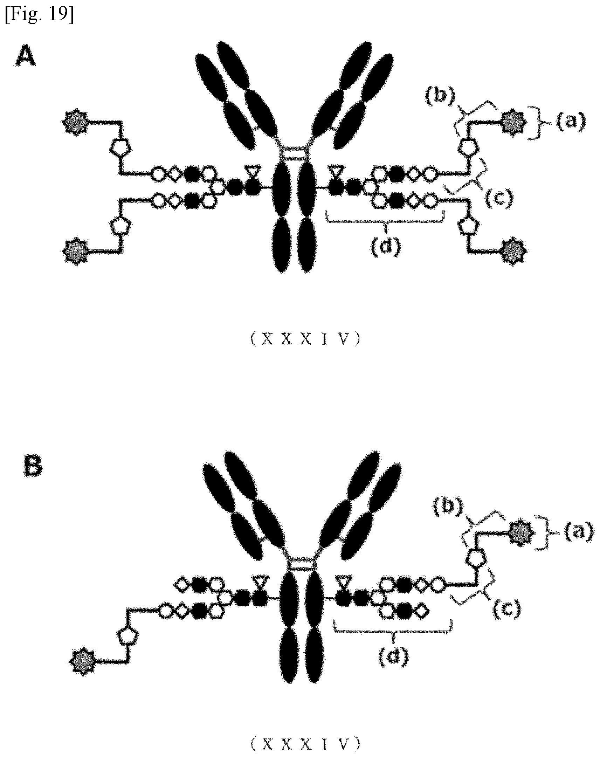

[Figure 19] Figure 19 schematically illustrates antibody-immunostimulant conjugates, namely an antibody-immunostimulant conjugate (a molecule (XXXIV) in Figure 1A) obtained from an SG-type glycan-remodeled antibody and an antibody-immunostimulant conjugate (a molecule (XXXIV) in Figure 1B) obtained from an MSG-type glycan-remodeled antibody. (a) denotes a immunostimulant D, (b) denotes a linker L, (c) denotes a PEG linker (L(PEG)), and (d) denotes a N297 glycan (where white circle is NeuAc (Sia), white hexagon is Man, black hexagon is GlcNAc, white diamond is Gal, and white inverted triangle is Fuc), respectively. The white pentagon denotes a triazole ring formed by the reaction of the linker L-derived alkyne with the PEG linker-derived azide group. The Y-shaped diagram represents an antibody Ab. The PEG linker is linked via an amide bond to the carboxyl group at 2-position of the sialic acid located at the non-reducing end. Such notation is applicable throughout this disclosure unless otherwise noted.
[Figure 20] Figure 20 shows the amino acid sequence of CDRL1 (SEQ ID NO: 57), the amino acid sequence of CDRL2 (SEQ ID NO: 58), the amino acid sequence of CDRL3 (SEQ ID NO: 59), the amino acid sequence of CDRH1 (SEQ ID NO: 60), the amino acid sequence of CDRH2 (SEQ ID NO: 61), and the amino acid sequence of CDRH3 (SEQ ID NO: 62) of pertuzumab. CDR sequences were determined by IMGT definition.
[Figure 21] Figure 21 shows the amino acid sequence of CDRL1 (SEQ ID NO: 63), the amino acid sequence of CDRL2 (SEQ ID NO: 64), the amino acid sequence of CDRL3 (SEQ ID NO: 65), the amino acid sequence of CDRH1 (SEQ ID NO: 66), the amino acid sequence of CDRH2 (SEQ ID NO: 67), and the amino acid sequence of CDRH3 (SEQ ID NO: 68) of anti-CDH6 antibody. CDR sequences were determined by Kabat's definition.

### Description of Embodiments

An aspect of the present invention provides a novel method for preparing a CDN derivative, namely an intermediate for an antibody-immunostimulant conjugate that has STING agonist activity and activates immune cells. WO2020/050406 (Patent Literature 6) discloses such an antibody-immunostimulant conjugate.

One embodiment of the present invention is a method for stereoselective synthesis of a CDN derivative having the structure of the following formula (Rp-VII) or formula (Sp-VII):

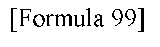

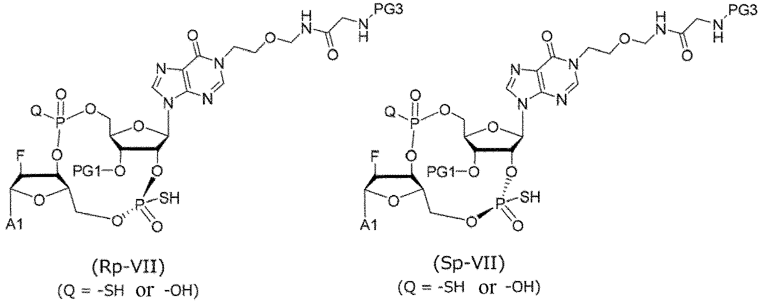

In one embodiment, a representative scheme of the present invention is outlined below while focusing on a novel method for preparing a CDN derivative of formula (Rp-VII).

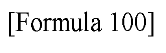

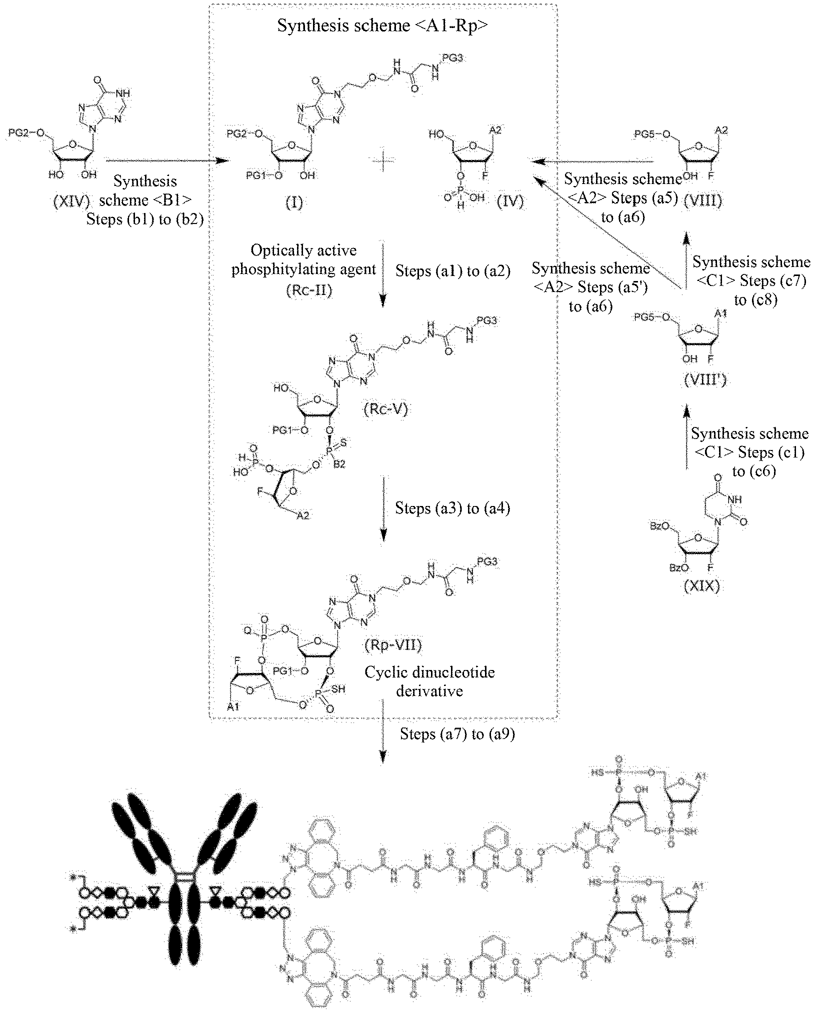

### (Rp,Rp-XIII) Antibody-immunostimulant conjugate

Hereinafter, preferable embodiments of the present invention will be described. Note that the below-described embodiments are representative examples of the embodiments of the present invention. The scope of the present invention should not be narrowly construed due to them. Note that as used herein, the compound numbers designated in each reaction scheme are used to indicate each compound. Specifically, for instance, the term "compound of formula (I)" or "compound (I)" is used. Also, the same applies to compounds with numbers other than the above.

### <1. Production of cyclic dinucleotide (CDN) and CDN-linker>

### <1-1. Method for preparing cyclic dinucleotide (compound (Rp-VII) or (Sp-VII)) and intermediate therefor>

In conventional preparation methods, the following four types of cyclic dinucleotides have been synthesized not in a stereoselective manner (see, for example, Patent Literature 6). The stereo configuration of these four different cyclic dinucleotides is denoted as (Rp,Rp), (Sp,Rp), (Rp,Sp), and (Sp,Sp) for the absolute stereo configuration on the two phosphorus atoms (absolute stereo configuration of phosphorus at 2-position, absolute stereo configuration of phosphorus at 10-position, respectively).

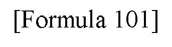

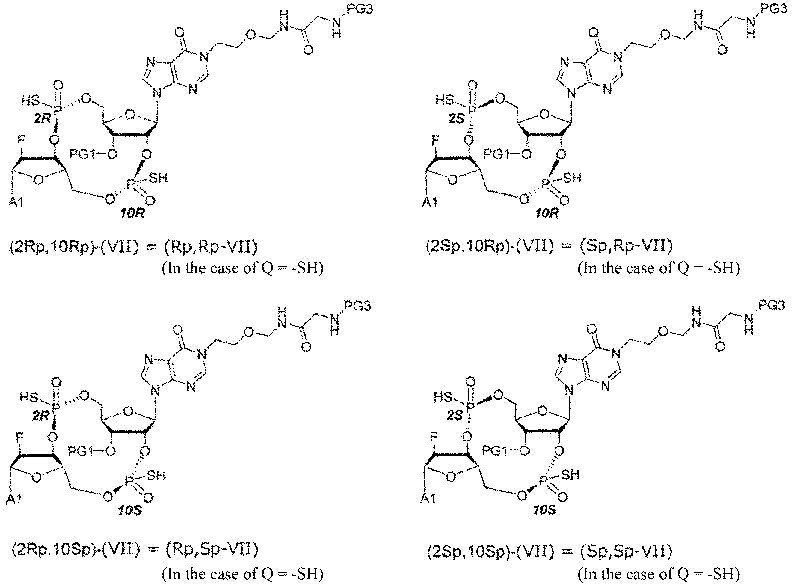

In one embodiment for the production of CDN in the present invention, a cyclic dinucleotide of formula (Rp-VII) may be produced according to the following synthesis scheme <A1-Rp>.

### [Synthesis Scheme <A1-Rp>]

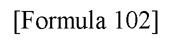

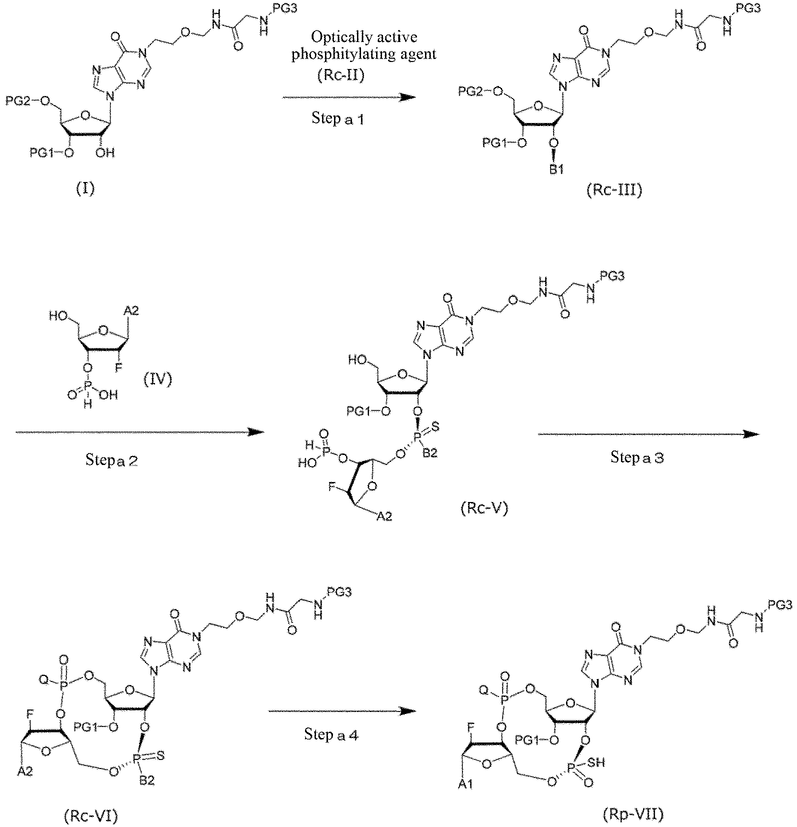

In another embodiment, a cyclic dinucleotide of formula (Sp-VII) may be produced according to the following synthesis scheme <A1-Sp>.

### [Synthesis Scheme <A1-Sp>]

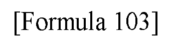

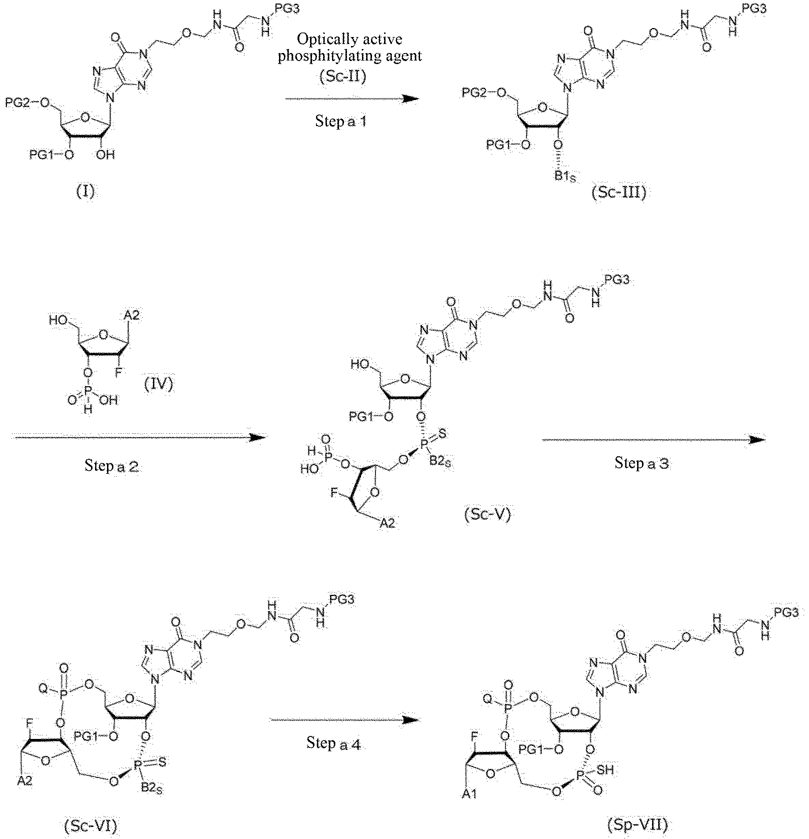

In the above [Synthesis Schemes <A1-Rp> and <A1-Sp>],
PG1 is a protecting group for a hydroxy group,
PG2 is a protecting group for a hydroxy group,
PG3 is a protecting group for an amino group,
A1 is 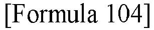 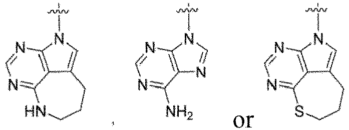
A2 is 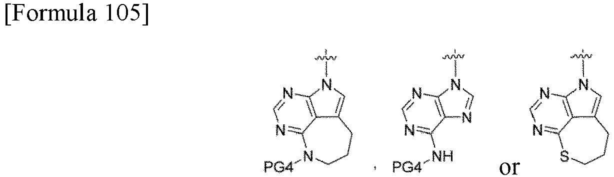 wherein PG4 is a protecting group for an amino group,
an optically active phosphitylating agent (Rc-II) is 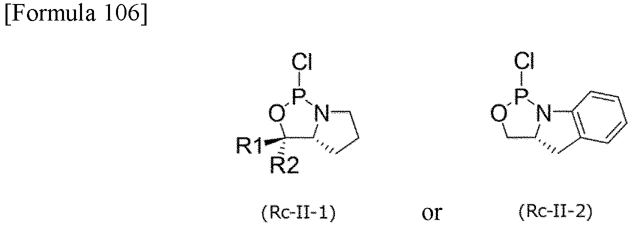
wherein R1 is hydrogen or methyl, R2 is hydrogen, C₁₋₃ alkyl or phenyl, wherein the alkyl is unsubstituted or substituted with one or more phenyl, tosyl or diphenylmethylsilyl, and the phenyl is unsubstituted or substituted with nitro or methoxy, and
an optically active phosphitylating agent (Sc-II) is 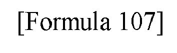 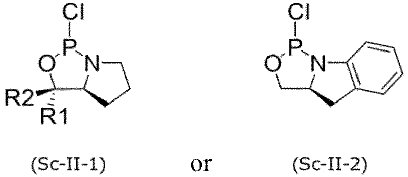
wherein R1 and R2 have the same meanings as of the above phosphitylating agent (Rc-II).

In the synthesis scheme <A1-Rp>, B1 is

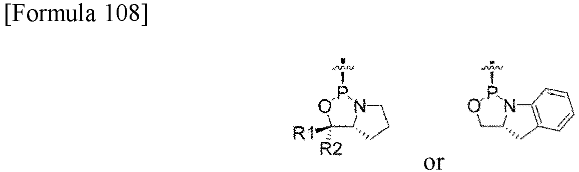

In the synthesis scheme <A1-Sp>, B1_{S} is

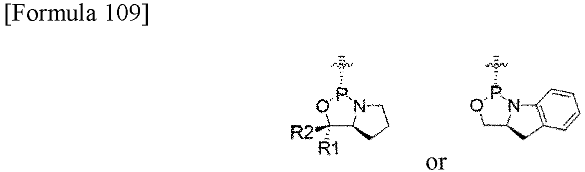

In the synthesis scheme <A1-Rp>, B2 is

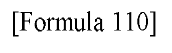

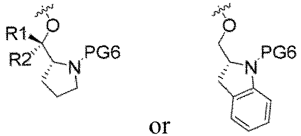

In the synthesis scheme <A1-Sp>, B2s is 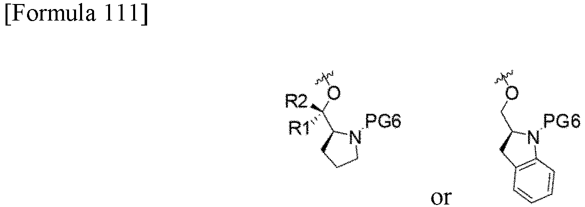
wherein PG6 is a protecting group for an amino group, and
Q is a thiol group or a hydroxy group.

In the present production method, examples of PG1 include *tert-*butyldimethylsilyl, trimethylsilyl, triethylsilyl, triisopropylsilyl, or *tert-*butyldiphenylsilyl. Preferred is tert-butyldimethylsilyl or trimethylsilyl. More preferred is tert-butyldimethylsilyl.

Examples of PG2 include 4,4'-dimethoxytrityl, 4-methoxytrityl, 2-chlorotrityl, or trityl. Preferred is 4,4'-dimethoxytrityl, 4-methoxytrityl, or trityl. More preferred is 4,4'-dimethoxytrityl.

Examples of PG3 include 2-(trimethylsilyl)ethoxycarbonyl, *tert-*butoxycarbonyl, 9-fluorenylmethyloxycarbonyl, allyloxycarbonyl, 2,2,2-trichloroethoxycarbonyl, or benzyloxycarbonyl. Preferred is 2-(trimethylsilyl)ethoxycarbonyl or allyloxycarbonyl. More preferred is 2-(trimethylsilyl)ethoxycarbonyl.

Examples of PG4 include benzyl, benzoyl, tert-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, or ethoxycarbonyl. Preferred is benzoyl or 2-(trimethylsilyl)ethoxycarbonyl.

Examples of PG6 include acetyl, trifluoroacetyl, benzoyl, *tert-*butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, methoxycarbonyl, or ethoxycarbonyl. Preferred is acetyl, trifluoroacetyl, benzoyl, 9-fluorenylmethyloxycarbonyl, allyloxycarbonyl, or 2-(trimethylsilyl)ethoxycarbonyl. More preferred is acetyl, trifluoroacetyl, allyloxycarbonyl, or 2-(trimethylsilyl)ethoxycarbonyl.

Hereinbelow, each step will be described.

### (Step a1)

In one embodiment, this step is a step of reacting a compound of formula (I) with an optically active phosphitylating agent (Rc-II) to obtain a compound of formula (Rc-III). Another embodiment involves a step of reacting with an optically active phosphitylating agent (Sc-II) to obtain a compound of formula (Sc-III).

In one embodiment, examples of an optically active phosphitylating agent (Rc-II) used in this step include a compound represented by the following formula (Rc-II-1) or (Rc-II-2): 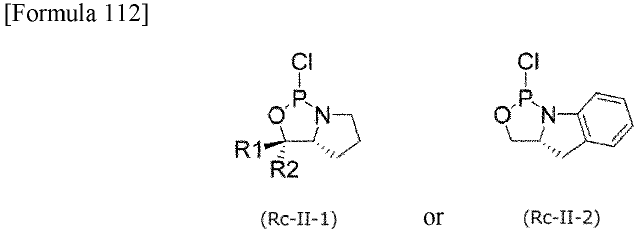 wherein
R1 is hydrogen or methyl, and
R2 is hydrogen, C₁₋₃ alkyl, or phenyl,
   wherein the alkyl is unsubstituted or substituted with one or more phenyl, tosyl, or diphenylmethylsilyl, and
   the phenyl is unsubstituted or substituted with nitro or methoxy.
The optically active phosphitylating agent is a reagent for stereoselective condensation of a compound of formula (I) and a compound of formula (IV) to produce a compound of formula (Rc-V). The optically active phosphitylating agent reacts stereoselectively with a compound of formula (I) to form an intermediate of formula (Rc-III).

Examples of the optically active phosphitylating agent (Rc-II) that can be used include (3aR)-1-chlorotetrahydro-1H,3H-pyrrolo[1,2-c][1,3,2]oxazaphosphole, (3aR)-1-chloro-3,3-dimethyltetrahydro-1H,3H-pyrrolo[1,2-c][1,3,2]oxazaphosphole, (3S,3aR)-1-chloro-3-methyl-3-phenyltetrahydro-1H,3H-pyrrolo[1,2-c][1,3,2]oxazaphosphole, (3S,3aR)-1-chloro-3-phenyltetrahydro-1H,3H-pyrrolo[1,2-c][1,3,2]oxazaphosphole, (3R,3aR)-1-chloro-3-[(4-methylbenzene-1-sulfonyl)methyl]tetrahydro-1H,3H-pyrrolo[1,2-c][1,3,2]oxazaphosphole, (3S,3aR)-1-chloro-3-[(4-nitrophenyl)methyl]tetrahydro-1H,3H-pyrrolo[1,2-c][1,3,2]oxazaphosphole, (3R,3aR)-1-chloro-3-{[methyl(diphenyl)silyl]methyl}tetrahydro-1H,3H-pyrrolo[1,2-c][1,3,2]oxazaphosphole, (3S,3aR)-1-chloro-3-[(4-methoxyphenyl)methyl]tetrahydro-1H,3H-pyrrolo[1,2-c][1,3,2]oxazaphosphole, (3S,3aR)-1-chloro-3-(diphenylmethyl)tetrahydro-1H,3H-pyrrolo[1,2-c][1,3,2]oxazaphosphole, or (3aR)-1-chloro-3a,4-dihydro-1H,3H-[1,3,2]oxazaphosphoro[3,4-a]indole. Preferably used is (3aR)-1-chlorotetrahydro-1H,3H-pyrrolo[1,2-c][1,3,2]oxazaphosphole, (3aR)-1-chloro-3,3-dimethyltetrahydro-1H,3H-pyrrolo[1,2-c][1,3,2]oxazaphosphole, (3S,3aR)-1-chloro-3-methyl-3-phenyltetrahydro-1H,3H-pyrrolo[1,2-c][1,3,2]oxazaphosphole, (3S,3aR)-1-chloro-3-phenyltetrahydro-1H,3H-pyrrolo[1,2-c][1,3,2]oxazaphosphole, or (3R,3aR)-1-chloro-3-{[methyl(diphenyl)silyl]methyl}tetrahydro-1H,3H-pyrrolo[1,2-c][1,3,2]oxazaphosphole. More preferably used is (3aR)-1-chlorotetrahydro-1H,3H-pyrrolo[1,2-c][1,3,2]oxazaphosphole, (3aR)-1-chloro-3,3-dimethyltetrahydro-1H,3H-pyrrolo[1,2-c][1,3,2]oxazaphosphole, (3S,3aR)-1-chloro-3-phenyltetrahydro-1H,3H-pyrrolo[1,2-c][1,3,2]oxazaphosphole, or (3R,3aR)-1-chloro-3-{[methyl(diphenyl)silyl]methyl}tetrahydro-1H,3H-pyrrolo[1,2-c][1,3,2]oxazaphosphole. The amount of the optically active phosphitylating agent used in this step is not limited as long as the reaction proceeds, but is preferably from 1 to 3 equivalents of the compound represented by formula (I).

The structure of the optically active phosphitylating agent (Rc-II) exemplified above is shown below.

**[Table 1]**

| Table A. Structure of phosphitylating agent (Rc-II) | | | |
|---|---|---|---|
| Compound name | Structure | Compound name | Structure |
| (3aR)-1-Chlorotetrahydro-1H,3H-pyrrolo[1,2-c] [1,3,2]oxazaphosphole | 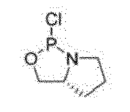 | (3S,3aR)-1-Chloro-3-[(4-nitrophenyl) methyl]tetrahydro-1H,3H-pyrrolo[1,2-c] [1,3,2]oxazaphosphole | 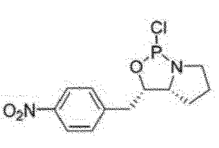 |
| (3aR)-1-Chloro-3,3-dimethyltetrahydro-1H,3H-pyrrolo[1,2-c] [1,3,2]oxazaphosphole | 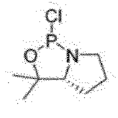 | (3R,3aR)-1-Chloro-3-{[methyl(diphenyl) silyl]methyl}tetrahydro -1H,3H-pyrrolo[1,2-c] [1,3,2]oxazaphosphole | 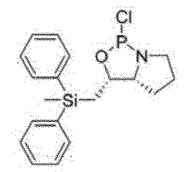 |
| (3S,3aR)-1-Chloro-3-methyl-3-phenyltetrahydro-1H,3H-pyrrolo[1,2-c] [1,3,2]oxazaphosphole | 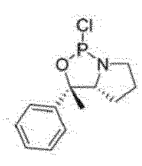 | (3S,3aR)-1-Chloro-3-[(4-methoxyphenyl) methyl]tetrahydro-1H,3H-pyrrolo[1,2-c] [1,3,2]oxazaphosphole | 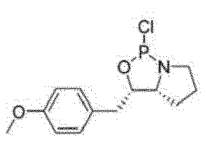 |
| (3S,3aR)-1-Chloro-3-phenyltetrahydro-1H,3H-pyrrolo[1,2-c] [1,3,2]oxazaphosphole | 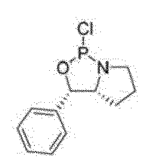 | (3S,3aR)-1-Chloro-3-(diphenylmethyl)tetrah ydro-1H,3H-pyrrolo[1,2-c] [1,3,2]oxazaphosphole | 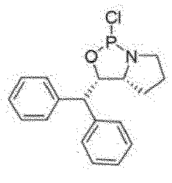 |
| (3R,3aR)-1-Chloro-3-[(4-methylbenzene-1-sulfonyl)methyl] tetrahydro-1H,3H-pyrrolo[1,2-c] [1,3,2]oxazaphosphole | 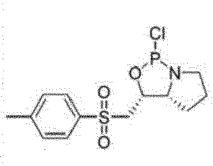 | (3aR)-1-Chloro-3a,4-dihydro-1H,3H-[1,3,2]oxazaphosphoro [3,4-a]indole | 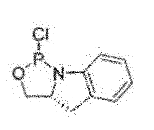 |

In addition, examples of an optically active phosphitylating agent (Sc-II) used in another embodiment can include a compound represented by the following formula (Sc-II-1) or (Sc-II-2): 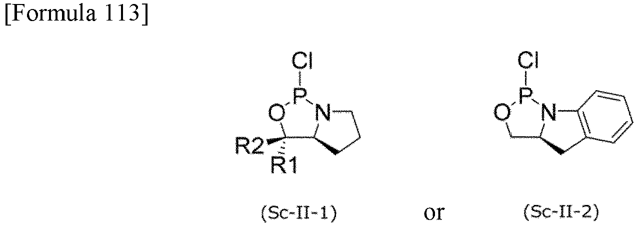 wherein
R1 is hydrogen or methyl, and
R2 is hydrogen, C₁₋₃ alkyl, or phenyl,
   wherein the alkyl is unsubstituted or substituted with one or more phenyl, tosyl, or diphenylmethylsilyl, and
   the phenyl is unsubstituted or substituted with nitro or methoxy.

Examples of the optically active phosphitylating agent (Sc-II) that can be used include (3aS)-1-chlorotetrahydro-1H,3H-pyrrolo[1,2-c][1,3,2]oxazaphosphole, (3aS)-1-chloro-3,3-dimethyltetrahydro-1H,3H-pyrrolo[1,2-c][1,3,2]oxazaphosphole, (3R,3aS)-1-chloro-3-methyl-3-phenyltetrahydro-1H,3H-pyrrolo[1,2-c][1,3,2]oxazaphosphole, (3R,3aS)-1-chloro-3-phenyltetrahydro-1H,3H-pyrrolo[1,2-c][1,3,2]oxazaphosphole, (3 S,3aS)-1-chloro-3-[(4-methylbenzene-1-sulfonyl)methyl]tetrahydro-1H,3H-pyrrolo[1,2-c][1,3,2]oxazaphosphole, (3R,3aS)-1-chloro-3-[(4-nitrophenyl)methyl]tetrahydro-1H,3H-pyrrolo[1,2-c][1,3,2]oxazaphosphole, (3S,3aS)-1-chloro-3-{[methyl(diphenyl)silyl]methyl}tetrahydro-1H,3H-pyrrolo[1,2-c][1,3,2]oxazaphosphole, (3R,3aS)-1-chloro-3-[(4-methoxyphenyl)methyl]tetrahydro-1H,3H-pyrrolo[1,2-c][1,3,2]oxazaphosphol, (3R,3aS)-1-chloro-3-(diphenylmethyl)tetrahydro-1H,3H-pyrrolo[1,2-c][1,3,2]oxazaphosphole, or (3aS)-1-chloro-3a,4-dihydro-1H,3H-[1,3,2]oxazaphosphoro[3,4-a]indole. Preferably used is (3aS)-1-chlorotetrahydro-1H,3H-pyrrolo[1,2-c][1,3,2]oxazaphosphole, (3aS)-1-chloro-3,3-dimethyltetrahydro-1H,3H-pyrrolo[1,2-c][1,3,2]oxazaphosphole, (3R,3aS)-1-chloro-3-methyl-3-phenyltetrahydro-1H,3H-pyrrolo[1,2-c][1,3,2]oxazaphosphole, (3R,3aS)-1-chloro-3-phenyltetrahydro-1H,3H-pyrrolo[1,2-c][1,3,2]oxazaphosphole, or (3S,3aS)-1-chloro-3-{[methyl(diphenyl)silyl]methyl}tetrahydro-1H,3H-pyrrolo[1,2-c][1,3,2]oxazaphosphole. More preferably used is (3aS)-1-chlorotetrahydro-1H,3H-pyrrolo[1,2-c][1,3,2]oxazaphosphole, (3aS)-1-chloro-3,3-dimethyltetrahydro-1H,3H-pyrrolo[1,2-c][1,3,2]oxazaphosphole, (3R,3aS)-1-chloro-3-phenyltetrahydro-1H,3H-pyrrolo[1,2-c][1,3,2]oxazaphosphole, or (3S,3aS)-1-chloro-3-{[methyl(diphenyl)silyl]methyl}tetrahydro-1H,3H-pyrrolo[1,2-c][1,3,2]oxazaphosphole. The amount of the optically active phosphitylating agent used in this step is not limited as long as the reaction proceeds, but is preferably from 0.5 to 10 equivalents and more preferably from 1 to 3 equivalents of the compound represented by formula (I).

The structure of the optically active phosphitylating agent (Sc-II) exemplified above is shown below.

**[Table 2]**

| Table B. Structure of phosphitylating agent (Sc-11) | | | |
|---|---|---|---|
| Compound name | Structure | Compound name | Structure |
| (3aS)-1-Chlorotetrahydro-1H,3H-pyrroto[1,2-c] [1,3,2]oxazaphosphole | 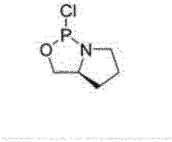 | (3R,3aS)-1-Chloro-3-[(4-nitrophenyl) methyl]tetrahydro-1H,3H-pyrrolo[1,2-c] [1,3,2]oxazaphosphole | 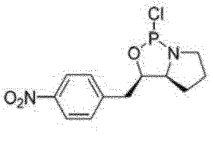 |
| (3aS)-1-Chloro-3,3-dimethyltetrahydro-1H,3H-pyrrolo[1,2-c] [1,3,2]oxazaphosphole | 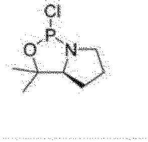 | (3S,3aS)-1-Chloro-3-{[methyl(diphenyl)sily l]methyl}tetrahydro-1H,3H-pyrrolo[1,2-c] [1,3,2]oxazaphosphole | 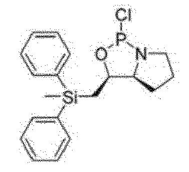 |
| (3R,3aS)-1-Chloro-3-methyl-3-phenyltetrahydro-1H,3H-pyrrolo[1,2-c] [1,3,2]oxazaphosphole | 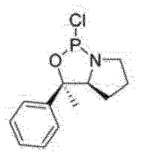 | (3R,3aS)-1-Chloro-3-[(4-methoxyphenyl) methyl]tetrahydro-1H,3H-pyrrolo[1,2-c] [1,3,2]oxazaphosphole | 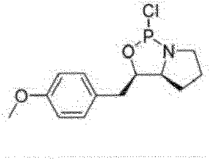 |
| (3R,3aS)-1-Chloro-3-phenyltetrahydro-1H,3H-pyrrolo[1,2-c] [1,3,2]oxazaphosphole | 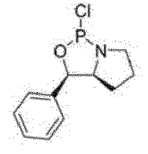 | (3R,3aS)-1-Chloro-3-(diphenylmethyl) tetrahydro-1H,3H-pyrrolo[1,2-c] [1,3,2]oxazaphosphole | 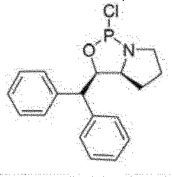 |
| (3S,3aS)-1-Chloro-3-[(4-methylbenzene-1-sulfonyl)methyl] tetrahydro-1H,3H-pyrrolo[1,2-c] [1,3,2]oxazaphosphole | 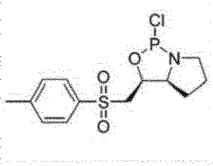 | (3aS)-1-Chloro-3a,4-dihydro-1H,3H-[1,3,2]oxazaphosphoro [3,4-a]indole | 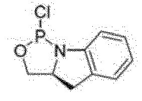 |

In this step, the reaction proceeds stereoselectively to be able to obtain a compound of formula (III) with the desired absolute stereo configuration. Here, the absolute stereo configuration of the compound of formula (III) is shown by using the absolute stereo configuration (Rc) or (Sc) on the chiral carbon atom in the prolinol skeleton, as exemplified below.

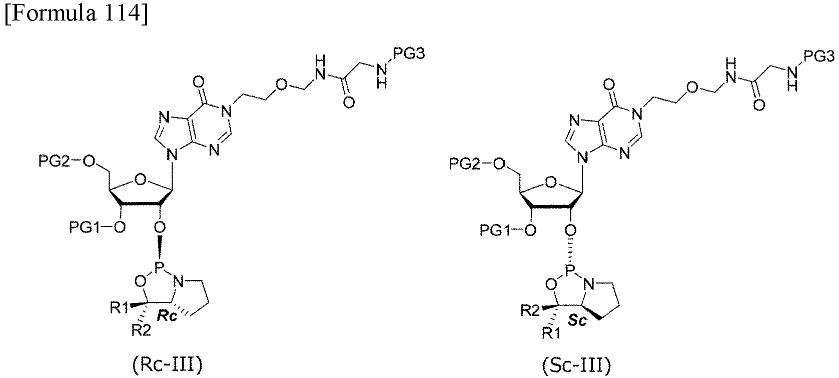

In one embodiment, an optically active phosphitylating agent (Rc-II) in which R1 and R2 are hydrogen atoms may be used. In this case, a compound of formula (Rc-III) with the absolute stereo configuration (Sp) on the phosphorus atom is produced, as shown below.

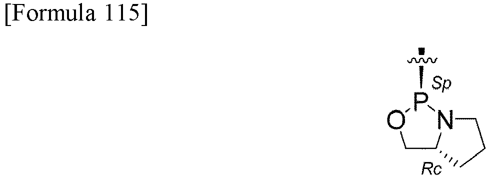

In another embodiment, an optically active phosphitylating agent (Sc-II) may be used. This case can result in a diastereomer having the opposite absolute stereo configuration with respect to the chiral point on the phosphorus.

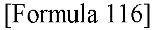

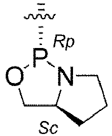

That is, the optically active phosphitylating agent with an absolute stereo configuration of (Rc) or (Sc) may be used to produce each desired diastereomer with a high selectivity of 90% or higher, preferably 95% or higher, and more preferably 98% or higher. In the conventional production method, the phosphorothioate bond is formed not in a stereoselective manner, resulting in the formation of (Rp) and (Sp) forms in an approximate 50 : 50 ratio, therefore the yield of compound with the desired absolute stereo configuration was half or less.

This step can preferably be performed in the presence of a base. Examples of the base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium tert-butoxide). Preferable examples include triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, or 1,4-diazabicyclo[2.2.2]octane. More preferable examples include triethylamine, diisopropylethylamine, or N-methylmorpholine. The amount of the base used in this step is not limited as long as the reaction proceeds, but is preferably from 0.5 to 10 equivalents and more preferably from 1 to 5 equivalents of the compound represented by formula (I).

The solvent that can be used in this step is not particularly limited as long as the reaction is not inhibited. Examples include acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is acetonitrile, dichloromethane, 1,2-dimethoxyethane, tetrahydrofuran, 2-methyltetrahydrofuran, or toluene. More preferred is dichloromethane or tetrahydrofuran.

The reaction temperature in this step is not limited as long as the reaction proceeds, but is preferably from -78°C to the boiling point of the solvent used in the reaction, and more preferably from -20°C to 30°C. The reaction time in this step is not limited as long as the reaction proceeds, but is preferably from 2 minutes to 10 hours, and more preferably from 5 to 180 minutes.

### (Step a2)

In one embodiment, this step is a step of reacting a compound of formula (Rc-III) and a compound of formula (IV) or a salt thereof in the presence of an activator, followed by treatment with an acylating or alkoxycarbonylating agent, by a further reaction with a sulfurizing agent, and then by removal of PG2 by deprotection to obtain a compound of formula (Rc-V). Optionally, the product may be isolated before the removal of PG2 by deprotection. Another embodiment involves a step of obtaining a compound of formula (Sc-V) from a compound of formula (Sc-III) by using substantially the same reaction. This step optionally includes treatment with an organic or inorganic base to convert the compound to a salt thereof.

In this step, an intermediate obtained after the reaction with the sulfurizing agent but before the removal of PG2 by deprotection is a compound represented by formula (Rc-V₀): 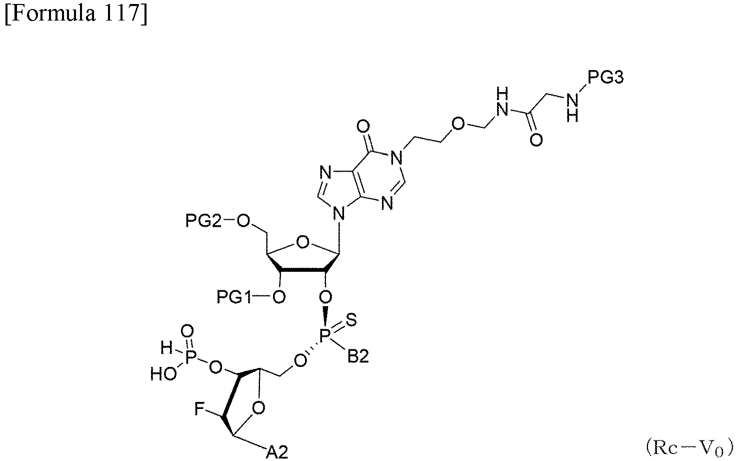 or a salt thereof, wherein A2, B2, PG1, PG2 and PG3 have the same meanings as the groups described in the synthesis scheme <A1-Rp>.

In another embodiment, an intermediate obtained after the reaction with the sulfurizing agent but before the removal of PG2 by deprotection is a compound represented by formula (Sc-V₀): 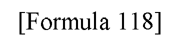 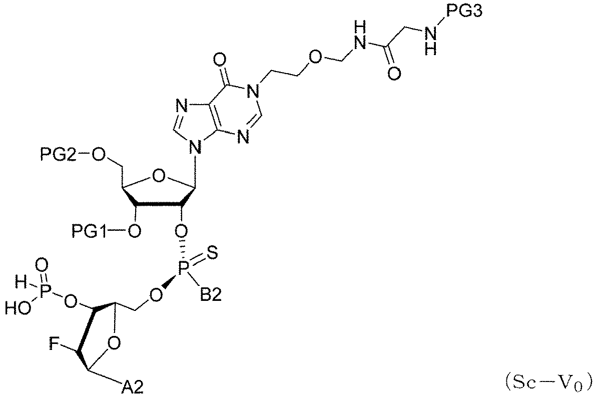 or a salt thereof, wherein A2, B2s, PG1, PG2 and PG3 have the same meanings as the groups described in the synthesis scheme <A1-Sp>.

In one embodiment, in this step, the reaction proceeds in a stereoselective manner, so that the compound of formula (Rc-V) with the desired absolute stereo configuration can be obtained through the intermediate of formula (Rc-V₀). Here, the absolute stereo configuration of formula (V) or (V₀) is shown by using the absolute stereo configuration on the chiral carbon atom contained in the prolinol skeleton in B2 in a manner similar to the above compound of formula (III).

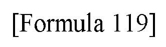

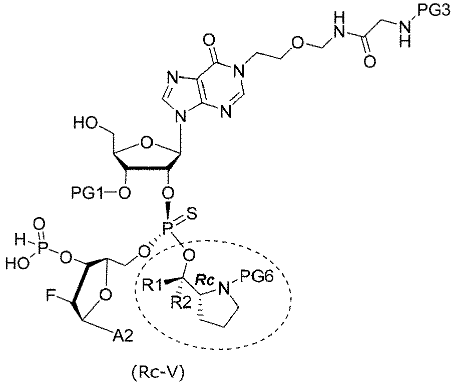

In this step, the reaction proceeds in a stereo-inverted manner with a selectivity of 70% or higher, 75% or higher, 80% or higher, 85% or higher, 90% or higher, 95% or higher, or 98% or higher while maintaining the chiral environment established in formula (Rc-III). This allows the desired diastereomer to be obtained with a high selectivity of 70% or higher, 75% or higher, 80% or higher, 85% or higher, 90% or higher, 95% or higher, or 98% or higher. In the conventional method, compounds of formula (I) and (IV) are condensed not in a stereoselective manner, so that a mixture of (Rp) and (Sp) forms, which corresponds to a compound of formula (V) and are difficult to separate, is obtained at a ratio of approximately 50 : 50. Therefore, about two-fold increase in the yield of the desired compound (Rc-V) is observed from the viewpoint of stereoselectivity. In addition, the productivity is expected to increase by a factor of 3 to 10 in view of the separation load of a large amount of difficult-to-separate diastereomers on the phosphorus.

On the other hand, in the case of using a compound of formula (Sc-III), the compound of formula (Sc-V), an inverted diastereomer, can be likewise obtained through the intermediate of formula (Sc-V₀). Thus, depending on the absolute stereo configuration of the chiral carbon on the prolinol skeleton of the optically active phosphitylating agent used, a compound with the desired absolute stereo configuration can be stereoselectively obtained.

The activator used in this step is not particularly limited as long as the reaction proceeds without impairing the absolute stereo configuration of the compound represented by formula (Rc-III) or formula (Sc-III). Examples include 1-phenylimidazole, benzimidazole, 1-methylbenzimidazole, 1-cyanomethylpiperidine, 1-pyrrolidineacetonitrile, 1-(cyanomethyl)imidazole, or a salt thereof. Preferred is 1-phenylimidazole, 1-methylbenzimidazole, 1-cyanomethylpiperidine, 1-(cyanomethyl)imidazole or a salt thereof. More preferred is 1-phenylimidazole, 1-methylbenzimidazole, or a salt thereof. The amount of the activator used in this step is not limited as long as the reaction proceeds, but is preferably from 1 to 3 equivalents of the compound represented by formula (Rc-III) or (Sc-III).

The amount of compound (IV) or a salt thereof used in this step is not limited as long as the reaction proceeds, but is preferably from 0.5 to 3 equivalents and more preferably from 0.8 to 1.2 equivalents of the compound represented by formula (Rc-III) or (Sc-III).

The acylating or alkoxycarbonylating agent used in this step is not particularly limited as long as the reaction proceeds. Examples include acetic anhydride, acetyl chloride, N-succinimidyl acetate, pentafluorophenyl acetate, 1-acetyl-1H-1,2,3-triazolo[4,5-b]pyridine, N-methoxydiacetamide, N-acetylimidazole, trifluoroacetic anhydride, bistrifluoroacetamide, ethyl trifluoroacetate, methyl trifluoroacetate, pentafluorophenyl trifluoroacetate, trifluoroacetyl benzotriazole, S-ethyl trifluorothioacetate , N-methylbistrifluoroacetamide, trifluoroacetyltriflate, 1-trifluoroacetylimidazole, benzoic anhydride, benzoyl chloride, pentafluorophenylbenzoate, pentafluorophenylbenzoate, benzoyl trifluoromethanesulfonate, 3-benzoylthiazolidine-2-thione, *tert-*butylphenylcarbonate, N-(*tert*-butoxycarbonyloxy)phthalimide, 2*-*(*tert-*butoxycarbonylthio)-4,6-dimethylpyridine, N-*tert*-butoxycarbonyl imidazole, *tert-*butylcarbazate, 2-(tert-butoxycarbonyloxyimino)-2-phenylacetonitrile, 1*-tert-*butoxycarbonyl-1,2,4-triazole, N-*tert*-butoxycarbonyl imidazole, di*-tert-*butyldicarbonate, 9-fluorenylmethylpentafluorophenylcarbonate, 9-fluorenylmethyl chloroformate, 9-fluorenylmethylcarbazate, 19-fluorenylmethylcarbamate, 1-[(9H-fluoren-9-ylmethoxy)carbonyloxy]benzotriazole, N-[(9H-fluoren-9-ylmethoxy)carbonyloxy]succinimide, allyl chloroformate, diallyl dicarbonate, N-(allyloxycarbonyloxy)succinimide, allyl phenyl carbonate, N-(2,2,2-trichloroethoxycarbonyloxy)succinimide, 2,2,2-trichloroethyl chloroformate, benzyl chloroformate, benzyl carbazate, benzyl phenyl carbonate, N-carbobenzyloxysuccinimide, dibenzyl dicarbonate, 4-[2-(trimethylsilyl)ethoxycarbonyloxy]nitrobenzene, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, ethyl chloroformate, diethyldicarbonate, ethyl imidazole-1-carboxylate, 2-ethoxy-1-(ethoxycarbonyl)-1,2-dihydroquinoline, methyl chloroformate, dimethyl carbonate, dimethyldicarbonate, or methyl imidazole-1-carboxylate. Preferred is acetic anhydride, 1-trifluoroacetylimidazole, benzoic anhydride, N-[(9H-fluoren-9-ylmethoxy)carbonyloxy]succinimide, N-(allyloxycarbonyloxy)succinimide, or N-[2-(trimethylsilyl)ethoxycarbonyloxy]succinimide. More preferred is acetic anhydride, 1-trifluoroacetylimidazole, N-(allyloxycarbonyloxy)succinimide, or N-[2-(trimethylsilyl)ethoxycarbonyloxy] succinimide. The amount of the acylating or alkoxycarbonylating agent used in this step is not limited as long as the reaction proceeds, but is preferably from 0.7 to 5 equivalents and more preferably from 1 to 3 equivalents of the compound represented by formula (Rc-III) or (Sc-III).

The temperature of the reaction with the acylating or alkoxycarbonylating agent in this step is not limited as long as the reaction proceeds, but is preferably from -20°C to the boiling point of the solvent used in the reaction, and more preferably from -20°C to 30°C. The reaction time in this step is not limited as long as the reaction proceeds, but is preferably from 2 minutes to 5 hours, and more preferably from 5 to 90 minutes.

The reaction with the activator, acylating or alkoxycarbonylating agent, and sulfurizing agent in this step may preferably be carried out in the presence of a dehydrating agent. The dehydrating agent used in this step is not particularly limited as long as the reaction proceeds. Examples include Molecular Sieve 3A, Molecular Sieve 4A, Molecular Sieve 5A, Molecular Sieve 13X, magnesium sulfate, sodium sulfate, or calcium chloride. Preferred is Molecular Sieve 3A, Molecular Sieve 4A, Molecular Sieve 5A, Molecular Sieve 13X, or sodium sulfate. More preferred is Molecular Sieve 3A or Molecular Sieve 4A. The amount of dehydrating agent used in this step is not limited as long as the reaction proceeds, but is a mass preferably 0.01 to 3 times and more preferably 0.01 to 1 times as the mass of the compound represented by formula (Rc-III) or (Sc-III).

The sulfurizing agent used in this step is not particularly limited as long as the reaction proceeds. Examples of the sulfurizing agent that can be used include xanthane hydride, bis(phenylacetyl)disulfide, 3H-1,2-benzodithiol-3-one-1,1-dioxide, 5-phenyl-3H-1,2,4-dithiazol-3-one, or [(N,N-dimethylaminomethylidene)amino]-3H-1,2,4-dithiazolin-3-thione. Preferably used is xanthane hydride, bis(phenylacetyl)disulfide, or [(N,N-dimethylaminomethylidene)amino]-3H-1,2,4-dithiazolin-3-thione. The amount of the sulfurizing agent used in this step is not limited as long as the reaction proceeds, but is preferably from 0.5 to 5 equivalents and more preferably from 1 to 2 equivalents of the compound represented by formula (Rc-III) or (Sc-III).

The temperature of the reaction with a sulfurizing agent in this step is not limited as long as the reaction proceeds, but is preferably from -20°C to the boiling point of the solvent used in the reaction, and more preferably from 0°C to 30°C. The reaction time in this step is not limited as long as the reaction proceeds, but is preferably from 5 minutes to 5 hours, and more preferably from 5 to 90 minutes.

This step can preferably be performed in the presence of a base. Examples of the base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium tert-butoxide). Preferable examples include triethylamine, tributylamine, diisopropylethylamine, N-methylmorpholine, N-methylpyrrolidine, or N-methylpiperidine. More preferable examples include triethylamine, diisopropylethylamine, or N-methylmorpholine. The amount of the base used in this step is not limited as long as the reaction proceeds, but is preferably from 0.5 to 20 equivalents and more preferably from 1 to 10 equivalents of the compound represented by formula (Rc-III) or (Sc-III).

Examples of the acid used in the deprotection reaction in this step include hydrochloric acid, sulfuric acid, formic acid, oxalic acid, acetic acid, trifluoroacetic acid, monochloroacetic acid, dichloroacetic acid, trichloroacetic acid, methanesulfonic acid, p-toluenesulfonic acid, or benzenesulfonic acid. Preferred is hydrochloric acid, acetic acid, monochloroacetic acid, or dichloroacetic acid. More preferred is hydrochloric acid or dichloroacetic acid. The pH used for deprotection is not limited as long as the reaction proceeds, but is preferably from 1 to 4. The reaction temperature in the deprotection reaction is not limited as long as the reaction proceeds, but is preferably from -30°C to the boiling point of the solvent used in the reaction, and more preferably from -20°C to 30°C. The reaction time in this reaction is not limited as long as the reaction proceeds, but is preferably from 0.5 to 48 hours, and more preferably from 1 to 24 hours.

The solvent that can be used in this step is not particularly limited as long as the reaction is not inhibited. Examples that can be used include acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is acetonitrile or dichloromethane. More preferred is acetonitrile.

Examples of the organic base used in the conversion of the compound of formula (Rc-V) or (Sc-V) to a salt thereof include triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, or 1,8-diazabicyclo[5.4.0]undec-7-ene. In addition, examples of the inorganic base include potassium carbonate, potassium hydroxide, potassium bicarbonate, sodium carbonate, sodium hydroxide, sodium bicarbonate, sodium acetate, potassium acetate, sodium methoxide, sodium ethoxide, or potassium tert-butoxide. Preferable examples include triethylamine, tributylamine, diisopropylethylamine, N-methylmorpholine, N-methylpyrrolidine, N-methylpiperidine, potassium carbonate, potassium bicarbonate, sodium carbonate, or sodium bicarbonate. More preferable examples include triethylamine, potassium carbonate, potassium bicarbonate, sodium carbonate, or sodium bicarbonate.

### (Step a3)

In one embodiment, this step is a step of cyclizing a compound of formula (Rc-V) or a salt thereof in the presence of a condensing agent, followed by a reaction with a sulfurizing or oxidizing agent to obtain a compound of formula (Rc-VI). Another embodiment involves a step of obtaining a compound of formula (Sc-VI) from a compound of formula (Sc-V) by using substantially the same reaction.

In this step, the reaction with a sulfurizing agent can result in a compound of formula (VI) where Q is a thiol group. Alternatively, in this step, the reaction with an oxidizing agent can result in a compound of formula (VI) where Q is a hydroxy group.

The condensing agent used in this step is not particularly limited as long as the reaction proceeds. Examples include 2-chloro-5,5-dimethyl-1,3,2-dioxaphosphorinan 2-oxide, dimethylchlorophosphate, diethylchlorophosphate, 2-chloro-2-oxo-1,3,2-dioxaphospholane, 1H-benzotriazol-1-yloxytripyrrolidino phosphonium hexafluorophosphate, chlorotripyrrolidino hexafluorophosphate, bromotripyrrolidino hexafluorophosphate, propylphosphonic anhydride, diisopropyl chlorophosphate, bis(2,6-dimethylphenyl)chlorophosphate, bis(dimethylamino)phosphoryl chloride, bis(2-oxo-3-oxolidinyl)phosphonate chloride, diphenylphosphinic acid chloride, diethylchlorothiophosphate, (7-azabenzotriazol-1-yloxy)tripyrrolidinophosphonium hexafluorophosphate, 6-trifluoromethyl-1H-benzotriazol-1-yloxy tripyrrolidinophosphonium hexafluorophosphate, diphenylphosphorochloridate, bis(2,2,2-trichloroethyl)phosphorochloridate, ethyl dichlorophosphate, o-phenylene phosphorochloridate, 3-(diethoxyphosphoryloxy)-1,2,3-benzotriazin-4(3H)-one, 2-chloro-1-methylpyridinium iodide, 1-[bis(dimethylamino)methylene]-1H-benzotriazolium 3-oxide hexafluorophosphate, (benzotriazol-1-yloxy)tris(dimethylamino)phosphonium hexafluorophosphate, 1-adamantanecarbonyl chloride, or pivaloyl chloride. Preferable examples include 2-chloro-5,5-dimethyl-1,3,2-dioxaphosphorinan 2-oxide, dimethylchlorophosphate, diethylchlorophosphate, 2-chloro-2-oxo-1,3,2-dioxaphospholane, or propylphosphonic anhydride. More preferable examples include 2-chloro-5,5-dimethyl-1,3,2-dioxaphosphorinan 2-oxide, 2-chloro-2-oxo-1,3,2-dioxaphospholane, or propylphosphonic anhydride. The amount of the condensing agent used in this step is not limited as long as the reaction proceeds, but is preferably from 0.5 to 10 equivalents and more preferably from 1 to 5 equivalents of the compound represented by formula (Rc-V) or (Sc-V).

The temperature of the reaction with a condensing agent in this step is not limited as long as the reaction proceeds, but is preferably from -78°C to the boiling point of the solvent used in the reaction, and more preferably from -20°C to 0°C. The reaction time in this step is not limited as long as the reaction proceeds, but is preferably from 2 minutes to 10 hours, and more preferably from 5 minutes to 2 hours.

The sulfurizing agent used in this step is not particularly limited as long as the reaction proceeds. Examples include xanthane hydride, bis(phenylacetyl)disulfide, 3H-1,2-benzodithiol-3-one-1,1-dioxide, 5-phenyl-3H-1,2,4-dithiazol-3-one, or [(N,N-dimethylaminomethylidene)amino]-3H-1,2,4-dithiazolin-3-thione. Preferably used is xanthane hydride, bis(phenylacetyl)disulfide, or [(N,N-dimethylaminomethylidene)amino]-3H-1,2,4-dithiazolin-3-thione. The amount of the sulfurizing agent used in this step is not limited as long as the reaction proceeds, but is preferably from 0.5 to 5 equivalents and more preferably from 1 to 2 equivalents of the compound represented by formula (Rc-V) or (Sc-V).

The temperature of the reaction with a sulfurizing agent in this step is not limited as long as the reaction proceeds, but is preferably from -40°C to the boiling point of the solvent used in the reaction, and more preferably from -20°C to 30°C. The reaction time in this step is not limited as long as the reaction proceeds, but is preferably from 2 minutes to 10 hours, and more preferably from 5 minutes to 3 hours.

The oxidizing agent used in this step is not particularly limited as long as the reaction proceeds. Examples of the oxidizing agent that can be used include iodine, bromine, tert-butyl hydroperoxide, 3-chloroperbenzoic acid, hydrogen peroxide, periodic acid, potassium permanganate, oxygen, or ozone. Preferred is iodine, 3-chloroperbenzoic acid, hydrogen peroxide, or oxygen. The amount of the oxidizing agent used in this step is not limited as long as the reaction proceeds, but is preferably from 0.5 to 10 equivalents and more preferably from 1 to 5 equivalents of the compound represented by formula (Rc-V) or (Sc-V).

The temperature of the reaction with an oxidizing agent in this step is not limited as long as the reaction proceeds, but is preferably from -40°C to the boiling point of the solvent used in the reaction, and more preferably from -20°C to 30°C. The reaction time in this step is not limited as long as the reaction proceeds, but is preferably from 2 minutes to 10 hours, and more preferably from 5 minutes to 3 hours.

The solvent that can be used in this step is not particularly limited as long as the reaction is not inhibited. Examples that can be used include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferable examples include pyridine, 2-methylpyridine, 3-methylpyridine, 4-methylpyridine, 2,6-lutidine, acetonitrile, dichloromethane, or a mixed solvent thereof. More preferred is pyridine.

The absolute stereo configuration of the compound of formula (VI) is indicated by the absolute stereo configuration of the chiral carbon atom on the prolinol skeleton in B2. For a compound of formula (VI) where Q is a thiol group, the absolute stereo configuration on the two phosphorus atoms is described in the following order (absolute stereo configuration of phosphorus at 2-position, absolute stereo configuration of phosphorus at 10-position), as shown below. In conjunction with the previous stereo position on the chiral carbon atom on the prolinol skeleton, they are denoted as (Rc,Rp,Rp), (Rc,Sp,Rp), (Rc,Rp,Sp), (Rc,Sp,Sp), (Sc,Rp,Rp), (Sc,Sp,Rp), (Sc,Rp,Sp), and (Sc,Sp,Sp).

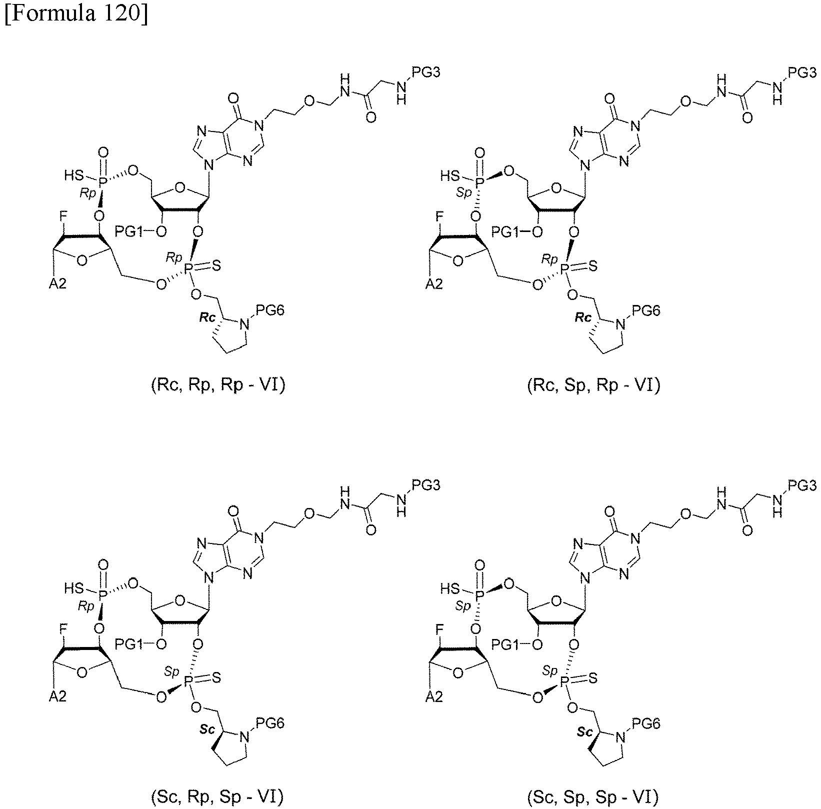

The cyclization reaction in this step is not controlled stereoselectively. However, based on the three-dimensional structure of the molecule itself, the formation of one diastereomer is predominant over the other. For example, as shown in Examples 3, 8 and A5-4 herein, in the cyclization reaction of a compound of formula (Rc-V) to a compound of formula (Rc-VI), the (Rc,Rp,Rp-VI) with the desired absolute stereo configuration was obtained with a selectivity of about 70 : 30 to 85 : 15 between the (Rc,Rp,Rp-VI) compound and the (Rc,Sp,Rp-VI) compound.

### (Step a4)

In one embodiment, this step is a step of removing B2, a protecting group for a thiophosphoric acid moiety in the compound of (Rc-VI), and PG4, a protecting group in A2 by deprotection, to obtain a compound of formula (Rp-VII). Another embodiment involves a step of obtaining a compound of formula (Sp-VII) from a compound of formula (Sc-VI) by using substantially the same reaction. This step optionally includes treatment with an organic or inorganic base to convert the compound to a salt thereof.

Ammonia used for the deprotection reaction in this step is not limited as long as the reaction proceeds. Preferably, 28% ammonia water may be used.

The reaction temperature in this step is not limited as long as the reaction proceeds, but is preferably from 0°C to the boiling point of the solvent used in the reaction, and more preferably from 30°C to 65°C. The reaction time in this step is not limited as long as the reaction proceeds, but is preferably from 1 to 96 hours, and more preferably from 5 to 48 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, pyridine, or dimethyl sulfoxide, or a mixed solvent thereof. Preferred is methanol, ethanol, acetonitrile, dichloromethane, or pyridine. More preferred is methanol or pyridine.

Examples of the organic base used in the conversion of the compound of formula (Rp-VII) or (Sp-VII) to a salt thereof include triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, or 1,8-diazabicyclo[5.4.0]undec-7-ene. In addition, examples of the inorganic base include potassium carbonate, potassium hydroxide, potassium bicarbonate, sodium carbonate, sodium hydroxide, sodium bicarbonate, sodium acetate, potassium acetate, sodium methoxide, sodium ethoxide, or potassium tert-butoxide. Preferable examples include triethylamine, tributylamine, diisopropylethylamine, N-methylmorpholine, N-methylpyrrolidine, N-methylpiperidine, potassium carbonate, potassium bicarbonate, sodium carbonate, or sodium bicarbonate. More preferable examples include triethylamine, potassium carbonate, potassium bicarbonate, sodium carbonate, or sodium bicarbonate.

### (Novel Intermediate)

A novel intermediate in the above [Synthesis Scheme <A1-Rp>] is a compound represented by the following formula (Rc-III): 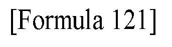 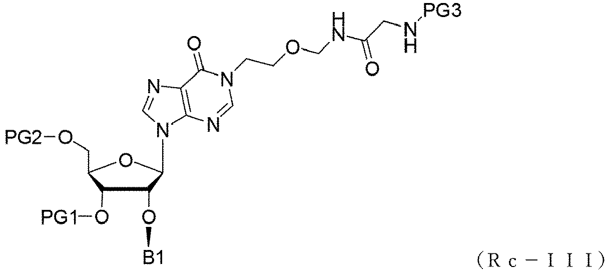 wherein preferably, B1 is 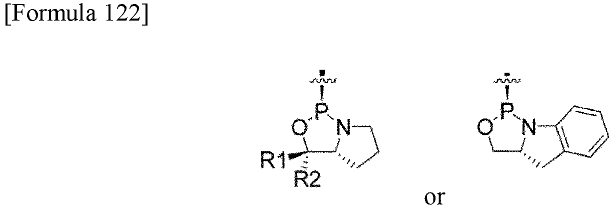 wherein R1 is hydrogen or methyl, R2 is hydrogen, C₁₋₃ alkyl or phenyl, wherein the alkyl is unsubstituted or substituted with one or more phenyl, tosyl or diphenylmethylsilyl groups, and the phenyl is preferably a group unsubstituted or substituted with nitro or methoxy,
wherein more preferably, B1 is 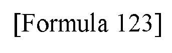 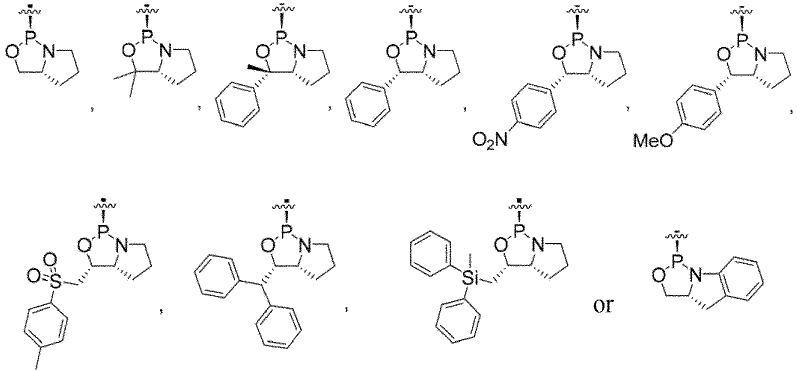 wherein still more preferably, B1 is 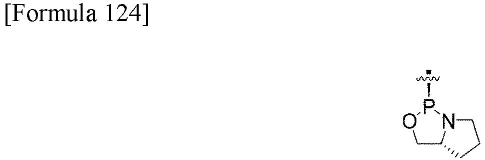
PG1 is preferably tert-butyldimethylsilyl, trimethylsilyl, triethylsilyl, triisopropylsilyl, or tert-butyldimethylsilyl, and more preferably *tert-*butyldimethylsilyl,
PG2 is preferably 4,4'-dimethoxytrityl, 4-methoxytrityl, 2-chlorotrityl, or trityl, and more preferably 4,4'-dimethoxytrityl.
PG3 is preferably 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl, and more preferably 2-(trimethylsilyl)ethoxycarbonyl.

In addition, examples of a novel intermediate in the above [Synthesis Scheme <A1-Rp>] include a compound represented by the following formula (Rc-V₀): 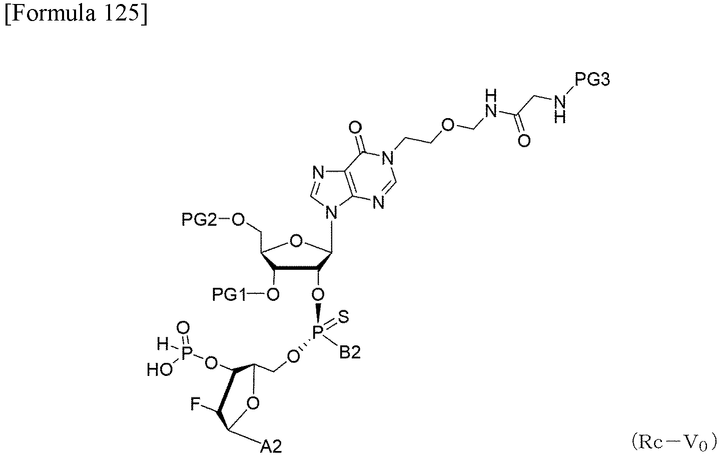 wherein A2 is preferably 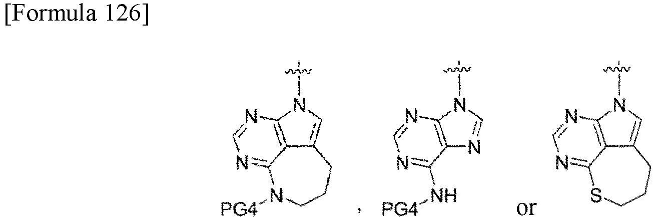 and more preferably 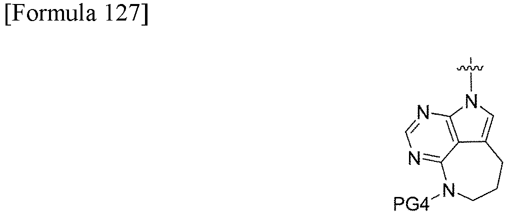
PG4 is a protecting group for an amino group, preferably benzyl, benzoyl, tert-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, or ethoxycarbonyl. More preferable is benzoyl or 2-(trimethylsilyl)ethoxycarbonyl.
B 1, PG1, PG2 and PG3 have the same meanings as the respective groups of the compound of formula (Rc-III) shown above.

Further, examples of a novel intermediate in the above [Synthesis Scheme <A1-Rp>] include a compound represented by the following formula (Rc-V): 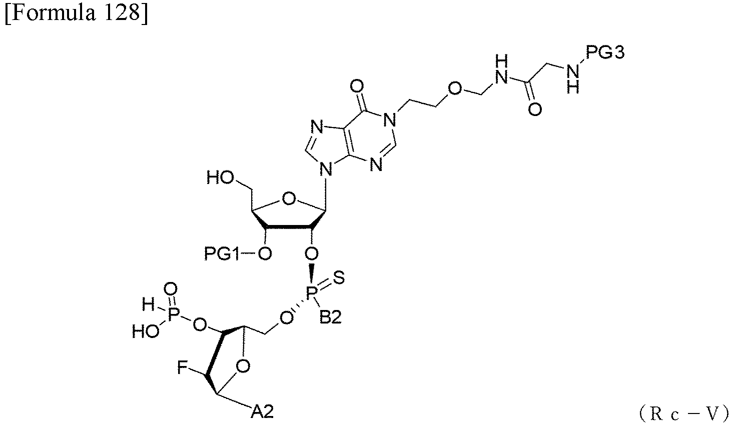
wherein A2 has the same meaning as A2 of the compound of formula (Rc-V₀) shown above,
wherein preferably, B2 is 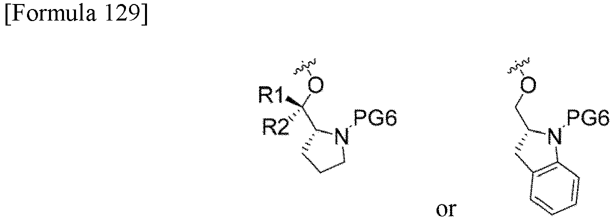 wherein R1 is hydrogen or methyl, R2 is hydrogen, C₁₋₃ alkyl or phenyl, wherein the alkyl is unsubstituted or substituted with one or more phenyl, tosyl or diphenylmethylsilyl, and the phenyl is preferably a group unsubstituted or substituted with nitro or methoxy,
wherein more preferably, B2 is 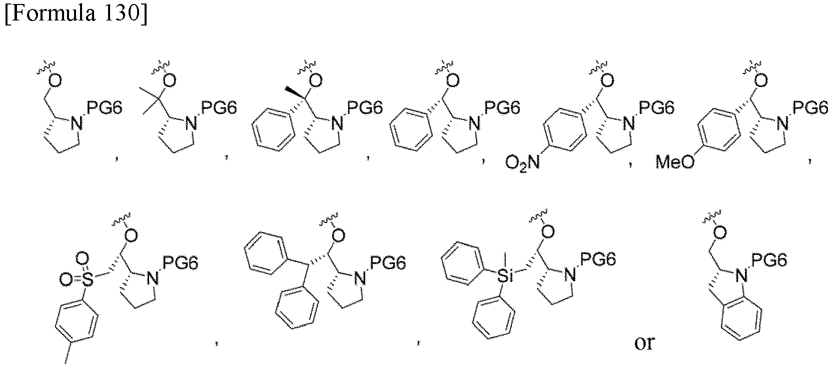
wherein still more preferably, B2 is 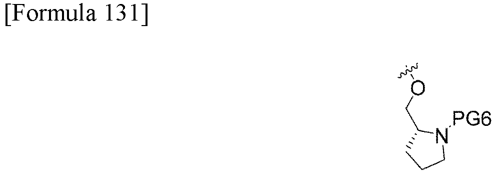
PG6 is a protecting group for an amino group, preferably acetyl, trifluoroacetyl, benzoyl, tert-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, methoxycarbonyl, or ethoxycarbonyl, and more preferably acetyl or trifluoroacetyl.
PG1 is preferably tert-butyldimethylsilyl, trimethylsilyl, triethylsilyl, triisopropylsilyl, or tert-butyldiphenylsilyl, and more preferably *tert-*butyldimethylsilyl.
PG3 is preferably 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl, and more preferably 2-(trimethylsilyl)ethoxycarbonyl.

In addition, this step a4 includes subjecting the compound of formula (Rp-VII) where Q is a thiol group to silica gel column chromatography prior to conversion to a salt thereof and/or crystallization after conversion to a salt thereof to obtain a compound of formula (Rp,Rp-VII'): 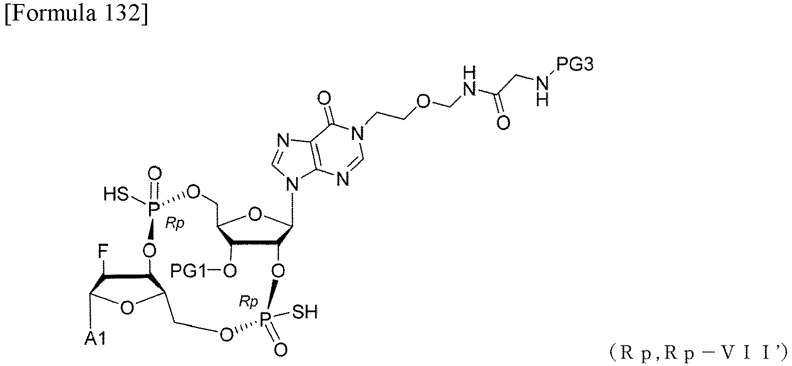 or a salt thereof.

The compound represented by formula (Rp-VII) or (Sp-VII) obtained by the production method of the present invention, in particular, the compound represented by formula (Rp,Rp-VII'), may also be preferably used for a CDN-linker represented by formula (Rp,Rp-XII) (hereinafter, sometimes referred to as a CDN conjugate precursor) and an antibody-immunostimulant conjugate in which the CDN conjugate precursor is further conjugated with an antibody. The compound of formula (Rp-VII) or (Sp-VII) obtained by the production method of the present invention is not limited thereto, and may also be used to produce an antibody-immunostimulant conjugate having another chemical structure or for other applications.

### <1-2. Comparison between method for preparing compound (Rp-VII) in the present invention and conventional methods (e.g., Patent Literature 6)>

In conventional methods, cyclic dinucleotides produced are a mixture of four different diastereomers in approximately the equal ratio. Accordingly, it is necessary to obtain a cyclic dinucleotide with the desired stereo configuration by column purification. The problem is that the yield is very low. The method for preparing a compound of formula (Rp-VII) according to the present invention makes it possible to selectively synthesize the desired stereo structure. As a result, it is possible to provide a production method with better effects, such as improved total yield and avoidance of column purification, than those of the synthesis scheme of Patent Literature 6 shown below.

### [Synthesis scheme of conventional method (from synthesis scheme of Example 77 in Patent Literature 6)]

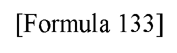

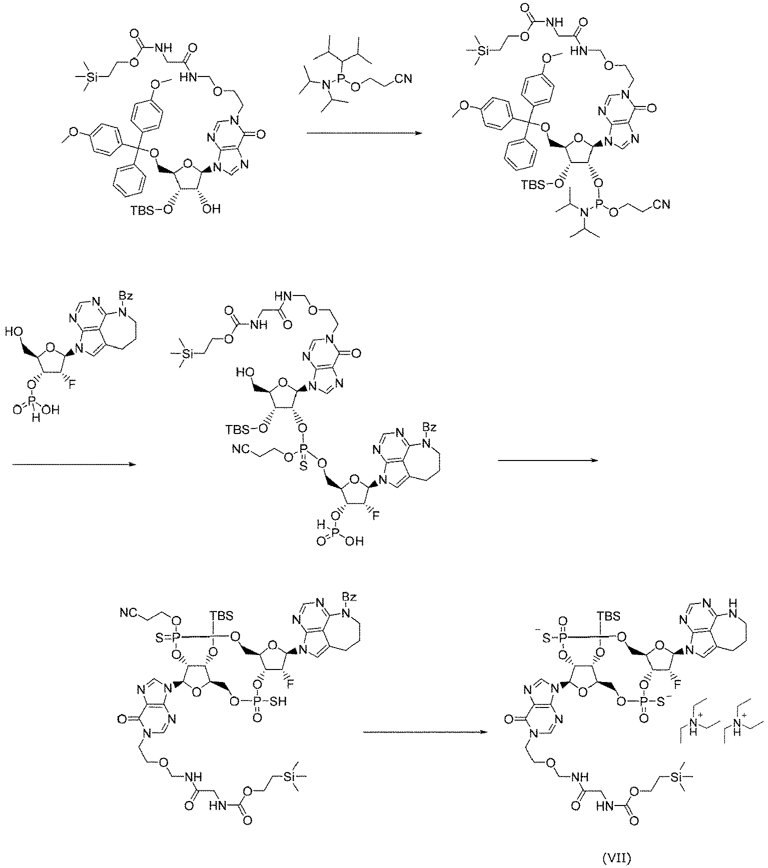

### [Effect of method for preparing formula (Rp-VII) according to the present invention]

In the conventional synthetic route (Patent Literature 6), the stereo structure on the phosphorus is not controlled by the reaction, but a mixture of four different diastereomers are synthesized and column purification is used to finally obtain the desired stereo structure. This method has an extremely low yield (total yield of 7.5% (containing impurities as described) after the 8 reaction steps).

In contrast, in the production method of the present invention, the phosphorothioate bonding in the first step can be performed stereoselectively. As a result, the total yield from the compound of formula (I) has been improved by about 20 to 40%, and the quality is also high, ranging from 92.4 to >99% by HPLC analysis.

Note that in the above synthesis scheme <A1-Rp> or <A1-Sp> (step a1), the compound of formula (I) used is the compound represented by the following formula (I-1) or formula (I-2): 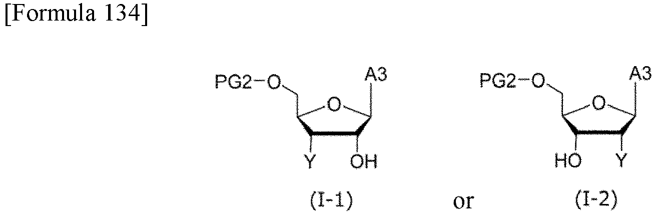 wherein
A3 is a substituted or unsubstituted deazapurine group, a substituted or unsubstituted purine base, or a substituted or unsubstituted pyrimidine base, and
Y is each independently H, F, or -O-PG1, where PG1 is a protecting group for a hydroxy group; and
in (step a2), a compound represented by formula (IV): formula (IV-1) or formula (IV-2): 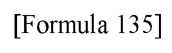 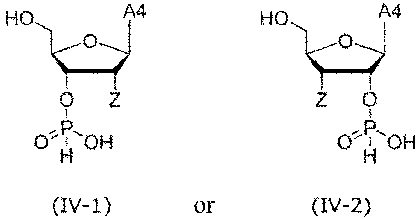 or a salt thereof, wherein
A4 is a substituted or unsubstituted deazapurine group, a substituted or unsubstituted purine base, or a substituted or unsubstituted pyrimidine base, and
Z is each independently H, F, or -O-PG1, where PG1 is a protecting group for a hydroxy group,
may be used to synthesize, according to the combination, four types of cyclic dinucleotide derivatives having the following 12-to-14-membered ring structure as corresponding to formula (Rp-VII) or four types of cyclic dinucleotide (not shown) having the 12-to-14-membered ring structure as corresponding to formula (Sp-VII):

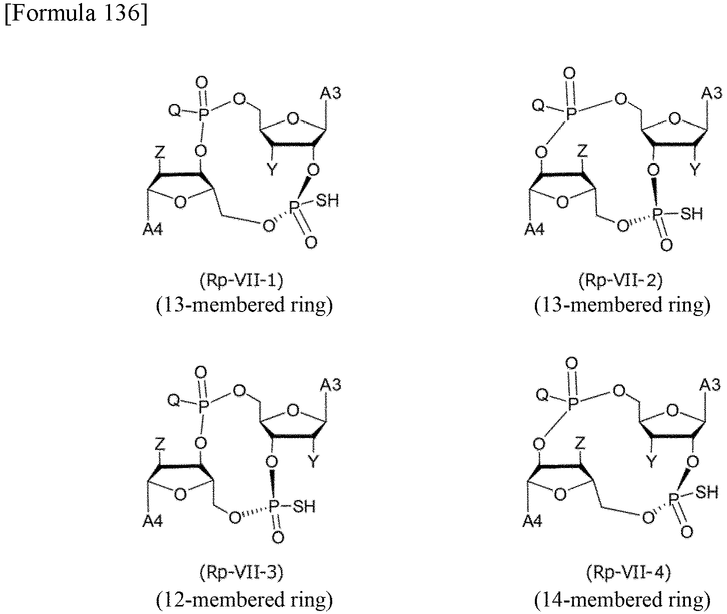

### <1-3. Method for preparing compound of formula (IV)>

The compound of formula (IV) or a salt thereof used for preparing the above cyclic dinucleotide may be produced according to the following synthesis scheme.

### [Synthesis Scheme <A2>]

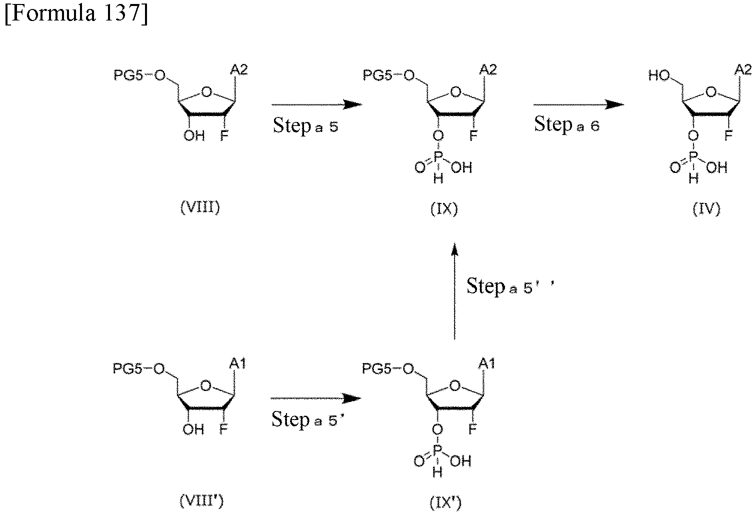

In the above [Synthesis Scheme <A2>],
A1 and A2 have the same meanings as in [Synthesis Scheme <A1>] above, and
PG5 is a protecting group for a hydroxy group.

In this production method, examples of PG5 include 4,4'-dimethoxytrityl, 4-methoxytrityl, 2-chlorotrityl, or trityl. Preferred is 4,4'-dimethoxytrityl or 4-methoxytrityl.

Hereinbelow, each step will be described.

### (Step a5) and (Step a5')

This step is a step of reacting a compound of formula (VIII) or formula (VIII') with a phosphite to obtain a compound of formula (IX) or formula (IX') or a salt thereof.

Examples of the phosphite used in this step include diphenyl phosphite, methyl phosphite, diethyl phosphite, or dibutyl phosphite. Preferred is diphenyl phosphite or methyl phosphite. More preferred is diphenyl phosphite. The amount of phosphite used in this step is not limited as long as the reaction proceeds, but is preferably from 0.5 to 20 equivalents and more preferably from 1 to 10 equivalents of the compound represented by formula (VIII) or (VIII').

The reaction temperature in this step is not limited as long as the reaction proceeds, but is preferably from -20°C to the boiling point of the solvent used in the reaction, and more preferably from -10°C to 30°C. The reaction time in this step is not limited as long as the reaction proceeds, but is preferably from 2 minutes to 120 hours, and more preferably from 5 minutes to 72 hours.

The solvent that can be used in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is pyridine, 2-methylpyridine, 3-methylpyridine, 4-methylpyridine, 2,6-lutidine, acetonitrile, or dichloromethane. More preferred is pyridine or acetonitrile.

Examples of the reagent used in the conversion of the compound of formula (IX) or (IX') to a salt thereof include triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene, propylamine, iso-propylamine, butylamine, isobutylamine, tert-butylamine, pentylamine, aniline, potassium carbonate, potassium hydroxide, potassium bicarbonate, sodium carbonate, sodium hydroxide, sodium bicarbonate, sodium acetate, potassium acetate, sodium methoxide, sodium ethoxide, potassium tert-butoxide, sodium chloride, or potassium chloride. Preferable examples include triethylamine, tributylamine, iso-propylamine, butylamine, isobutylamine, tert-butylamine, potassium carbonate, potassium bicarbonate, sodium carbonate, sodium bicarbonate, sodium chloride, or potassium chloride. More preferable examples include triethylamine, tert-butylamine, potassium carbonate, potassium bicarbonate, sodium carbonate, sodium bicarbonate, sodium chloride, or potassium chloride. The amount of the base used for the conversion to the salt is not limited as long as the reaction proceeds, but is preferably from 0.5 to 10 equivalents and more preferably from 3 to 8 equivalents of the compound represented by formula (2).

### (Step a5")

This step is a step of reacting the compound of formula (IX') or a salt thereof with an acylating or alkoxycarbonylating agent to obtain a compound of formula (IX) or a salt thereof.

The acylating or alkoxycarbonylating agent used in this step is not particularly limited as long as the reaction proceeds. Examples include acetic anhydride, acetyl chloride, N-succinimidyl acetate, pentafluorophenyl acetate, 1-acetyl-1H-1,2,3-triazolo[4,5-b]pyridine, N-methoxydiacetamide, N-acetylimidazole, trifluoroacetic anhydride, bistrifluoroacetamide, ethyl trifluoroacetate, methyl trifluoroacetate, pentafluorophenyl trifluoroacetate, trifluoroacetyl benzotriazole, S-ethyl trifluorothioacetate, N-methylbistrifluoroacetamide, trifluoroacetyltriflate, 1-trifluoroacetylimidazole, benzoic anhydride, benzoyl chloride, pentafluorophenylbenzoate, pentafluorophenylbenzoate, benzoyl trifluoromethanesulfonate, 3-benzoylthiazolidine-2-thione, *tert-*butylphenylcarbonate, N-(*tert*-butoxycarbonyloxy)phthalimide, 2-(*tert-*butoxycarbonylthio)-4,6-dimethylpyridine, N-*tert*-butoxycarbonyl imidazole, *tert-*butylcarbazate, 2-(*tert*-butoxycarbonyloxyimino)-2-phenylacetonitrile, 1-tert-butoxycarbonyl-1,2,4-triazole, N-*tert*-butoxycarbonyl imidazole, di-*tert-*butyldicarbonate, 9-fluorenylmethylpentafluorophenylcarbonate, 9-fluorenylmethyl chloroformate, 9-fluorenylmethylcarbazate, 19-fluorenylmethylcarbamate, 1-[(9H-fluoren-9-ylmethoxy)carbonyloxy]benzotriazole, N-[(9H-fluoren-9-ylmethoxy)carbonyloxy]succinimide, allyl chloroformate, diallyl dicarbonate, N-(allyloxycarbonyloxy)succinimide, allyl phenyl carbonate, N-(2,2,2-trichloroethoxycarbonyloxy)succinimide, 2,2,2-trichloroethyl chloroformate, benzyl chloroformate, benzyl carbazate, benzyl phenyl carbonate, N-carbobenzyloxysuccinimide, dibenzyl dicarbonate, 4-[2-(trimethylsilyl)ethoxycarbonyloxy]nitrobenzene, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, ethyl chloroformate, diethyldicarbonate, ethyl imidazole-1-carboxylate, 2-ethoxy-1-(ethoxycarbonyl)-1,2-dihydroquinoline, methyl chloroformate, dimethyl carbonate, dimethyldicarbonate, or methyl imidazole-1-carboxylate. Preferable examples include acetic anhydride, 1-trifluoroacetylimidazole, benzoic anhydride, N-[(9H-fluoren-9-ylmethoxy)carbonyloxy]succinimide, N-(allyloxycarbonyloxy)succinimide, 4-[2-(trimethylsilyl)ethoxycarbonyloxy]nitrobenzene, or N-[2-(trimethylsilyl)ethoxycarbonyloxy]succinimide. More preferable examples include 1-trifluoroacetylimidazole, 4-[2-(trimethylsilyl)ethoxycarbonyloxy]nitrobenzene, 2-(trimethylsilyl)ethyl-3-nitro-1H-1,2,4-triazole-1-carboxylate, or N-[2-(trimethylsilyl)ethoxycarbonyloxy]succinimide. The amount of the acylating or alkoxycarbonylating agent used in this step is not limited as long as the reaction proceeds, but is preferably from 1 to 15 equivalents and more preferably from 3 to 8 equivalents of the compound represented by formula (IX').

This step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium *tert*-butoxide). Preferable examples include triethylamine, tributylamine, diisopropylethylamine, N-methylmorpholine, N-methylpyrrolidine, or N-methylpiperidine. More preferable examples include triethylamine, diisopropylethylamine, or N-methylmorpholine. The amount of the base used in this step is not limited as long as the reaction proceeds, but is preferably from 1 to 20 equivalents and more preferably from 4 to 10 equivalents of the compound represented by formula (IX').

The temperature of the reaction with the acylating or alkoxycarbonylating agent in this step is not limited as long as the reaction proceeds, but is preferably from -20°C to the boiling point of the solvent used in the reaction, and more preferably from 40°C to 80°C. The reaction time in this step is not limited as long as the reaction proceeds, but is preferably from 1 hour to 96 hours, and more preferably from 24 hours to 72 hours.

The solvent that can be used in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is pyridine, 4-methylpyridine, 2-methyltetrahydrofuran, acetonitrile, or 1-methyl-2-pyrrolidone. More preferred is 2-methyltetrahydrofuran or acetonitrile.

The same reagents and amounts as in step a5 above may be used for the conversion of the resulting compound of formula (IX) to a salt thereof.

### (Step a6)

This step is a step of removing PG5, a protecting group for a 5' hydroxyl group in the compound of formula (IX) by deprotection, to obtain a compound of formula (IV).

Examples of the acid used in the deprotection reaction in this step include acetic acid, chloroacetic acid, dichloroacetic acid, trichloroacetic acid, trifluoroacetic acid, methanesulfonic acid, toluenesulfonic acid, or sulfuric acid. Preferred is acetic acid, chloroacetic acid, dichloroacetic acid, or trichloroacetic acid. More preferred is dichloroacetic acid. The amount of the acid used in this step is not limited as long as the reaction proceeds, but is preferably from 0.05 to 500 equivalents and more preferably from 0.1 to 200 equivalents of the compound represented by formula (IX).

The reaction temperature in this step is not limited as long as the reaction proceeds, but is preferably from -20°C to the boiling point of the solvent used in the reaction, and more suitably from -10°C to 40°C. The reaction time in this step is not limited as long as the reaction proceeds, but is suitably from 1 hour to 96 hours, and more suitably from 5 hours to 48 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is methanol, acetonitrile, or dichloromethane. More preferred is dichloromethane.

### <1-4. Method for preparing CDN-linker (compound (Rp,Rp-XII))>

The compound of formula (Rp,Rp-XII) or a salt thereof may be produced according to the following synthesis scheme. The compound of formula (Rp,Rp-XII) is a production precursor for an antibody-immunostimulant conjugate.

### [Synthesis Scheme <A3>]

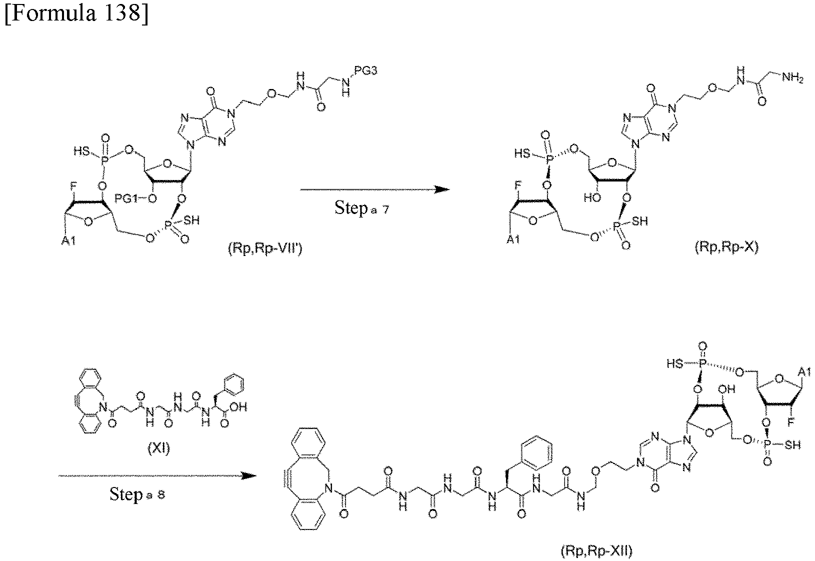

In the above [Synthesis Scheme <A3>],
PG1, PG3, and A1 have the same meanings as in [Synthesis Scheme <A1-Rp>] above.

Hereinbelow, each step will be described.

### (Step a7)

This step is a step of removing PG1 and PG3, protecting groups in the compound of formula (Rp,Rp-VII') by deprotection, to obtain a compound of formula (Rp,Rp-X).

Examples of the deprotectant used in this step include ammonium fluoride, tetra-n-butylammonium fluoride, pyridine hydrofluoride, or triethylamine trihydrofluoride. Preferred is ammonium fluoride or tetra-n-butylammonium fluoride. More preferred is tetra-n-butylammonium fluoride. The amount of the deprotectant used in this step is not limited as long as the reaction proceeds, but is preferably from 0.5 to 60 equivalents and more preferably from 1 to 30 equivalents of the compound represented by formula (Rp,Rp-VII').

The reaction temperature in this step is not limited as long as the reaction proceeds, but is preferably from 0°C to the boiling point of the solvent used in the reaction, and more preferably from 10°C to 40°C. The reaction time in this step is not limited as long as the reaction proceeds, but is preferably from 30 minutes to 240 hours, and more preferably from 1 hour to 120 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is methanol, acetonitrile, tetrahydrofuran, dichloromethane, or dimethyl sulfoxide. More preferred is tetrahydrofuran or dimethyl sulfoxide.

### (Step a8)

This step is a step of condensing the compound of formula (Rp,Rp-X) with a compound of formula (XI) to obtain a compound of formula (Rp,Rp-XII).

The compound represented by formula (XI) may preferably be condensed with the compound represented by formula (Rp,Rp-X) by derivation to an active ester. The amount of the compound that is represented by formula (XI) and used in this step is not limited as long as the reaction proceeds, but is preferably from 0.3 to 3 equivalents and more preferably from 0.7 to 1.3 equivalents of the compound represented by formula (Rp,Rp-X).

The derivation to the active ester in this step is not limited as long as the reaction proceeds. For example, a condensing agent (e.g., 1-ethyl-3-(3-dimethylaminopropyl)carbodiimide hydrochloride (WSCD-HCl) or N,N'-dicyclohexylcarbodiimide (DCC)) may be used; also, an additive (e.g., 1-hydroxybenzotriazole (HOBt), 1-hydroxy-7-azabenzotriazole (HOAt), N-hydroxysuccinimide, cyano(hydroxyimino)ethyl acetate, or p-nitrophenol) may be used for the derivation. Alternatively, a condensing agent (e.g., 1-[bis(dimethylamino)methylene]-1H-benzotriazolium 3-oxide hexafluorophosphate (HBTU), 1-[bis(dimethylamino)methylene]-1H-1,2,3-triazolo[4,5-b]pyridinium 3-oxide hexafluorophosphate (HATU), (1-cyano-2-ethoxy-2-oxoethylideneaminooxy)dimethylamino-morpholino-carbenium hexafluorophosphate (COMU), or 4-(4,6-dimethoxy-1,3,5-triazin-2-yl)-4-methylmorpholinium chloride (DMT-MM)) may be used. Preferably, 1-ethyl-3-(3-dimethylaminopropyl)carbodiimide hydrochloride and 1-hydroxybenzotriazole (HOBt), or 1-ethyl-3-(3-dimethylaminopropyl)carbodiimide hydrochloride and 1-hydroxy-7-azabenzotriazole, or 4-(4,6-dimethoxy-1,3,5-triazine-2-yl)-4-methylmorpholinium chloride (DMT-MM) may be used for the derivation. The amount of 1-ethyl-3-(3-dimethylaminopropyl)carbodiimide hydrochloride used in this step is not limited as long as the reaction proceeds, but is preferably from 0.5 to 5 equivalents and more preferably from 0.7 to 2 equivalents of the compound represented by formula (Rp,Rp-X). The amount of 1-hydroxybenzotriazole or 1-hydroxy-7-azabenzotriazole used in this step is not limited as long as the reaction proceeds, but is preferably from 0.05 to 4 equivalents and more preferably from 0.1 to 2 equivalents of the compound represented by formula (Rp,Rp-X). The amount of 4-(4,6-dimethoxy-1,3,5-triazine-2-yl)-4-methylmorpholinium chloride used in this step is not limited as long as the reaction proceeds, but is preferably from 0.3 to 5 equivalents and more preferably from 0.7 to 2 equivalents of the compound represented by formula (Rp,Rp-X).

This step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium *tert*-butoxide). Preferable examples include triethylamine, tributylamine, diisopropylethylamine, N-methylmorpholine, potassium carbonate, potassium hydroxide, potassium bicarbonate, sodium carbonate, sodium hydroxide, sodium bicarbonate, or sodium acetate. More preferable examples include triethylamine or N-methylmorpholine. The amount of the base used in this step is not limited as long as the reaction proceeds, but is preferably from 0.5 to 10 equivalents and more preferably from 1 to 5 equivalents of the compound represented by formula (Rp,Rp-X).

The reaction temperature in this step is not limited as long as the reaction proceeds, but is preferably from 0°C to the boiling point of the solvent used in the reaction, and more preferably from -10°C to 40°C. The reaction time in this step is not limited as long as the reaction proceeds, but is preferably from 5 minutes to 72 hours, and more preferably from 1 hour to 24 hours.

The solvent that can be used in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is water, tetrahydrofuran, N,N-dimethylformamide, acetonitrile, or dimethyl sulfoxide, or a mixed solvent thereof.

Examples of the base used in the conversion of formula (Rp,Rp-XII) to a salt thereof include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, propylamine, iso-propylamine, butylamine, iso-butylamine. *tert*-butylamine, pentylamine, aniline), or an inorganic base (e.g., potassium 2-ethylhexanoate, potassium carbonate, potassium hydroxide, potassium bicarbonate, sodium 2-ethylhexanoate, sodium carbonate, sodium hydroxide, sodium bicarbonate, sodium acetate, potassium acetate, sodium methoxide, sodium ethoxide, potassium *tert*-butoxide, sodium chloride, potassium chloride). Preferable examples include triethylamine, *tert*-butylamine, potassium 2-ethylhexanoate, potassium carbonate, potassium bicarbonate, sodium 2-ethylhexanoate, sodium carbonate, sodium bicarbonate, sodium chloride, or potassium chloride. More preferable examples include triethylamine, potassium 2-ethylhexanoate, or sodium carbonate.

Examples of the solvent used in the conversion of formula (Rp,Rp-XII) to a salt thereof include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferable examples include water, 2-propanol, acetonitrile, cyclopentyl methyl ether, or ethyl acetate, or a mixed solvent thereof.

### <2. Production of raw material compound (I) used for preparing cyclic dinucleotide>

### <2-1. Method for preparing compound of formula (I)>

The compound of formula (I) used for preparing the above cyclic dinucleotide may be produced according to the following synthesis scheme.

### [Synthesis Scheme <B1>]

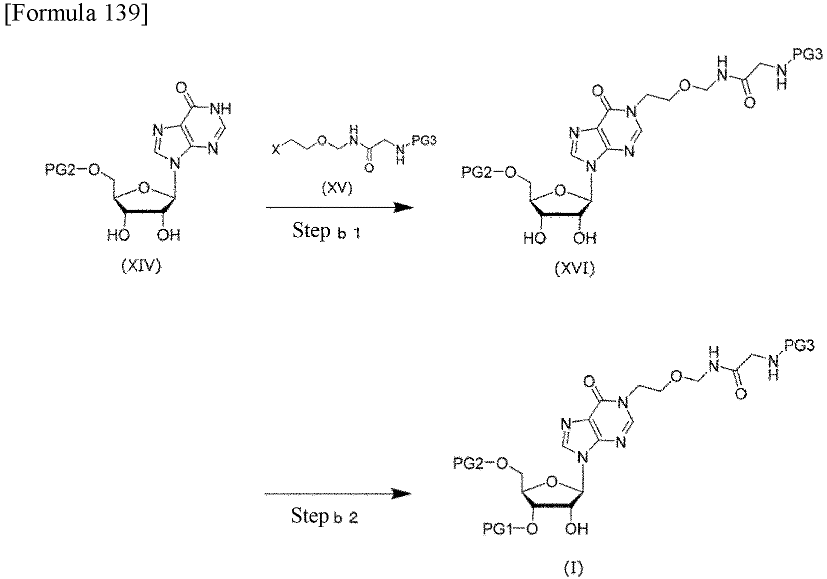

In the above [Synthesis Scheme <B1>],
PG1, PG2, and PG3 have the same meanings as in [Synthesis Scheme <A1-Rp>] above, and
X is Cl, Br, or I.

In this production method,
PG1 represents a protecting group for a hydroxy group, and examples of the protecting group include *tert*-butyldimethylsilyl, trimethylsilyl, triethylsilyl, triisopropylsilyl, or *tert*-butyldiphenylsilyl. Preferred is *tert*-butyldimethylsilyl or trimethylsilyl. More preferred is *tert*-butyldimethylsilyl.
PG2 represents a protecting group for a hydroxy group, and examples of the protecting group include 4,4'-dimethoxytrityl, 4-methoxytrityl, 2-chlorotrityl, or trityl. Preferred is 4,4'-dimethoxytrityl, 4-methoxytrityl, or trityl. More preferred is 4,4'-dimethoxytrityl.
PG3 represents a protecting group for an amino group, and examples of the protecting group include 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl. Preferred is 2-(trimethylsilyl)ethoxycarbonyl or allyloxycarbonyl. More preferred is 2-(trimethylsilyl)ethoxycarbonyl.

Hereinbelow, each step will be described.

### (Step b1)

This step is a step of reacting the compound of formula (XIV) with the compound of formula (XV) to obtain a compound of formula (XVI).

The reaction in this step is carried out in the presence of a base. The base used may be 1,1,3,3-tetramethylguanidine, triethylamine, diisopropylethylamine, or 1,8-diazabicyclo[5.4.0]-7-undecene. Preferably used is 1,1,3,3-tetramethylguanidine or 1,8-diazabicyclo[5.4.0]-7-undecene. More preferred is 1,1,3,3-tetramethylguanidine. The amount of the base used in this step is not limited as long as the reaction proceeds, but is preferably from 0.5 to 10 equivalents and more preferably from 1 to 3 equivalents of the compound represented by formula (XIV).

The reaction temperature in this step is not limited as long as the reaction proceeds, but is preferably from 10°C to the boiling point of the solvent used in the reaction, and more preferably from 20°C to 50°C. The reaction time in this step is not limited as long as the reaction proceeds, but is preferably from 30 minutes to 72 hours, and more preferably from 5 hours to 36 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is N,N-dimethylformamide, N,N-dimethylacetamide, 1-methyl-2-pyrrolidone, or 1,3-dimethyl-2-imidazolidinone. More preferred is 1,3-dimethyl-2-imidazolidinone.

### (Step b2)

This step is a step of reacting the compound of formula (XVI) with a silylating agent to obtain a mixture of the following compounds of formula (I') and formula (XVII): 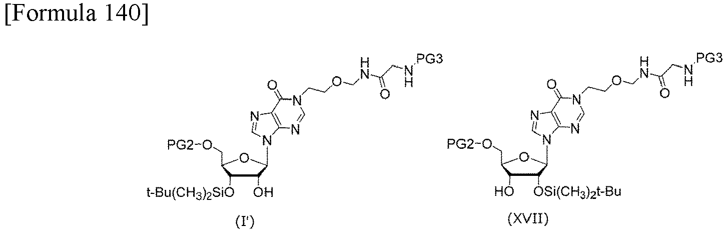 and then converting the compound of formula (XVII) in the mixture to the compound of formula (I') in the presence of a base to obtain the compound of formula (I').

Here, in this step, the reaction of the compound of formula (XVI) with the silylating agent is carried out in the presence of a first base to obtain a mixture of the compound of formula (I') and the compound of formula (XVII), and then the compound of formula (XVII) in the mixture is converted in the presence of a second base to the compound of formula (I') to obtain the compound of formula (I').

The compound of formula (XVII) is converted to the compound of formula (I') by taking advantage of the equilibrium reaction in the mixture solution and the difference in solubility between the compound of formula (XVII) and the compound of formula (I'). The conversion is thus carried out by crystallization of the compound of formula (I') from the mixture solution.

Here, examples of the first base may include 1,1,3,3-tetramethylguanidine, triethylamine, diisopropylethylamine, 2,6-lutidine, and 1,8-diazabicyclo[5.4.0]-7-undecene. Preferred is 1,1,3,3-tetramethylguanidine, 2,6-lutidine, or 1,8-diazabicyclo[5.4.0]-7-undecene. More preferred is 1,1,3,3-tetramethylguanidine. The amount of the first base used in this step is not limited as long as the reaction proceeds, but is preferably from 0.5 to 10 equivalents and more preferably from 1 to 5 equivalents of the compound represented by formula (XVI).

The reaction temperature of the reaction using the first base in this step is not limited as long as the reaction proceeds, but is preferably from 10°C to the boiling point of the solvent used in the reaction, and more preferably from 40°C to 70°C. The reaction time in this step is not limited as long as the reaction proceeds, but is preferably from 30 minutes to 72 hours, and more preferably from 5 hours to 36 hours.

The solvent that can be used for the reaction using the first base in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is N,N-dimethylformamide, N,N-dimethylacetamide, 1-methyl-2-pyrrolidone, or 1,3-dimethyl-2-imidazolidinone. More preferred is 1,3-dimethyl-2-imidazolidinone.

Examples of the silylating agent in this step include *tert-*butyldimethylchlorosilane or *tert*-butyldimethylsilyl triflate. Preferred is *tert-*butyldimethylchlorosilane. The amount of the silylating agent used in this step is not limited as long as the reaction proceeds, but is preferably from 0.5 to 10 equivalents and more preferably from 2 to 4 equivalents of the compound represented by formula (XIV).

Here, examples of the second base may include 1,1,3,3-tetramethylguanidine, triethylamine, diisopropylethylamine, 2,6-lutidine, and 1,8-diazabicyclo[5.4.0]-7-undecene. Preferred is 1,1,3,3-tetramethylguanidine, 2,6-lutidine, or 1,8-diazabicyclo[5.4.0]-7-undecene. More preferred is 1,1,3,3-tetramethylguanidine. The amount of the second base used in this step is not limited as long as the reaction proceeds, but is preferably from 0.01 to 3 equivalents and more preferably from 0.05 to 1 equivalent of the compound represented by formula (XIV).

The reaction temperature of the reaction using the second base in this step is not limited as long as the reaction proceeds, but is preferably from 10°C to the boiling point of the solvent used in the reaction, and more preferably from 10°C to 40°C. The reaction time in this step is not limited as long as the reaction proceeds, but is preferably from 2 minutes to 10 hours, and more preferably from 5 minutes to 90 minutes.

The first solvent that can be used for the reaction using the second base in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is tetrahydrofuran, N,N-dimethylformamide, N,N-dimethylacetamide, 1-methyl-2-pyrrolidone, or 1,3-dimethyl-2-imidazolidinone. More preferred is tetrahydrofuran.

The second solvent that can be used for the reaction using the second base in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is tetrahydrofuran, heptane, N,N-dimethylformamide, N,N-dimethylacetamide, 1-methyl-2-pyrrolidone, or 1,3-dimethyl-2-imidazolidinone. More preferred is heptane.

### <2-2. Method for preparing compound of formula (XV) and intermediate therefor>

The compound of formula (XV) used for preparing the above formula (I) may be produced according to the following synthesis scheme.

### [Synthesis Scheme <B2>]

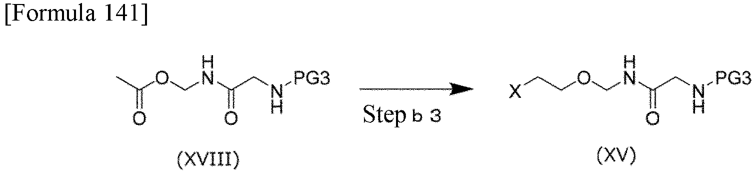

In the above [Synthesis Scheme <B2>],
PG3 has the same meaning as in [Synthesis Scheme <A1>] above, and
X has the same meaning as in [Synthesis Scheme <B1>] above.

Hereinbelow, step b3 will be described.

### (Step b3)

This step is a step of reacting the compound of formula (XVIII) with 2-haloethanol in the presence of an acid or a base to obtain a compound of formula (XV).

Examples of the base in this step include sodium hydroxide, potassium hydroxide, lithium hydroxide, triethylamine, diisopropylethylamine, or 1,8-diazabicyclo[5.4.0]-7-undecene. Preferred is sodium hydroxide, potassium hydroxide, triethylamine, or diisopropylethylamine. More preferred is sodium hydroxide. The amount of the base used in this step is not limited as long as the reaction proceeds, but is preferably from 0.5 to 2 equivalents of glycylglycine, namely a starting material for the compound of (XVIII). The compound (XVIII) can be obtained from glycylglycine by protection of the amino group and conversion of the carboxy group to an acetyloxy group.

Examples of 2-haloethanol used in this step include 2-chloroethanol, 2-bromoethanol, or 3-iodoethanol. Preferred is 2-bromoethanol or 2-iodoethanol. More preferred is 2-bromoethanol. The amount of 2-haloethanol used in this step is not limited as long as the reaction proceeds, but is preferably from 0.5 to 10 equivalents and more preferably from 1 to 4 equivalents of glycylglycine, namely a starting material for the compound of (XVIII).

The reaction temperature in this step is not limited as long as the reaction proceeds, but is preferably from -20°C to the boiling point of the solvent used in the reaction, and more preferably from -10°C to 20°C. The reaction time in this step is not limited as long as the reaction proceeds, but is preferably from 5 minutes to 36 hours, and more preferably from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is 1,2-dimethoxyethane, 2-methyltetrahydrofuran, or 1,4-dioxane. More preferred is 1,2-dimethoxyethane.

### (Novel Intermediate)

Examples of a novel intermediate in the above [Synthesis Scheme <B1>] include a compound represented by the following formula (XV): 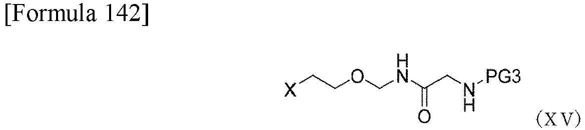
wherein PG3 is 2-(trimethylsilyl)ethoxycarbonyl, and
X is preferably Cl, Br, or I, and more preferably Br.

### <2-3. Comparison between method for preparing compound (I) in the present invention and conventional methods (e.g., Patent Literature 6)>

The method for preparing the compound of formula (I) according to the present invention enables synthesis of the product in which the 3' hydroxyl group among the hydroxyl groups at 2'- and 3'-positions is selectively *tert-*butyldimethylsilylated, which could not be achieved by the conventional method. As a result, it is possible to provide a production method with better effects, such as improved total yield, a reduced number of steps, and avoidance of column purification, than those of the synthesis scheme of Patent Literature 6 shown below.

### [Synthesis scheme of conventional method (from synthesis scheme of Example 77 in Patent Literature 6)]

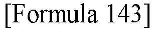

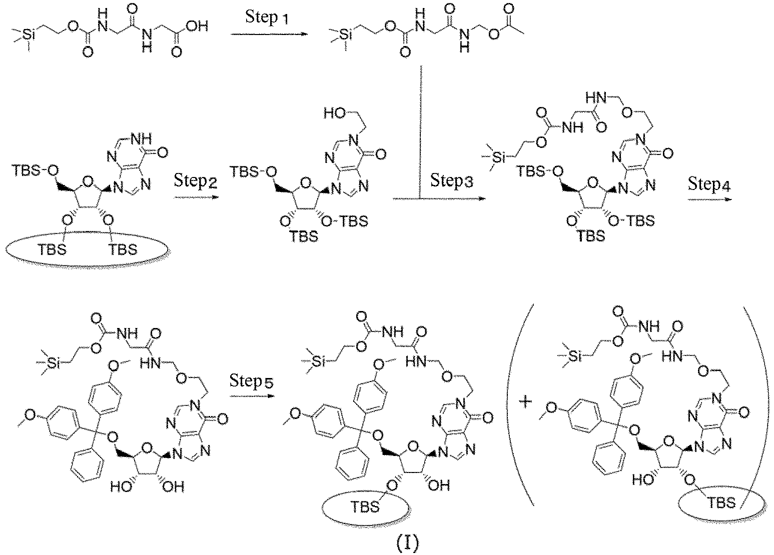

### [Effect of method for preparing formula (I) according to the present invention]

### (Effect 1) Synthesis of product (I) in which the hydroxyl group at 3'-position among hydroxyl groups at 2'- and 3'-positions is selectively tert-butyldimethylsilylated

In the conventional synthetic route (Patent Literature 6), in the *tert-*butyldimethylsilylation step (step 5), the 3'-position among the hydroxyl groups at 2'-and 3'-positions cannot be selectively *tert-*butyldimethylsilylated. The 2'-position is *tert*-butyldimethylsilylated to obtain a byproduct. Consequently, the yield of the target compound of formula (I) was as low as about 35%. In addition, as a reaction of selective silylation of hydroxyl groups at the 2'- and 3'-positions, a method for selective triethylsilylation at the 3'-position by using a chiral imidazole is known (see Org. Lett., Vol. 15, No. 18, 2013, pp. 4710-4713). However, the chiral imidazole is special and not readily available. Further, selective *tert*-butyldimethylsilylation using a silver reagent is also known (see Tetrahedron Lett., Vol. 22, No. 52, 1981, pp. 5423-5246). However, an expensive silver reagent has to be used in an amount equivalent or more to the amount of the reaction substrate. This is not favorable. Although the same conditions were also applied with this substrate, the desired selectivity was unable to be obtained.

Meanwhile, in the production method of the present invention, the condition has been found that the TBS group transfers between 2'- and 3'-positions after nonselective introduction of the TBS group on the hydroxyl group at 2'- or 3'-position. The condition has also been found to crystalize only a compound in which the target hydroxyl group at 3'-position represented by formula (I) is protected by TBS group. This made it possible to predominantly obtain the target compound of formula (I).

### (Effect 2) Reduced number of steps, improved yield, avoidance of column purification

In the conventional synthetic route (Patent Literature 6), an extra deprotection step was required during the N-alkylation of the amide group of the nucleic acid base because the hydroxyl groups at 2'- and 3'-positions were protected on the reaction substrate.

On the other hand, in the production method of the present invention, an alkylating agent (compound of formula (XV)) has been found that allows N-alkylation of the amide group of the nucleic acid base to proceed while leaving the hydroxyl groups at 2'- and 3'-positions unprotected. This eliminates the need for protection and deprotection for the hydroxyl groups at 2'- and 3'-positions, thereby capable of providing a production method with two shorter steps.

### (Effect 3) Improved yield and avoidance of column purification in view of overall scheme

In the conventional synthetic route (Patent Literature 6), the total yield from the starting material 2',3',5'-tris-O-[*tert*-butyl(dimethyl)silyl]inosine to the compound of formula (I) was about 13% (5 steps). However, in the production method of the present invention, the compound of formula (I) can be synthesized from the starting material inosine in a yield of about 60% (3 steps). In addition, in the conventional synthetic route (Patent Literature 6), the product was obtained by silica gel column chromatography purification, which increases the amount of waste (e.g., silica gel, an eluent (e.g., hexane, ethyl acetate)) and prolongs the lead time throughout all the steps. In contrast, in the production method of the present invention, silica gel column chromatography is avoided, and it is thus possible to reduce the environmental burden and improve productivity.

### <3. Production of raw material compounds (VIII) and formula (VIII') used for preparing cyclic dinucleotide>

### <3-1. Method for preparing compounds of formula (VIII) and formula (VIII') and intermediate therefor>

The compounds of formula (VIII) and formula (VIII') used for preparing the above cyclic dinucleotide may be produced according to the following synthesis scheme.

### [Synthesis Scheme <C1>]

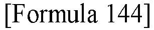

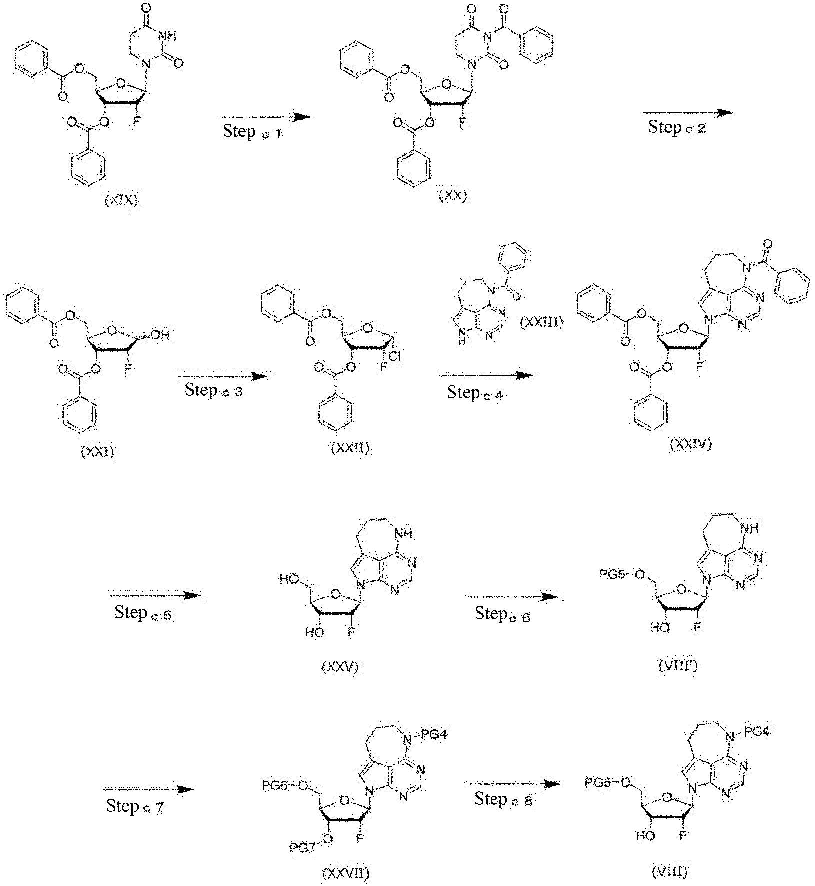

In the above [Synthesis Scheme <C1>],
PG4 is benzoyl, 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl,
PG5 is 4,4'-dimethoxytrityl, 4-methoxytrityl, 2-chlorotrityl, or trityl, and
PG7 is trimethylsilyl, triethylsilyl, triisopropylsilyl, *tert*-butyldimethylsilyl, *tert*-butyldiphenylsilyl, or triphenylsilyl.

Hereinbelow, each step will be described.

### (Step c1)

This step is a step of reacting the compound of formula (XIX) with a benzoylating agent to obtain a compound of formula (XX).

Examples of the benzoylating agent used in this step include benzoyl chloride, benzoyl bromide, benzoic anhydride, or benzoyl trifluoromethanesulfonate. Preferred is benzoyl chloride or benzoyl bromide. More preferred is benzoyl chloride. The amount of the benzoylating agent used in this step is not limited as long as the reaction proceeds, but is preferably from 0.5 to 5 equivalents and more preferably from 1 to 2 equivalents of the compound represented by formula (XIX).

This step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium tert-butoxide), or a mixture thereof. Preferable examples include triethylamine, 2,6-methylpyridine, 4-dimethylaminopyridine, potassium carbonate, potassium hydroxide, potassium bicarbonate, sodium carbonate, sodium hydroxide, sodium bicarbonate, or sodium acetate, or a mixture thereof. More preferable examples include a mixture of triethylamine and 4-dimethylaminopyridine. The amount of the base used in this step is not limited as long as the reaction proceeds, but is preferably from 0.1 to 10 equivalents and more preferably from 0.2 to 5 equivalents of the compound represented by formula (XIX).

The reaction temperature in this step is not limited as long as the reaction proceeds, but is preferably from -40°C to the boiling point of the solvent used in the reaction, and more preferably from -10°C to 35°C. The reaction time in this step is not limited as long as the reaction proceeds, but is preferably from 5 minutes to 24 hours, and more preferably from 0.5 hours to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is acetonitrile or N,N-dimethylformamide. More preferred is acetonitrile.

### (Step c2)

This step is a step of hydrolyzing the compound represented by formula (XX) to obtain a compound of formula (XXI).

Examples of the acid used in this step include hydrochloric acid, acetic acid, chloroacetic acid, dichloroacetic acid, trichloroacetic acid, trifluoroacetic acid, methanesulfonic acid, p-toluenesulfonic acid, or sulfuric acid. Preferred is hydrochloric acid, methanesulfonic acid, p-toluenesulfonic acid, or sulfuric acid. More preferred is p-toluenesulfonic acid. The amount of the acid used in this step is not limited as long as the reaction proceeds, but is preferably from 0.5 to 20 equivalents and more preferably from 1 to 10 equivalents of the compound represented by formula (XX).

The reaction temperature in this step is not limited as long as the reaction proceeds, but is preferably from 0°C to the boiling point of the solvent used in the reaction, and more preferably from 40°C to 80°C. The reaction time in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is 1,2-dimethoxyethane, 2-methyltetrahydrofuran, tetrahydrofuran, cyclopentyl methyl ether, or a mixed solvent thereof. More preferred is a mixture solvent of cyclopentyl methyl ether and water.

### (Step c3)

This step is a step of reacting the compound of formula (XXI) with a chlorinating agent to obtain a compound of formula (XXII).

Examples of the chlorinating agent in this step include trichloroisocyanuric acid, chloroisocyanuric acid, dichloroisocyanuric acid, N-chlorosuccinimide, 1,3-dichloro-5,5-dimethylhydantoin, N-chlorosaccharin, N-N-dichloro-p-toluenesulfonamide, N-N-dichlorobenzenesulfonamide, or carbon tetrachloride. Preferred is trichloroisocyanuric acid, chloroisocyanuric acid, or dichloroisocyanuric acid. More preferred is trichloroisocyanuric acid. The amount of the chlorinating agent used in this step is not limited as long as the reaction proceeds, but is preferably from 0.1 to 6 equivalents and more preferably from 0.3 to 3 equivalents of the compound represented by formula (XXI).

In addition, the reaction in this step can preferably be performed in the presence of a phosphorus reagent. The phosphorus reagent used in this step is not particularly limited as long as the reaction proceeds. Examples include tris(2,4-di-*tert*-butylphenyl)phosphite, tri-o-tolyl phosphite, triphenyl phosphite, triethyl phosphite, tributylphosphine, triphenylphosphine, tris(dimethylamino)phosphine, or tris(diethylamino)phosphine. Preferred is tris(2,4-di-*tert*-butylphenyl)phosphite, tri-O-tolyl phosphite, or triphenyl phosphite. More preferred is tris(2,4-di-*tert-*butylphenyl)phosphite. The amount of the phosphorus reagent used in this step is not limited as long as the reaction proceeds, but is preferably from 0.5 to 6 equivalents and more preferably from 1 to 3 equivalents of the compound represented by formula (XXI).

The reaction temperature in this step is not limited as long as the reaction proceeds, but is preferably from -80°C to the boiling point of the solvent used in the reaction, and more preferably from -20°C to 30°C. The reaction time in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is dichloromethane, 1,2-dimethoxyethane, 2-methyltetrahydrofuran, 1,4-dioxane, cyclopentyl methyl ether, or a mixed solvent thereof. More preferred is dichloromethane or cyclopentyl methyl ether, or a mixed solvent thereof.

### (Step c4)

This step is a step of reacting the compound of formula (XXII) with formula (XXIII) to obtain a compound of formula (XXIV).

The reaction in this step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), a base (e.g., cesium carbonate, potassium carbonate, potassium hydroxide, potassium bicarbonate, sodium carbonate, sodium hydroxide, sodium bicarbonate, sodium acetate, potassium acetate, sodium methoxide, sodium ethoxide, potassium *tert*-butoxide), or a mixture thereof. Preferred is cesium carbonate, potassium carbonate, or sodium carbonate. More preferred is cesium carbonate. The amount of the base used in this step is not limited as long as the reaction proceeds, but is preferably from 0.5 to 10 equivalents and more preferably from 1 to 5 equivalents of the compound represented by formula (XXII).

The reaction temperature in this step is not limited as long as the reaction proceeds, but is preferably from -20°C to the boiling point of the solvent used in the reaction, and more preferably from 10°C to 50°C. The reaction time in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is N,N-dimethylformamide, N,N-dimethylacetamide, 1-methyl-2-pyrrolidone, 1,3-dimethyl-2-imidazolidinone, or dimethyl sulfoxide. More preferred is dimethyl sulfoxide.

### (Step c5)

This step is a step of deprotecting the compound of formula (XXIV) to remove a benzoyl group, to obtain a compound of formula (XXV) or a salt thereof.

This step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., cesium carbonate, potassium carbonate, potassium hydroxide, potassium bicarbonate, sodium carbonate, sodium hydroxide, sodium bicarbonate, sodium acetate, potassium acetate, sodium methoxide, sodium ethoxide, potassium *tert*-butoxide), or a mixture thereof. Preferable examples include 1,8-diazabicyclo[5.4.0]undec-7-ene, sodium methoxide, sodium ethoxide, potassium carbonate, sodium carbonate, sodium hydroxide, or potassium hydroxide. More preferable examples include sodium hydroxide, sodium methoxide, or sodium ethoxide. The amount of the base used in this step is not limited as long as the reaction proceeds, but is preferably from 0.001 to 10 equivalents and more preferably from 0.01 to 5 equivalents of the compound represented by formula (XXIV).

The reaction temperature in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 24 hours, and more suitably from 1 hour to 10 hours.

The solvent that can be used in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is water, methanol, ethanol, or tetrahydrofuran, or a mixed solvent thereof. More preferred is a mixed solvent of tetrahydrofuran and ethanol or water.

Examples of the acid used in the conversion of the compound of formula (XXV) to a salt thereof include formic acid, acetic acid, propionic acid, oxalic acid, maleic acid, benzoic acid, methanesulfonic acid, p-toluenesulfonic acid, hydrochloric acid, nitric acid, sulfuric acid, or phosphoric acid. Preferred is acetic acid, p-toluenesulfonic acid, or hydrochloric acid. More preferred is p-toluenesulfonic acid or hydrochloric acid. The amount of the acid used for the conversion to the salt is not limited as long as the reaction proceeds, but is preferably from 0.5 to 5 equivalents and more preferably from 1 to 3 equivalents of the compound represented by formula (XXIV).

### (Step c6)

This step is a step of reacting the compound of formula (XXV) or a salt thereof with a tritylating agent to obtain a compound of formula (VIII') or a salt thereof.

Examples of the tritylating agent in this step include 4,4-dimethoxytrityl chloride, 4-methoxytrityl chloride, 2-chlorotrityl chloride, or trityl chloride. Preferred is 4,4-dimethoxytrityl chloride or 4-methoxytrityl chloride. The amount of the tritylating agent used in this step is not limited as long as the reaction proceeds, but is preferably from 0.5 to 6 equivalents and more preferably from 1 to 3 equivalents of the compound represented by formula (XXV).

The reaction temperature in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 72 hours, and more preferably from 1 hour to 36 hours.

The solvent that can be used in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is pyridine, 2-methylpyridine, 3-methylpyridine, 4-methylpyridine, 2,6-lutidine, acetonitrile, or dichloromethane. More preferred is pyridine.

### (Step c7)

This step is a step of reacting the compound represented by formula (VIII') or a salt thereof with a silylating agent and then with an acylating or alkoxycarbonylating agent to obtain a compound of formula (XXVII).

Examples of the silylating agent in this step include trimethylchlorosilane, trimethylsilyl triflate, triethylchlorosilane, triethylsilyl triflate, triisopropylchlorosilane, triisopropyl silyl triflate, *tert*-butyldimethylchlorosilane, *tert*-butyldimethylsilyl triflate, *tert*-butyldiphenylchlorosilane, *tert*-butyldiphenylsilyl triflate, triphenylchlorosilane, or triphenylsilyl triflate. Preferred is *tert-*butyldimethylsilyl triflate.

Examples of the acylating or alkoxycarbonylating agent in this step include a benzoylating agent, a 2-(trimethylsilyl)ethoxycarbonylating agent, a *tert-*butoxycarbonylating agent, a 9-fluorenylmethyloxycarbonylating agent, an allyloxycarbonylating agent, a 2,2,2-trichloroethoxycarbonylating agent, or a benzyloxycarbonylating agent. Preferred is a benzoylating agent or a 2-(trimethylsilyl)ethoxycarbonylating agent. The amount of the acylating or alkoxycarbonylating agent used in this step is not limited as long as the reaction proceeds, but is preferably from 0.5 to 6 equivalents and more preferably from 1 to 3 equivalents of the compound represented by formula (VIII') or a salt thereof.

The temperature of the reaction with the acylating or alkoxycarbonylating agent in this step is not limited as long as the reaction proceeds, but is preferably from -20°C to the boiling point of the solvent used in the reaction, and more preferably from 0°C to 40°C. The time of the reaction with the acylating or alkoxycarbonylating agent in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is pyridine, 2-methylpyridine, 3-methylpyridine, 4-methylpyridine, 2,6-lutidine, acetonitrile, or dichloromethane. More preferred is pyridine.

### (Step c8)

This step is a step of removing PG7, a protecting group in the compound of formula (XXVII) by deprotection, to obtain a compound of formula (VIII).

Examples of the deprotectant used in this step include ammonium fluoride, tetra-n-butylammonium fluoride, pyridine hydrofluoride, or triethylamine trihydrofluoride. Preferred is ammonium fluoride or tetra-n-butylammonium fluoride. More preferred is tetra-n-butylammonium fluoride. The amount of the deprotectant used in this step is not limited as long as the reaction proceeds, but is preferably from 0.1 to 5 equivalents and more preferably from 0.2 to 2 equivalents of the compound represented by formula (XXVII).

The reaction temperature in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is methanol, acetonitrile, tetrahydrofuran, or dichloromethane. More preferred is tetrahydrofuran.

### (Novel Intermediate)

Examples of a novel intermediate in the above [Synthesis Scheme <C1>] include a compound represented by the following formula (XX):

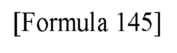

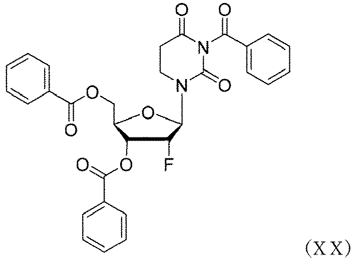

Examples of the novel intermediate in the above [Synthesis Scheme <C1>] include a compound represented by the following formula (XXII):

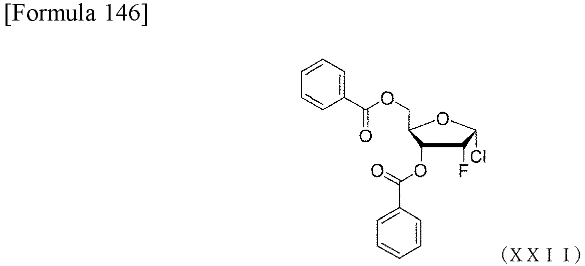

Examples of the novel intermediate in the above [Synthesis Scheme <C1>] include a compound represented by the following formula (XXIV):

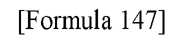

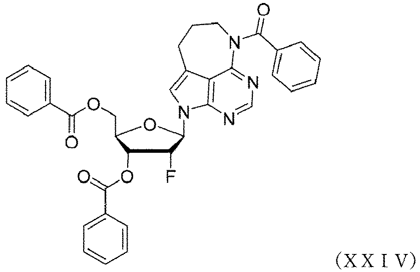

Examples of the novel intermediate in the above [Synthesis Scheme <C1>] include a compound represented by the following formula (XXV): 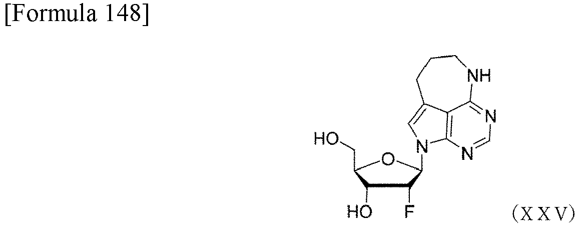 or a salt thereof.

Examples of the novel intermediate in the above [Synthesis Scheme <C1>] include a compound represented by the following formula (VIII'-1): 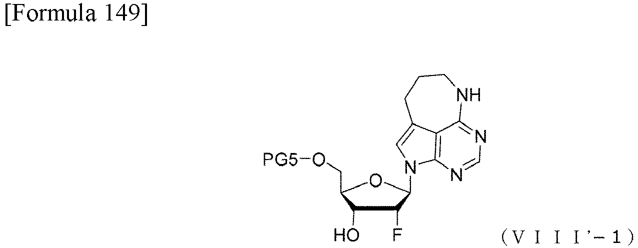 or a salt thereof, wherein PG5 is preferably 4,4'-dimethoxytrityl, 4-methoxytrityl, 2-chlorotrityl, or trityl.

Examples of the novel intermediate in the above [Synthesis Scheme <C1>] include a compound represented by the following formula (XXVII): 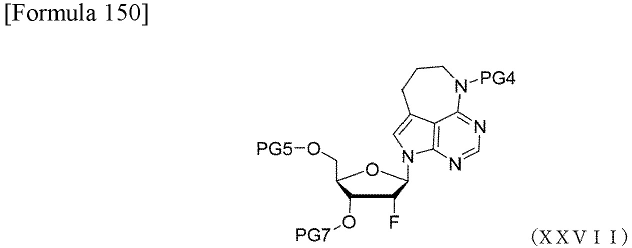
wherein PG4 is preferably benzoyl, 2-(trimethylsilyl)ethoxycarbonyl, *tert-*butoxycarbonyl, 9-fluorenylmethyloxycarbonyl, allyloxycarbonyl, 2,2,2-trichloroethoxycarbonyl, or benzyloxycarbonyl, and more preferably benzoyl or 2-(trimethylsilyl)ethoxycarbonyl,
PG5 is preferably 4,4'-dimethoxytrityl, 4-methoxytrityl, 2-chlorotrityl, or trityl, and more preferably 4,4'-dimethoxytrityl or 4-methoxytrityl, and
PG7 is preferably hydroxy(oxo)-λ⁵-phosphanyl, trimethylsilyl, triethylsilyl, triisopropylsilyl, *tert*-butyldimethylsilyl, *tert*-butyldiphenylsilyl, or triphenylsilyl, and more preferably *tert*-butyldimethylsilyl.

### <3-2. Method for preparing compound of formula (XXIII) and intermediate therefor>

The compound of formula (XXIII) used for preparing the above formula (VIII) may be produced according to the following synthesis scheme.

### [Synthesis Scheme <C2>]

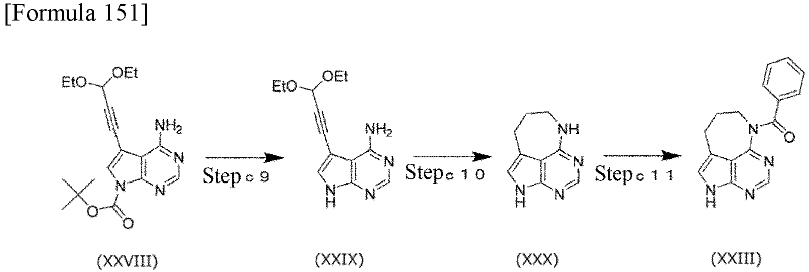

Hereinbelow, each step will be described.

### (Step c9)

This step is a step of deprotecting the compound represented by formula (XXVIII) to remove a *tert*-butoxycarbonyl group, to obtain a compound of formula (XXIX).

This step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium *tert*-butoxide). Preferred is potassium hydroxide, potassium bicarbonate, sodium carbonate, or sodium hydroxide. More preferred is sodium hydroxide. The amount of the base used in this step is not limited as long as the reaction proceeds, but is preferably from 0.5 to 6 equivalents and more preferably from 1 to 3 equivalents of the compound represented by formula (XXVIII).

The reaction temperature in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 24 hours, and from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is methanol, ethanol, 1-propanol, or tetrahydrofuran. More preferred is ethanol.

### (Step c10)

This step is a step of subjecting the compound of formula (XXIX) to alkyne reduction reaction in the presence of a catalyst, followed by reductive amination reaction to obtain a compound of formula (XXX).

A metal catalyst can be used as a catalyst for the alkyne reduction reaction in this step. The metal catalyst used in this step is not particularly limited as long as it catalyzes hydrogenation. Preferred is a ruthenium, rhodium, palladium, platinum, or nickel catalyst. More preferred is a palladium catalyst. The amount of the metal catalyst used in this step is not limited as long as the reaction proceeds, but is a mass preferably as 0.01 to 1 times, more preferably 0.02 to 0.4 times, more preferably 0.05 to 0.2 times as the mass of the compound represented by formula (XXVIII). The hydrogen gas pressure is usually from 100 to 1000 kPa, preferably from 100 to 700 kPa, and more preferably from 200 to 500 kPa. The reaction temperature in this step is not limited as long as the reaction proceeds, but is preferably from 0°C to the boiling point of the solvent used in the reaction, and more preferably from 20°C to 80°C. The reaction time in this step is not limited as long as the reaction proceeds, but is preferably from 5 minutes to 72 hours, and more preferably from 1 hour to 24 hours.

The solvent that can be used for the alkyne reduction reaction in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, acetic acid, N,N-dimethylformamide, N,N-dimethylacetamide, 1-methyl-2-pyrrolidone, or dimethyl sulfoxide, or a mixed solvent thereof. Preferred is N,N-dimethylformamide, N,N-dimethylacetamide, or 1-methyl-2-pyrrolidone, or a mixed solvent thereof. More preferred is 1-methyl-2-pyrrolidone.

A metal catalyst can be used as a catalyst for the reductive amination reaction in this step. The metal catalyst used in this step is not particularly limited as long as it catalyzes hydrogenation. Preferred is a ruthenium, rhodium, palladium, platinum, or nickel catalyst. More preferred is a palladium catalyst. The amount of the metal catalyst used in this step is not limited as long as the reaction proceeds, but is a mass preferably as 0.01 to 1 times, more preferably 0.02 to 0.4 times, more preferably 0.05 to 0.2 times as the mass of the compound represented by formula (XXVIII). The hydrogen gas pressure is usually from 100 to 1000 kPa, preferably from 100 to 700 kPa, and more preferably from 200 to 500 kPa. The reaction temperature in this step is not limited as long as the reaction proceeds, but is preferably from 0°C to the boiling point of the solvent used in the reaction, and more preferably from 20°C to 80°C. The reaction time in this step is not limited as long as the reaction proceeds, but is preferably from 5 minutes to 72 hours, and more preferably from 1 hour to 24 hours.

The solvent that can be used for the reductive amination reaction in this step is not particularly limited as long as the reaction is not inhibited. Examples of the solvent include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, acetic acid, N,N-dimethylformamide, N,N-dimethylacetamide, 1-methyl-2-pyrrolidone, and dimethyl sulfoxide, and a mixed solvent thereof. Preferred is N,N-dimethylformamide, N,N-dimethylacetamide, 1-methyl-2-pyrrolidone, water, or acetic acid, or a mixed solvent thereof. More preferred is a mixed solvent of 1-methyl-2-pyrrolidone, water, and acetic acid.

### (Step c11)

This step is a step of reacting the compound of formula (XXX) with a benzoylating agent, and then subjecting the resulting compound to base treatment for debenzoylation to obtain a compound of formula (XXIII).

Examples of the benzoylating agent in this step include benzoyl chloride, benzoyl bromide, benzoic anhydride or benzoyl trifluoromethanesulfonate. Preferred is benzoyl chloride. The amount of the benzoylating agent used in this step is not limited as long as the reaction proceeds, and is preferably from 0.5 to 10 equivalents and more preferably from 1 to 5 equivalents of the compound represented by formula (XXX).

The reaction with a benzoylating agent in this step can preferably be performed in the presence of a base. The base used in this step is not particularly limited as long as the reaction proceeds. Examples thereof include organic bases, such as triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, and 1,8-diazabicyclo[5.4.0]undec-7-ene, and potassium carbonate, potassium hydroxide, potassium bicarbonate, sodium carbonate, sodium hydroxide, sodium bicarbonate, sodium acetate, potassium acetate, sodium methoxide, sodium ethoxide, and potassium *tert*-butoxide, and mixed bases thereof. Preferred is triethylamine, diisopropylethylamine, 4-dimethylaminopyridine, 1,4-diazabicyclo[2.2.2]octane, or 1,8-diazabicyclo[5.4.0]undec-7-ene, or a mixed base thereof. More preferred is a mixed base of triethylamine, diisopropylethylamine, and 4-dimethylaminopyridine. The amount of the base used in this step is not limited as long as the reaction proceeds, and is preferably from 0.5 to 10 equivalents and more preferably from 1 to 5 equivalents of the compound represented by formula (XXVIII).

The temperature of the reaction with a benzoylating agent in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 72 hours, and more preferably from 1 hour to 24 hours.

The solvent that can be used for the reaction with a benzoylating agent in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is N,N-dimethylformamide, N,N-dimethylacetamide, 1-methyl-2-pyrrolidone, or 1,3-dimethyl-2-imidazolidinone. More preferred is 1,3-dimethyl-2-imidazolidinone.

The base used for the base treatment in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, or potassium *tert*-butoxide). Preferred is potassium hydroxide, potassium bicarbonate, sodium carbonate, or sodium hydroxide. More preferred is triethylamine. The amount of the base used for the base treatment in this step is not limited as long as the reaction proceeds, but is preferably from 0.5 to 10 equivalents and more preferably from 1 to 5 equivalents of the compound represented by formula (XXVIII).

The reaction temperature of the base treatment in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 72 hours, and more preferably from 1 hour to 24 hours.

The solvent that can be used for the base treatment in this step is not particularly limited as long as the reaction is not inhibited. Examples include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is N,N-dimethylformamide, N,N-dimethylacetamide, 1-methyl-2-pyrrolidone, or 1,3-dimethyl-2-imidazolidinone. More preferred is a mixed solvent of methanol and 1,3-dimethyl-2-imidazolidinone.

Examples of the intermediate in the above [Synthesis Scheme <C2>] include a compound represented by the following formula (XXVIII):

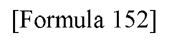

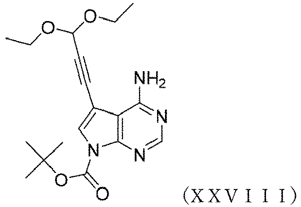

### <3-3. Method for preparing compound of formula (XXVIII)>

The compound of formula (XXVIII) used for preparing the above formula (XXIII) may be produced according to the following synthesis scheme.

### [Synthesis Scheme <C3>]

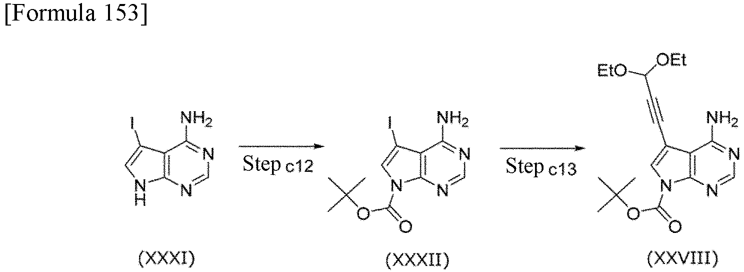

Hereinbelow, each step will be described.

### (Step c12)

This step is a step of reacting the compound of formula (XXXI) with a *tert-*butoxycarbonylating agent in the presence of 1-methylimidazole to obtain a compound of formula (XXXII).

Examples of the *tert*-butoxycarbonylating agent in this step include di-*tert-*butyl dicarbonate, N-*tert*-butoxycarbonyl imidazole, N-*tert*-butoxycarbonyl-1,2,4-triazole, or N-(*tert*-butoxycarbonyloxy)phthalimide. Preferred is di-*tert*-butyl dicarbonate. The amount of the *tert*-butoxycarbonylating agent used in this step is not limited as long as the reaction proceeds, but is preferably from 0.5 to 6 equivalents and more preferably from 1 to 3 equivalents of the compound represented by formula (XXXI).

The reaction temperature in this step is not limited as long as the reaction proceeds, but is preferably from -20°C to the boiling point of the solvent used in the reaction, and more preferably from 0°C to 40°C. The reaction time in this step is not limited as long as the reaction proceeds, but is preferably from 5 minutes to 24 hours, and more preferably from 1 hour to 10 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is acetonitrile, tetrahydrofuran, ethyl acetate, benzene, or toluene. More preferred is toluene.

### (Step c13)

This step is a step of reacting the compound represented by formula (XXXII) with propargylaldehyde diethyl acetal to obtain a compound of formula (XXVIII).

This step can preferably be performed in the presence of a transition metal catalyst. It is preferable to be performed in the presence of a palladium catalyst. The palladium catalyst used in this step is not particularly limited as long as the reaction proceeds. Examples include a divalent palladium salt or a complex thereof (e.g., palladium(II) acetate, palladium(II) trifluoroacetate, palladium(II) chloride, palladium(II) bromide, palladium(II) iodide, bis(triphenylphosphine)palladium(II) dichloride), or a zero-valent palladium metal or a complex thereof (e.g., palladium black, palladium carbon, tetrakistriphenylphosphine palladium(0), bis(dibenzylideneacetone)palladium(0)). Preferred is bis(triphenylphosphine)palladium(II) dichloride. The amount of the palladium catalyst used in this step is not limited as long as the reaction proceeds, but is preferably from 0.0001 to 1 equivalents and more preferably from 0.005 to 0.05 equivalents of the compound represented by formula (XXXII).

Further, this step can preferably be carried out in the presence of a copper catalyst other than the palladium catalyst mentioned above. The copper catalyst that can be used in this step may be copper(I) chloride, copper(I) bromide, or copper(I) iodide. Preferably used is copper(I) iodide. The amount of the copper catalyst used in this step is not limited as long as the reaction proceeds, but is preferably from 0.0001 to 1 equivalents and more preferably from 0.005 to 0.05 equivalents of the compound represented by formula (XXXII).

In addition, this step can preferably be performed in the presence of a base. The base used in this step is not particularly limited as long as the reaction proceeds. Examples of the base include organic bases, such as triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, and 1,8-diazabicyclo[5.4.0]undec-7-ene), and potassium carbonate, potassium hydroxide, potassium bicarbonate, sodium carbonate, sodium hydroxide, sodium bicarbonate, sodium acetate, potassium acetate, sodium methoxide, sodium ethoxide, and potassium *tert*-butoxide, and mixed bases thereof. Preferred is triethylamine, diisopropylethylamine, 1,4-diazabicyclo[2.2.2]octane, or 1,8-diazabicyclo[5.4.0]undec-7-ene. More preferred is triethylamine. The amount of the base used in this step is not limited as long as the reaction proceeds, and is preferably from 0.5 to 10 equivalents and more preferably from 1 to 5 equivalents of the compound represented by formula (XXXII).

The reaction temperature in this step is not limited as long as the reaction proceeds, but is preferably from 0°C to the boiling point of the solvent used in the reaction, and more preferably from 15°C to 50°C. The reaction time in this step is not limited as long as the reaction proceeds, but is preferably from 5 minutes to 72 hours, and more preferably from 1 hour to 24 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is N,N-dimethylformamide, N,N-dimethylacetamide, or 1-methyl-2-pyrrolidone. More preferred is N,N-dimethylformamide.

### <3-4. Method for preparing compound of formula (XXIV)>

The compound of formula (XXIV) used for preparing the above formulas (VIII) and (VIII') may be produced according to the following synthesis scheme.

### [Synthesis Scheme <C4>]

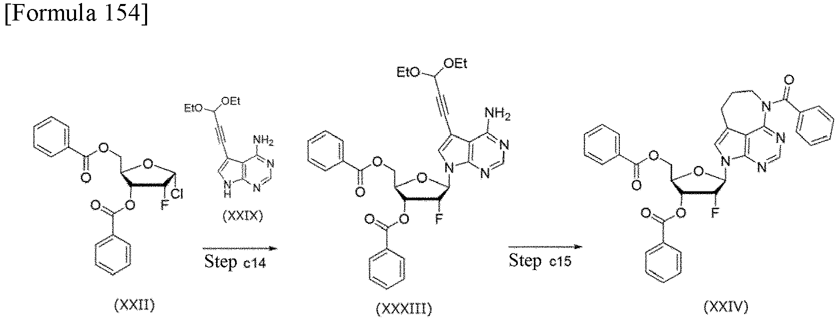

Hereinbelow, each step will be described.

### (Step c14)

This step is a step of reacting the compound of formula (XXII) with a compound of formula (XXIX) to obtain a compound of formula (XXXIII).

The reaction in this step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), a base (e.g., cesium carbonate, potassium carbonate, potassium hydroxide, potassium bicarbonate, sodium carbonate, sodium hydroxide, sodium bicarbonate, sodium acetate, potassium acetate, sodium methoxide, sodium ethoxide, potassium *tert*-butoxide), or a mixture thereof. Preferred is cesium carbonate, potassium carbonate, or sodium carbonate. More preferred is cesium carbonate. The amount of the base used in this step is not limited as long as the reaction proceeds, but is preferably from 0.5 to 10 equivalents and more preferably from 1 to 5 equivalents of the compound represented by formula (XXII).

The reaction temperature in this step is not limited as long as the reaction proceeds, but is preferably from -20°C to the boiling point of the solvent used in the reaction, and more preferably from 10°C to 50°C. The reaction time in this step is not limited as long as the reaction proceeds, but is preferably from 5 minutes to 72 hours, and more preferably from 1 hour to 24 hours.

The solvent that can be used in this step is not particularly limited as long as the reaction is not inhibited. Examples include methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, cyclopentyl methyl ether, ethyl acetate, hexane, pentane, heptane, cyclohexane, ethylcyclohexane, benzene, toluene, chlorobenzene, acetone, 2-butanone, N,N-dimethylformamide, N,N-dimethylacetamide, 1-methyl-2-pyrrolidone, 1,3-dimethyl-2-imidazolidinone, or dimethyl sulfoxide, or a mixed solvent thereof. Preferred is acetonitrile, N,N-dimethylformamide, N,N-dimethylacetamide, 1-methyl-2-pyrrolidone, 1,3-dimethyl-2-imidazolidinone, or dimethyl sulfoxide. More preferred is dimethyl sulfoxide.

### (Step c15)

This step is a step of subjecting the compound represented by formula (XXXIII) to alkyne reduction reaction in the presence of a catalyst, followed by reductive amination reaction and a further reaction with a benzoylating agent to obtain a compound of formula (XXIV).

A metal catalyst can be used as a catalyst for the alkyne reduction reaction and the reductive amination reaction in this step. The metal catalyst used in this step is not particularly limited as long as it catalyzes hydrogenation. Preferred is a ruthenium, rhodium, palladium, platinum, or nickel catalyst. More preferred is a palladium catalyst. The amount of the metal catalyst used in this step is not limited as long as the reaction proceeds, and is a mass preferably as 0.01 to 1 times, more preferably 0.02 to 1 times, more preferably 0.05 to 0.5 times as the mass of the compound represented by formula (XXXIII). The hydrogen gas pressure is usually from 100 to 1000 kPa, preferably from 100 to 900 kPa, and from 200 to 700 kPa. The reaction temperature in this step is not limited as long as the reaction proceeds, but is preferably from 0°C to the boiling point of the solvent used in the reaction, and more preferably from 15°C to 80°C. The reaction time in this step is not limited as long as the reaction proceeds, and is preferably from 5 minutes to 96 hours, and more preferably from 1 hour to 36 hours.

The solvent that can be used for the alkyne reduction reaction in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, acetic acid, N,N-dimethylformamide, N,N-dimethylacetamide, 1-methyl-2-pyrrolidone, or dimethyl sulfoxide, or a mixed solvent thereof. Preferred is ethanol, methanol, 1-propanol, 2-propanol, N,N-dimethylformamide, N,N-dimethylacetamide, or 1-methyl-2-pyrrolidone, or a mixed solvent thereof. More preferred is ethanol.

The solvent that can be used for the reductive amination reaction in this step is not particularly limited as long as the reaction is not inhibited. Examples include water, methanol, ethanol, 1-propanol, 2-propanol, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, acetic acid, N,N-dimethylformamide, N,N-dimethylacetamide, 1-methyl-2-pyrrolidone, or dimethyl sulfoxide, or a mixed solvent thereof. Preferred is ethanol, water, or acetic acid, or a mixed solvent thereof. More preferred is a mixed solvent of water and acetic acid.

Examples of the benzoylating agent used for the benzoylation reaction in this step include benzoic anhydride, benzoyl chloride, benzoyl bromide, or benzoyl trifluoromethanesulfonate. Preferred is benzoyl chloride or benzoyl bromide. More preferred is benzoyl chloride. The amount of the benzoylating agent used in this step is not limited as long as the reaction proceeds, but is preferably from 0.5 to 10 equivalents and more preferably from 1 to 5 equivalents of the compound represented by formula (XIX).

The benzoylation in this step can preferably be performed in the presence of a base. The base used in this step is not particularly limited as long as the reaction proceeds. Examples include an organic base (e.g., triethylamine, tributylamine, diisopropylethylamine, N-methylmorpholine, N-methylpyrrolidine, N-methylpiperidine, pyridine, 2-methylpyridine, 2,6-methylpyridine, 4-dimethylaminopyridine, 1,4-diazabicyclo[2.2.2]octane, 1,8-diazabicyclo[5.4.0]undec-7-ene), or a base (e.g., potassium carbonate, potassium hydroxide, potassium bicarbonate, sodium carbonate, sodium hydroxide, sodium bicarbonate, sodium acetate, potassium acetate, sodium methoxide, sodium ethoxide, potassium tert-butoxide). Preferable examples include triethylamine, 2,6-methylpyridine, 4-dimethylaminopyridine, potassium carbonate, potassium hydroxide, potassium bicarbonate, sodium carbonate, sodium hydroxide, sodium bicarbonate, or sodium acetate. More preferable examples include 4-dimethylaminopyridine. The amount of the base used in this step is not limited as long as the reaction proceeds, but is preferably from 0.05 to 10 equivalents and more preferably from 0.2 to 5 equivalents of the compound represented by formula (XIX).

The reaction temperature of the benzoylation in this step is not limited as long as the reaction proceeds, but is preferably from -20°C to the boiling point of the solvent used in the reaction, and more preferably from 0°C to 90°C. The reaction time in this step is not limited as long as the reaction proceeds, but is preferably from 5 minutes to 24 hours, and more preferably from 1 hour to 10 hours.

The solvent that can be used for the benzoylation in this step is not particularly limited as long as the reaction is not inhibited. Examples that can be used include pyridine, 2-methylpyridine, 3-methylpyridine, 4-methylpyridine, 2,6-lutidine, acetonitrile, dichloromethane, chloroform, diethyl ether, 1,2-dimethoxyethane, tetrahydrofuran, 2-methyltetrahydrofuran, 1,4-dioxane, ethyl acetate, hexane, pentane, cyclohexane, ethylcyclohexane, benzene, toluene, chlorobenzene, acetone, 2-butanone, N,N-dimethylformamide, N,N-dimethylacetamide, 1-methyl-2-pyrrolidone, or dimethyl sulfoxide, or a mixed solvent thereof. Preferred is pyridine, 2-methylpyridine, 3-methylpyridine, 4-methylpyridine, 2,6-lutidine, acetonitrile, or dichloromethane. More preferred is pyridine.

### <3-5. Comparison between method for preparing compound (VIII) in the present invention and conventional methods (e.g., Patent Literature 6)>

The method for preparing a compound of formula (VIII) according to the present invention makes it possible to provide a production method with better effects, such as improved total yield, a reduced number of steps, and avoidance of column purification, than those of the synthesis scheme of Patent Literature 6 shown below.

### [Synthesis scheme 1 of conventional method (from synthesis schemes of Examples 1 and 44 in Patent Literature 6)]

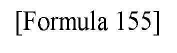

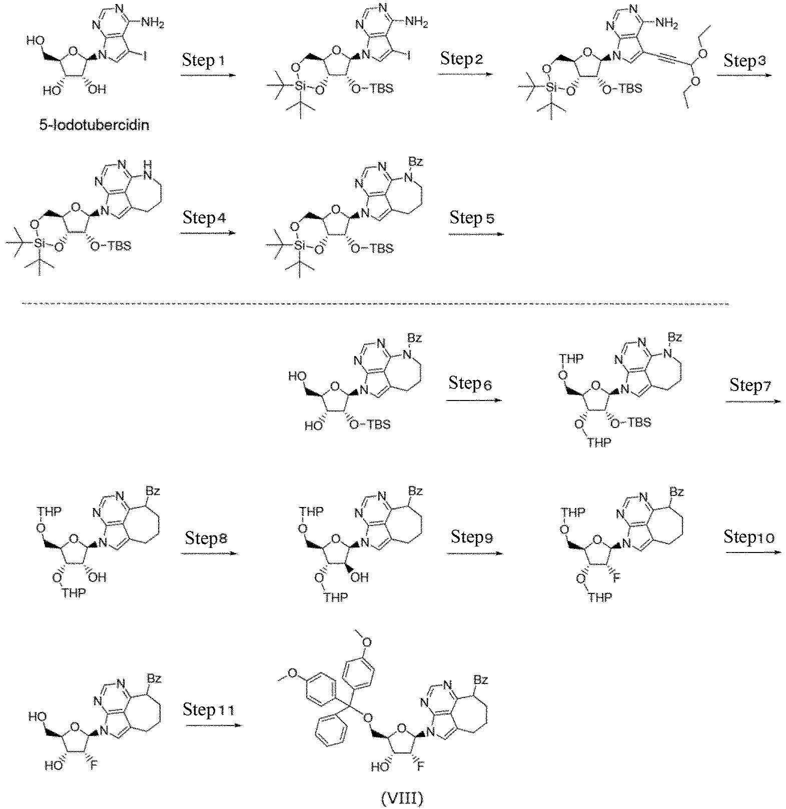

### [Synthesis scheme 2 of conventional method (from synthesis scheme of Example 42 in Patent Literature 6)]

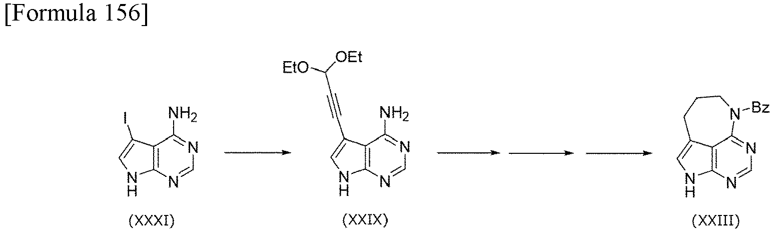

### [Effect of method for preparing formula (VIII) according to the present invention]

### (Effect 1) Improved synthetic route (improved yield, avoidance of column purification)

In the conventional synthetic route (Patent Literature 6/Conventional synthetic scheme 1), the compound of formula (VIII) was synthesized in 11 steps from 5-Iodotubercidin, which is commercially available in the market. However, there remains a problem that the total yield was low (5%) because of the linear synthesis.

On the other hand, in the present method, the convergent synthesis was adopted, and the number of steps from available raw materials, compound (XXXI) and 2'-deoxy-2'-fluorouridine, was reduced to 10 steps. The total yield was improved to 17% from compound (XXXI) and 9% from 2'-deoxy-2'-fluorouridine.

### (Effect 2) Synthesis of 2-fluororibose derivative by deglycosylation of compound (XX)

Conventionally, the acid hydrolysis method has been known in which a dihydrouridine derivative (e.g., 2'-deoxy-2'-fluoro-5,6-dihydrouridine) was hydrolyzed to synthesize a 2-deoxy-2-fluororibose derivative (e.g., compound (XXI)) (see Carbohydrate Res., I, 1966, pp. 455-466). However, when this method was applied to compound (XIX) to synthesize compound (XXI), there remains a problem that a side reaction involved removal of the benzoyl group at the hydroxyl group by deprotection, resulting in a low yield.

On the other hand, in the production method of the present invention, when the uridine moiety of compound (XIX) is benzoylated to obtain compound (XX), the reactivity during hydrolysis is improved. This enables deglycosylation under milder conditions, and the yield has been improved by suppressing the removal of the benzoyl group by deprotection.

### (Effect 3) Synthesis of compound (XXII) by selective α-chlorination of compound (XXI)

Conventionally, there is a report about chlorination of a 2-fluororibose derivative, in which the hydroxyl groups at 2- and 5-positions are each protected by a TBDMS group, by treatment under mesylchloride/triethylamine conditions. Unfortunately, in this method, the chlorinated sugar is obtained as a mixture of α/β (see J. Org. Chem., 2009, 74, pp. 5779-5789, and WO 2011/003018). Another reported method involves treatment of a 2-fluororibose derivative, in which the hydroxyl groups at 2- and 5-positions are each protected by a benzyl group, with hexamethyltriamide phosphite/carbon tetrachloride. However, this method produced a β-form and could not be applied to the synthesis of α-form (see WO 2014/124430 and WO 2015/038596).

On the other hand, the synthesis method of α-chloro-2-fluororibose derivative was investigated in the production method of the present invention. As a result, it has been found that the reaction proceeds in a highly α-selective manner (α/β = 15/1) by chlorination of a 2-fluororibose derivative (compound (XXI)), in which the hydroxyl groups at 2- and 5-positions are each protected by a benzoyl group, with triarylphosphite or triarylphosphine in combination with a chlorinating agent such as trichloroisocyanuric acid. Then, an efficient method for synthesizing a α-chloro-2-fluororibose derivative has been successfully established.

Meanwhile, as a conventional method, chlorination of alcohol using triarylphosphine and a chlorinating agent has been known for a long time. For example, the chlorination using the combination of triphenylphosphine and carbon tetrachloride has been widely known as the Appel reaction (see Angew. Chem. Int. Edit., Vol. 14, No. 12, 1975, pp. 801-811). However, in this Appel reaction, triphenylphosphine oxide is generated after the reaction, so that purification by silica gel column is required for the removal. This caused a problem in the mass synthesis.

On the other hand, in the production method of the present invention, 2,4-di-*tert*-butylphenyl phosphite is used as triarylphosphite. The by-product phosphate ester can be removed by liquid separation, and the column purification can successfully be avoided.

### (Effect 4) Synthesis of compound (XXIII) by Boc-protection route

In the conventional synthetic route (Patent Literature 6/conventional synthetic scheme 2), the yield of the Sonogashira reaction step to synthesize compound (XXIX) from compound (XXXI) was low, therefore there was a problem that the total yield was as low as 18%. In the production method of the present invention, the yield of the Sonogashira reaction step has been increased via compound (XXXII) by using the Boc protecting group, and the total yield has been improved to 53%.

### <4. Method for producing antibody-immunostimulant conjugate (Rp,Rp-XIII)>

### <4-1. Antibody and antibody glycan remodeling>

### <4-1-1. Antibody>

As used herein, the "functional fragment of antibody" is sometimes called an "antigen-binding fragment of antibody", and means a partial fragment of antibody having antigen-binding activity. Examples include an Fab, F(ab')₂, Fv, scFv, diabody, or linear antibody or multispecific antibody formed using an antibody fragment(s). Also included is an Fab', which is a monovalent fragment of a variable region of antibody, obtained by treating F(ab')₂ under reducing conditions. Provided that the fragment is not limited to these molecules as long as having an ability to bind to an antigen. In addition, these antigen-binding fragments include not only those obtained by treating a full-length molecule of antibody protein with appropriate enzyme, but also proteins produced in appropriate host cells while a genetically engineered antibody gene is used.

Examples of the "functional fragment" herein include a functional fragment having asparagine (Asn297) and surrounding amino acids, which asparagine is modified by well-conserved N-linked glycan and in the Fc region of IgG heavy chain, and having an ability to bind to an antigen.

The antibody used for producing an antibody-immunostimulant conjugate of the present invention means an immunoglobulin, and is a molecule containing an antigen-binding site where an antigen can be immunospecifically bound. The antibody in the present invention may be of any of class IgG, IgE, IgM, IgD, IgA, or IgY. Preferred is IgG. In addition, its subclass may be IgG1, IgG2, IgG3, IgG4, IgA1, or IgA2, and preferred is IgG1, IgG2, or IgG4 (including an antibody with an IgG heavy chain Fc region having a mutation affecting the ADCC and/or ADCP activities).

If IgG1 is used as the isotype of antibody used for producing an antibody-immunostimulant conjugate in the present invention, the effector function may be adjusted by substituting a part of amino acid residues in the constant region (see, WO88/07089, WO94/28027, WO94/29351). Examples of variants of IgG1 include IgG1 LALA mutant (IgG1-L234A, L235A). The L234A, L235A indicates substitution of leucine with alanine at the 234- and 235-positions specified by EU-index numbering (Proceedings of the National Academy of Sciences of the United States of America, Vol. 63, No. 1 (May 15, 1969), pp. 78-85).

It is known that each of heavy chains or light chains of an antibody molecule has three complementarity determining regions (CDRs). CDRs, which are also called a hypervariable region, are located in variable regions of heavy chains or light chains of an antibody and is a site with particularly high variation of the primary structure. Three CDRs are separately located in the primary structure of the polypeptide chain of each of heavy chains or light chains. Regarding CDRs of antibodies, herein, CDRs of a heavy chain refer to CDRH1, CDRH2, and CDRH3 from the amino terminus of the heavy chain amino acid sequence, and CDRs of a light chain refer to CDRL1, CDRL2, and CDRL3 from the amino terminus of the light chain amino acid sequence. These sites are located in the proximity of each other in the three-dimensional structure, determining specificity to an antibody to bind.

The antibody may be derived from any species. Preferable examples include a human, a rat, a mouse, or a rabbit. If the antibody is derived from a species other than human, it is preferable to chimerize or humanize the antibody using well-known techniques. The antibody of the present invention may be a polyclonal or monoclonal antibody, and is preferably a monoclonal antibody. Examples of the monoclonal antibody include each monoclonal antibody derived from a non-human animal (e.g., a rat, a mouse, and a rabbit antibody), a chimeric antibody, a humanized antibody, a human antibody, or a functional fragment thereof, or a modified antibody thereof.

The antibody is preferably an antibody against a tumor cell or an immune cell as a target. The target, however, is not limited to them. The antibody is more preferably an antibody against a tumor cell as a target.

The binding activity of the antibody against tumor cells can be confirmed using flow cytometry. The incorporation of the antibody into tumor cells can be checked using (1) an assay of visualizing an antibody incorporated in cells under a fluorescence microscope by using a (fluorescently labeled) secondary antibody binding to the therapeutic antibody (Cell Death and Differentiation (2008) 15, 751-761), (2) an assay of measuring a fluorescence intensity incorporated in cells by using a (fluorescently labeled) secondary antibody binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may also be used.

When an antibody against tumor cells is used in an antibody-immunostimulant conjugate of the present invention, it is desirable, but not essential, that the antibody itself has an anti-tumor effect.

The anti-tumor activity of the immunostimulant or the antibody-immunostimulant conjugate refers to cytotoxic activity on tumor cells, anti-cellular effects, and regression of the tumor volume. The anti-tumor activity can be checked by using a known *in vitro* or *in vivo* evaluation system.

The immunostimulatory activity of the immunostimulant or the antibody-immunostimulant conjugate refers to the increased sensitivity of tumor cells to immune cells or the activation of immune cells via tumor cells. The immunostimulatory activity can be checked by using a known *in vitro* or *in vivo* evaluation system.

Examples of the antibody used for producing an antibody-immunostimulant conjugate of the present invention include, but are not limited to, anti-HER2 antibody, anti-HER3 antibody, anti-DLL3 antibody, anti-FAP antibody, anti-CDH11 antibody, anti-CDH6 antibody, anti-A33 antibody, anti-CanAg antibody, anti-CD19 antibody, anti-CD20 antibody, anti-CD22 antibody, anti-CD30 antibody, anti-CD33 antibody, anti-CD56 antibody, anti-CD70 antibody, anti-CD98 antibodies, anti-TROP2 antibody, anti-CEA antibody, anti-Cripto antibody, anti-EphA2 antibody, anti-G250 antibody, anti-MUC1 antibody, anti-GPNMB antibody, anti-Integrin antibody, anti-PSMA antibody, anti-Tenascin-C antibody, anti-SLC44A4 antibody, anti-Mesothelin antibody, anti-ENPP3 antibody, anti-CD47 antibody, anti-EGFR antibody, anti-GPR20 antibody, or anti-DR5 antibody. Preferable examples of the antibody in the present invention include anti-HER2 antibody (e.g., trastuzumab or pertuzumab), anti-CDH6 antibody, anti-CD33 antibody, anti-EphA2 antibody, anti-CD70 antibody, anti-TROP2 antibody, or anti-EGFR antibody. More preferable examples include anti-HER2 antibody, anti-CDH6 antibody, anti-CD70 antibody, anti-TROP2 antibody, or anti-EGFR antibody.

The antibody used for producing an antibody-immunostimulant conjugate of the present invention can be obtained by immunizing an animal with a polypeptide that serves as an antigen, and collecting and purifying the antibody produced *in vivo,* while using the protocols routinely implemented in the art. The origin of the antigen is not limited to a human, and each animal can also be immunized with the antigen derived from a non-human animal (e.g., a mouse, a rat). In this case, any antibody applicable to human diseases can be selected by testing the cross-reactivity between the obtained antibody that can bind to a heterologous antigen and the corresponding human antigen.

Alternatively, antibody-producing cells which produce antibodies against the antigen are fused with myeloma cells according to the method known in the art (e.g., Kohler and Milstein, Nature (1975) 256, p. 495-497; Kennett, R. ed., Monoclonal Antibodies, p. 365-367, Plenum Press, N.Y. (1980)) to establish hybridomas, from which monoclonal antibodies can in turn be obtained.

Note that the antigen can be obtained by genetical engineering a host cell to produce an antigen protein encoded by a gene of interest.

The humanized antibody used for producing an antibody-immunostimulant conjugate of the present invention can be obtained according to the known protocols (e.g., Proc. Natl. Acad. Sci. U.S.A., 81, 6851-6855, (1984), Nature (1986) 321, p. 522-525, WO90/07861).

For example, anti-HER2 antibody (e.g., US5821337, WO2004/008099, WO2020/050406), anti-CD33 antibody (e.g., WO2014/057687, WO2020/050406), anti-EphA2 antibody (e.g., WO2009/028639, WO2020/050406), anti-CDH6 antibody (e.g., WO2018/212136, WO2020/050406), anti-CD70 antibody (e.g., WO2004/073656, WO2007/038637, WO2021/177438), anti-TROP2 antibody (e.g., WO2015/098099, WO2021/177438), or anti-EGFR antibody (e.g., WO1998/050433, WO2002/092771, WO2021/177438) can be obtained by known means.

The anti-HER2 antibody used for producing an antibody-immunostimulant conjugate of the present invention is not particularly limited, and should have, for example, the following characteristics.
(1) An anti-HER2 antibody having the following characteristics of:
   (a) binding specifically to HER2; and
   (b) having activity to internalize into an HER2-expressing cell by binding to HER2.
(2) The antibody described in (1), wherein the antibody can bind to an extracellular domain of HER2.
(3) The antibody described in (1) or (2), wherein the antibody is a monoclonal antibody.
(4) The antibody described in any one of (1) to (3), wherein the antibody has antibody-dependent cell-mediated cytotoxicity (ADCC) and/or complement-dependent cytotoxicity (CDC).
(5) The antibody described in any one of (1) to (4), wherein the antibody is a mouse monoclonal antibody, a chimeric monoclonal antibody, or a humanized monoclonal antibody.
(6) The antibody described in any one of (1) to (3), wherein a heavy chain constant region thereof is a heavy chain constant region of human IgG1 and can cause a decrease in ADCC and ADCP activities.
(7) The antibody described in any one of (1) to (4), wherein the antibody is a humanized monoclonal antibody comprising a heavy chain consisting of the amino acid sequence set forth in SEQ ID NO: 50 and a light chain consisting of the amino acid sequence set forth in SEQ ID NO: 49.
(8) The antibody described in (6), wherein a heavy chain constant region thereof is a heavy chain constant region of human IgG1 and leucine residues at the 234- and 235-positions specified by EU Index numbering are substituted with alanine residues.
(9) The antibody described in (8), wherein the antibody is a humanized monoclonal antibody comprising a heavy chain consisting of the amino acid sequence set forth in SEQ ID NO: 51 and a light chain consisting of the amino acid sequence set forth in SEQ ID NO: 49.
(10) The antibody described in any one of (1) to (4), wherein the antibody is a humanized monoclonal antibody comprising a heavy chain consisting of the amino acid sequence set forth in SEQ ID NO: 53 and a light chain consisting of the amino acid sequence set forth in SEQ ID NO: 52.
(11) The antibody described in (8), wherein the antibody is a humanized monoclonal antibody comprising a heavy chain consisting of the amino acid sequence set forth in SEQ ID NO: 54 and a light chain consisting of the amino acid sequence set forth in SEQ ID NO: 52.
(12) The antibody described in any one of (1) to (3), (6), or (8), wherein the antibody is a humanized monoclonal antibody comprising: a light chain comprising CDRL1 consisting of the amino acid sequence set forth in SEQ ID NO: 57, CDRL2 consisting of the amino acid sequence set forth in SEQ ID NO: 58, and CDRL3 consisting of the amino acid sequence set forth in SEQ ID NO: 59; and a heavy chain comprising CDRH1 consisting of the amino acid sequence set forth in SEQ ID NO: 60, CDRH2 consisting of the amino acid sequence set forth in SEQ ID NO: 61, and CDRH3 consisting of the amino acid sequence set forth in SEQ ID NO: 62.
(13) The antibody described in any one of (1) to (12), wherein one or two amino acids are deleted at a heavy chain carboxyl terminus.
(14) An antibody obtained by a method for producing the antibody, the method comprising the steps of culturing a host cell transfected with an expression vector containing a polynucleotide encoding the antibody described in any one of (1) to (13), and collecting the antibody of interest from the resulting culture obtained in the former step.

The anti-CD70 antibody used for producing an antibody-immunostimulant conjugate of the present invention is not particularly limited, and should have, for example, the following characteristics.
(1) An anti-CD70 antibody capable of specifically binding to CD70.
(2) The antibody described in (1), wherein the antibody can bind to an extracellular domain of CD70.
(3) The antibody described in (1) or (2), wherein the antibody is a monoclonal antibody.
(4) The antibody described in any one of (1) to (3), wherein the antibody has antibody-dependent cell-mediated cytotoxicity (ADCC) and/or complement-dependent cytotoxicity (CDC).
(5) The antibody described in any one of (1) to (4), wherein the antibody is a mouse monoclonal antibody, a chimeric monoclonal antibody, or a humanized monoclonal antibody.
(6) The antibody described in any one of (1) to (3), wherein a heavy chain constant region thereof is a heavy chain constant region of human IgG1 and can cause a decrease in ADCC and ADCP activities.
(7) The antibody described in (6), wherein a heavy chain constant region thereof is a heavy chain constant region of human IgG1 and leucine residues at the 234- and 235-positions specified by EU Index numbering are substituted with alanine residues.
(8) The antibody described in (7), wherein the antibody is a humanized monoclonal antibody comprising a heavy chain consisting of the amino acid sequence set forth in SEQ ID NO: 2 and a light chain consisting of the amino acid sequence set forth in SEQ ID NO: 1.
(9) The antibody described in (7), wherein the antibody is a humanized monoclonal antibody comprising a heavy chain consisting of the amino acid sequence set forth in SEQ ID NO: 4 and a light chain consisting of the amino acid sequence set forth in SEQ ID NO: 3.
(10) The antibody described in any one of (1) to (3), (6), or (7), wherein the antibody is a humanized monoclonal antibody comprising: a light chain comprising CDRL1 consisting of the amino acid sequence set forth in SEQ ID NO: 13, CDRL2 consisting of the amino acid sequence set forth in SEQ ID NO: 14, and CDRL3 consisting of the amino acid sequence set forth in SEQ ID NO: 15; and a heavy chain comprising CDRH1 consisting of the amino acid sequence set forth in SEQ ID NO: 16, CDRH2 consisting of the amino acid sequence set forth in SEQ ID NO: 17, and CDRH3 consisting of the amino acid sequence set forth in SEQ ID NO: 18.
(11) The antibody described in any one of (1) to (3), (6), or (7), wherein the antibody is a humanized monoclonal antibody comprising: a light chain comprising CDRL1 consisting of the amino acid sequence set forth in SEQ ID NO: 19, CDRL2 consisting of the amino acid sequence set forth in SEQ ID NO: 20, and CDRL3 consisting of the amino acid sequence set forth in SEQ ID NO: 21; and a heavy chain comprising CDRH1 consisting of the amino acid sequence set forth in SEQ ID NO: 22, CDRH2 consisting of the amino acid sequence set forth in SEQ ID NO: 23, and CDRH3 consisting of the amino acid sequence set forth in SEQ ID NO: 24.
(12) The antibody described in any one of (1) to (11), wherein one or two amino acids are deleted at a heavy chain carboxyl terminus.
(13) An antibody obtained by a method for producing the antibody, the method comprising the steps of culturing a host cell transfected with an expression vector containing a polynucleotide encoding the antibody described in any one of (1) to (12), and collecting the antibody of interest from the resulting culture obtained in the former step.

The anti-TROP2 antibody used for producing an antibody-immunostimulant conjugate of the present invention is not particularly limited, and should have, for example, the following characteristics.
(1) An anti-TROP2 antibody capable of specifically binding to TROP2.
(2) The antibody described in (1), wherein the antibody can bind to an extracellular domain of TROP2.
(3) The antibody described in (1) or (2), wherein the antibody is a monoclonal antibody.
(4) The antibody described in any one of (1) to (3), wherein the antibody has antibody-dependent cell-mediated cytotoxicity (ADCC) and/or complement-dependent cytotoxicity (CDC).
(5) The antibody described in any one of (1) to (4), wherein the antibody is a mouse monoclonal antibody, a chimeric monoclonal antibody, or a humanized monoclonal antibody.
(6) The antibody described in any one of (1) to (3), wherein a heavy chain constant region thereof is a heavy chain constant region of human IgG1 and can cause a decrease in ADCC and ADCP activities.
(7) The antibody described in any one of (1) to (4), wherein the antibody is a humanized monoclonal antibody comprising a heavy chain consisting of the amino acid sequence set forth in SEQ ID NO: 6 and a light chain consisting of the amino acid sequence set forth in SEQ ID NO: 5.
(8) The antibody described in (6), wherein a heavy chain constant region thereof is a heavy chain constant region of human IgG1 and leucine residues at the 234- and 235-positions specified by EU Index numbering are substituted with alanine residues.
(9) The antibody described in (8), wherein the antibody is a humanized monoclonal antibody comprising a heavy chain consisting of the amino acid sequence set forth in SEQ ID NO: 8 and a light chain consisting of the amino acid sequence set forth in SEQ ID NO: 7.
(10) The antibody described in any one of (1) to (3), (6), or (8), wherein the antibody is a humanized monoclonal antibody comprising: a light chain comprising CDRL1 consisting of the amino acid sequence set forth in SEQ ID NO: 25, CDRL2 consisting of the amino acid sequence set forth in SEQ ID NO: 26, and CDRL3 consisting of the amino acid sequence set forth in SEQ ID NO: 27; and a heavy chain comprising CDRH1 consisting of the amino acid sequence set forth in SEQ ID NO: 28, CDRH2 consisting of the amino acid sequence set forth in SEQ ID NO: 29, and CDRH3 consisting of the amino acid sequence set forth in SEQ ID NO: 30.
(11) The antibody described in any one of (1) to (3), (6), or (8), wherein the antibody is a humanized monoclonal antibody comprising: a light chain comprising CDRL1 consisting of the amino acid sequence set forth in SEQ ID NO: 31, CDRL2 consisting of the amino acid sequence set forth in SEQ ID NO: 32, and CDRL3 consisting of the amino acid sequence set forth in SEQ ID NO: 33; and a heavy chain comprising CDRH1 consisting of the amino acid sequence set forth in SEQ ID NO: 34, CDRH2 consisting of the amino acid sequence set forth in SEQ ID NO: 35, and CDRH3 consisting of the amino acid sequence set forth in SEQ ID NO: 36.
(12) The antibody described in any one of (1) to (11), wherein one or two amino acids are deleted at a heavy chain carboxyl terminus.
(13) An antibody obtained by a method for producing the antibody, the method comprising the steps of culturing a host cell transfected with an expression vector containing a polynucleotide encoding the antibody described in any one of (1) to (12), and collecting the antibody of interest from the resulting culture obtained in the former step.

The anti-EGFR antibody used for producing an antibody-immunostimulant conjugate of the present invention is not particularly limited, and should have, for example, the following characteristics.
(1) An anti-EGFR antibody capable of specifically binding to EGFR.
(2) The antibody described in (1), wherein the antibody can bind to an extracellular domain of EGFR.
(3) The antibody described in (1) or (2), wherein the antibody is a monoclonal antibody.
(4) The antibody described in any one of (1) to (3), wherein the antibody has antibody-dependent cell-mediated cytotoxicity (ADCC) and/or complement-dependent cytotoxicity (CDC).
(5) The antibody described in any one of (1) to (4), wherein the antibody is a mouse monoclonal antibody, a chimeric monoclonal antibody, or a humanized monoclonal antibody.
(6) The antibody described in any one of (1) to (3), wherein a heavy chain constant region thereof is a heavy chain constant region of human IgG1 and can cause a decrease in ADCC and ADCP activities.
(7) The antibody described in (6), wherein a heavy chain constant region thereof is a heavy chain constant region of human IgG1 and leucine residues at the 234- and 235-positions specified by EU Index numbering are substituted with alanine residues.
(8) The antibody described in (7), wherein the antibody is a humanized monoclonal antibody comprising a heavy chain consisting of the amino acid sequence set forth in SEQ ID NO: 10 and a light chain consisting of the amino acid sequence set forth in SEQ ID NO: 9.
(9) The antibody described in (7), wherein the antibody is a humanized monoclonal antibody comprising a heavy chain consisting of the amino acid sequence set forth in SEQ ID NO: 12 and a light chain consisting of the amino acid sequence set forth in SEQ ID NO: 11.
(10) The antibody described in any one of (1) to (3), (6), or (7), wherein the antibody is a humanized monoclonal antibody comprising: a light chain comprising CDRL1 consisting of the amino acid sequence set forth in SEQ ID NO: 37, CDRL2 consisting of the amino acid sequence set forth in SEQ ID NO: 38, and CDRL3 consisting of the amino acid sequence set forth in SEQ ID NO: 39; and a heavy chain comprising CDRH1 consisting of the amino acid sequence set forth in SEQ ID NO: 40, CDRH2 consisting of the amino acid sequence set forth in SEQ ID NO: 41, and CDRH3 consisting of the amino acid sequence set forth in SEQ ID NO: 42.
(11) The antibody described in any one of (1) to (3), (6), or (7), wherein the antibody is a humanized monoclonal antibody comprising: a light chain comprising CDRL1 consisting of the amino acid sequence set forth in SEQ ID NO: 43, CDRL2 consisting of the amino acid sequence set forth in SEQ ID NO: 44, and CDRL3 consisting of the amino acid sequence set forth in SEQ ID NO: 45; and a heavy chain comprising CDRH1 consisting of the amino acid sequence set forth in SEQ ID NO: 46, CDRH2 consisting of the amino acid sequence set forth in SEQ ID NO: 47, and CDRH3 consisting of the amino acid sequence set forth in SEQ ID NO: 48.
(12) The antibody described in any one of (1) to (11), wherein one or two amino acids are deleted at a heavy chain carboxyl terminus.
(13) An antibody obtained by a method for producing the antibody, the method comprising the steps of culturing a host cell transfected with an expression vector containing a polynucleotide encoding the antibody described in any one of (1) to (12), and collecting the antibody of interest from the resulting culture obtained in the former step.

The anti-CDH6 antibody used for producing an antibody-immunostimulant conjugate of the present invention is not particularly limited, and should have, for example, the following characteristics.
(1) An anti-CDH6 antibody capable of specifically binding to CDH6.
(2) The antibody described in (1), wherein the antibody can bind to an extracellular domain of CDH6.
(3) The antibody described in (1) or (2), wherein the antibody is a monoclonal antibody.
(4) The antibody described in any one of (1) to (3), wherein the antibody has antibody-dependent cell-mediated cytotoxicity (ADCC) and/or complement-dependent cytotoxicity (CDC).
(5) The antibody described in any one of (1) to (4), wherein the antibody is a mouse monoclonal antibody, a chimeric monoclonal antibody, or a humanized monoclonal antibody.
(6) The antibody described in any one of (1) to (3), wherein a heavy chain constant region thereof is a heavy chain constant region of human IgG1 and can cause a decrease in ADCC and ADCP activities.
(7) The antibody described in (6), wherein a heavy chain constant region thereof is a heavy chain constant region of human IgG1 and leucine residues at the 234- and 235-positions specified by EU Index numbering are substituted with alanine residues.
(8) The antibody described in (7), wherein the antibody is a humanized monoclonal antibody comprising a heavy chain consisting of the amino acid sequence set forth in SEQ ID NO: 56 and a light chain consisting of the amino acid sequence set forth in SEQ ID NO: 55.
(9) The antibody described in any one of (1) to (3), (6), or (7), wherein the antibody is a humanized monoclonal antibody comprising: a light chain comprising CDRL1 consisting of the amino acid sequence set forth in SEQ ID NO: 63, CDRL2 consisting of the amino acid sequence set forth in SEQ ID NO: 64, and CDRL3 consisting of the amino acid sequence set forth in SEQ ID NO: 65; and a heavy chain comprising CDRH1 consisting of the amino acid sequence set forth in SEQ ID NO: 66, CDRH2 consisting of the amino acid sequence set forth in SEQ ID NO: 67, and CDRH3 consisting of the amino acid sequence set forth in SEQ ID NO: 68.
(10) The antibody described in any one of (1) to (9), wherein one or two amino acids are deleted at a heavy chain carboxyl terminus.
(11) An antibody obtained by a method for producing the antibody, the method comprising the steps of culturing a host cell transfected with an expression vector containing a polynucleotide encoding the antibody described in any one of (1) to (10), and collecting the antibody of interest from the resulting culture obtained in the former step.

### <4-1-2. Antibody glycan remodeling>

It has recently been reported that the heterogeneous glycans of antibodies are remodeled by enzymatic reactions to introduce functionalized glycans uniformly (ACS Chem. Biol. 2012, 7, 110-122, ACS Med. Chem. Lett. 2016, 7, 1005-1008). Using this glycan remodeling technology, attempts have been made to synthesize a homogeneous ADC by introducing a drug(s) in a site-specific manner (Bioconjugate Chem. 2015, 26, 2233-2242; Angew. Chem. Int. Ed. 2016, 55, 2361-2367, US2016361436).

In the glycan remodeling, heterogeneous glycans added to a protein (e.g., an antibody) are cleaved off, using hydrolase, to leave only GlcNAc at each terminus thereby producing a homogenous protein moiety with GlcNAc (hereinafter, referred to as an "acceptor"). Subsequently, a given glycan separately prepared (hereinafter, referred to as a "donor") is provided, and the acceptor and the donor are linked together by using transglycosidase. Thereby, a homogeneous glycoprotein with a given glycan structure can be synthesized.

As used herein, a "glycan" refers to a structural unit of two or more monosaccharides bonded together via glycosidic bonds. Specific monosaccharides and glycans are occasionally abbreviated, for example, as "GlcNAc-", "SG-", and so on. When any of these abbreviations is used in a structural formula, the abbreviation is shown with an intention that an oxygen atom or nitrogen atom involved in a glycosidic bond at the reducing terminal to another structural unit is not included in the abbreviation indicating the glycan, unless specifically defined.

As used herein, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic bond) is defined as the 1-position (the 2-position only for sialic acids), unless otherwise specified. The names of compounds in Examples are each provided in view of the chemical structure as a whole, and that rule is not necessarily applied.

When a glycan is herein indicated as a sign (e.g., SG, MSG, GlcNAc), the sign is intended, unless otherwise defined, to include carbon atoms ranging to the reducing terminal and not to include N or O involved in an N- or O-glycosidic bond.

The antibody-immunostimulant conjugate used for producing an antibody-immunostimulant conjugate of the present invention is represented by the following formula (XXXIV):

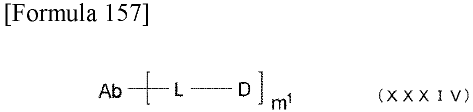

The antibody Ab or its functional fragment is bonded to L directly from a side chain of its amino acid residue (e.g., cysteine, lysine) or from a glycan or remodeled glycan of Ab. The side chain of the amino acid residue may be modified with, for example, an azide group.

Here, Ab denotes an antibody or a functional fragment of the antibody, the antibody optionally having a remodeled glycan,
m¹ ranges from 1 to 10,
L denotes a linker for linking Ab and D,
the linker L is denoted by -Lb-La-Lp-Lc-*
   wherein the asterisk indicates bonding to the immunostimulant D;
   Lp is -GGFG-, where G denotes glycine and F denotes phenylalanine;
   La represents -C(=O)-CH₂CH₂-C(=O)-;
   Lb represents the following formula: 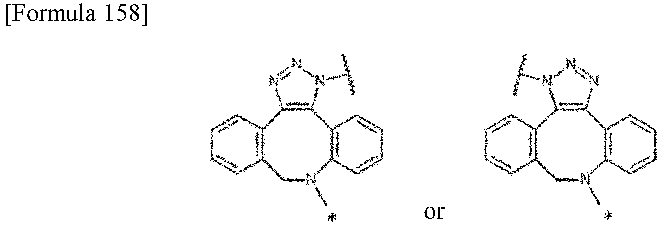
   wherein, in the structural formula for Lb shown above, the asterisk indicates bonding to La, and the wavy line indicates bonding to a glycan or remodeled glycan of Ab;
   Lc represents -NH-CH₂-; and
D represents

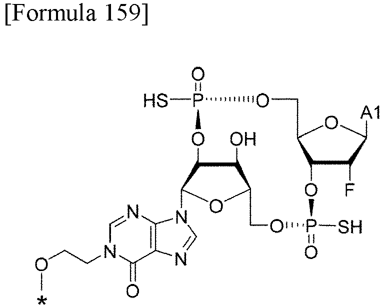

Glycans in Ab herein are N-linked glycans or O-linked glycans, and preferably N-linked glycans.

N-linked glycans and O-linked glycans each bond to an amino acid side chain of an antibody via an N-glycosidic bond and an O-glycosidic bond, respectively.

The Ab herein is IgG, and preferably IgG1, IgG2 or IgG4.

IgG contains a well-conserved N-linked glycan (hereinafter, referred to as "Asn297 glycan or N297 glycan") at the 297th asparagine residue ("Asn297 or N297") in the Fc region of its heavy chain, and the glycan can contribute to the activity and kinetics of the antibody molecule (Eon-Duval, A. et al, Biotechnol. Prog. 2012, 28, 608-622; Sanglier-Cianferani, S., Anal. Chem. 2013, 85, 715-736).

The amino acid sequence in the constant region of IgG is well conserved, and each amino acid is specified by EU Index numbering in Edelman et al. (Proc. Natl. Acad. Sci. U.S.A., 63, 78-85, (1969)). For example, Asn297, to which an N-linked glycan is added, in the Fc region, corresponds to 297-position in the EU numbering, and even if the actual amino acid position changes due to fragmentation or loss of the region of the molecule, the amino acid can be uniquely identified by using the EU numbering.

The diagram below shows the case where in an antibody-immunostimulant conjugate of the present invention, the N297 glycan of the antibody or its functional fragment is linked to L. Here, m² is an integer of 1 or 2.

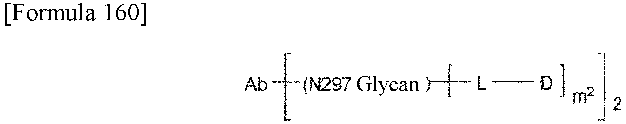

Note that an antibody having a remodeled glycan is called a glycan-remodeled antibody.

SGP (α2,6-SGP), an abbreviation for sialyl glycopeptide, is a representative N-linked glycopeptide. SGP can be separated/purified from the yolk of a hen egg, for example, by using a method described in WO 2011/027868. Purified products of SGP are commercially available from TOKYO CHEMICAL INDUSTRY CO., LTD. or FUSHIMI Pharmaceutical Co., Ltd. As used herein, the glycan moiety of SGP is denoted as SG, and the glycan lacking one GlcNAc at the reducing end of SG is denoted as SG(10). SG(10) can be prepared by enzymatic hydrolysis of SGP (see the report by Umekawa et al. (Biochim. Biophys. Acta 2010, 1800, 1203-1209). SG(10) can also be purchased from TOKYO CHEMICAL INDUSTRY CO., LTD. or FUSHIMI Pharmaceutical Co., Ltd.

As used herein, MSG(9) denotes the glycan structure in which the non-reducing terminal sialic acid is absent in only one of the branched chains of β-Man in SG(10); MSG1 denotes the structure having sialic acid in only 1-3 glycan of the branched chains; and MSG2 denotes the structure having sialic acid in only 1-6 glycan of the branched chains.

The remodeled glycan used in an antibody-immunostimulant conjugate of the present invention is N297-(Fuc)SG, N297-(Fuc)MSG1, N297-(Fuc)MSG2, or a mixture of N297-(Fuc)MSG1 and N297-(Fuc)MSG2. The N297glycan is preferably N297-(Fuc)SG or N297-(Fuc)MSG1 or N297-(Fuc)MSG2, and more preferably N297-(Fuc)SG or N297-(Fuc)MSG1.

N297-(Fuc)SG is represented by the following structural formula or sequence formula:

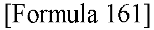

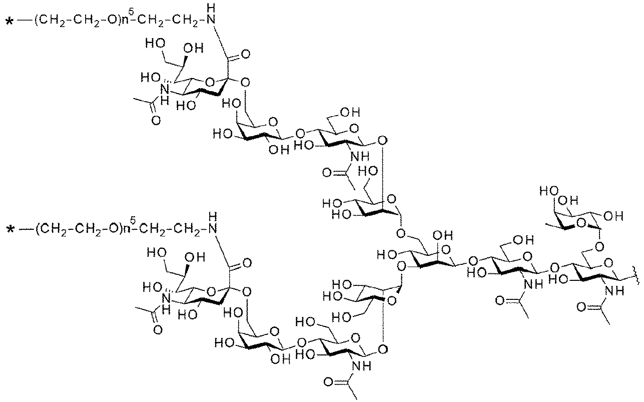

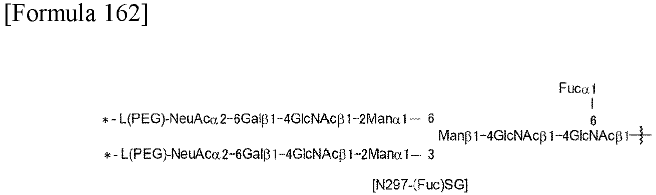

In the above formulas, the wavy line indicates bonding to Asn297 of the antibody;

L(PEG) represents -(CH₂-CH₂-O)n⁵-CH₂-CH₂-NH-, an amino group at a right end of the L(PEG) is bonded through an amide bond to a carboxyl group at 2-position of sialic acid at a non-reducing end of each on both 1-3 and 1-6 glycan sides of branched chains of β-Man in the N297 glycan, and
the asterisk denotes bonding to the nitrogen atom at the 1- or 3-position on 1,2,3-triazole ring of the linker L, in particular, Lb in the above linker L,
where n⁵ is an integer from 2 to 10, and preferably from 2 to 5.

N297-(Fuc)MSG1 is represented by the following structural formula or sequence formula:

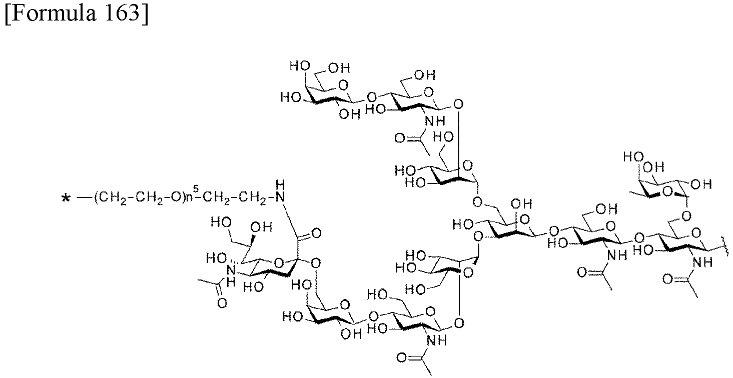

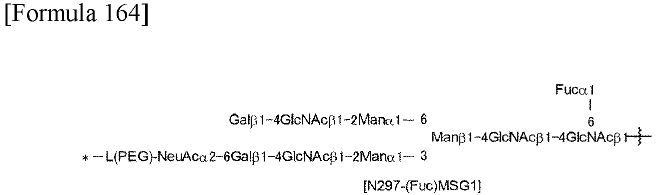

In the above formulas, the wavy line indicates bonding to Asn297 of the antibody;
L(PEG) represents -(CH₂-CH₂-O)n⁵-CH₂-CH₂-NH-, an amino group at a right end of the L(PEG) is bonded through an amide bond to a carboxyl group at 2-position of sialic acid at a non-reducing end on a 1-3 glycan side of branched chains of β-Man in the N297 glycan, and
the asterisk denotes bonding to the nitrogen atom at the 1- or 3-position on 1,2,3-triazole ring of the linker L, in particular, Lb in the above linker L,
where n⁵ is an integer from 2 to 10, and preferably from 2 to 5.

N297-(Fuc)MSG2 is represented by the following structural formula or sequence formula:

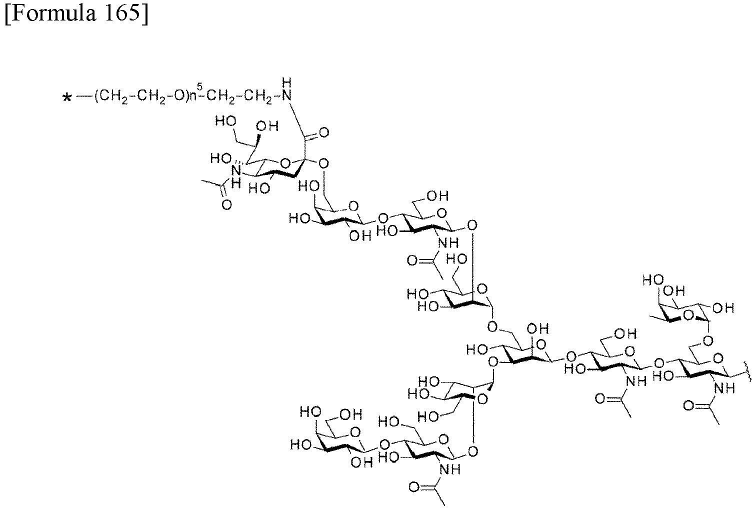

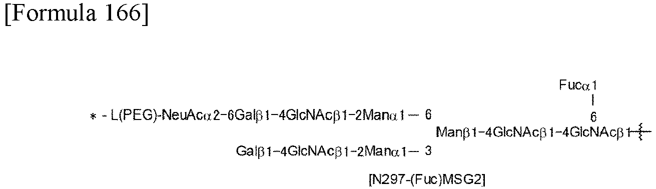

In the above formulas, the wavy line indicates bonding to Asn297 of the antibody;
L(PEG) represents -(CH₂-CH₂-O)n⁵-CH₂-CH₂-NH-, the amino group at the right end of the L(PEG) is bonded through an amide bond to the carboxyl group at the 2-position of the sialic acid at the non-reducing end on the 1-6 glycan side of the branched chains of β-Man in the N297 glycan; and
the asterisk denotes bonding to the nitrogen atom at the 1- or 3-position on 1,2,3-triazole ring of the linker L, in particular, Lb in the above linker L,
where n⁵ is an integer from 2 to 10, and preferably from 2 to 5.

The N297 glycan of the antibody in the antibody-immunostimulant conjugate of the present invention may be N297-(Fuc)SG. In this case, since the antibody is a dimer, the antibody-immunostimulant conjugate is a molecule in which four linkers L and four immunostimulant D are linked (m² = 2).

The N297 glycan of the antibody in the antibody-immunostimulant conjugate of the present invention may be N297-(Fuc)MSG1 or N297-(Fuc)MSG2, or a mixture thereof. In this case, since the antibody is a dimer, the antibody-immunostimulant conjugate is a molecule in which two linkers L and two immunostimulant D are linked (m² = 1) (See Fig. 19).

The N297glycan is preferably N297-(Fuc)SG or N297-(Fuc)MSG1 or N297-(Fuc)MSG2, more preferably N297-(Fuc) SG or N297-(Fuc)MSG1, and still more preferably N297-(Fuc)SG.

The N297 glycan of the antibody in the antibody-immunostimulant conjugate of the present invention may be N297-(Fuc)SG or N297-(Fuc)MSG1 or N297-(Fuc)MSG2. In this case, a highly homogeneous ADC may be obtained.

### <4-2. To produce glycan-remodeled antibody>

A glycan-remodeled antibody can be produced by the method illustrated in the following scheme in accordance with the procedure described in, for instance, WO2018/003983, WO2020/050406, WO2021/177438, or PLos ONE 2018, 13, e0193534.

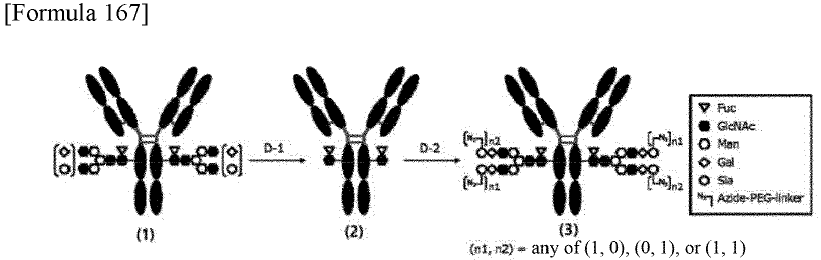

### (Step D-1)

This step is a step of producing a glycan-cleaved antibody by using a known enzymatic reaction to hydrolyze and cleave a glycosidic linkage between GlcNAcβ1-4GlcNAc of the chitobiose structure at the reducing end of the N-linked glycan (N297-linked glycan) attached to asparagine at 297-position of the amino acid sequence of an antibody. The hydrolysis of the glycosidic linkage between GlcNAcβ1 and 4GlcNAc at the reducing end of the chitobiose structure of a desired antibody (1) (10 mg/mL) was carried out using a hydrolase (e.g., wild-type EndoS enzyme) in a buffer solution (e.g., a phosphate buffer) at a temperature of 0°C to 40°C. The reaction time is from 10 min to 72 h and preferably from 1 h to 6 h. The amount of wild-type EndoS enzyme used was from 0.1 mg to 10 mg and preferably from 0.1 mg to 3 mg per 100 mg of the antibody (1). After completion of the reaction, the antibody was purified by affinity chromatography (HiTrap rProtein A FF (5 ml) (manufactured by GE Healthcare)) and/or hydroxyapatite column (Bio-Scale Mini CHT Type I cartridge (5 ml) (manufactured by BIO-RAD)). A (Fucα1,6)GlcNAc antibody (2) was thus obtained.

### (Step D-2)

This step is a step of producing a glycan-remodeled antibody (3) by using a known enzymatic reaction to link the (Fucα1,6)GlcNAc antibody (2) obtained in step D-1 with an SG- or MSG (MSG1, MSG2)-type glycan oxazoline moiety having an azide group-containing PEG linker (hereinafter referred to as a "azido-glycan oxazoline moiety").

The glycosyltransfer was conducted by reacting the antibody (2) with the azido-glycan oxazoline moiety in the presence of glycosyltransferase (e.g., EndoS (D233Q/Q303L)) in a buffer solution (e.g., a phosphate buffer) at a temperature of 0°C to 40°C. The reaction time is from 10 min to 72 h and preferably from 1 h to 6 h. The amount of the EndoS enzyme (D233Q/Q303L) used was from 1 mg to 10 mg and preferably from 1 mg to 3 mg per 100 mg of the antibody; and 2 to excess equivalents and preferably 4 to 20 equivalents of the azido-glycan oxazoline moiety was used. After completion of the reaction, the antibody was purified by affinity chromatography (HiTrap rProtein A FF (5 ml) (manufactured by GE Healthcare)) and hydroxyapatite column (Bio-Scale Mini CHT Type I cartridge (5 ml) (manufactured by BIO-RAD)). A glycan-remodeled antibody (3) was thus obtained.

In the above preparation of the glycan-remodeled antibody, the below-described common protocols A to C may be used to concentrate the antibody aqueous solution, measure the concentration, and exchange the buffer.

Note that the SG-type azido-glycan oxazoline moiety was synthesized according to the procedure described in WO2018/003983. As an example, how to synthesize [N₃-PEG(3)]₂-SG(10)-Ox (compound 1-10 as described in WO2018/003983) is illustrated in the following scheme.

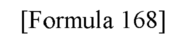

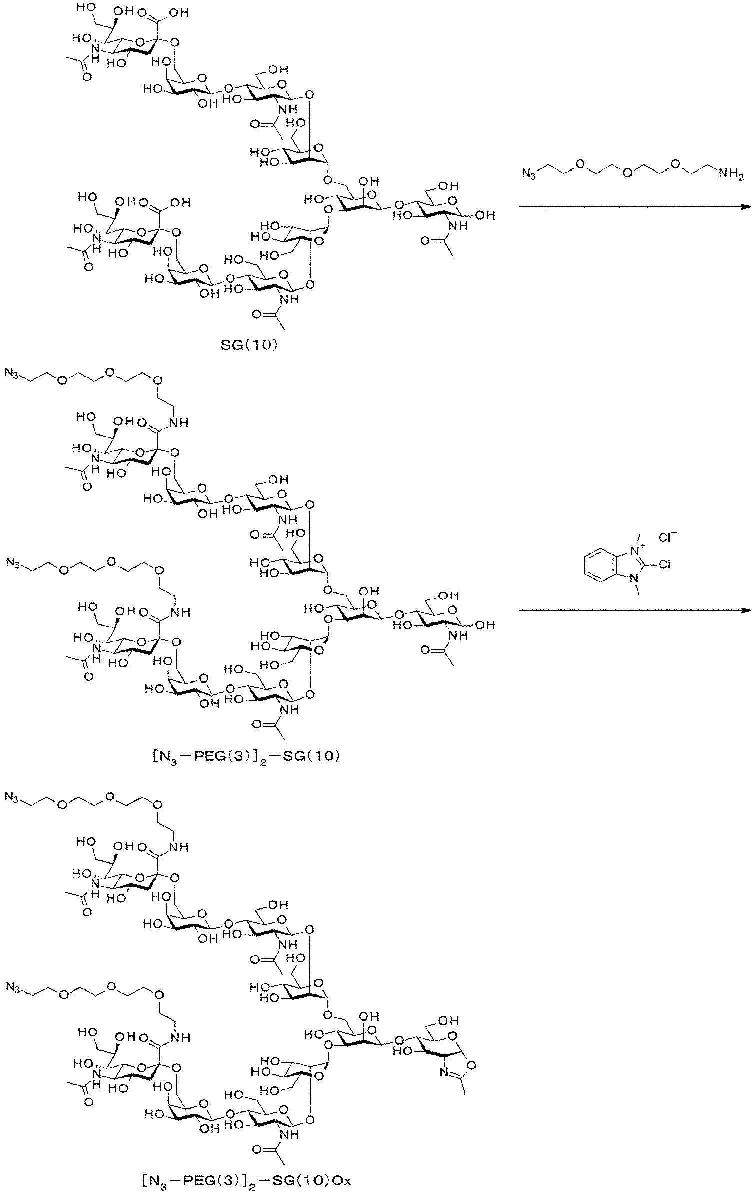

The MSG-type azido-glycan oxazoline moiety was also synthesized according to the procedure described in WO2018/003983. As an example, how to synthesize [N₃-PEG(3)]₂-MSG1(9)-Ox (compound 1-11 described in WO2018/003983) is illustrated in the following scheme.

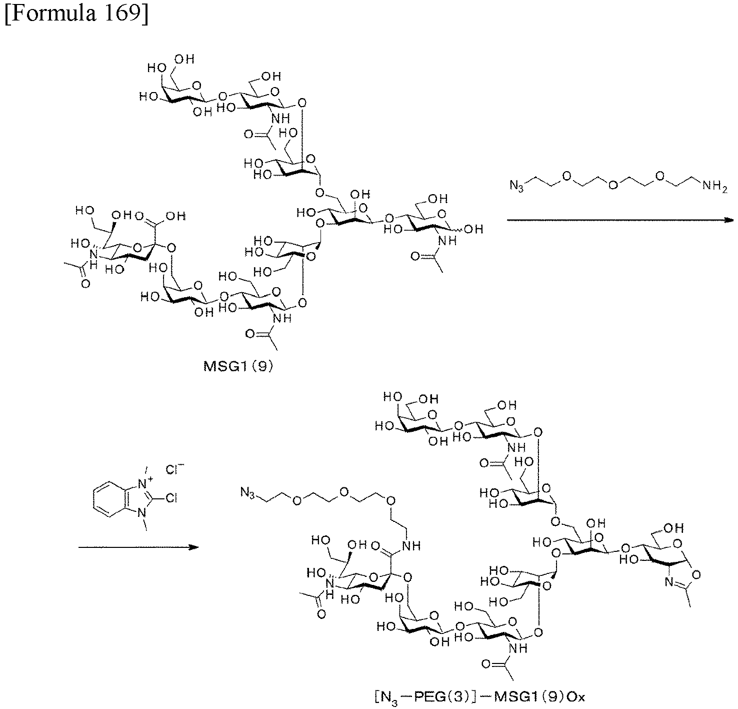

### (Step D-3)

This step is a step of subjecting the (Fucα1,6)GlcNAc antibody (2) obtained in step D-1 to a glycosyltransfer reaction using two types of Endo enzymes to produce a glycan-remodeled antibody (3). The two types of enzymes may be used simultaneously to directly transfer a glycan to the N297 glycan of the antibody while SGP and/or (SG)Asn, the reducing ends of which are not activated, is used as a glycan donor. For the two types of Endo enzymes used, enzyme A (EndoM-like enzyme) and enzyme B (EndoS-like enzyme) can be used in combination, if appropriate.

Examples of the enzyme A include EndoM, EndoOm, or EndoCC, or an EndoM, EndoOm, or EndoCC mutant with reduced hydrolytic activity. The preferred enzyme A is EndoM N175Q, EndoCC N180H, or EndoOm N194Q.

Examples of the enzyme B include EndoS or EndoS2 (EndoS49), or an EndoS or EndoS2(EndoS49) mutant with reduced hydrolytic activity. The preferred enzyme B is, for instance, EndoS D233Q, EndoS D233Q/Q303L, EndoS D233Q/E350A, EndoS D233Q/E350Q, EndoS D233Q/E350D, EndoS D233Q/E350N, EndoS D233Q/D405A, EndoS2 D184M, or EndoS2 T138Q.

The glycan donor used may be, for instance, ([N₃-PEG(3)]₂-SG)-Asn-PEG(3)-N₃, [N₃-PEG(3)]-MSG1-Asn-PEG(3)-N₃, or [N₃-PEG(3)]-MSG2-Asn-PEG(3)-N₃.

The antibody (2) was reacted with ([N₃-PEG(3)]₂-SG)-Asn-PEG(3)-N₃ in the presence of glycosyltransferases, enzyme A (EndoM-like enzyme) and enzyme B (EndoS-like enzyme), in a buffer solution (e.g., tris buffered saline) for glycosyltransfer. The reaction temperature can be selected, if appropriate, according to the optimum temperature for the enzymes used, but is usually from 15 to 50°C and preferably from 25 to 40°C. The reaction time can be selected from 2 to 48 hours.

After the reaction, a purification method (e.g., affinity chromatography, hydroxyapatite column) and an ultrafiltration method (ultrafiltration membrane) suitable for the scale of the reaction were selected to obtain a glycan remodeled antibody (3).

In the above preparation of the glycan-remodeled antibody, the below-described common protocols A to C may be used to concentrate the antibody aqueous solution, measure the concentration, and exchange the buffer.

Note that ([N₃-PEG(3)]₂-SG)-Asn-PEG(3)-N₃ was synthesized according to the method described in WO2018/003983. In step 1-2A described in WO2018/003983, Fmoc-(SG-)Asn-free form, which was prepared from Fmoc-(SG-)Asn (1S2S-11NC-Asn-Fmoc, manufactured by GlyTech, Inc.), was reacted with 11-azido-3,6,9-trioxaundecan-1-amine to form ([N₃-PEG(3)]₂-SG)-Asn-PEG(3)-N₃ (compound 1-13 as described in WO2018/003983).

The MSG-type glycan donor [N₃-PEG(3)]-MSG1-Asn-PEG(3)-N₃ or [N₃-PEG(3)]-MSG2-Asn-PEG(3)-N₃ can also be synthesized according to the method described in steps 1 to 3 of Example 154 in WO2019065964.

### <4-3. To conjugate antibody and immunostimulant>

The antibody-immunostimulant conjugate of formula (Rp,Rp-XIII) can be produced according to the following method. 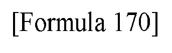 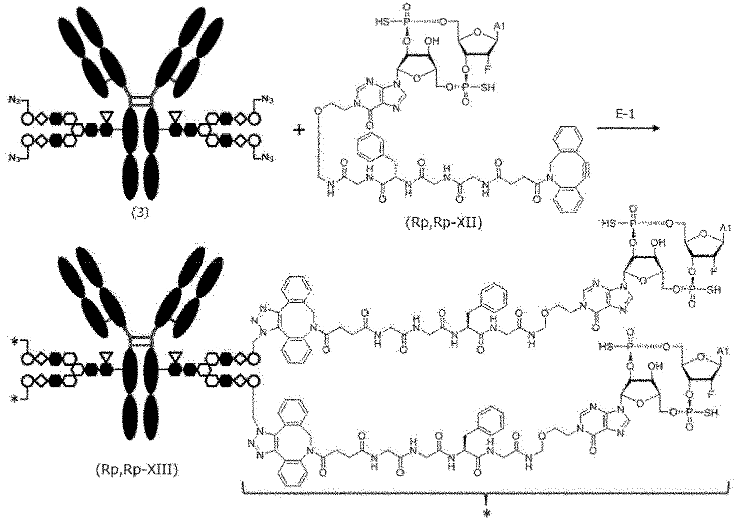 wherein the two asterisks (*) on the left side of the antibody-immunostimulant conjugate (Rp,Rp-XIII) each indicate a CDN-linker portion denoted by the asterisk on the right side.

The antibody-immunostimulant conjugate (Rp,Rp-XIII) can be produced by subjecting the glycan-remodeled antibody (3) and the CDN conjugate precursor (Rp,Rp-XII) to a cycloaddition reaction for conjugation. Examples of the cycloaddition reaction include the Diels-Alder reaction or 1,3-dipolar cycloaddition reaction. The 1,3-dipolar cycloaddition reaction may be preferably used for the production. Examples of the 1,3-dipolar cycloaddition reaction include a cycloaddition reaction of azide with terminal alkyne or SPAAC (strain-promoted azide-alkyne cycloaddition: J. Am. Chem. Soc. 2004, 126, 15046-15047). The SPAAC reaction may be preferably used for the production.

This production method is a method for producing an antibody-immunostimulant conjugate (Rp,Rp-XIII) by subjecting the glycan-remodeled antibody (3) and the CDN conjugate precursor (Rp,Rp-XII) as obtained in the above steps D-2 or D-3 to an SPAAC reaction for conjugation.

### (Step E-1) (Step a9)

The SPAAC reaction was carried out by mixing the glycan-remodeled antibody (3)-containing buffer solution (e.g., phosphate buffer, acetate buffer, borate buffer) with a solution in which the CDN conjugate precursor (Rp,Rp-XII) had been dissolved in an appropriate solvent (dimethyl sulfoxide, N,N-dimethylformamide, N,N-dimethylacetamide, N-methylpyrrolidone, propylene glycol, or a mixed solvent thereof). For each mole of the glycan-remodeled antibody (3), 2 to excess moles and preferably 4 to 30 moles of the CDN conjugate precursor (Rp,Rp-XII) are used; and the percentage of the organic solvent is preferably from 1% to 200% (v/v) based on the antibody buffer solution. The reaction temperature is from 0°C to 37°C and preferably from 15°C to 25°C, and the reaction time is from 1 hour to 150 hours and preferably from 6 hours to 72 hours. The pH of the reaction solution is preferably from 5 to 9. The reaction solution was subjected to purification by the procedure described in common protocol D to obtain the antibody-immunostimulant conjugate (Rp,Rp-XIII).

The below-described common protocols D to G may be used for the antibody-immunostimulant conjugate to exchange the buffer, purify the conjugate, measure the antibody concentration, and measure the average number of immunostimulants conjugated per antibody molecule. In this way, the antibody-immunostimulant conjugate can be identified.

### Common Protocol A: To concentrate antibody aqueous solution

An antibody or antibody-immunostimulant conjugate solution was placed in an Amicon (registered trademark) Ultra centrifugal filter device (50,000 NMWL, Merck Millipore, Ltd.) and centrifuged (at 2000 G to 4000 G for 5 to 20 min) using a centrifuge (Allegra X-15R Beckman Coulter, Inc.). In this way, the antibody or antibody-immunostimulant conjugate solution was concentrated.

### Common Protocol B: To measure antibody concentration

The antibody concentration was measured using a UV meter (Nanodrop 1000, Thermo Fisher Scientific, Inc.) in accordance with the protocol specified by the manufacturer. At that time, the absorbance coefficients at 280 nm (1.3 mLmg⁻¹ cm⁻¹ to 1.8 mLmg⁻¹ cm⁻¹) were different for each antibody.

### Common Protocol C: To exchange buffer for antibody

A buffer solution (e.g., phosphate buffered saline (pH 6.0), phosphate buffer (pH 6.0)) was added to the antibody aqueous solution. The mixture was concentrated according to the protocol described in common protocol A. The operation was repeated several times, and the antibody concentration was then measured by the protocol described in common protocol B. A buffer solution (e.g., phosphate buffered saline (pH 6.0), phosphate buffer (pH 6.0)) was added, if appropriate, to the antibody buffer solution. In this way, the antibody buffer solution at a desired concentration (e.g., about 10 mg/mL) was prepared.

### Common Protocol D: To purify antibody-immunostimulant conjugate (gel filtration chromatography)

Each NAP column (NAP-5, NAP-10, or NAP-25 (manufactured by GE Healthcare)) was equilibrated with acetate buffer (10 mM acetate buffer, 5% sorbitol, pH 5.5; herein referred to as ABS) or another suitable buffer solution. The NAP column was charged with the antibody-immunostimulant conjugate reaction solution. A buffer solution at a volume specified by the manufacturer was allowed to flow down. Then, the antibody fractions were collected. The fractions were recharged into the NAP column. Subsequently, a buffer solution at a volume specified by the manufacturer was allowed to flow down, and the antibody fractions were then collected. This operation was repeated a total of 2 to 3 times to yield an antibody-immunostimulant conjugate from which an unbound immunostimulant linker, dimethyl sulfoxide, and propyleneglycol had been removed. The concentration of the antibody-immunostimulant conjugate solution was optionally adjusted using common protocols A and C.

Common Protocol E: To measure antibody concentration and average number of immunostimulants conjugated per antibody molecule in antibody-immunostimulant conjugate (UV method).

The concentration of immunostimulant conjugated in the antibody-immunostimulant conjugate can be calculated by using a spectrophotometer (UV/VIS Spectrometer Lambda 25, PerkinElmer, Inc.) to measure absorbance of the aqueous solution of the antibody-immunostimulant conjugate at two wavelengths, 280 nm and 250 nm, according to the method described in WO2020/050406 or WO2021/177438.

Common Protocol F: To measure antibody concentration and average number of immunostimulants conjugated per antibody molecule in antibody-immunostimulant conjugate (reverse-phase high performance liquid chromatography method: RP-HPLC).

The antibody concentration and the average number of immunostimulants conjugated per antibody molecule in the antibody-immunostimulant conjugate can be calculated by the above-described common protocol E as well as high performance liquid chromatography analysis according to the method described in WO2020/050406 or WO2021/177438.

Common Protocol G: To measure antibody concentration and average number of immunostimulants conjugated per antibody molecule in antibody-immunostimulant conjugate (hydrophobic interaction-high performance liquid chromatography method: HI-HPLC).

The antibody concentration and the average number of immunostimulants conjugated per antibody molecule in the antibody-immunostimulant conjugate can be calculated by the above-described common protocols E and F as well as high performance liquid chromatography analysis according to the method described in WO2020/050406 or WO2021/177438.

The antibody-immunostimulant conjugate or its production intermediate as produced by the method of the present invention may contain stereoisomers, optical isomers caused by asymmetric carbon atoms, geometric isomers, tautomers, or optical isomers (e.g., d-, l-, or atrop-isomers). However, all of these isomers, optical isomers, and mixtures of them are included in the present invention.

In the antibody-immunostimulant conjugate produced by the method of the present invention, the number of immunostimulants conjugated per antibody molecule is an important factor affecting the efficacy and safety of the conjugate. The antibody-immunostimulant conjugate is produced by specifying the reaction conditions (e.g., the amounts of raw materials or reagents to be used in the reaction), so that the number of immunostimulants conjugated can be constant. However, unlike the chemical reaction using small molecule compounds, a mixture, in which the number of immunostimulants conjugated is different, is usually obtained. The number of immunostimulants conjugated per antibody molecule can be specified as an average value, i.e., the average number of immunostimulants conjugated (DAR: Drug to Antibody Ratio). The number of cyclic dinucleotide derivatives conjugated to the antibody molecule is controllable. Here, the number of cyclic dinucleotide derivatives conjugated may be in the range of 1 to 10 as the number of immunostimulants conjugated per antibody. The number is preferably from 1 to 8 and more preferably from 1 to 5.

In an antibody-immunostimulant conjugate produced by the method of the present invention, the antibody Ab may be provided such that the remodeled glycan of the antibody Ab is linked to L. In this case, m², which indicates the number of immunostimulants conjugated per antibody molecule in the antibody-immunostimulant conjugate, is an integer of 1 or 2. The glycan may be N297 glycan that is N297-(Fuc)SG. In this case, m² is 2, and DAR is in the range of 3 to 5 (preferably in the range of 3.2 to 4.8, and more preferably in the range of 3.5 to 4.2). The N297 glycans may be N297-(Fuc)MSG1, N297-(Fuc)MSG2, or a mixture of N297-(Fuc)MSG1 and N297-(Fuc)MSG2. In this case, m² is 1, and DAR is in the range of 1 to 3 (preferably in the range of 1.0 to 2.5, and more preferably in the range of 1.2 to 2.2).

Note that a person skilled in the art can design, from the description of the Examples in the present application, a reaction in which the necessary number of immunostimulants can be conjugated to the antibody. Thus, it is possible to obtain an antibody in which the number of cyclic dinucleotide derivatives conjugated is controlled.

Note that an antibody-immunostimulant conjugate or its production intermediate as produced by the method of the present invention may be left in the air or recrystallized. This may cause moisture absorption, moisture adsorption, or a hydrate. Such a moisturized compound and a salt thereof are also included in the present invention.

The antibody-immunostimulant conjugate or its production intermediate as produced by the method of the present invention may be converted into a pharmaceutically acceptable salt, as desired, if having a basic group such as an amino group. Examples of such salts may include, hydrogen halide salts such as hydrochlorides and hydroiodides; inorganic acid salts such as nitrates, perchlorates, sulfates, and phosphates; lower alkanesulfonates such as methanesulfonates, trifluoromethanesulfonates, and ethanesulfonates; arylsufonates such as benzenesulfonates and p-toluenesulfonates; organic acid salts such as formates, acetates, malates, fumarates, succinates, citrates, tartrates, oxalates, and maleates; and amino acid salts such as omithinates, glutamates, and aspartates.

The antibody-immunostimulant conjugate or its production intermediate as produced by the method of the present invention contains a phosphate group and/or a thiophosphate group in its structure, so that a base addition salt can be generally formed. In addition, if the production intermediate has an acidic group such as a carboxy group, it is generally possible to form a base addition salt. Examples of pharmaceutical acceptable salts may include, alkali metal salts such as sodium salts, potassium salts, and lithium salts; alkali earth metal salts such as calcium salts and magnesium salts; inorganic salts such as ammonium salts; and organic amine salts such as dibenzylamine salts, morpholine salts, phenylglycine alkyl ester salts, ethylenediamine salts, N-methylglucamates, diethylamine salts, triethylamine salts, *tert*-butylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts, and tris(hydroxymethyl)aminomethane salts.

The antibody-immunostimulant conjugate or its production intermediate as produced by the method of the present invention may exist as a hydrate, for example, by absorbing moisture in the air. The solvate in the present invention is not limited to a particular solvate and may be any pharmaceutically acceptable solvate, and specifically hydrates, ethanol solvates, 2-propanol solvates, and so on are preferred. The antibody-immunostimulant conjugate or its production intermediate in the present invention may be in an N-oxide form if a nitrogen atom is present therein, and these solvates and N-oxide forms are included in the scope of the present invention. In addition, the antibody-immunostimulant conjugate or its production intermediate in the present invention may be in an sulfoxide form if a sulfur atom is present therein, and these solvates and sulfoxide forms are included in the scope of the present invention.

The antibody-immunostimulant conjugate and its production intermediate as produced by the method of the present invention also includes a compound labeled with each radioactive or non-radioactive isotope. The antibody-immunostimulant conjugate or its production intermediate as produced by the method of the present invention may contain one or more constituent atoms in which atomic isotopes are present at a non-natural ratio. Examples of atomic isotopes may include deuterium (2H), tritium (3H), iodine-125 (125I), and carbon-14 (14C). The compound in the present invention may be radiolabeled with a radioactive isotope such as tritium (3H), iodine-125 (125I), or carbon-14 (14C). The radiolabeled compound is useful as a therapeutic or prophylactic agent, a reagent for research such as an assay reagent, and a diagnostic agent such as a diagnostic agent for *in vivo* imaging. Isotopic variants of the antibody-immunostimulant conjugate of the present invention are all included in the scope of the present invention, regardless of whether they are radioactive or not.

### <5. Medicine>

The antibody-immunostimulant conjugate as produced by the method of the present invention exhibits anti-tumor immunity or cytotoxicity against cancer cells. Thus, the conjugate can be used as a medicine, especially as a therapeutic and/or prophylactic agent against cancer, or as an anti-tumor agent.

Examples of the type of cancer indicated for the antibody-immunostimulant conjugate produced by the method of the present invention include lung cancer (e.g., non-small cell lung cancer, small cell lung cancer), renal cancer, urothelial cancer, colorectal cancer, prostate cancer, glioblastoma multiforme, ovarian cancer (e.g., superficial epithelial tumor, stromal tumor, germ cell tumor), pancreatic cancer, breast cancer, melanoma, liver cancer, bladder cancer, gastric cancer, esophageal cancer, endometrial cancer, testicular cancer (seminoma, non-seminoma), cervical cancer, placental choriocarcinoma, brain tumor, head and neck cancer, thyroid cancer, mesothelioma, Gastrointestinal Stromal Tumor (GIST), gall bladder cancer, bile duct cancer, adrenal cancer, pharyngeal cancer, tongue cancer, auditory organ cancer, thymus cancer, small intestine cancer, squamous-cell carcinoma, leukemia, malignant lymphoma, plasmacytoma, myeloma, or sarcoma. However, the antibody-immunostimulant conjugate is not limited to the above cancer cells as long as cancer cells of treatment subject express a protein that can be recognized by the antibody in the antibody-immunostimulant conjugate.

The antibody-immunostimulant conjugate produced by the method of the present invention can be preferably administered to mammals, and are more preferably administered to humans.

Substances used in a pharmaceutical composition containing the antibody-immunostimulant conjugate produced by the method of the present invention may be suitably applied after selected from formulation additives or the like that are generally used in the field in view of the dose or concentration for administration.

The antibody-immunostimulant conjugate produced by the method of the present invention may be administered as a pharmaceutical composition containing one or more pharmaceutically applicable components. For example, the pharmaceutical composition typically contains one or more pharmaceutical carriers (e.g., sterilized liquid (including water and oil (petroleum oil and oil of animal origin, plant origin, or synthetic origin (such as peanut oil, soybean oil, mineral oil, and sesame oil)))). Water is a more typical carrier when the pharmaceutical composition above is intravenously administered. Saline solution, an aqueous dextrose solution, and an aqueous glycerol solution can be also used as a liquid carrier, in particular, for an injection solution. Suitable pharmaceutical vehicles are known in the art. If desired, the composition above may also contain a trace amount of a moisturizing agent, an emulsifying agent, or a pH buffering agent. Examples of suitable pharmaceutical carriers are disclosed in "Remington's Pharmaceutical Sciences" by E.W. Martin. The formulations correspond to the administration mode.

Various delivery systems are known, and they may be used for administering the antibody-immunostimulant conjugate of the present invention. Examples of the administration route may include, but not limited to, intradermal, intramuscular, intraperitoneal, intravenous, and subcutaneous routes. The administration may be made by injection or bolus injection, for example. According to a specific preferred embodiment, the administration of the above antibody-immunostimulant conjugate is done by injection. Parenteral administration is a preferred administration route.

According to a representative embodiment, the pharmaceutical composition containing the antibody-immunostimulant conjugate is prescribed, as a pharmaceutical composition suitable for intravenous administration to humans, according to conventional procedures. The composition for intravenous administration is typically a solution in a sterile and isotonic aqueous buffer. If necessary, the medicine may contain a solubilizing agent and a local anesthetic to alleviate pain at an injection site (e.g., lignocaine). Generally, the ingredients above are provided either individually as a dried lyophilized powder or an anhydrous concentrate contained in each container which is obtained by sealing in an ampoule or a sachet with indication of the amount of the active agent, or as a mixture in a unit dosage form. When the pharmaceutical composition is to be administered by injection, it may be administered from an injection bottle containing water or saline of sterile pharmaceutical grade. When the medicine is administered by injection, an ampoule of sterile water or saline for injection may be provided so that the aforementioned ingredients are admixed with each other before administration. The above pharmaceutical composition may be provided as a solution.

A pharmaceutical composition containing an antibody-immunostimulant conjugate produced by the method of the present invention may be a pharmaceutical composition containing only an antibody-immunostimulant conjugate produced by the method of the present invention. The pharmaceutical composition may contain an antibody-immunostimulant conjugate produced by the method of the present invention and another cancer treating agent. The antibody-immunostimulant conjugate produced by the method of the present invention may be administered in combination with other cancer treating agents, and thereby the anti-tumor effect may be enhanced. Other cancer treating agents used for such purpose may be administered to an individual simultaneously with, separately from, or subsequently to the antibody-immunostimulant conjugate, and may be administered while varying the administration interval for each. Examples of such cancer treating agents may include antimetabolites, alkylating agents, microtubule inhibitors, and other chemotherapeutic agents (e.g., abraxane, carboplatin, cisplatin, gemcitabine, irinotecan (CPT-11), paclitaxel, docetaxel, pemetrexed, vinblastin, agents described in International Publication No. WO 2003/038043), hormone regulators (e.g., LH-RH analogs (e.g., leuprorelin, goserelin, estramustine), estrogen antagonists (e.g., tamoxifen, raloxifene)), aromatase inhibitors (e.g., anastrozole, letrozole, exemestane), kinase inhibitors, PARP inhibitors, bone destruction inhibitors, osteogenesis promoters, metastasis inhibitors, molecular target drugs (e.g., an anti-EGFR antibody, an anti-VEGF antibody, an anti-VEGFR antibody), immune checkpoint inhibitors (e.g., an anti-PD-1 antibody (e.g., nivolumab, pembrolizumab), an anti-PD-L1 antibody (e.g., atezolizumab, avelumab, durvalumab), an anti-PD-L2 antibody, an anti-CTLA4 antibody (e.g., ipilimumab), an anti-A2aR antibody, an A2a receptor antagonist, an anti-LAG3 antibody, an anti-TIM3 antibody), anti-regulatory T-cell drugs (e.g., an anti-CTLA4 antibody, an anti-CD25 antibody, an anti-GITR antibody, an anti-GARP antibody, an anti-TIGIT antibody, an anti-CCR8 antibody), immune activators (e.g., an anti-4-1BB antibody, an anti-OX40 antibody, an anti-CD40 antibody, an anti-CD3 antibody, an anti-CD28 antibody, an IL-2 analog, cytokines, an TLR agonist), immunomodulators (e.g., an anti-CD47 antibody, an anti-SIRPα antibody, inhibitory myeloid modulators), antibody medicines with ADCC (Antibody Dependent Cellular Cytotoxicity) activity, ADCP (Antibody Dependent Cellular Phagocytosis) activity, and/or complement activity, BiTE (Bi-specific T-cell engagers), antibody-drug-conjugates (ADCs) (e.g., a drug conjugate containing Deruxtecan, DM1, pyrrolobenzodiazepine, MMAF, etc. (an anti-HER2-ADC, an anti-TROP2-ADC, an anti-HER3-ADC, etc.)), ADCs in combination with photodynamic therapy, as well as anti-tumor vaccines, anti-tumor cell therapy (e.g., CAR-T, TCR-T, dendritic cells, NK cells), anti-tumor bacterial treatment, or anti-tumor viral treatment. However, the cancer treating agents are not limited if the agents have antitumor activity. The antibody-immunostimulant conjugate of the present invention may be administered in combination with another antibody-immunostimulant conjugate of the present invention, and thereby the anti-tumor effect may be enhanced. Besides, the antibody-immunostimulant conjugate of the present invention can enhance anti-tumor effects not only by the immunostimulant alone, but also by use in combination with anti-tumor effect-exerting treatment (e.g., radiation, heavy particle radiation, surgery, bone marrow transplantation). The treatment is not limited if having anti-tumor effects.

The pharmaceutical composition can be formulated into a lyophilization formulation or a liquid formulation as a formulation having the selected composition and required purity. When formulated as a lyophilization formulation, it may be a formulation containing suitable formulation additives that are used in the art. Also, for a liquid formulation, it may be formulated as a liquid formulation containing various formulation additives that are used in the art.

The composition and concentration of the pharmaceutical composition may vary depending on the administration method. However, the antibody-immunostimulant conjugate contained in the pharmaceutical composition containing the antibody-immunostimulant conjugate produced by the method of the present invention can exhibit a pharmaceutical effect even at a small dosage when the antibody-immunostimulant conjugate has a higher affinity for an antigen, that is, a higher affinity (lower Kd value) in terms of the dissociation constant (Kd value) for the antigen. Thus, for determining the dosage of the antibody-immunostimulant conjugate, the dosage may be set in view of the situation relating to the affinity of the antibody-immunostimulant conjugate toward the antigen. When the antibody-immunostimulant conjugate of the present invention is administered to a human, for example, about 0.001 to 100 mg/kg can be administered once or administered in several portions with intervals of 1 to 180 days.

Hereinbelow, the present invention will be described with reference to Examples. However, the present invention is not limited to them. In the following, the term "compound (I)" or "(I)" in the synthesis scheme indicates that it corresponds to "compound of formula (I)", and the same applies to subsequent compound numbers.

### Examples

In the following Examples, the room temperature is from 15°C to 35°C, unless otherwise specified. Dehydrated dichloromethane used was dichloromethane (super dehydrated) sold by FUJIFILM Wako Pure Chemical Corporation. Dehydrated acetonitrile used was acetonitrile (super dehydrated) sold by FUJIFILM Wako Pure Chemical Corporation. Dehydrated pyridine used was pyridine (dehydrated) sold by KANTO CHEMICAL CO., INC. Silica gel chromatography was performed using Biotage Sfar HC D (20 µm; manufactured by Biotage), amino silica gel column chromatography was performed using Biotage Sfar Amino D (50 µm; manufactured by Biotage), and preparative HPLC was performed using Agilent Preparative HPLC System (manufactured by Agilent Technology). The preparative column used was XBridge Prep OBD (5 µm, C18, 130 A, 250 × 30 mm; manufactured by Waters).

The following instruments were used to measure various spectral data. ¹H-NMR and ³¹P-NMR spectra were measured using JEOL ECZ500R. Mass spectra were measured using Shimadzu LCMS-2010 and LCMS-2020 (manufactured by Shimadzu Corporation). LC/MS measurements were performed under the following conditions [column: XBridge C18, 3.5 µm, 150 × 4.6 mm (manufactured by Waters); mobile phase: 10 mM ammonium acetate/acetonitrile, acetonitrile: 10%-97.5% (0 min-42 min)]

### [A. To synthesize cyclic dinucleotide (CDN)]

### A-1 Synthesis 1 of CDN1

A cyclic dinucleotide (CDN1) was synthesized according to the following synthesis scheme A-1. In synthesis scheme A-1, A2 = a Bz-tetraazabenzo[cd]azulene group, Q = a thiol group, PG1 = TBS, PG2 = DMTr, PG3 = Teoc, and PG6 = Ac. (3aR)-1-Chlorotetrahydro-1H,3H-pyrrolo[1,2-c][1,3,2]oxazaphosphole was used as (Rc-II).

### [Synthesis Scheme A-1]

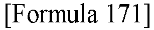

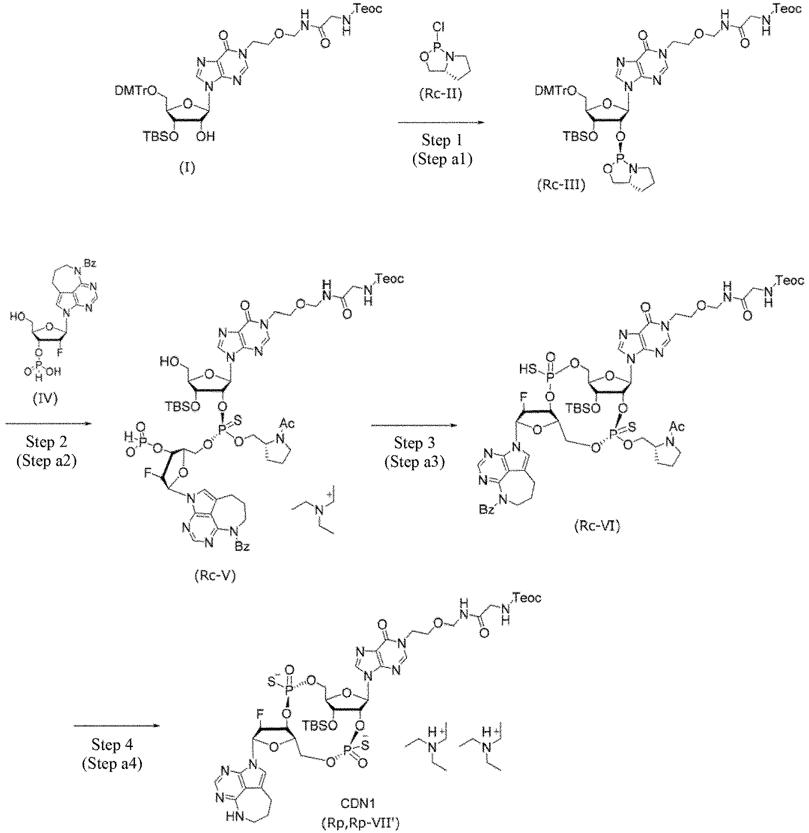

### Example 1:

### Synthesis of 5'-O-[bis(4-methoxyphenyl)(phenyl)methyl]-3'-O-[tert-butyl(dimethyl)silyl]-2'-O-[(1S,3aR)-tetrahydro-1H,3H-pyrrolo[1,2-c][1,3,2]oxazaphosphol-1-yl]-1-(2- f [(N- f [2-(trimethylsilyl)ethoxy]carbonyl}glycyl)amino]methoxy}ethyl)inosine (corresponding to a compound of formula (Rc-III))

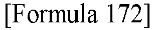

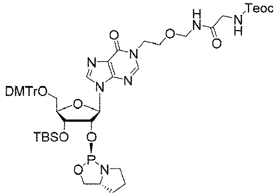

### (Step 1)

Dehydrated dichloromethane (150 mL) was added to 5'-O-[bis(4-methoxyphenyl)(phenyl)methyl]-3'-O-[tert-butyl(dimethyl)silyl]-1-(2- f [(N- f [2-(trimethylsilyl)ethoxy]carbonyl}glycyl)amino]methoxy}ethyl)inosine (10.0 g). The mixture was cooled to -5°C and triethylamine (1.73 mL) was added thereto. To the above solution was added dropwise (3aR)-1-chlorotetrahydro-1H,3H-pyrrolo[1,2-c][1,3,2]oxazaphosphole-containing dehydrated dichloromethane (50 mL) as described in a known publication (Tetrahedron Letter, 1998, 39, 2491-2494). The mixture was stirred at the same temperature for 30 minutes. The temperature was raised to room temperature, the mixture was stirred for 30 minutes, and the reaction solution was then concentrated. The concentrated crude product was dissolved and diluted in dichloromethane and purified by amino silica gel chromatography [dichloromethane/ethyl acetate] to obtain the target compound (10.4 g).
MS (ESI) m/z: 1088(M+H)⁺, 1086(M-H)⁻.
¹H-NMR (MeCN-d₃) δ: 7.94 (1H, s), 7.93 (1H, s), 7.44-7.42 (3H, m), 7.31 (4H, d, J = 9.0 Hz), 7.27 (2H, t, J = 7.0 Hz), 7.21 (1H, t, J = 7.0 Hz), 6.84 (4H, d, J = 7.0 Hz), 5.88 (1H, d, J = 6.5 Hz), 5.83 (1H, brm), 4.94 (1H, m), 4.56 (2H, m), 4.33 (1H, m), 4.17-4.02 (6H, m), 3.74 (6H, s), 3.69 (2H, m), 3.61-3.57 (3H, m), 3.43 (1H, dd, J = 5.0 Hz), 3.28-3.19 (3H, m), 2.48-2.44 (1H, m), 1.65-1.49 (3H, m) 1.26-1.17 (1H, m), 0.94-0.90 (2H, m), 0.87 (9H, s), 0.10 (3H, s), 0.06 (3H, s), 0.00 (9H, s).
³¹P-NMR (MeCN-d₃) δ: 151.93.

### Example 2:

### Synthesis of N,N-diethylethanaminium(2R,3R,4R,5R)-2-({[(R)-{[(2R)-1-acetylpyrrolidin-2-yl]methoxy}{[(2R,3R,4R,5R)-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy}phosphorothioyl]oxy}methyl)-5-(6-benzoyl-6,7,8,9-tetrahydro-2H-2,3,5,6-tetraazabenzo[cd]azulene-2-yl)-4-fluorooxolan-3-yl phosphate (corresponding to a compound of formula (Rc-V))

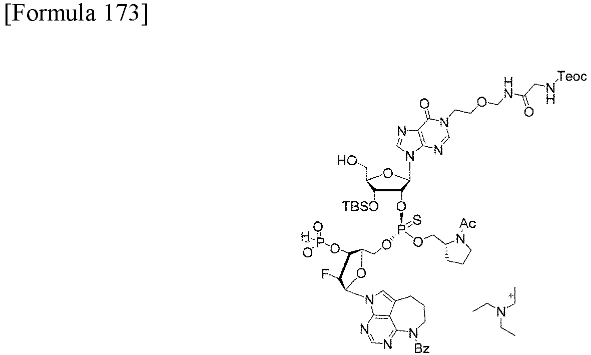

### (Step 2)

A triethylamine salt (260 mg) of 6-benzoyl-2-{2-deoxy-2-fluoro-3-O-[hydroxy(oxo)-λ⁵-phosphanyl]-β-D-ribofuranosyl}-6,7,8,9-tetrahydro-2H-2,3,5,6-tetraazabenzo[cd]azulene was dissolved in dehydrated acetonitrile (2.7 mL), Molecular Sieve 4A (12.5 mg) was added, and the mixture was then stirred for 30 minutes. In a separate vessel, the compound (540 mg) obtained in Example 1 was dissolved in dehydrated acetonitrile (2.7 mL), Molecular Sieve 4A (25 mg) was added, and the mixture was then stirred for 30 minutes. The previously prepared triethylamine salt of 6-benzoyl-2-{2-deoxy-2-fluoro-3-O-[hydroxy(oxo)-λ⁵-phosphanyl]-β-D-ribofuranosyl}-6,7,8,9-tetrahydro-2H-2,3,5,6-tetraazabenzo[cd]azulene in an acetonitrile solution was added thereto. A dehydrated acetonitrile solution (2.7 mL) containing 1-phenylimidazolium triflate prepared at 0.4 mol/L was added, and the mixture was stirred at room temperature for 30 minutes. Next, a dehydrated acetonitrile solution (0.45 mL) containing acetic anhydride prepared at 1 mol/L was added, and triethylamine (55 mg) was then added. The resulting mixture was stirred for 30 minutes. Subsequently, xanthane hydride (74 mg) was added and stirred for 30 min. The reaction solution was filtered to remove the Molecular Sieve. The filtrate was cooled to 0°C, and 20% brine (2.7 mL) was added. The pH was then adjusted with hydrochloric acid to 2.5. The solution was stirred overnight at the same temperature. The reaction solution was separated, and 20% brine (2.7 mL) was added to the resulting organic layer, which was neutralized with triethylamine to pH 7. After that, the separated organic layer was concentrated, and the crude product was purified by silica gel chromatography [acetonitrile/methanol] and concentrated to dryness to obtain the target compound. The diastereomers on the phosphorus atom as formed during the reaction were at a ratio of 98.5 : 1.5 as measured by HPLC.
MS (ESI) m/z: 1336(M+H)⁺, 1334(M-H)⁻.
   ³¹P-NMR (MeCN-d₃) δ: 69.41, 2.13 (Rotamer δ: 68.23, 1.91).
³¹P-NMR data was measured using an isolated product obtained by reversed-phase HPLC [10 mM aqueous ammonium acetate solution/acetonitrile].

### Example 3:

### Synthesis of 2-(trimethylsilyl)ethyl (2-{[(2-{9-[(5R, 7R, 8R, 10R, 12aR, 14R, 15R, 15aR, 16R)-10-{ [(2R)-1 -acetylpyrrolidin-2-yl]methoxy}-14-(6-benzoyl-6,7,8,9-tetrahydro-2H-2,3,5,6-tetraazabenzo[cd]azulen-2-yl)-16-{[tert-butyl(dimethyl)silyl]oxy}-15-fluoro-2-oxo-2-sulfanyl-10-sulfaniliden-octahydro-2H, 10H, 12H-5,8-methano-2λ⁵, 10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]amino}-2-oxoethyl)carbamate (corresponding to a compound of formula (Rc-VI))

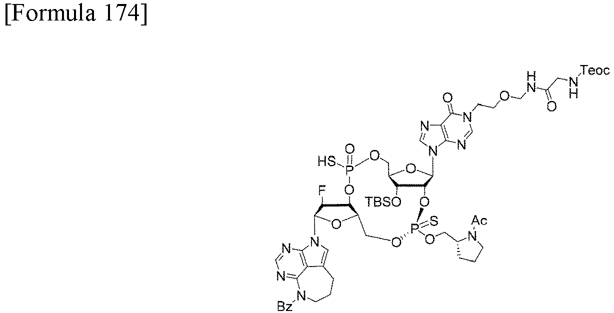

### (Step 3)

The compound (320 mg) obtained in Example 2 was azeotropically dehydrated with dehydrated pyridine (50 mL) to prepare 16 mL of pyridine solution, which was cooled to -20°C. In a separate vessel, 2-chloro-2-oxo-1,3,2-dioxaphosphorane (171 mg) was weighed and cooled to -20°C. The above pyridine solution was added to the vessel of 2-chloro-1,3,2-dioxaphosphorane-2-oxide, and the mixture was stirred at -20°C for 1 hour. The diastereomers on the phosphorus atom as generated during cyclization were at a ratio of 75 : 25. Water (43 mg) was added, and the mixture was stirred at the same temperature for 10 minutes. Xanthane hydride (40 mg) was then added, and the temperature was raised to room temperature. The mixture was stirred for 1 hour. The resulting reaction solution was quenched with 5% sodium bicarbonate water (3 mL). Then, ethyl acetate (12 mL) and 20% brine (3 mL) were added, and the mixture was liquid-separated. The aqueous layer was re-extracted with ethyl acetate (3 mL). The organic layer obtained by the first separation was combined with the organic layer obtained by re-extraction. The resulting organic layer was washed with a mixture of 20% brine (1.5 mL) and 5% sodium bicarbonate water (1 mL). Subsequently, the organic layer was concentrated to dryness, and methanol (30 mL) was added. The mixture was concentrated again to dryness to obtain the target compound.
MS (ESI) m/z: 1350(M+H)⁺, 1348(M-H)⁻.
   ³¹P-NMR (MeCN-d₃) δ: 65.93, 56.60 (Rotamer δ: 65.51, 57.12).
³¹P-NMR data was measured using an isolated product obtained by reversed-phase HPLC [10 mM aqueous triethylammonium acetate solution/acetonitrile].

### Example 4:

### Synthesis of bis(N,N-diethylethanaminium)(2R,5R,7R,8R,10R,12aR, 14R,15R,15aR,16R)-16-{[tert-butyl(dimethyl)silyl]oxy}-15-fluoro-2,10-dioxo-7-[6-oxo-1-(2-{[(N-f[2-(trimethylsilyl)ethoxy]carbonyl}glycyl)amino]methoxy}ethyl)-1,6-dihydro-9H-purin-9-yl]-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-2,10-bis(thiolate) (corresponding to a compound of formula (Rp,Rp-VII'))

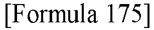

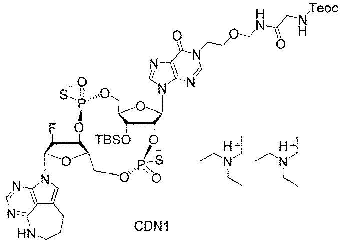

### (Step 4)

Methanol (12 mL) was added to the compound obtained in Example 2 to prepare a methanol solution. Next, 28% aqueous ammonia (4.5 mL) was added thereto, and the mixture was stirred at 60°C for 30 hours. The resulting reaction solution was concentrated, and the residue was purified by preparative HPLC [10 mM aqueous triethylammonium acetate solution/acetonitrile] to obtain 84 mg of the target compound as a single diastereomer (Rp,Rp form) on the phosphorus atom. Note that the HPLC purity of the isolated compound was 92.4%.
MS (ESI) m/z: 1121(M+H)⁺, 1119(M-H)⁻.
¹H-NMR (MeCN-d₃) δ: 8.61 (1H, s), 8.10 (1H, s), 8.07 (1H, brs), 7.84-7.82 (1H, m), 7.50 (1H, brs), 7.03 (1H, brs), 6.37 (1H, dd, J = 16.5, 3.0 Hz), 6.10 (1H, d, J = 8.0 Hz), 6.06 (1H, brm), 5.51 (1H, dt, J = 52.5, 4.0 Hz), 5.37 (1H, m), 5.31 (1H, m), 4.57 (2H, d, J = 7.0 Hz), 4.52 (1H, d, J = 3.5 Hz), 4.28 (2H, m), 4.19 (3H, m), 4.07 (3H, m), 3.91 (1H, ddd, J = 12.0, 5.0, 2.0 Hz), 3.84 (1H, ddd, J = 12.0, 5.0, 2.0 Hz), 3.70 (2H, m), 3.62 (2H, m), 3.46 (2H, m), 2.99 (6H, q, J = 7.0 Hz), 2.86 (1H, m), 2.73 (1H, m), 1.95 (2H, m), 1.17 (18H, t, J = 7.0 Hz), 0.95-0.92 (2H, m), 0.94 (9H, s), 0.23 (3H, s), 0.20 (3H, s), 0.00 (9H, s).
³¹P-NMR (MeCN-d₃) δ: 58.31, 57.22.

### A-2 Synthesis of compound (IV) (where A2 = Bz-tetraazabenzo[cd]azulene group)

The raw material compound (IV) used in the above synthesis scheme A-1 was synthesized according to the following scheme.

### [Synthesis Scheme A-2]

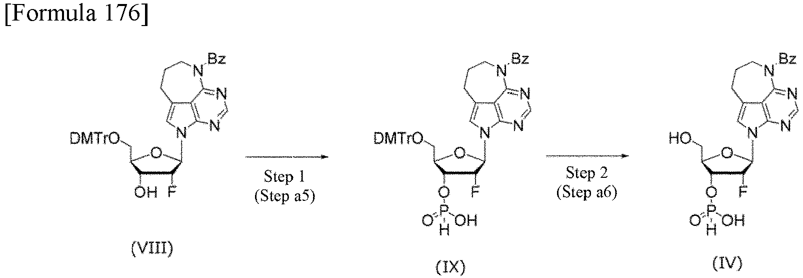

### Example 5:

### Synthesis of 6-benzoyl-2- {5-O-[bis(4-methoxyphenyl)(phenyl)methyl]-2-deoxy-2-fluoro-3-O-[hydroxy(oxo)-λ⁵-phosphanyl]-β-D-ribofuranosyl}-6,7,8,9-tetrahydro-2H-2,3,5,6-tetraazabenzo[cd]azulene (corresponding to a compound of formula (IX))

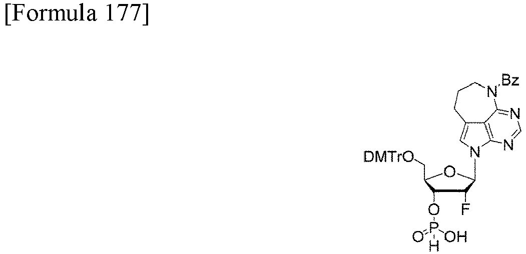

### (Step 1)

First, 6-benzoyl-2-{5-O-[bis(4-methoxyphenyl)(phenyl)methyl]-2-deoxy-2-fluoro-β-D-ribofuranosyl}-6,7,8,9-tetrahydro-2H-2,3,5,6-tetraazabenzo[cd]azulene (800 mg) was dissolved in pyridine (6 mL), and the mixture was cooled to 0°C. Diphenyl phosphite (1.31 g) was added, and the mixture was stirred for 1 hour while cooling. Subsequently, water (3 mL) was added, and triethylamine (3 mL) was then added. The resulting mixture was stirred at room temperature for 2 hours. The reaction solution was admixed with dichloromethane (8 mL) and 5% sodium bicarbonate water (2 mL), and was liquid-separated. The organic layer was washed twice with 5% sodium bicarbonate water (4 mL). The organic layer was then dried over anhydrous sodium sulfate to obtain the target compound.

MS (ESI) m/z: 779(M+H)⁺, 777(M-H)⁻.

### Example 6:

### Synthesis of 6-benzoyl-2-{2-deoxy-2-fluoro-3-O-[hydroxy(oxo)-λ⁵-phosphanyl]-β-D-ribofuranosyl}-6,7,8,9-tetrahydro-2H-2,3,5,6-tetraazabenzo[cd]azulene (corresponding to a compound of formula (IV))

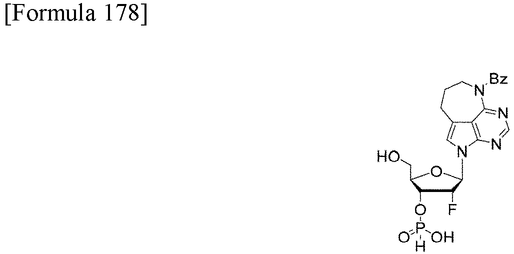

### (Step 2)

A solution containing the compound obtained in Example 5 was cooled to 0°C, and dichloroacetic acid (12 mL) was added. The mixture was then stirred for 24 hours while cooling. The reaction solution was subjected to purification by silica gel chromatography [acetonitrile/methanol] and concentrated and prepared as an acetonitrile solution to obtain the target compound (total quantitative value (2 steps) of 358 mg by NMR).

MS (ESI) m/z: 477(M+H)⁺, 475(M-H)⁻.

### A-3 Synthesis of CDN2

A cyclic dinucleotide (CDN2) was synthesized according to the following synthesis scheme A-3. In synthetic scheme A-3, A2 = a Bz-adenine group, Q = a thiol group, PG1 = TBS, PG2 = DMTr, PG3 = Teoc, and PG6 = Ac.

### [Synthesis Scheme A-3]

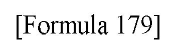

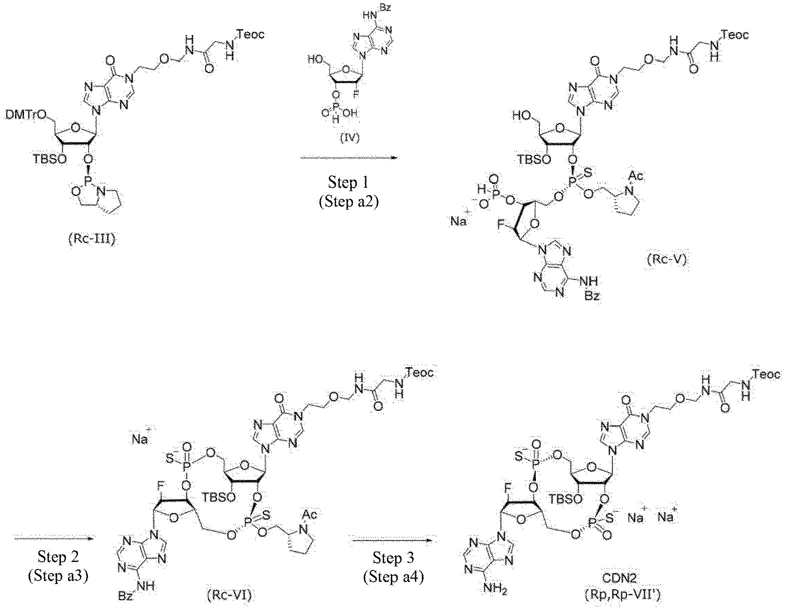

### Example 7:

### Synthesis of sodium (2R,3R,4R,5R)-2-({[(R)-{[(2R)-1-acetylpyrrolidin-2-yl]methoxy}{[(2R,3R,4R,SR)-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy}phosphorothiol]oxy}methyl)-5-(6-benzamido-9H-purin-9-yl)-4-fluorooxolan-3-yl phosphonate (corresponding to a compound of formula (Rc-V))

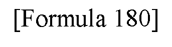

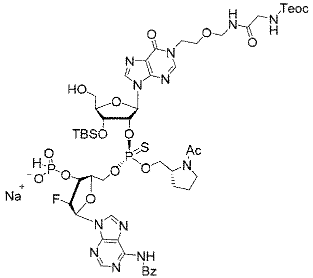

### (Step 1)

(2R,3R,4R, SR)-5-(6-Benzamido-9H-purin-9-yl)-4-fluoro-2-(hydroxymethyl)tetrahydrofuran-3-yl hydrogen phosphonate (400 mg), described in Patent Document WO2015/185565, was dissolved in dehydrated acetonitrile (4 mL). Triethylamine (97 mg) and Molecular Sieve 4A (20 mg) were added, and the mixture was stirred for 30 minutes. In a separate vessel, the compound (1.1 g) obtained in Example 1 was dissolved in dehydrated acetonitrile (5.5 mL). Molecular sieve 4A (25 mg) was then added, and the mixture was stirred for 30 minutes. An acetonitrile solution containing a triethylamine salt of the previously prepared (2R,3R,4R,5R)-5-(6-benzamido-9H-purin-9-yl)-4-fluoro-2-(hydroxymethyl)tetrahydrofuran-3-yl hydrogen phosphonate was added thereto. A dehydrated acetonitrile solution (5.5 mL) containing 1-phenylimidazolium triflate prepared at 0.4 mol/L was added, and the mixture was stirred at room temperature for 30 minutes. Next, a dehydrated acetonitrile solution (1.14 mL) containing acetic anhydride prepared at 1 mol/L was added, and triethylamine (139 mg) was then added. The resulting mixture was stirred for 30 minutes. Subsequently, xanthane hydride (154 mg) was added and stirred for 30 min. The reaction solution was filtered to remove the Molecular Sieve. The removed Molecular Sieve was washed with dehydrated acetonitrile (5 mL). The filtrate and the resulting washing liquid were combined and cooled to 0°C, and 20% brine (6 mL) was added. The pH was then adjusted with hydrochloric acid to 2.5. The solution was stirred overnight at the same temperature. The reaction solution was liquid-separated, and 20% brine (6 mL) was added to the resulting organic layer, which was neutralized with triethylamine to pH 7. After that, the separated organic layer was concentrated, and the crude product was purified by silica gel chromatography [ethyl acetate/methanol/0.4% triethylamine]. The collected fractions were concentrated and purified by preparative HPLC [10 mM aqueous ammonium acetate solution/acetonitrile], and the acetonitrile was removed by concentration. Salt was added to the resulting aqueous solution. The solution was extracted with tetrahydrofuran, and concentrated to dryness to obtain the target compound (776 mg). The diastereomers on the phosphorus atom as formed during the reaction were at a ratio of 98.5 : 1.5 as measured by HPLC.
MS (ESI) m/z: 1297(M+H)⁺, 1295(M-H)⁻.
³¹P-NMR (MeOH-d₃) δ: 70.20, 4.17.

### Example 8:

### Synthesis of sodium (5R,7R,8R,10R,12aR,14R,15R,15aR, 16R)-10-{[(2R)-1-acetylpyrrolidin-2-yl]methoxy}14-(6-benzamido-9H-purin-9-yl)-16- { [tert-butyl(dimethyl)silyl]oxy}-15-fluoro-2-oxo-7-[6-oxo-1-(2-{[(N-{[2-(trimethylsilyl)ethoxy]carbonyl}glycyl)amino]methoxy}ethyl)-1,6-dihydro-9H-purin-9-yl]-10-sulfaniliden-octahydro-2H,10H,12H-5,8-methano-2λ⁵, 10X⁵-furo[3.2-1][1,3,6,9,11,2,10]pentaoxadiphosphacyclotetradecin-2-thiolate (corresponding to a compound of formula (Rc-VI))

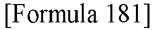

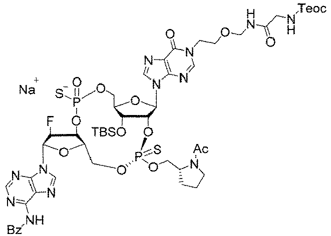

### (Step 2)

The compound (18 mg) obtained in Example 7 was dissolved in dehydrated pyridine (1 mL), and Molecular Sieve 4A (3 mg) was added. The diastereomers on the phosphorus atom as generated during cyclization were at a ratio of 70 : 30. Then, 2-chloro-5,5-dimethyl-1,3,2-dioxaphosphorinane 2-oxide (5.7 mg) was added. The mixture was stirred for 30 minutes, and the Molecular Sieve was filtered off. Water (10 mg) was added to the filtrate, and the mixture was stirred for 5 minutes. Xanthane hydride (2.6 mg) was then added, and the mixture was stirred for 30 minutes. The reaction was stopped by adding 5% sodium bicarbonate water (2 mL) to the reaction solution. Ethyl acetate was added, and the mixture was liquid-separated for extraction. The organic layer was concentrated to dryness and subjected to purification by preparative HPLC [10 mM aqueous ammonium acetate solution/acetonitrile], and the acetonitrile was removed by concentration. Salt was added to the resulting aqueous solution. The solution was extracted with tetrahydrofuran, and concentrated to dryness to obtain the target compound (4 mg) as a single diastereomer on the phosphorus.
MS (ESI) m/z: 1311(M+H)⁺, 1309(M-H)⁻.
³¹P-NMR (MeOH-d₃) δ: 66.89, 58.93.

### Example 9:

### Synthesis of disodium (2R,5R,7R,8R,10R,12aR,14R,15R,15aR, 16R)-14-(6-amino-9H-purin-9-yl)-16-{[tert-butyl(dimethyl)silyl]oxy}-15-fluoro-2,10-dioxo-7-[6-oxo-1-(2-{[(N-{[2-(trimethylsilyl)ethoxy]carbonyl}glycyl)amino]methoxy}ethyl)-1,6-dihydro-9H-purin-9-yl]octahydro-2H,10H,12H-5,8-methano-2λ⁵,10λ⁵-furo[3.2-1][1,3,6,9,11,2,10]pentaoxadiphosphacyclotetradecin-2-bisthiolate (corresponding to a compound of formula (Rp,Rp-VII'))

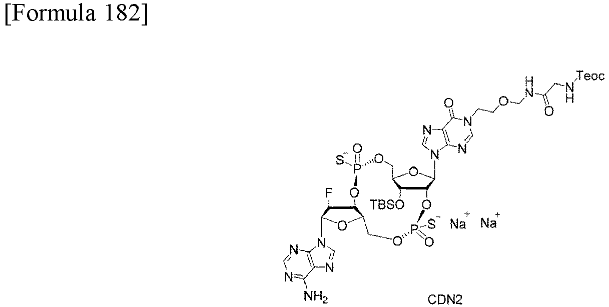

### (Step 3)

The compound (1.6 mg) obtained in Example 8 was dissolved in MeOH (0.1 mL). Next, 28% aqueous ammonia (0.05 mL) was added thereto, and the mixture was stirred at 50°C for 24 hours. The resulting reaction solution was analyzed by LC-MS, and the target compound was found to be formed as a single diastereomer (Rp,Rp form) on the phosphorus atom.

MS (ESI) m/z: 1121(M+H)⁺, 1119(M-H)⁻.

### A-4 Synthesis of compound (IV) (where A2 = Bz-adenine group)

The raw material compound (IV) used in the above synthesis scheme A-3 was synthesized according to the following scheme.

### [Synthesis Scheme A-4]

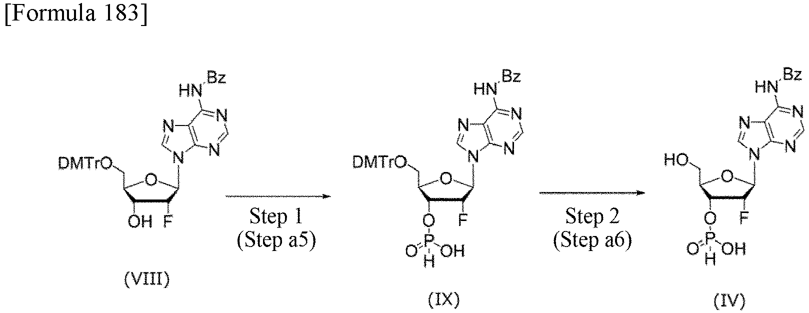

### Example 10:

### Synthesis of N-benzoyl-5'-O-[bis(4-methoxyphenyl)(phenyl)methyl]-2'-deoxy-2'-fluoro-3'-O-[hydroxy(oxo)-λ⁵-phosphanyl]adenosine (corresponding to a compound of formula (IX))

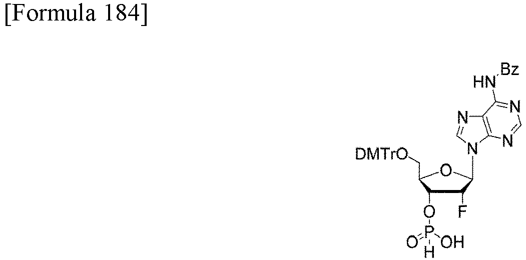

### (Step 1)

Commercially available N-benzoyl-5'-O-[bis(4-methoxyphenyl)(phenyl)methyl]-2'-deoxy-2'-fluoroadenosine (1 g) was dissolved in pyridine (7.5 mL), and the mixture was cooled to 0°C. Diphenyl phosphite (1.73 g) was added, and the mixture was stirred for 1 hour while cooling. Subsequently, water (3.7 mL) was added, and triethylamine (3.7 mL) was then added. The resulting mixture was stirred at room temperature for 2 hours. The reaction solution was concentrated to 5 mL to obtain the target compound.

MS (ESI) m/z: 740(M+H)⁺, 738(M-H)⁻.

### Example 11:

### Synthesis of N-benzoyl-2'-deoxy-2'-fluoro-3'-O-[hydroxy(oxo)-λ⁵-phosphanyl]adenosine (corresponding to a compound of formula (IV))

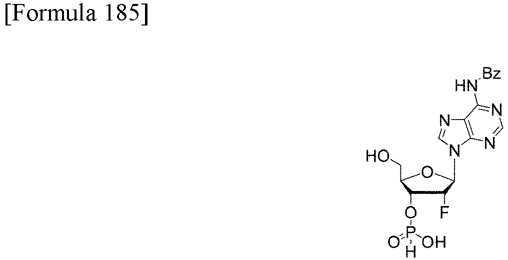

### (Step 2)

To the solution obtained in Example 10 was added dichloromethane (10 mL), and the mixture was cooled to 0°C. While cooling, dichloroacetic acid was added until pH 3, and the mixture was stirred for 24 hours. The reaction solution was subjected to purification by silica gel chromatography [ethyl acetate/methanol] and concentrated and prepared as an acetonitrile solution. Then, concentrated hydrochloric acid was used to adjust the pH to 2 or lower. The white crystals that precipitated with pH adjustment were filtered, washed with 80% acetonitrile solution, and dried to obtain the target compound (amount: 0.24 g; yield: 37%).
MS (ESI) m/z: 438(M+H)⁺, 436(M-H)⁻.
¹H-NMR (DMSO-d₆) δ: 11.29 (1H, brs), 8.78 (1H, s), 8.73 (1H, s), 8.05 (2H, m), 7.65 (2H, t, J = 7.5 Hz), 7.60 (1H, d, J = 1.5 Hz), 7.56 (2H, t, J = 7.5 Hz), 6.45 (1H, dd, J = 16.5, 3.5 Hz), 6.24 (1H, d, J = 1.5 Hz), 5.88-5.76 (1H, m), 5.22 (1H, m), 4.25 (1H, m), 3.80 (1H, dd, J = 12.5, 4.0 Hz), 3.67 (1H, dd, J = 12.5, 4.0 Hz).

### A-5 Synthesis 2 of CDN1

A cyclic dinucleotide (CDN1) was synthesized according to the following synthesis scheme A-5. In synthesis scheme A-5, A2 = a Teoc-tetraazabenzo[cd]azulene group, Q = a thiol group, PG1 = TBS, PG2 = DMTr, PG3 = Teoc, and PG6 = TFAc. (3aR)-1-chlorotetrahydro-1H,3H-pyrrolo[1,2-c][1,3,2]oxazaphosphole was used as (Rc-II).

### [Synthesis Scheme A-5]

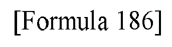

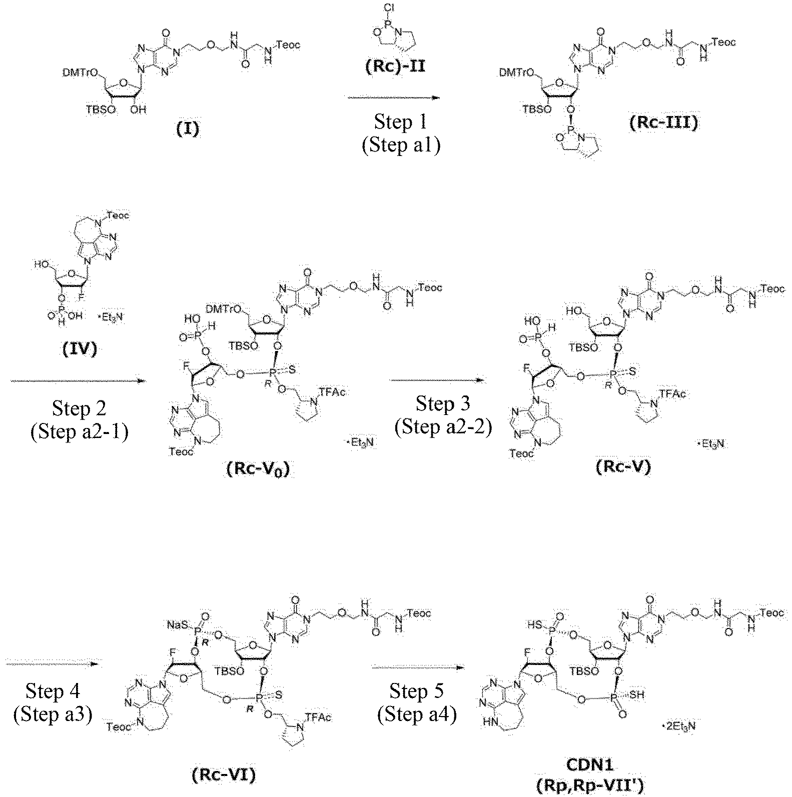

### Example A5-1:

### Synthesis of 5'-O-[bis(4-methoxyphenyl)(phenyl)methyl]-3'-O-[tert-butyl(dimethyl)silyl]-2'-O-[(1S,3aR)-tetrahydro-1H,3H-pyrrolo[1,2-c][1,3,2]oxazaphosphol-1-yl]-1-(2-{[(N-{[2-(trimethylsilyl)ethoxy]carbonyl}glycyl)amino]methoxy}ethyl)inosine (corresponding to a compound of formula (Rc-III))

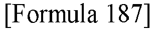

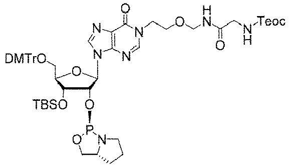

### (Step 1)

A dichloromethane solution (0.8 mL) containing 5'-O-[bis(4-methoxyphenyl)(phenyl)m ethyl] -3 '-O-(2,3,3 -trimethylbutan-2-yl)-1 -(2- {[(N- {[2-(trimethylsilyl)ethoxy]carbonyl}glycyl)amino]methoxy}ethyl)inosine (372.7 mg, 0.39 mmol) and Molecular Sieve 3A (80 mg) were stirred under a nitrogen atmosphere at room temperature for 2 hours. The mixture was then cooled to 0°C, and triethylamine (58.7 µL, 0.42 mmol) was added thereto. To the mixture was added a dichloromethane solution (0.6 mL) containing (3aR)-1-chlorotetrahydro-1H,3H-pyrrolo[1,2-c][1,3,2]oxazaphosphole (64.3 mg, 0.39 mmol) described in a known publication (Tetrahedron Letter, 1998, 39, 2491-2494). The resulting mixture was stirred at room temperature for 1.5 hours to obtain a dichloromethane solution of the target compound.

### Example A5-2:

### Synthesis ofN,N-diethylethanaminium(2R,3R,4R,5R)-2-({[(R)-({(2R,3R,4R,5R)-5-{[bis(4-methoxyphenyl)(phenyl)methoxy]methyl}-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]oxolan-3-yl}oxy){[(2R)-1-(trifluoroacetyl)pyrrolidin-2-yl]methoxy}phosphorothioyl]oxy}methyl)-5-(6-{[2-[trimethylsilyl)ethoxy]carbonyl}-6,7,8,9-tetrahydro-2H-2,3,5,6-tetraazabenzo[cd]azulen-2-yl)-4-fluorooxolan-3-yl phosphonate (corresponding to a compound of formula (Rc-V₀))

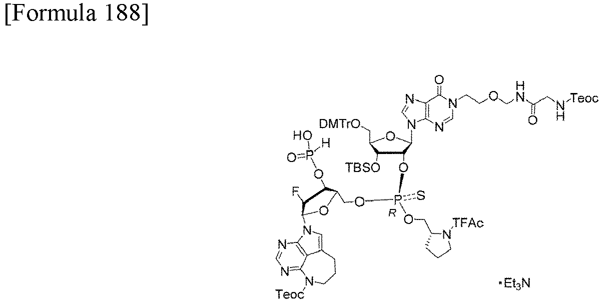

### (Step 2)

To an acetonitrile solution (2.0 mL) containing N,N-diethylethanaminium 2-(trimethylsilyl)ethyl 2-{2-deoxy-2-fluoro-3-O-[oxido(oxo)-λ⁵-phosphanyl]-β-D-ribofuranosyl}-2,7,8,9-tetrahydro-6H-2,3,5,6-tetraazabenzo[cd]azulene-6-carboxylate (200 mg, 0.32 mmol) was added Molecular Sieve 3A (60 mg), and the mixture was stirred at room temperature for 2 hours. The solution was then cooled to -10°C, and triethylamine (18.1 µL, 0.13 mmol) was then added thereto. A dichloromethane solution containing the compound obtained in Example A5-1 was added dropwise, and the mixture was washed with 0.2 mL of dichloromethane. An acetonitrile solution (809.4 µL, 0.65 mmol) containing 0.8 mol/L 1-methylbenzimidazolium triflate was added, and the mixture was stirred for 1.5 hours. An acetonitrile solution (121.4 µL, 0.10 mmol) containing 0.8 mol/L 1-methylbenzimidazolium triflate was then further added. After the reaction was found to be stopped, triethylamine (158.0 µL, 1.13 mmol) and 1-(trifluoroacetyl)imidazole (66.3 µL, 0.58 mmol) were added at -10°C, and the temperature was raised to room temperature. After stirring for about 30 minutes, xanthane hydride (63.2 mg, 0.42 mmol) was added and the mixture was stirred at room temperature for 15 minutes. The reaction solution was concentrated to about 2.5 mL and subjected to purification by silica gel column chromatography (0-12% methanol acetonitrile with 0.4% triethylamine) to obtain the target compound as a pale yellow solid (536.0 mg; yield: 90%). The diastereomers on the phosphorus atom as formed during the reaction were at a ratio of 94.7 : 5.3 as measured by HPLC.
MS (ESI) m/z: 1734(M+H)⁺, 1732(M-H)⁻.
1H-NMR (500 MHz, DMSO-d₆) δ: 9.22 (1H, s), 9.10-9.02 (1H, m), 8.50 (1H, s), 8.45 (1H, s), 8.39 (1H, s), 7.37-7.28 (4H, m), 7.26-7.16 (7H, m), 6.85-6.80 (4H, m), 6.42 (1H, dd, J = 18.6, 2.0 Hz), 6.25 (1H, d, J = 3.4 Hz), 6.16 (1H, s), 5.53-5.45 (1H, m), 5.42-5.28 (1H, m), 4.89-4.78 (1H, m), 4.73-4.67 (1H, m), 4.57-4.49 (2H, m), 4.42-4.33 (1H, m), 4.28-4.19 (3H, m), 4.18-4.03 (6H, m), 4.02-3.84 (5H, m), 3.72 (6H, s), 3.69-3.56 (4H, m), 3.53-3.41 (2H, m), 3.16-3.04 (7H, m), 2.89-2.72 (2H, m), 1.97-1.89 (2H, m), 1.84-1.65 (4H, m), 1.17 (9H, t, J = 7.2 Hz), 0.98-0.94 (2H, m), 0.92-0.86 (2H, m), 0.71 (9H, s), 0.03- -0.09 (minus 0.09) (24H, m).
31P-NMR (DMSO-d₆) δ: 69.43, 1.45.

### Example A5-3:

### Synthesis ofN,N-diethylethanaminium(2R,3R,4R,5R)-2-({[(R)-{[(2R,3R,4R,SR)-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy}({2-[(2R)-1-(trifluoroacetyl)pyrrolidin-2-yl]methoxy}phosphorothioyl]oxy}methyl)-5-(6-{[2-(trimethylsilyl)ethoxy]carbonyl}-6,7,8,9-tetrahydro-2H-2,3,5,6-tetraazabenzo[cd]azulen-2-yl)-4-fluorooxolane-3-yl phosphonate (corresponding to a compound of formula (Rc-V))

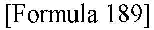

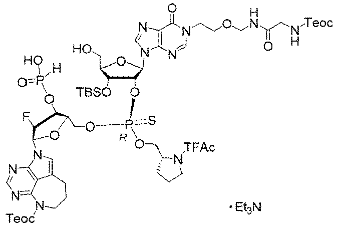

### (Step 3)

To a dichloromethane solution (100 mL) containing the compound (4.1 g, 2.25 mmol) obtained in Example A5-2 were added acetonitrile (15 mL) and ethanol (4.1 g, 89.84 mmol) at room temperature. Dichloroacetic acid (0.9 g, 6.74 mmol) was added, and the mixture was stirred at room temperature for 15 hours. Triethylamine (0.91 g, 8.98 mmol) and water (50 mL) were added, and the mixture was then stirred. The aqueous layer was discarded, and acetonitrile (22.5 mL), ethanol (6.2 g, 134.8 mmol), and water (50 mL) were added. The aqueous layer was discarded, and the solvent was distilled away under reduced pressure to about 15 mL. Acetonitrile (100 mL) was added, and the solvent was distilled away twice under reduced pressure to about 15 mL to obtain an acetonitrile solution containing the target compound (13.95 g).

### Example A5-4:

Synthesis of sodium (2R,5R,7R,8R,10R, 12aR,14R,15R,15aR,16R)-16-{ *[tert-*butyl(dimethyl)silyl]oxy}-15-fluoro-2-oxo-7-[6-oxo-1-(2-{(N-{[2-(trimethylsilyl)ethoxy]carbonyl}glycyl)amino]methoxy}ethyl)-1,6-dihydro-9H-purin-9-yl]-10-sulfaniliden-14-(6-{[2-(trimethylsilyl)ethoxy]carbonyl}-6,7,8,9-tetrahydro-2H-2,3,5,6-tetraazabenzo[cd]azulen-2-yl)-10-{[(2R)-1-(trifluoroacetyl)pyrrolidin-2-yl]methoxy}octahydro-2H,10H,12H-5,8-methano-2λ⁵,10λ⁵-furo[3,2-1][1,3,6,9,11,2,10]pentaoxadiphosphacyclotetradecin-2-thiolate (corresponding to a compound of formula (Rc-VI))

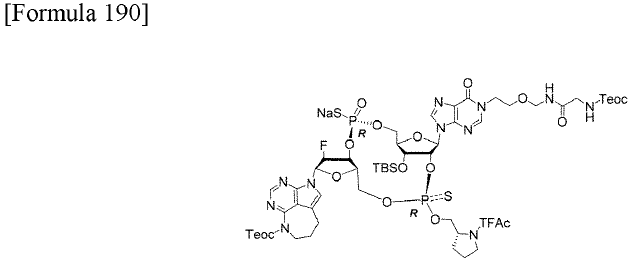

### (Step 4)

To a mixture of pyridine (42 mL) and dichloromethane (42 mL) was added a T3P-containing acetonitrile solution (1.7 mol/L, 4.9 mL, 8.30 mmol). The mixture was then cooled to -20°C. An acetonitrile solution (13.67 g) containing the compound obtained in Example A5-3 was added dropwise, and the mixture was stirred at -20°C for 1 hour. The diastereomers on the phosphorus atom as generated during cyclization were at a ratio of 85 : 15. Xanthane hydride (0.62 g, 4.15 mmol) was added, and the mixture was heated to about 0°C and stirred for 3 hours. Then, 5% aqueous sodium bicarbonate solution (22.5 mL), ethyl acetate (40 mL), and salt (1.2 g) were added, and the mixture was stirred. The aqueous layer was discarded, and the remainder was washed with a solution of water (22.5 mL), sodium bicarbonate (1.1 g), and salt (2.4 g). The resulting organic layer was subjected to distillation and the solvent was distilled away under reduced pressure to about 15 mL to obtain a pyridine solution containing the target compound.

### Example A5-5:

### Synthesis of bis(N,N-diethylethanaminium)(2R,5R,7R,8R,10R, 12aR, 14R,15R,15aR,16R)-16-{ [tert-butyl(dimethyl)silyl]oxy}-15-fluoro-2,10-dioxo-7-[6-oxo-1-(2-{[(N-{[2-(trimethylsilyl)ethoxy]carbonyl}glycyl)amino]methoxy}ethyl)-1,6-dihydro-9H-purin-9-yl]-14-(6,7,8,9)-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-2,10-bis(thiolate) (corresponding to a compound of formula (Rp,Rp-VII'))

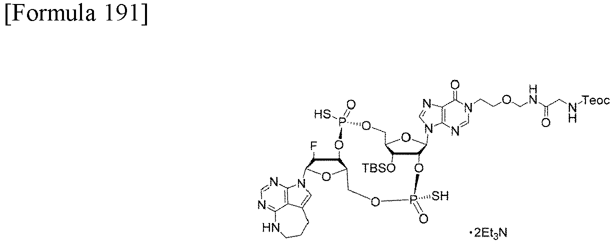

### (Step 5)

To a pyridine solution containing the compound obtained in Example A5-4 was added 28% aqueous ammonia (35 mL), and the mixture was stirred at about 45°C for 15 hours. After cooling to room temperature, heptane (35 mL) was added and the mixture was stirred. The organic layer was discarded, and the resulting aqueous layer was washed with heptane (35 mL). The aqueous layer was subjected to purification by preparative HPLC [10 mM aqueous triethylamine acetate solution/acetonitrile], and the fractions obtained were diluted with water. The solution was subjected to solid-phase extraction using silica gel, and eluted with 0.1% triethylamine/acetonitrile solution. For the resulting fractions, the solvent was distilled away under reduced pressure to about 15 mL. Ethyl acetate (100 mL) was added, and the solvent was distilled away under reduced pressure to about 15 mL. This process was repeated twice, and ethyl acetate (10 mL) was then added. This ethyl acetate solution was added dropwise to heptane (175 mL) at room temperature, and the mixture was stirred for 30 minutes. A solid was filtered from the resulting suspension and washed with heptane (25 mL). The solid was dried at 40°C overnight to obtain the target compound as white powder (1.9 g; yield: 55%; HPLC: >99%).

### A-6 Synthesis of compound (IV) (where A2 = Teoc-tetraazabenzo[cd]azulene group)

The raw material compound (IV) used in the above synthesis scheme A-5 was synthesized according to the following scheme.

### [Synthesis Scheme A-6]

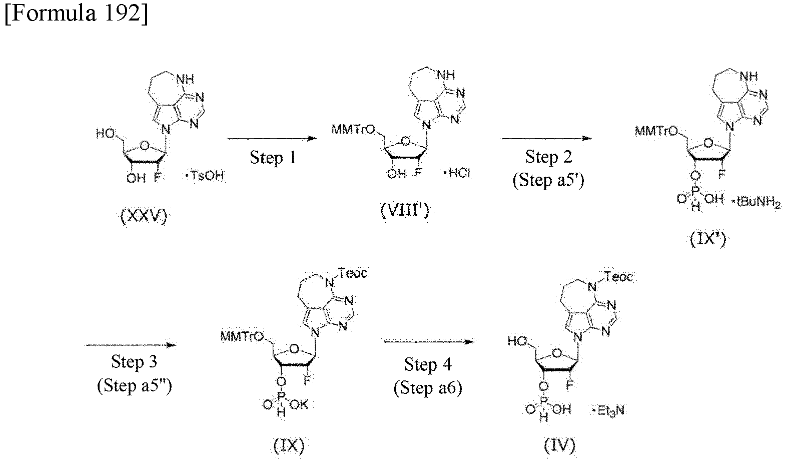

### Reference Example A6-1:

### Synthesis of 2-{2-deoxy-2-fluoro-5-O-[(4-methoxyphenyl)(diphenyl)methyl]-β-D-ribofuranosyl }-6,7, 8, 9-tetrahydro-2H-2,3, 5,6-tetraazabenzo[cd] azulene hydrochloride (corresponding to a hydrochloride salt of compound of formula (VIII'))

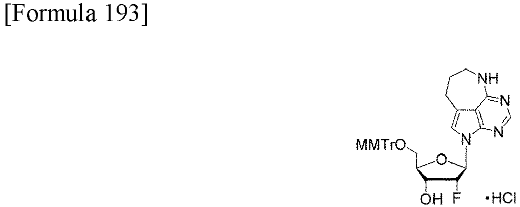

### (Step 1)

Here, 4-methoxytrityl chloride (26.4 g, 85.4 mmol) was added at room temperature to a pyridine solution (171 mL) containing p-toluenesulfonate (34.2 g, 71.2 mmol) of the compound obtained in Example 25-2. After stirring at room temperature for about 24 hours, 5% aqueous sodium bicarbonate solution (171 mL) was added dropwise. Toluene (684 mL) was added, and the aqueous layer was discarded after stirring. The resulting organic layer was washed with 10% brine (171 mL) to obtain an organic layer. Pyridine hydrochloride (8.2 g, 71.2 mmol) was added and the mixture was stirred for 1 hour. Pyridine hydrochloride (12.3 g, 106.8 mmol) was further added, and the mixture was stirred for about 23 hours. Crystals were filtered from the resulting suspension and washed with toluene (103 mL) to obtain wet crystals of hydrochloride salt of the target compound.

### Example A6-1:

### Synthesis of 2-methylpropan-2-aminium 2-{2-deoxy-2-fluoro 5-O-[(4-methoxyphenyl)(diphenyl)methyl]-3-O-[oxido(oxo)-λ⁵-phosphanyl]-β-D-ribofuranosyl}-2,7,8,9-tetrahydro-6H-2,3,5,6-tetraazabenzo[cd]azulene-6-carboxylate (corresponding to a tert-butylamine salt of compound of formula (IX'))

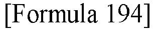

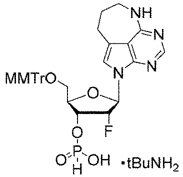

### (Step 2)

To the wet crystals of hydrochloride obtained in Reference Example A6-1 were added ethyl acetate (342 mL) and 5% sodium bicarbonate (342 mL). The mixture was then stirred at room temperature. The aqueous layer was discarded, and the resulting organic layer was washed with 10% brine (171 mL). Acetonitrile (171 mL) was added to the organic layer, and the solvent was distilled away under reduced pressure to about 170 mL. Acetonitrile (342 mL) was further added, the solvent was distilled away under reduced pressure to about 170 mL, and triethylamine (61.2 g, 605.2 mmol) was added. Diphenyl phosphite (43.4 g, 185.1 mmol) was added dropwise, and the mixture was stirred at room temperature for 47 hours. After addition of water (34.2 mL), the mixture was stirred for about 5 hours. Ethyl acetate (513 mL) and 23% potassium bicarbonate (239 mL) were added and stirred, and the aqueous layer was then discarded. The organic layer was washed twice with 20% potassium bicarbonate (239 mL), and the solvent in the resulting organic layer was distilled away under reduced pressure to about 170 mL. Acetonitrile (342 mL) was added, and the solvent was distilled away twice under reduced pressure to about 170 mL. Then, acetonitrile (342 mL) was added. Subsequently, *tert*-butylamine (2.3 mL, 21.4 mmol) was added, and seed crystals (34.2 mg) of the *tert*-butylamine salt of the target compound were added. The mixture was then stirred overnight. Further, tert-butylamine (42.8 mL, 405.8 mmol) was added dropwise in three portions (5.3 mL, 15.0 mL, 22.5 mL). The mixture was stirred at room temperature and cooled to -1°C. Crystals were filtered from the suspension obtained by stirring at -1°C for about 6 hours, and washed with acetonitrile (102 mL). The crystals were dried at 40°C overnight to obtain the *tert-*butylamine salt of the target compound as white crystals (35.7 g; yield: 70%).
MS (ESI) m/z: 645(M+H)⁺, 643(M-H)⁻.
1H-NMR (500 MHz, DMSO-d₆) δ: 8.04 (1H, s), 7.84 (2H, brs), 7.60 (1H, t, J = 3.4 Hz), 7.42-7.37 (4H, m), 7.32-7.23 (8.5H, m), 7.07 (1H, s), 6.88 (2H, td, J = 6.0, 3.4 Hz), 6.35 (1H, dd, J = 17.2, 2.3 Hz), 6.07 (0.5H, d, J = 1.7 Hz), 5.30 (0.5H, q, J = 2.1 Hz), 5.20 (0.5H, q, J = 2.3 Hz), 4.90-4.79 (1H, m), 4.16-4.13 (1H, m), 3.74 (3H, s), 3.31-3.26 (2H, m), 2.66-2.53 (2H, m), 1.87-1.82 (2H, m), 1.22 (9H, s).

### Example A6-2:

### Synthesis of potassium 2-(trimethylsilyl)ethyl 2-{2-deoxy-2-fluoro-5-O-[(4-methoxyphenyl)(diphenyl)methyl]-3-O-[oxido(oxo)-λ⁵-phosphanyl]-β-D-ribofuranosyl}-2,7,8,9-tetrahydro-6H-2,3,5,6-tetraazabenzo[cd]azulene-6-carboxylate (corresponding to a potassium salt of compound of formula (IX))

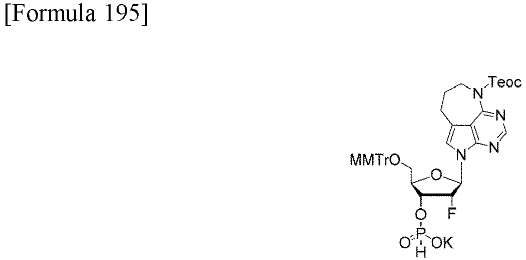

### (Step 3)

An acetonitrile solution (125 mL) containing 4-[2-(trimethylsilyl)ethoxycarbonyloxy]nitrobenzene (59.2 g, 209.0 mmol) and N,N-diisopropylethylamine (36.0 g, 278.6 mmol) was heated to 60°C. To the solution was added the *tert*-butylamine salt (25.0 g, 34.8 mmol) obtained in Example A6-1 in three portions. The mixture was stirred at 60°C for about 2 days and cooled to room temperature. Then, isopropylamine (10.3 g, 174.2 mmol) was added dropwise, and the mixture was stirred for 2.5 hours. Isopropyl acetate (250 mL) and 10% aqueous ammonium chloride solution (250 mL) were added and stirred, and the aqueous layer was then discarded. The organic layer was washed with 10% aqueous ammonium chloride solution (250 mL) and then washed twice with 10% aqueous potassium carbonate solution (500 mL). The organic layer was further washed with 10% aqueous potassium chloride solution (250 mL), and the solvent in the organic layer was distilled away under reduced pressure to about 125 mL to obtain an acetonitrile solution containing the potassium salt of the target compound (123.9 g).

### Example A6-3:

### Synthesis of N,N-diethylethanaminium 2-(trimethylsilyl)ethyl 2-{2-deoxy-2-fluoro-3-O-[oxido(oxo)-λ⁵-phosphanyl]-β-D-ribofuranosyl}-2,7,8,9-tetrahydro-6H-2,3,5,6-tetraazabenzo[cd]azulene-6-carboxylate (corresponding to a compound of formula (IV))

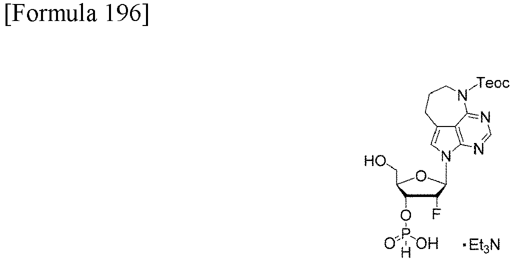

### (Step 4)

To an acetonitrile solution (121.2 g) containing the potassium salt obtained in Example A6-2 were added dichloromethane (417 mL) and methanol (55.4 mL). Next, dichloroacetic acid (22.0 g, 170.7 mmol) was added dropwise at room temperature. After stirring for about 27 hours, triethylamine (20.7 g, 204.8 mmol) was added dropwise to obtain a solution of the target compound (658.1 g). The solution of the target compound (26.9 g) was distilled under reduced pressure to remove the solvent to about 5 mL. Then, acetonitrile (3 mL), water (10 mL), and n-heptane (10 mL) were added and stirred to obtain an aqueous layer. The aqueous layer was washed with n-heptane (10 mL), dichloromethane (20 mL) was added, and the pH was adjusted to 8.5 with triethylamine (97 µL). Triethylamine hydrochloride (500 mg) was added, and the mixture was stirred to obtain an organic layer. To the organic layer were added water (10 mL) and triethylamine hydrochloride (500 mg). After the mixture was stirred, the aqueous layer was discarded. The resulting organic layer was distilled under reduced pressure to remove the solvent to about 5 mL. Acetonitrile (10 mL) was added, and the solvent was distilled away twice under reduced pressure to about 5 mL to obtain an acetonitrile solution containing the triethylamine salt of the target compound (632.6 mg; yield: 74%).
MS (ESI) m/z: 517(M+H)⁺, 515(M-H)⁻.
1H-NMR (500 MHz, DMSO-d₆) δ: 8.51 (1H, s), 7.56 (1H, s), 7.30 (0.5H, s), 6.42 (1H, dd, J = 17.8, 1.7 Hz), 6.12 (0.5H, s), 5.80 (1H, brs), 5.31-5.30 (0.5H, m), 5.20-5.19 (0.5H, m), 4.81-4.72 (1H, m), 4.27-4.23 (2H, m), 4.02-3.91 (3H, m), 3.72-3.63 (2H, m), 3.07 (6H, s), 2.89 (2H, t, J = 6.30 Hz), 1.99 (2H, t, J = 4.6 Hz), 1.17 (9H, t, J = 7.5 Hz), 1.00-0.97 (2H, m), -0.01 (9H, s).

### A-7 Synthesis of CDN-linker

The CDN-linker was synthesized according to the following scheme.

### [Synthesis Scheme A-7]

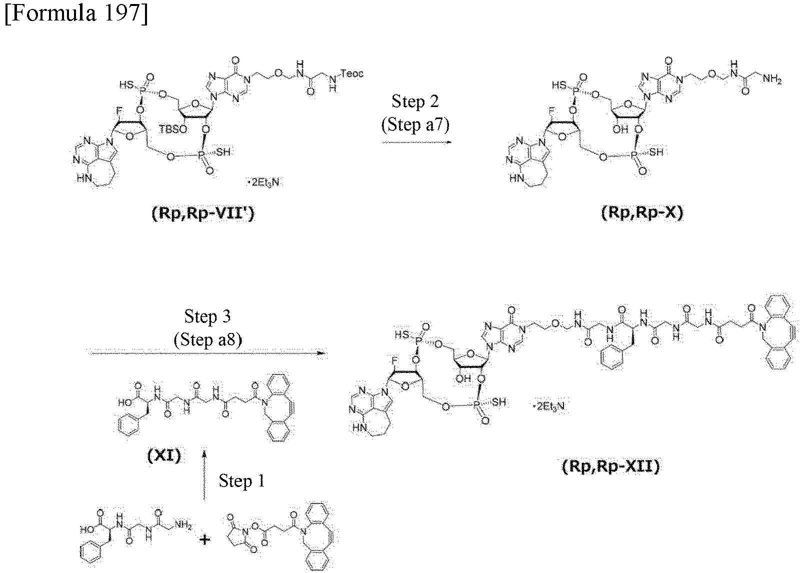

### Reference Example A7-1:

### Synthesis ofN-[4-(11,12-Didehydrodibenzo[b,f]azocin-5(6H)-yl)-4-oxobutanoyl]glycylglycyl-L-phenylalanine (corresponding to a compound of formula (XI))

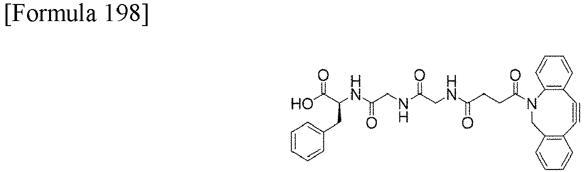

### (Step 1)

To a suspension containing 1-{[4-(11,12-didehydrodibenzo[b,f]azocin-5(6H)-yl)-4-oxobutanoyl]oxy}pyrrolidine-2,5-dione (5.0 g, 12.4 mmol) and glycylglycyl-L-phenylalanine (4.2 g, 14.9 mmol) in acetonitrile (50 mL) and water (50 mL) was added triethylamine (2.3 mL, 16.1 mmol) at room temperature. The mixture was stirred for about 4 hours. After 1 mol/L aqueous hydrochloric acid solution (16.2 mL) was added, seed crystals of the target compound (4.6 mg) were added. After stirring overnight at room temperature, water (100 mL) was added dropwise to the resulting suspension over 2 hours. After stirring at 40°C for about 1.5 hours, 1 mol/L aqueous hydrochloric acid solution (16.2 mL) was added. The mixture was stirred for 30 minutes, cooled to room temperature, and stirred for another 2 hours. Crystals were filtered from the suspension, and washed with acetonitrile/water (1/3, 50 mL). The crystals were dried at 30°C overnight to obtain the target compound as white crystals (6.7 g; yield: 95%).
1H-NMR (500 MHz, DMSO-d₆) δ: 12.8 (1H, brs), 8.15-7.95 (3H, m), 7.68-7.17 (13H, m), 5.01 (1H, J = 14.2 Hz), 4.41-4.37 (1H, m), 3.74-3.57 (5H, m) 3.05-3.01 (1H, m), 2.87 (1H, dd, J = 14.2, 9.3 Hz), 2.68-2.59 (1H, m), 2.32-2.25 (1H, m), 2.09-2.03 (1H, m), 1.82-1.76 (1H, m).

### Example A7-1:

### Synthesis 1 of N-[(2-{9-[(2R,5R,7R,8R,10R,12aR,14R,15R,15aR,16R)-15-fluoro-16-hydroxy-2,10-dioxo-2,10-bis(sulfanyl)-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]glycinamide (corresponding to a compound of formula (Rp,Rp-X))

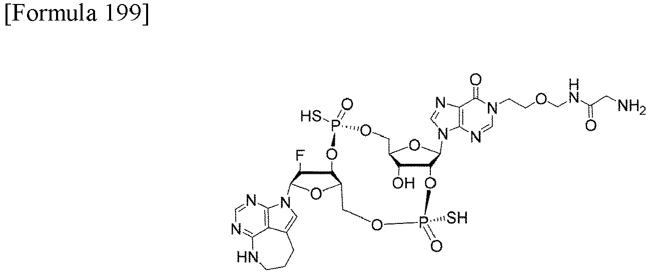

### (Step 2)

To a dimethyl sulfoxide solution (3 mL) containing the compound (1 g, 0.76 mmol) obtained in Example A5-4 was added 70% aqueous tetrabutylammonium fluoride solution (3 mL) at room temperature. The mixture was then stirred at 30°C for 2 days. Acetonitrile (12 mL) and calcium chloride (0.59 g, 5.32 mmol) were added thereto, and the mixture was stirred at room temperature for 14 hours. Triethylamine (1 mL) and water (2 mL) were then added. After stirring at room temperature for 2 hours, 5 mol/L aqueous hydrochloric acid water (1.2 mL) was added in 6 portions. Triethylamine (60 µL) was added, and the insoluble material was filtered off. The resulting filtrate was distilled under reduced pressure to remove the solvent to about 10 mL. Acetonitrile (20 mL) was added, and the solvent was distilled away twice under reduced pressure to about 10 mL. Acetonitrile (50 mL) was then added, and the solvent was distilled away twice under reduced pressure to about 10 mL. To the resulting solution was added 5 mol/L aqueous hydrochloric acid water (165 µL), and after addition of acetonitrile (50 mL), the solution was cooled to 0°C and stirred for 2 hours. Solids were filtered from the resulting suspension, and washed with acetonitrile (5 mL). The solids were dried at 25°C overnight to obtain the target compound as white crystals (0.46 g; yield: 71%).
MS (ESI) m/z: 863(M+H)⁺, 861(M-H)⁻.
1H-NMR (500 MHz, DMSO-d₆) δ: 9.18 (1H, t, J = 6.5 Hz), 9.02 (1H, brs), 8.69 (1H, s), 8.37 (1H, s), 8.20 (1H, s), 8.09 (2H, brs), 7.47 (1H, s), 6.40 (1H, dd, J = 15.0, 2.0 Hz), 6.06 (1H, d, J = 8.0 Hz), 5.42 (1H, d, J = 52.5 Hz), 5.22 (1H, m), 5.09 (1H, m), 4.56 (1H, dd, J = 10.0, 6.5 Hz), 4.43-4.40 (2H, m), 4.30 (2H, m), 4.19-4.08 (3H, m), 3.91 (2H, m), 3.77 (1H, dd, J = 11.0, 3.5 Hz), 3.66 (2H, m), 3.54 (2H, m), 3.41 (2H, m), 2.58 (2H, m), 1.86 (2H, m).
31P-NMR (DMSO-d₆) δ: 55.36, 51.31.

### Example A7-2:

### Synthesis 2 of N-[(2-{9-[(2R,5R,7R,8R,10R,12aR,14R,15R,15aR, 16R)-15-fluoro-16-hydroxy-2,10-dioxo-2,10-bis(sulfanyl)-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]glycinamide (corresponding to a compound of formula (Rp,Rp-X))

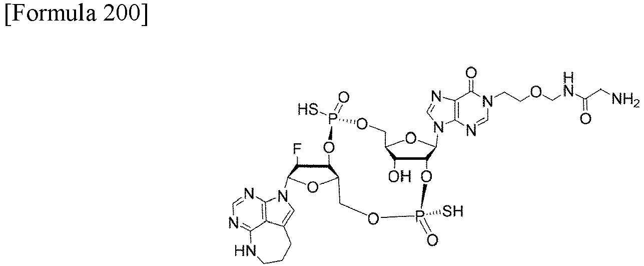

### (Step 2')

To a dimethyl sulfoxide solution (2.4 mL) containing the compound (0.8 g, 0.60 mmol) obtained in Example A5-4 was added 70% aqueous tetrabutylammonium fluoride solution (2.4 mL) at room temperature. The mixture was then stirred at room temperature for 4 days. Acetonitrile (9.6 mL), calcium carbonate (0.67 g, 6.65 mmol), and DOWEX 50Wx8 (2.24 g, 2.42 mmol) were added, and the mixture was stirred at room temperature for 1.5 hours. After the reaction solution was degassed under reduced pressure, triethylamine (0.8 mL) was added and the reaction solution was stirred for 1 hour. The insoluble material was filtered off and washed with acetonitrile (8.0 mL). The resulting filtrate was distilled under reduced pressure to remove the solvent to about 4 mL. Acetonitrile was added to adjust the solution to 8 mL. To the resulting solution (8 mL) was added 5 mol/L aqueous hydrochloric acid (280 µL). Acetonitrile (9.6 mL) was then added. After that, the mixture was stirred for 1 hour. Solids were filtered from the resulting suspension, and washed with acetonitrile (4.0 mL). The solids were dried at room temperature overnight to obtain the target compound as white crystals (0.48 g; yield: 91%).

### Example A7-3:

### Synthesis of bis(N,N-diethylethanaminium) N-[4-(11,12-didehydrodibenzo[b,f]azocin-5(6H)-yl)-4-oxobutanoyl]glycylglycyl-L-phenylalanyl-N-[(2-{9-[(2R,5R,7R,8R,10R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]glycinamide (corresponding to a compound of formula (Rp,Rp-XII))

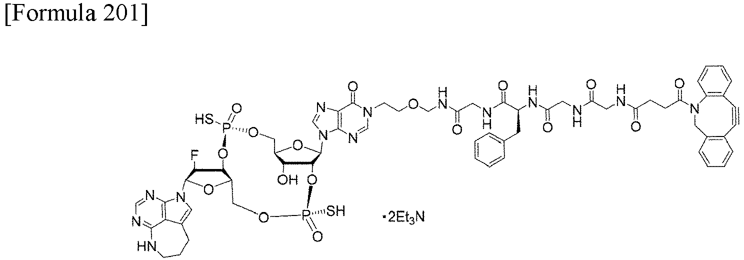

### (Step 3)

To a suspension containing the compound (132.6 mg, 0.23 mmol) obtained in Reference Example A7-1 in acetonitrile (1.8 mL) and water (0.44 mL) was added triethylamine (52.6 mg, 0.52 mmol). Afterwards, the compound (222.0 mg, 0.26 mmol) obtained in Example A7-2 was added. Next, 4-(4,6-dimethoxy-1,3,5-triazine-2-yl)-4-methylmorpholinium chloride (93.5 mg, 0.34 mmol) was added at room temperature, and the mixture was stirred at room temperature for 18 hours. Acetonitrile (2.2 mL), cyclopentyl methyl ether (2.2 mL), and 13.8% aqueous sodium sulfate solution (3.3 mL) were added to the reaction solution, and the mixture was stirred. The aqueous layer was discarded and the resulting organic layer was distilled under reduced pressure to remove the solvent. The residue was purified by silica gel column chromatography to obtain the target compound as a white solid (222 mg; yield: 59%).
1H-NMR (500 MHz, CD₃OD) δ: 8.58 (1H, s), 8.09 (1H, s), 8.04 (1H, s), 7.57-7.49 (2H, m), 7.43-7.34 (3H, m), 7.32-7.08 (9H, m), 6.47 (1H, d, J = 16.9 Hz), 6.23 (1H, d, J = 7.9 Hz), 5.56-5.37 (2H, m), 5.31-5.17 (1H, m), 5.03 (1H, d, J = 13.9 Hz), 4.79 (1H, d, J = 4.2 Hz), 4.64-4.38 (6H, m), 4.36-4.21 (4H, m), 4.05-3.60 (10H, m), 3.53-3.42 (3H, m), 3.18 (12H, q, J = 7.3 Hz), 3.01-2.92 (1H, m), 2.86-2.73 (1H, m), 2.70-2.54 (2H, m), 2.37-2.16 (2H, m), 2.06-1.77 (3H, m), 1.28 (18H, t, J = 7.3 Hz).

### Example A7-4:

### Synthesis of dipotassium N-[4-(11,12-didehydrodibenzo[b,f]azocin-5(6H)-yl)-4-oxobutanoyl]glycylglycyl-L-phenylalanyl-N-[(2-{9-[(2R,5R,7R,8R,10R, 12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]glycinamide (corresponding to a compound of formula (Rp,Rp-XII))

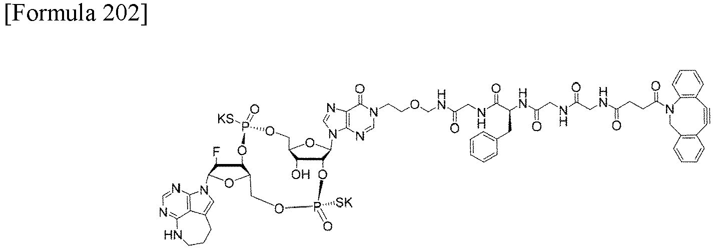

### (Step 3')

To a suspension containing the compound (177.3 mg, 0.31 mmol) obtained in Reference Example A7-1 in acetonitrile (3.9 mL) and water (0.78 mL) was added triethylamine (70.8 mg, 0.70 mmol). Afterwards, the compound (300.0 mg, 0.35 mmol) obtained in Example A7-2 was added. Next, 4-(4,6-dimethoxy-1,3,5-triazine-2-yl)-4-methylmorpholinium chloride (125.1 mg, 0.45 mmol) was added at room temperature, and the mixture was stirred at room temperature for 5.5 hours. Acetonitrile (3.9 mL), cyclopentyl methyl ether (2.0 mL), and 13.8% aqueous sodium sulfate solution (5.8 mL) were added to the reaction solution, and the mixture was stirred. The aqueous layer was then discarded to obtain an organic layer. The organic layer was added dropwise at room temperature to a 2-propanol solution (19.5 mL) containing potassium 2-ethylhexanoate (380.4 mg, 2.09 mmol), and the mixture was stirred for 1 hour. Cyclopentyl methyl ether (7.8 mL) was added, and the mixture was stirred for about 15 hours. Solids were filtered from the resulting suspension and washed with 2-propanol/acetonitrile/cyclopentyl methyl ether (5/2/2, 3 mL). The solids were dried at room temperature under reduced pressure for about 8 hours to obtain the target compound as a white solid (384.6 mg; yield: 74%).
1H-NMR (500 MHz, DMSO-d₆) δ: 8.68 (brs, 1H), 8.61 (dd, J = 13.2, 6.3 Hz, 1H), 8.35 (dt, J = 10.9, 5.7 Hz, 1H), 8.25 (brs, 1H), 8.17 (dt, J = 16.6, 5.7 Hz, 1H), 8.13-7.99 (m, 3H), 7.70-7.62 (m, 1H), 7.60-7.55 (m, 2H), 7.50-7.46 (m, 1H), 7.46-7.41 (m, 2H), 7.38-7.27 (m, 3H), 7.26-7.20 (m, 4H), 7.18-7.13 (m, 1H), 7.11 (brs, 1H), 6.37 (dd, J = 14.6, 3.7 Hz, 1H), 6.03 (d, J = 8.0 Hz, 1H), 5.48 (dt, J = 51.0, 3.9 Hz, 1H), 5.22-5.16 (m, 1H), 5.12-5.04 (m, 1H), 5.00 (d, J = 13.7 Hz, 1H), 4.93 (brs, 1H), 4.62-4.57 (m, 1H), 4.56-4.50 (m, 2H), 4.49-4.43 (m, 1H), 4.36 (brs, 1H), 4.26-4.17 (m, 3H), 4.06-3.96 (m, 3H), 3.82-3.51 (m, 11H), 3.04 (dd, J = 13.7, 4.6 Hz, 1H), 2.79 (dd, J = 13.7, 9.7 Hz, 1H), 2.70-2.57 (m, 3H), 2.29 (dt, J = 15.5, 7.7 Hz, 1H), 2.12-2.02 (m, 1H), 1.91-1.74 (m, 3H), 1.59-1.52 (m, 1H).

### Example A7-5:

### Synthesis of disodium N-[4-(11,12-didehydrodibenzo[b,f]azocin-5(6H)-yl)-4-oxobutanoyl]glycylglycyl-L-phenylalanyl-N-[(2-{9-[(2R,5R,7R,8R,10R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-7-yl]-6-oxo-6,9-dihydro-1H-purin-1-yl}ethoxy)methyl]glycinamide (corresponding to a compound of formula (Rp,Rp-XII))

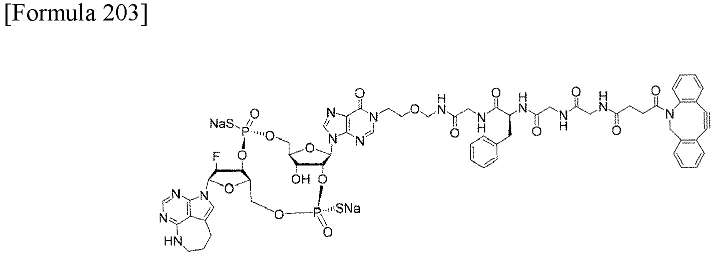

### (Step 3")

To an acetonitrile suspension (0.5 mL) containing the compound (18.1 mg, 0.03 mmol) obtained in Reference Example A7-1 were added triethylamine (12.1 µL, 0.09 mmol) and 4-(4,6-dimethoxy-1,3,5-triazine-2-yl)-4-methylmorpholinium chloride (10.4 mg, 0.04 mmol) at room temperature. Afterwards, the compound (25.0 mg, 0.03 mmol) obtained in Example A7-2 was added at room temperature, and the mixture was stirred for 2 hours. The reaction solution was concentrated under reduced pressure, and ethyl acetate (0.75 mL) and 5% aqueous sodium carbonate solution (0.25 mL) were added to the resulting residue. An oil component was separated from the two-layer solution, and acetonitrile (1 mL) was added to the oil component. The mixture was then stirred at room temperature for 6 hours. A supernatant component was removed from the resulting suspension to obtain a solid. The solid was dried under reduced pressure for about 5 hours to obtain the target compound as a white solid (38.2 mg; yield: 91%).

MS (ESI) m/z: 1411(M+H)⁺, 1409(M-H)⁻.

### A-8 Synthesis 3 of CDN1

A cyclic dinucleotide (CDN1) was synthesized according to the following synthesis scheme A-8. In synthesis scheme A-8, A2 = a Teoc-tetraazabenzo[cd]azulene group, Q = a thiol group, PG1 = TBS, PG2 = DMTr, PG3 = Teoc, and PG6 = TFAc. (3aR)-1-chloro-3,3-dimethyltetrahydro-1H,3H-pyrrolo[1,2-c][1,3,2]oxazaphosphole was used as (Rc-II).

### [Synthesis Scheme A-8]

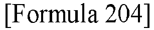

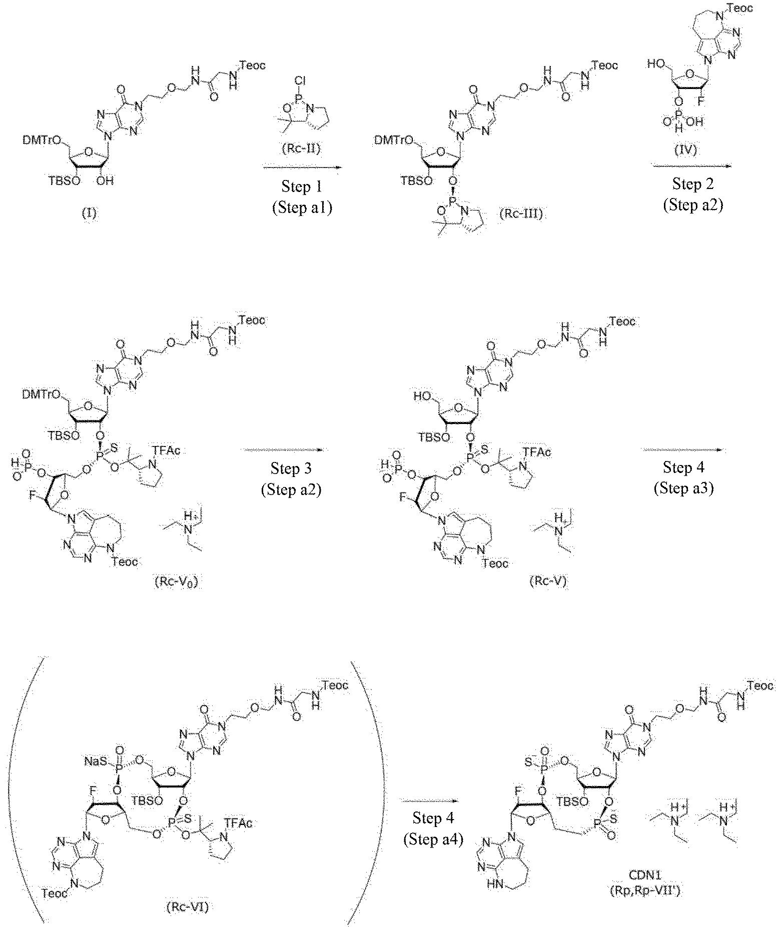

In the following Examples A8-1 to A8-4, preparative HPLC was performed using an Agilent Preparative HPLC System (manufactured by Agilent Technology). The preparative column used was YMC-Actus Triart C18 (250*20 mm I.D.S-5 µm, 12 nm).

The following instruments were used to measure various spectral data. Mass spectra were measured using Shimadzu LCMS-2020 (manufactured by Shimadzu Corporation). LC/MS measurements were performed under the following conditions [column: XBridge C18, 3.5 µm, 150 × 4.6 mm (manufactured by Waters); mobile phase: 10 mM ammonium acetate (or triethylamine acetate)/acetonitrile].

### Example A8-1:

### Synthesis of 5'-O-[bis(4-methoxyphenyl)(phenyl)methyl]-3'-O-[tert-butyl(dimethyl)silyl]-2'-O-3,3-dimethyltetrahydro-1H,3H-pyrrolo[1,2-c][1,3,2]oxazaphosphol-1-yl]-1-(2-{[(N-{[2-[trimethylsilyl)ethoxy]carbonyl}glycyl]amino]methoxy}ethyl)inosine (corresponding to a compound of formula (Rc-III))

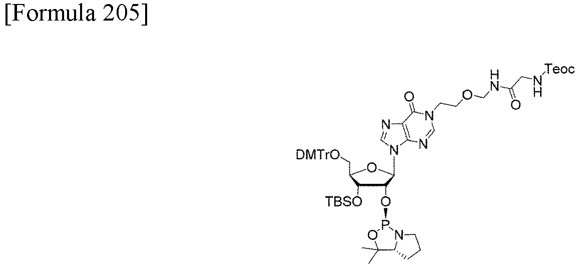

### (Step 1)

Dehydrated dichloromethane (14 mL) and Molecular Sieve 3A (200 mg) were added to 5'-O-[bis(4-methoxyphenyl)(phenyl)methyl]-3'-O-[tert-butyl(dimethyl)silyl]-1 -(2- {[(N- {[2-(trimethylsilyl)ethoxy]carbonyl}glycyl)amino]methoxy}ethyl)inosine (1.00 g). The mixture was stirred for 2 hours. The reaction system was then cooled to -10°C and triethylamine (0.58 mL) was added thereto. To the above solution was added dropwise a dehydrated dichloromethane solution (6 mL) containing (3aR)-1-chloro-3,3-dimethyltetrahydro-1H,3H-pyrrolo[1,2-c][1,3,2]oxazaphosphole (0.504 g) described in a known publication (Angewandte Chemie International Edition, 2009, 48, 496-499). The reaction solution was stirred at room temperature for 1 hour, and concentrated. The concentrated crude product was dissolved and diluted in dichloromethane and purified by amino silica gel chromatography [dichloromethane/ethyl acetate] to obtain the target compound (1.06 g).
MS (ESI) m/z: 1148(M+H)⁺, 1146(M-H)⁻.
³¹P-NMR (MeCN-d₃) δ: 149.68.

### Example A8-2:

### Synthesis ofN,N-diethylethanaminium(2R,3R,4R,5R)-2-({[(R)-({(2R,3R,4R,5R)-5-{[bis(4-methoxyphenyl)(phenyl)methoxy]methyl}-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]oxolan-3-yl}oxy)({2-[(2R)-1 - (trifluoroacetyl)pyrrolidin-2-yl]propan-2-yl}oxy)phosphorothioyl]oxy}methyl)-4-fluoro-5-(6-{ [2-[trimethylsilyl]ethoxy]carbonyl}-6,7,8,9-tetrahydro-2H-2,3,5,6-tetraazabenzo[cd]azulen-2-yl)-3-yl phosphonate (corresponding to a compound of formula (Rc-V₀))

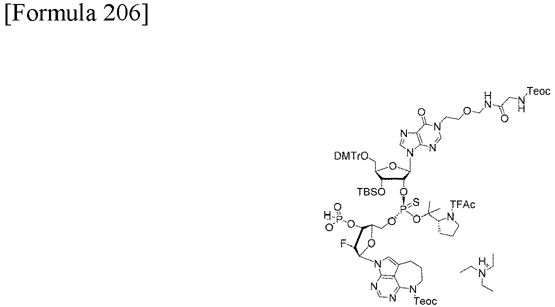

### (Step 2)

The triethylamine salt (250 mg) of 2-(trimethylsilyl)ethyl-2-{2-deoxy-2-fluoro-3-O-[hydroxy(oxo)-λ⁵-phosphanyl]-β-D-ribofuranosyl}-2,7,8,9-tetrahydro-6H-2,3,5,6-tetraazabenzo[cd]azulene was dissolved in dehydrated acetonitrile (2.5 mL). Molecular Sieve 3A (75 mg) was then added, and the mixture was stirred for 1 hour. In a separate vessel, the compound (497 mg) obtained in Example A8-1 was dissolved in dehydrated acetonitrile (2.5 mL). Molecular Sieve 3A (50 mg) was then added, and the mixture was stirred for 1 hour. This was then added to an acetonitrile solution containing the triethylamine salt of 2-(trimethylsilyl)ethyl-2-{2-deoxy-2-fluoro-3-O-[hydroxy(oxo)-λ⁵-phosphanyl]-β-D-ribofuranosyl}-2,7,8,9-tetrahydro-6H-2,3,5,6-tetraazabenzo[cd]azulene as previously prepared and cooled to -10°C. A dehydrated acetonitrile solution (1.01 mL) containing 1-methylbenzimidazole triflate prepared at 0.8 mol/L was added, and the mixture was stirred at-10°C for 1 hour. Triethylamine (164 mg) was then added, followed by 1-trifluoroacetylimidazole (119 mg), and the mixture was stirred for 1 hour. Subsequently, xanthane hydride (58 mg) was added and stirred for 1 hour. The reaction solution was filtered to remove the Molecular Sieve. The filtrate was concentrated and subjected to purification by silica gel chromatography [methanol/acetonitrile (0.4% triethylamine)] to obtain the target compound. The diastereomers on the phosphorus atom as formed during the reaction were at a ratio of >99 : 1 as measured by HPLC.
MS (ESI) m/z: 1762(M+H)⁺, 1760(M-H)⁻.
³¹P-NMR (MeCN-d₃) δ: 61.43, 2.03.

### Example A8-3:

### Synthesis ofN,N-diethylethanaminium(2R,3R,4R,5R)-2-({[(R)-{[(2R,3R,4R,5R)-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy}({2-[(2R)-1-(trifluoroacetyl)pyrrolidin-2-yl]propan-2-yl}oxy)phosphorothioyl]oxy}methyl)-5-(6-{[2-(trimethylsilyl)ethoxy]carbonyl}-6,7,8,9-tetrahydro-2H-2,3,5,6-tetraazabenzo[cd]azulen-2-yl)-4-fluorooxolane-3-yl phosphonate (corresponding to a compound of formula (Rc-V))

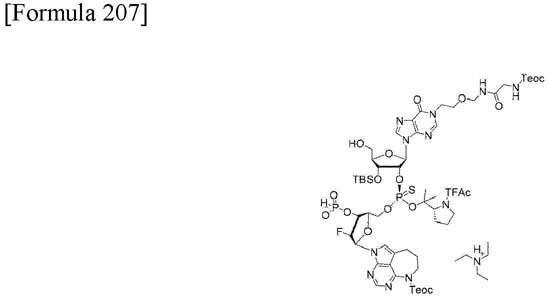

### (Step 3)

The compound (180 mg) obtained in Example A8-2 was dissolved in dehydrated acetonitrile (0.54 mL) and dichloromethane (3.6 mL). Ethanol (0.23 mL) and dichloroacetic acid (38.0 mg) were added, and the mixture was stirred at room temperature overnight. Triethylamine (39.7 mg) was then added and the mixture was stirred for 5 min. After that, water (1.8 mL) was added and the mixture was liquid-separated. Water (1.8 mL), acetonitrile (0.54 mL), and ethanol (0.23 mL) were added to the organic layer, and the mixture was liquid-separated again. Subsequently, the organic layer was concentrated to dryness, and acetonitrile (4 mL) was added. The mixture was concentrated again to dryness to obtain the target compound.
MS (ESI) m/z: 1459(M+H)⁺, 1457(M-H)⁻.
³¹P-NMR (MeCN-d₃) δ: 60.77, 1.72.

### Example A8-4:

### Synthesis of bis(N,N-diethylethanaminium)(2R,5R,7R,8R,10R, 12aR,14R,15R,15aR,16R)-16-{[tert-butyl(dimethyl)silyl]oxy}-15-fluoro-2,10-dioxo-7-[6-oxo-1-(2-{[(N-{[2-(trimethylsilyl)ethoxy]carbonyl}glycyl)amino]methoxy}ethyl)-1,6-dihydro-9H-purin-9-yl]-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-2,10-bis(thiolate) (corresponding to a compound of formula (Rp,Rp-VII'))

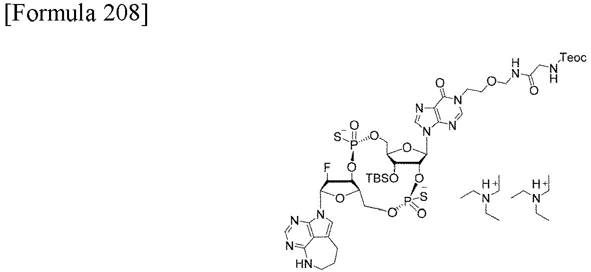

### (Step 4)

To the crude product obtained in Example A8-3 was added dehydrated acetonitrile (0.54 mL), and the mixture was cooled to -15°C. To another vessel were added dehydrated pyridine (1.53 mL), dichloromethane (1.53 mL), and 50% acetonitrile solution (176 µL) containing propylphosphonic anhydride, and the mixture was cooled to -15°C. The above acetonitrile solution was added to a vessel of propylphosphonic anhydride solution, and the mixture was stirred at -15°C for 1 hour. Xanthane hydride (23 mg) was then added, and the mixture was heated to 0°C and stirred for 1 hour. To the resulting reaction solution were added an aqueous solution (0.8 mL) containing sodium bicarbonate (43 mg) and salt (43 mg) and ethyl acetate (1.6 mL), and the mixture was liquid-separated. The organic layer was washed with an aqueous solution (0.8 mL) containing sodium bicarbonate (43 mg) and salt (43 mg). The organic layer was then concentrated to dryness to obtain sodium (2R,5R,7R,8R,10R,12aR,14R,15R,15aR,16R)-16-{ *[tert-*butyl(dimethyl)silyl]oxy}-15-fluoro-2-oxo-7-[6-oxo-1-(2-{[(N-{[2-(trimethylsilyl)ethoxy]carbonyl}glycyl)amino]methoxy}ethyl)-1,6-dihydro-9H-purin-9-yl]-10-sulfaniliden-14-(6-{[2-(trimethylsilyl)ethoxy]carbonyl}-6,7,8,9-tetrahydro-2H-2,3,5,6-tetraazabenzo[cd]azulen-2yl)-10-({2-[(2R)-1-(trifluoroacetyl)pyrrolidin-2-yl]propan-2-yl}oxy)octahydro-2H,10H,12H-5,8-methano-2λ⁵,10λ⁵-furo[3,2-1][1,3,6,9,11,2,10]pentaoxadiphosphacyclotetradecin-2-thiolate (corresponding to a compound of formula (VI)). To the resulting compound were added pyridine (0.48 mL) and 28% ammonia water (1.01 mL), and the mixture was stirred at 53°C for 15 hours. The resulting reaction solution was washed twice with n-heptane. The aqueous layer was then concentrated. The residue was purified by preparative HPLC [10 mM aqueous triethylamine acetate solution/acetonitrile] to obtain 66.1 mg of the target compound as a single diastereomer (Rp,Rp form) on the phosphorus atom. Note that the HPLC purity of the isolated compound was 99.8%.
MS (ESI) m/z: 1121(M+H)⁺, 1119(M-H)⁻.
¹H-NMR (MeCN-d₃) δ: 8.61 (1H, s), 8.10 (1H, s), 8.07 (1H, brs), 7.84-7.82 (1H, m), 7.50 (1H, brs), 7.03 (1H, brs), 6.37 (1H, dd, J = 16.5, 3.0 Hz), 6.10 (1H, d, J = 8.0 Hz), 6.06 (1H, brm), 5.51 (1H, dt, J = 52.5, 4.0 Hz), 5.37 (1H, m), 5.31 (1H, m), 4.57 (2H, d, J = 7.0 Hz), 4.52 (1H, d, J = 3.5 Hz), 4.28 (2H, m), 4.19 (3H, m), 4.07 (3H, m), 3.91 (1H, ddd, J = 12.0, 5.0, 2.0 Hz), 3.84 (1H, ddd, J = 12.0, 5.0, 2.0 Hz), 3.70 (2H, m), 3.62 (2H, m), 3.46 (2H, m), 2.99 (6H, q, J = 7.0 Hz), 2.86 (1H, m), 2.73 (1H, m), 1.95 (2H, m), 1.17 (9H, t, J = 7.0 Hz), 0.95-0.92 (2H, m), 0.94 (9H, s), 0.23 (3H, s), 0.20 (3H, s), 0.00 (9H, s).
³¹P-NMR (MeCN-d₃) δ: 58.31, 57.22.

### A-9 Synthesis of CDN3

A cyclic dinucleotide (CDN3) was synthesized according to the following synthesis scheme A-9. In synthesis scheme A-9, A2 = a Teoc-tetraazabenzo[cd]azulene group, Q = a hydroxy group, PG1 = TBS, PG3 = Teoc, and PG6 = TFAc.

### [Synthesis Scheme A-9]

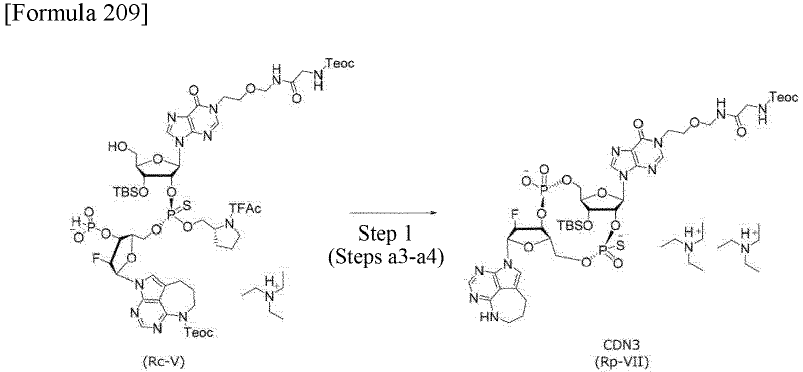

In Example A9-1 below, the preparative column used was YMC-Actus Triart C18 (250*20 mm I.D.S-5 µm, 12 nm).

The following instruments were used to measure various spectral data. Mass spectra were measured using Shimadzu LCMS-2020 (manufactured by Shimadzu Corporation). LC/MS measurements were performed under the following conditions [column: XBridge C18, 3.5 µm, 150 × 4.6 mm (manufactured by Waters); mobile phase: 10 mM ammonium acetate (or triethylamine acetate)/acetonitrile].

### Example A9-1:

### Synthesis of bis(N,N-diethylethanaminium)(5R,7R,8R,10R,12aR,14R,15R,15aR,16R)-16-{ [tert-butyl(dimethyl)silyl]oxy}-15-fluoro-2,10-dioxo-7-[6-oxo-1-(2-{[(N-{[2-(trimethylsilyl)ethoxy]carbonyl}glycyl)amino]methoxy}ethyl)-1,6-dihydro-9H-purin-9-yl]-10-sulfide-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecin-2-olate (corresponding to a compound of formula (Rp-VII))

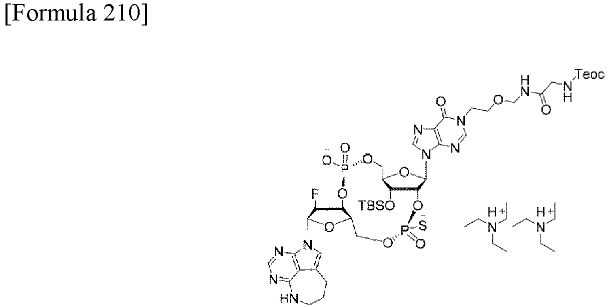

### (Step 1)

To a dehydrated pyridine solution (1.7 mL) containing N,N-diethylethanaminium(2R,3R,4R,5R)-2-({ [(R)-{ [(2R)-1-acetylpyrrolidin-2-yl]methoxy}{[(2R,3R,4R,5R)-4-{[*tert*-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy}phosphorothioyl]oxy}methyl)-5-(6-benzoyl-6,7,8,9-tetrahydro-2H-2,3,5,6-tetraazabenzo[cd]azulen-2-yl)-4-fluorooxolan-3-yl phosphonate (170 mg) was added dehydrated acetonitrile (0.54 mL), and the mixture was cooled to -15°C. To another vessel were added dehydrated dichloromethane (1.7 mL) and 50% acetonitrile solution (192 µL) containing propylphosphonic anhydride, and the mixture was cooled to -15°C. The above dehydrated pyridine-acetonitrile solution was added to a vessel of propylphosphonic anhydride solution, and the mixture was stirred at -15°C for 1 hour. Iodine (85 mg) was then added, and the mixture was heated to room temperature and stirred for 1 hour. To the resulting reaction solution was added water (0.5 mL), and the mixture was stirred for 10 minutes. Iodine was quenched by adding a saturated solution of sodium sulfite. Thereafter, ethyl acetate (1.8 mL) was added and the mixture was liquid-separated. The organic layer was then washed with 20% brine (1.0 mL). Subsequently, the organic layer was concentrated to dryness. Pyridine (0.6 mL) and 28% ammonia water (1.26 mL) were then added. After that, the mixture was stirred at 53°C for 15 hours. The resulting reaction solution was washed twice with n-heptane. The aqueous layer was then concentrated. The residue was purified by preparative HPLC [10 mM aqueous triethylamine acetate solution/acetonitrile] to obtain 52 mg of the target compound as a single diastereomer (Rp form) on the phosphorus atom. Note that the HPLC purity of the isolated compound was 98.7%.
MS (ESI) m/z: 1105(M+H)⁺, 1103(M-H)⁻.
¹H-NMR (MeCN-d₃) δ: 8.63 (1H, brs), 8.18 (1H, brs), 8.08 (2H, s), 7.18 (1H, brs), 6.35 (1H, d, J = 21.5 Hz), 6.11 (1H, d, J = 5.0 Hz), 6.10 (1H, brm), 5.42 (1H, brm), 5.17 (1H, m), 4.56-4.47 (3H, m), 4.29-4.18 (4H, m), 4.07 (3H, m), 3.97 (1H, m), 3.94 (1H, m), 3.83 (1H, dd, 3.5, 10.0 Hz), 3.69-3.66 (3H, m), 3.46 (2H, brs), 2.90 (9H, q, J = 7.5 Hz), 2.67 (2H, brm), 1.93 (5H, m), 1.11 (15H, t, J = 7.5 Hz), 0.95 (9H, s), 0.92-0.91 (2H, m), 0.25 (3H, s), 0.22 (3H, s), 0.00 (9H, s).
³¹P-NMR (MeCN-d₃) δ: 55.08, -1.44.

### A-10 Synthesis of CDN4

A cyclic dinucleotide (CDN4) was synthesized according to the following synthesis scheme A-10. In synthesis scheme A-10, A2 = a Teoc-tetraazabenzo[cd]azulene group, Q = a thiol group, PG1 = TBS, PG2 = DMTr, PG3 = Teoc, and PG6 = TFAc. (3aS)-1-chlorotetrahydro-1H,3H-pyrrolo[1,2-c][1,3,2]oxazaphosphole was used as (Sc-II).

### [Synthesis Scheme A-10]

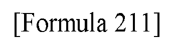

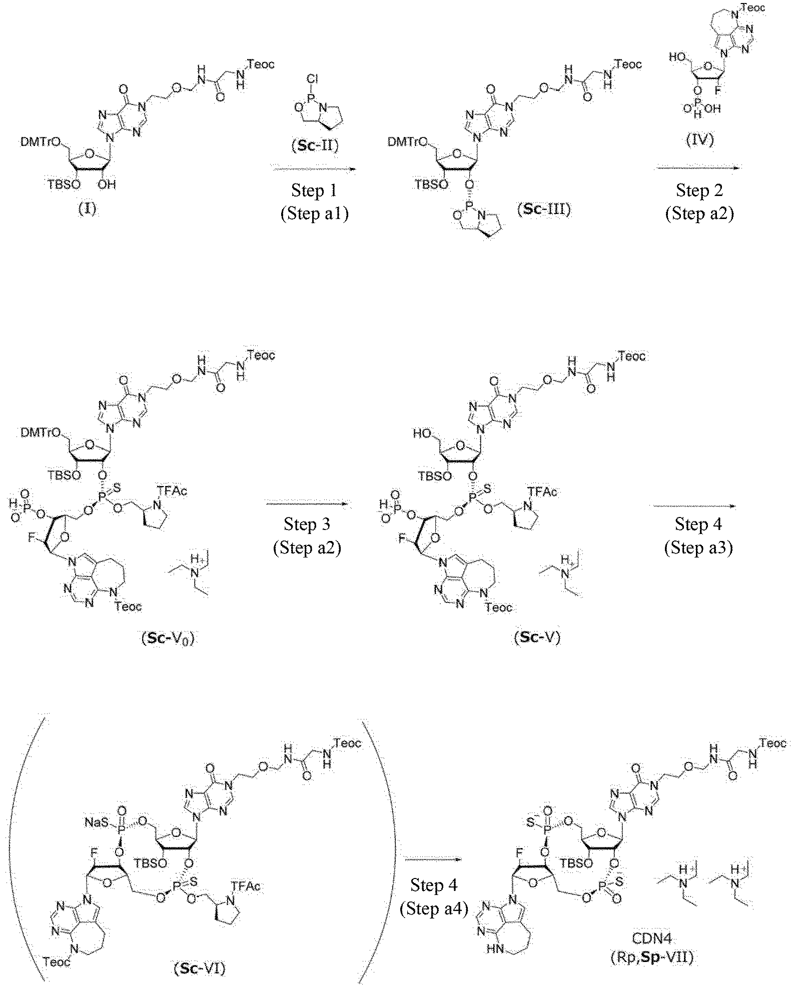

In Examples A10-1 to A10-4 below, the preparative column used was YMC-Actus Triart C18 (250*20 mm I.D.S-5 µm, 12 nm).

The following instruments were used to measure various spectral data. LC/MS measurements were performed under the following conditions [column: XBridge C18, 3.5 µm, 150 × 4.6 mm (manufactured by Waters); mobile phase: 10 mM ammonium acetate (or triethylamine acetate)/acetonitrile].

### Example A10-1:

### Synthesis of 5'-O-[bis(4-methoxyphenyl)(phenyl)methyl]-3'-O-[tert-butyl(dimethyl)silyl]-2'-O-[(1R,3aS)-tetrahydro-1H,3H-pyrrolo[1,2-c][1,3,2]oxazaphosphol-1-yl]-1-(2-{[(N-{[2-(trimethylsilyl)ethoxy]carbonyl}glycyl)amino]methoxy}ethyl)inosine (corresponding to a compound of formula (Sc-III))

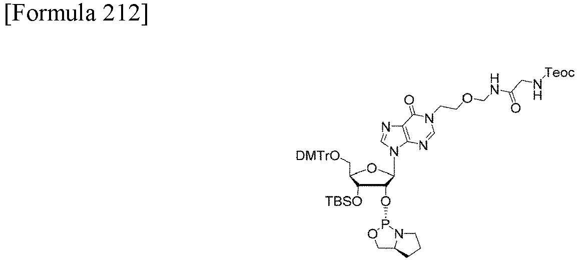

### (Step 1)

Dehydrated dichloromethane (42 mL) and Molecular Sieve 3A (600 mg) were added to 5'-O-[bis(4-methoxyphenyl)(phenyl)methyl]-3'-O-[tert-butyl(dimethyl)silyl]-1-(2-{[(N-{[2-(trimethylsilyl)ethoxy]carbonyl}glycyl)amino]methoxy}ethyl)inosine (3.00 g). The mixture was stirred for 2 hours. The reaction system was then cooled to -10°C and triethylamine (0.575 mL) was added thereto. To the above solution was added dropwise (3aS)-1-chlorotetrahydro-1H,3H-pyrrolo[1,2-c][1,3,2]oxazaphosphole-containing dehydrated dichloromethane (9 mL) as described in a known publication (Tetrahedron Letter, 1998, 39, 2491-2494). The mixture was stirred at room temperature for 1 hour. The reaction solution was then concentrated. The concentrated crude product was dissolved and diluted in dichloromethane and purified by amino silica gel chromatography [dichloromethane/ethyl acetate] to obtain the target compound (3.37 g).
MS (ESI) m/z: 1088(M+H)⁺, 1086(M-H)⁻.
¹H-NMR (MeCN-d₃) δ: 7.93 (2H, s), 7.43-7.41 (2H, m), 7.31-7.26 (7H, m), 7.22-7.21 (1H, m), 6.84 (4H, d, J = 8.5 Hz), 5.88 (1H, m), 5.72 (1H, brm), 4.97 (1H, m), 4.55-4.53 (2H, m), 4.37 (1H, s), 4.17-4.02 (6H, m), 3.74 (6H, s), 3.69 (2H, s), 3.58-3.56 (2H, m), 3.44-3.42 (2H, m), 3.31-3.22 (3H, m), 2.71-2.70 (1H, m), 1.67-1.61 (2H, m), 1.48-1.46 (1H, m) 1.31-1.30 (1H, m), 0.94-0.91 (2H, m), 0.86 (9H, s), 0.08 (3H, s), 0.03 (3H, s), 0.00 (9H, s).
³¹P-NMR (MeCN-d₃) δ: 154.01.

### Example A10-2:

### Synthesis ofN,N-diethylethanaminium(2R,3R,4R,5R)-2-({[(S)-({(2R,3R,4R,5R)-5-{[bis(4-methoxyphenyl)(phenyl)methoxy]methyl}-4-{[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]oxolan-3-yl}oxy){ [(2S)-1-(trifluoroacetyl)pyrrolidin-2-yl]methoxy}phosphorothioyl]oxy)methyl)-5-(6-{[2-[trimethylsilyl]ethoxy]carbonyl }-6,7,8,9-tetrahydro-2H-2,3,5,6-tetraazabenzo[cd]azulen-2-yl)-4-fluorooxolan-3-yl phosphonate (corresponding to a compound of formula (Sc-V₀))

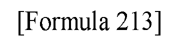

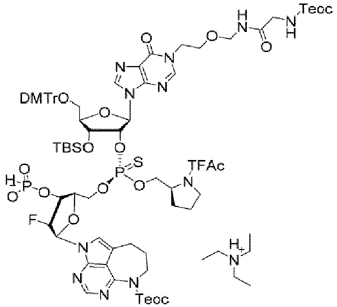

### (Step 2)

A triethylamine salt (200 mg) of 2-(trimethylsilyl)ethyl -2-{2-deoxy-2-fluoro-3-O-[hydroxy(oxo)-λ⁵-phosphanyl]-β-D-ribofuranosyl}-2,7,8,9-tetrahydro-6H-2,3,5,6-tetraazabenzo[cd]azulene was dissolved in dehydrated acetonitrile (1.0 mL). Molecular Sieve 3A (60 mg) was then added, and the mixture was stirred for 1 hour. In a separate vessel, the compound (388 mg) obtained in Example A10-1 was dissolved in dehydrated acetonitrile (1.0 mL). Molecular Sieve 3A (40 mg) was then added, and the mixture was stirred for 1 hour. This was then added to an acetonitrile solution containing the above triethylamine salt of 2-(trimethylsilyl) ethyl-2-{2-deoxy-2-fluoro-3-O-[hydroxy(oxo)-λ⁵-phosphanyl]-β-D-ribofuranosyl}-2,7,8,9-tetrahydro-6H-2,3,5,6-tetraazabenzo[cd]azulene as cooled to -10°C. A dehydrated acetonitrile solution (1.21 mL) containing N-phenylimidazolium triflate prepared at 0.4 mol/L was added, and the mixture was stirred at -10°C for 1 hour. Triethylamine (131 mg) was then added. Thereafter, 1-trifluoroacetylimidazole (96 mg) was added, and the mixture was stirred for 30 minutes. Subsequently, xanthane hydride (58 mg) was added and stirred for 30 min. The reaction solution was filtered to remove the Molecular Sieve. The filtrate was concentrated, subjected to purification by silica gel chromatography [methanol/acetonitrile (0.4% triethylamine)], and concentrated to dryness to obtain the target compound. The diastereomers on the phosphorus atom as formed during the reaction were at a ratio of 78.6 : 21.3 as measured by HPLC. The resulting target diastereomers were selected as 84.7 : 15.3.
MS (ESI) m/z: 1734(M+H)⁺, 1732(M-H)⁻.
³¹P-NMR (MeCN-d₃) δ: 69.19 (Major), 68.11 (Minor), 1.55.

### Example A10-3:

### Synthesis ofN,N-diethylethanaminium(2R,3R,4R,5R)-2-({[(S)-{[(2R,3R,4R,5R)-4-[tert-butyl(dimethyl)silyl]oxy}-2-[1-(2,2-dimethyl-6,9-dioxo-5,12-dioxa-7,10-diaza-2-silatetradecan-14-yl)-6-oxo-1,6-dihydro-9H-purin-9-yl]-5-(hydroxymethyl)oxolan-3-yl]oxy} f [(2S)-1-(trifluoroacetyl)pyrrolidin-2-yl]methoxy}phosphorothioyl]oxy}methyl)-5-(6-{[2-(trimethylsilyl)ethoxy]carbonyl}-6,7,8,9-tetrahydro-2H-2,3,5,6-tetraazabenzo[cd]azulen-2-yl)-4-fluorooxolane-3-yl phosphonate (corresponding to a compound of formula (Sc-V))

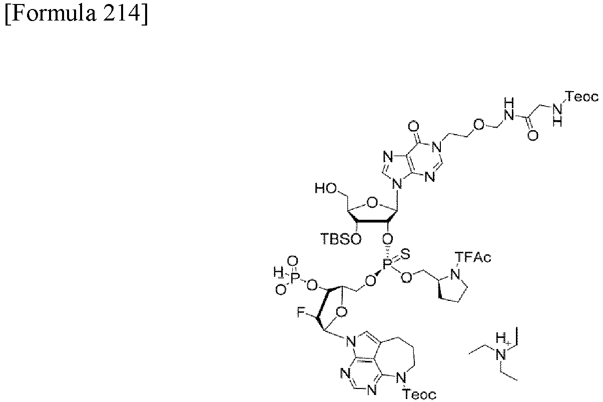

### (Step 3)

The compound (180 mg) obtained in Example A10-2 was dissolved in dehydrated acetonitrile (0.54 mL) and dichloromethane (3.6 mL). Ethanol (0.23 mL) and dichloroacetic acid (38.0 mg) were added, and the mixture was stirred at room temperature overnight. Triethylamine (39.7 mg) was then added and the mixture was stirred for 5 min. After that, water (1.8 mL) was added and the mixture was liquid-separated. Water (1.8 mL), acetonitrile (0.54 mL), and ethanol (0.23 mL) were added to the organic layer, and the mixture was liquid-separated again. Subsequently, the organic layer was concentrated to dryness, and acetonitrile (4 mL) was added. The mixture was concentrated again to dryness to obtain the target compound.
MS (ESI) m/z: 1430(M+H)⁺, 1428(M-H)⁻.
³¹P-NMR (MeCN-d₃) δ: 69.30 (Major), 68.80 (Minor), 1.39.

### Example A10-4:

### Synthesis of bis(N,N-diethylethanaminium)(2R,5R,7R,8R,10S,12aR,14R,15R,15aR,16R)-16-{[tert-butyl(dimethyl)silyl]oxy}-15-fluoro-2,10-dioxo-7-[6-oxo-1-(2-{[(N-{[2-(trimethylsilyl)ethoxy]carbonyl}glycyl)amino]methoxy}ethyl)-1,6-dihydro-9H-purin-9-yl]-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-l][1,3,6,9,11,2,10]pentaoxadiphosphacyclotetradecin-2,10-bis(thiolate) (corresponding to a compound of formula (Rp,Sp-VII'))

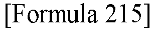

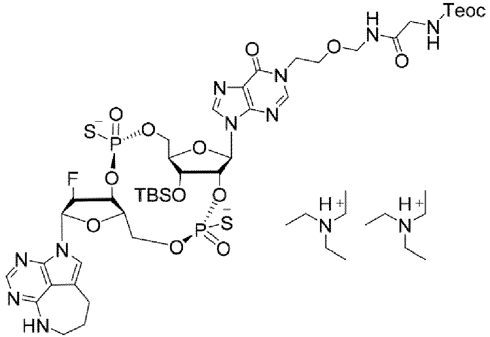

### (Step 4)

To the crude product obtained in Example A10-3 was added dehydrated acetonitrile (0.54 mL), and the mixture was cooled to -15°C. To another vessel were added dehydrated pyridine (1.53 mL), dichloromethane (1.53 mL), and 50% acetonitrile solution (172 µL) containing propylphosphonic anhydride, and the mixture was cooled to -15°C. The above acetonitrile solution containing the compound of Example A10-3 was added to a vessel of propylphosphonic anhydride solution, and the mixture was stirred at -15°C for 1 hour. Xanthane hydride (23 mg) was then added, and the mixture was heated to 0°C and stirred overnight. To the resulting reaction solution were added an aqueous solution (0.8 mL) containing sodium bicarbonate (43 mg) and salt (43 mg) and ethyl acetate (1.6 mL), and the mixture was liquid-separated. The organic layer was washed with an aqueous solution (0.8 mL) containing sodium bicarbonate (43 mg) and salt (43 mg). The organic layer was then concentrated to dryness to obtain sodium (2R,5R,7R,8R,10R,12aR,14R,15R,15aR,16R)-16-{[*tert*-butyl(dimethyl)silyl]oxy}-15-fluoro-2-oxo-7-[6-oxo-1-(2- f [(N- f [2-(trimethylsilyl)ethoxy]carbonyl}glycyl)amino]methoxy}ethyl)-1,6-dihydro-9H-purin-9-yl]-10-sulfaniliden-14-(6-{[2-(trimethylsilyl)ethoxy]carbonyl}-6,7,8,9-tetrahydro-2H-2,3,5,6-tetraazabenzo[cd]azulen-2-yl)-10-{[(2R)-1-(trifluoroacetyl)pyrrolidin-2-yl]methoxy}octahydro-2H,10H,12H-5,8-methano-2λ⁵,10λ⁵-furo[3,2-1][1,3,6,9,11,2,10]pentaoxadiphosphacyclotetradecin-2-thiolate (corresponding to a compound of formula (VI)). To the resulting compound were added pyridine (0.48 mL) and 28% ammonia water (1.01 mL), and the mixture was stirred at 53°C for 15 hours. The resulting reaction solution was washed twice with n-heptane. The aqueous layer was then concentrated. The residue was purified by preparative HPLC [10 mM aqueous triethylamine acetate solution/acetonitrile] to obtain 47.6 mg of the target compound as a single diastereomer (Rp,Sp form) on the phosphorus atom. Note that the HPLC purity of the isolated compound was 95.9%.
MS (ESI) m/z: 1121(M+H)⁺, 1119(M-H)⁻.
¹H-NMR (MeCN-d₃) δ: 8.94 (1H, brs), 8.22 (1H, brs), 8.08 (1H, s), 7.57 (1H, brs), 7.25 (1H, brs),, 6.30 (1H, dd, J = 13, 5.0 Hz), 6.11 (1H, d, J = 8.0 Hz), 6.03 (1H, brm), 5.61 (1H, brm), 5.40 (1H, m),, 4.56 (2H, d, J = 6.5 Hz), 4.90 (2H, d, J = 4.0 Hz), 4.31 (1H, m), 4.21-4.05 (7H, m), 3.97 (1H, dd, J = 11.0, 5.5 Hz), 3.78 (1H, m), 3.74-3.59 (4H, m), 3.50 (2H, m), 3.01 (10H, q, J = 7.5 Hz), 2.76 (2H, brm), 1.95 (2H, m), 1.19 (15H, t, J = 7.5 Hz), 0.95-0.92 (2H, m), 0.95 (9H, s), 0.20 (3H, s), 0.19 (3H, s), 0.00 (9H, s)
³¹P-NMR (MeCN-d₃) δ: 58.74, 56.83.

### [B. Synthesis of raw material compound (I)]

### B-1 Synthesis of compound (XV) (where X = Br)

The raw material compound (XV) used in synthesis scheme B-2 below was synthesized according to the following scheme.

### [Synthesis Scheme B-1]

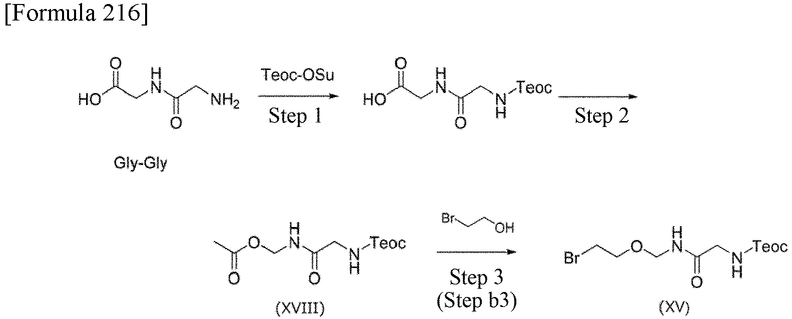

### Reference Example 1:

### Synthesis of [(N-{ [2-(trimethylsilyl)ethoxy]carbonyl}glycyl)amino]methyl acetate (corresponding to a compound of formula (XVIII))

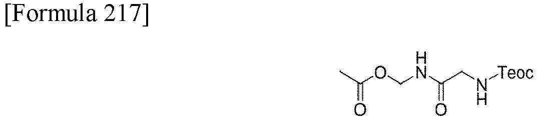

### (Step 1)

A solution containing glycylglycine (60 g, 0.45 mol), triethylamine (69 g, 0.68 mol), water (600 mL), and tetrahydrofuran (600 mL) was cooled to 0°C. Then, N-[2-(trimethylsilyl)ethoxycarbonyloxy]succinimide (130 g, 0.50 mol) was added, and the mixture was stirred at 25°C for 22 hours. The reaction solution was concentrated under reduced pressure to 900 mL, and ethyl acetate (600 mL) was added. After triethylamine (69 g, 0.68 mol) was added and stirred, the organic layer was discarded. Ethyl acetate (600 mL) was added to the resulting aqueous layer, and concentrated hydrochloric acid (116 g) was added. The aqueous layer was discarded, and the resulting organic layer was washed twice with 10 wt% brine (600 mL). The organic layer was concentrated under reduced pressure to obtain an ethyl acetate solution (120 mL) containing N-{[2-(trimethylsilyl)ethoxy]carbonyl}glycylglycine.

### (Step 2)

A solution containing lead tetraacetate (302 g, 0.68 mol), acetic acid (360 mL), and tetrahydrofuran (600 mL) was heated to 35°C. To this solution was added dropwise a solution prepared by adding tetrahydrofuran (600 mL) to N-{[2-(trimethylsilyl)ethoxy]carbonyl}glycylglycine-containing ethyl acetate solution (120 mL). After stirring at 35°C for 1 hour, the precipitate was filtered off and washed with ethyl acetate (600 mL). The filtrate was washed 7 times with 20 wt% aqueous trisodium citrate dihydrate solution (300 mL). Next, 20 wt% brine (180 mL) was added to the resulting organic layer. The pH was then set to 6.5 by adding an appropriate amount of 25 wt% aqueous sodium hydroxide solution. The aqueous layer was discarded. The resulting organic layer was concentrated under reduced pressure to about 600 mL. Thereafter, 1,2-dimethoxyethane (1200 mL) was added, and the mixture was concentrated under reduced pressure to about 600 mL. This procedure was repeated to obtain a 1,2-dimethoxyethane solution (about 600 mL) containing the target compound.

### Example 12:

### Synthesis of N-[(2-bromoethoxy)methyl]-N²-{[2-(trimethylsilyl)ethoxy]carbonyl}glycinamide (corresponding to a compound of formula (XV))

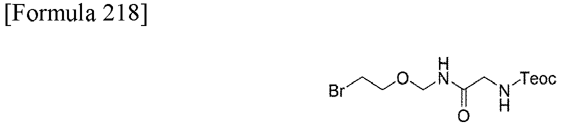

### (Step 3)

To a 1,2-dimethoxyethane solution (about 600 mL) containing the compound obtained in Reference Example 1 was added 1,2-dimethoxyethane (300 mL). Next, 2-bromoethanol (114 g, 0.91 mol) was added at room temperature. The mixture was cooled to 0°C. Then, 10 mol/L aqueous sodium hydroxide solution (66 g, 0.50 mol) was added, and the mixture was stirred for 3.5 hours. Acetic acid (41 g, 0.68 mol) was added to the reaction solution. Water (300 mL) was further added, seed crystals of the target compound (60 mg) were added, and water (600 mL) was then added. Crystals were filtered from the resulting suspension and washed with 50% hydrous 1,2-dimethoxyethane (360 mL). The crystals were stirred at 40°C overnight to obtain the target compound as white crystals (15 g; yield: 80%).
¹H-NMR (500 MHz, DMSO-d₆) δ 8.66 (1H, t, J = 6.6 Hz), 7.23 (1H, t, J = 6.0 Hz), 4.58 (2H, d, J = 6.9 Hz), 4.06-4.01 (2H, m), 3.69 (2H, t, J = 6.0 Hz), 3.58 (2H, d, J = 6.3 Hz), 3.56 (2H, t, J = 5.7 Hz), 0.95-0.90 (2H, m), 0.02 (9H, s).

### B-2. Synthesis 1 of compound (I)

The raw material compound (I) used in the above synthesis schemes A-1, A-5, A-8, and A-10 was synthesized according to the following scheme.

### [Synthesis Scheme B-2]

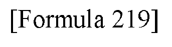

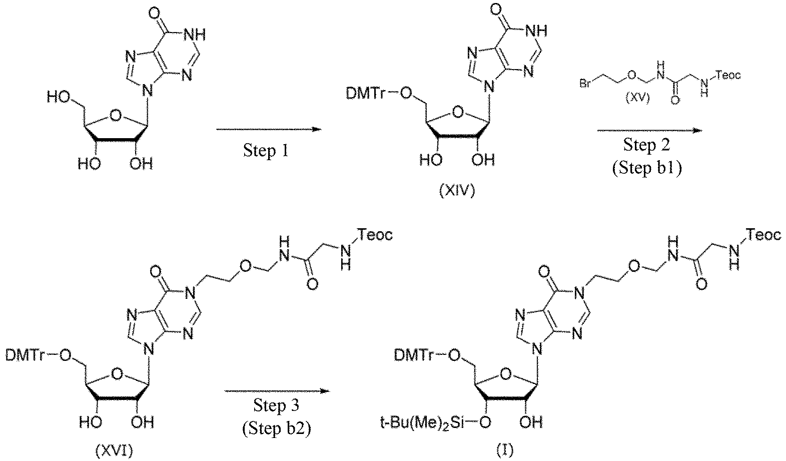

### Reference Example 2:

### Synthesis of 5'-O-[bis(4-methoxyphenyl)(phenyl)methyl]inosine (corresponding to a compound of formula (XIV))

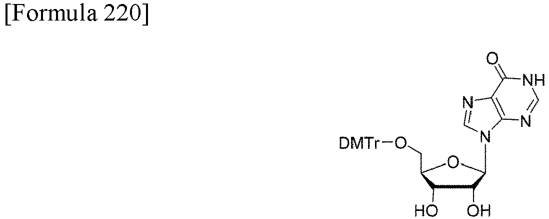

### (Step 1)

To a solution containing inosine (5.0 g, 18.4 mmol), pyridine (30 mL), and dimethyl sulfoxide (20 mL) was added 4,4'-dimethoxytrityl chloride (7.0 g, 20.5 mmol) at room temperature under nitrogen flow. After stirring for 2 hours, 4,4'-dimethoxytrityl chloride (1.3 g, 3.7 mmol) was further added. After stirring for 4 hours, 5 wt% aqueous sodium bicarbonate solution (50 mL), toluene (50 mL), and 20 wt% brine (25 mL) were added to the reaction solution. The aqueous layer was discarded, and 5 wt% aqueous sodium bicarbonate solution (50 mL) and 20 wt% brine (25 mL) were added to the organic layer. The aqueous layer was discarded, and the organic layer was washed with 20 wt% brine (50 mL). The resulting organic layer was concentrated under reduced pressure to about 20 mL and ethyl acetate (100 mL) was added dropwise to the resulting solution. The mixture was stirred at 50°C for 1 hour, cooled to room temperature, and then stirred overnight. Crystals were filtered from the resulting suspension and washed with ethyl acetate (40 mL). The crystals were dried under reduced pressure at 40°C to obtain the target compound as white crystals (9.0 g; yield: 85%).
¹H-NMR (500 MHz, DMSO-d₆) δ 12.39 (1H, brs), 8.19 (1H, s), 8.00 (1H, s), 7.35 (2H, d, J = 7.4 Hz), 7.26 (2H, t, J = 7.4 Hz), 7.24-7.18 (5H, m), 6.86-6.81 (4H, m), 5.91 (1H, d, J = 4.6 Hz), 5.60 (1H, brs), 5.23 (1H, brs), 4.58 (1H, t, J = 4.9 Hz) 4.24 (1H, t, J = 5.2 Hz), 4.06 (1H, q, J = 4.8 Hz), 3.73 (6H, s), 3.23-3.17 (2H, m).

### Example 13:

### Synthesis of 1,3-dimethyl-2-imidazolidinone containing 5'-O-[bis(4-methoxyphenyl)(phenyl)m ethyl]-1 -(2- {[(N- {[2-(trimethylsilyl)ethoxy]carbonyl}glycyl)amino]methoxy}ethyl)inosine (corresponding to a compound of (XVI))

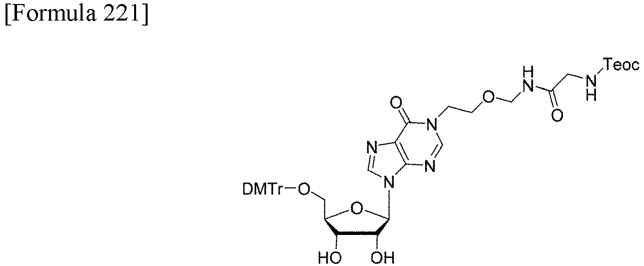

### (Step 2)

A solution containing the compound (3.0 g, 5.3 mmol) obtained in Reference Example 2, N-[(2-bromoethoxy)methyl]-N²-{[2-(trimethylsilyl)ethoxy]carbonyl}glycinamide (5.6 g, 15.7 mmol), and 1,3-dimethyl-2-imidazolidinone (30 mL) was heated to 35 to 40°C. Next, 1,1,3,3-tetramethylguanidine (1.5 mL, 11.8 mmol) was added dropwise. The mixture was stirred at 35-40°C for 16 hours, and cooled to room temperature. After toluene (45 mL) and 10 wt% brine (30 mL) were added and stirred, the aqueous layer was discarded. Subsequently, 1,3-dimethyl-2-imidazolidinone (15 mL) and 10 wt% brine (15 mL) were added and stirred, and the aqueous layer was then discarded. The resulting organic layer was washed twice with 10 wt% brine (30 mL), and concentrated under reduced pressure to about 15 mL. To the concentrated solution was added 1,3-dimethyl-2-imidazolidinone (15 mL). The mixture was concentrated under reduced pressure to obtain a 1,3-dimethyl-2-imidazolidinone solution (about 24 mL) containing the target compound.

### Example 14:

### Synthesis of 5'-O-[bis(4-methoxyphenyl)(phenyl)methyl]-3'-O-(2,3,3-trimethylbutan-2-yl)-1-(2-{[(N-{[2-(trimethylsilyl)ethoxy]carbonyl}glycyl)amino]methoxy}ethyl)inosine (corresponding to a compound of formula (I))

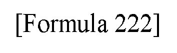

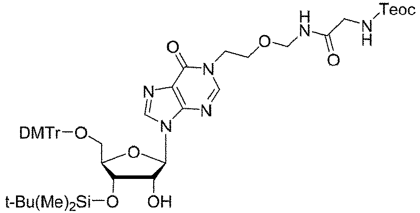

### (Step 3)

To the 1,3-dimethyl-2-imidazolidinone solution (about 24 mL) containing the compound obtained in Example 13 was added 1,3-dimethyl-2 imidazolidinone (15 mL). Next, 1,1,3,3-tetramethylguanidine (4.0 mL, 31.5 mmol) and *tert-*butyldimethylchlorosilane (2.2 g, 14.5 mmol) were added thereto. The mixture was heated to 55-60°C, stirred for 7 hours, and then cooled to room temperature. Toluene (60 mL) and water (30 mL) were added to the reaction solution, and the mixture was stirred. The aqueous layer was discarded. The resulting organic layer was concentrated under reduced pressure to about 15 mL. Thereafter, tetrahydrofuran (30 mL) was added to the concentrated solution, and the mixture was concentrated under reduced pressure to about 15 mL. The same procedure was repeated twice to obtain a tetrahydrofuran solution (about 15 mL) containing a (6:4) mixture of 5'-O-[bis(4-methoxyphenyl)(phenyl)methyl]-3'-O-(2,3,3-trimethylbutan-2-yl)-1-(2-{[(N-{[2-(trimethylsilyl)ethoxy]carbonyl}glycyl)amino]methoxy}ethyl]inosine (target compound) and 5'-O-[bis(4-methoxyphenyl)(phenyl)methyl]-2'-O-(2,3,3-trimethylbutan-2-yl]-I-(2-{ [(N-{ [2-(trimethylsilyl)ethoxy]carbonyl}glycyl)amino]methoxy}ethyl)inosine. Tetrahydrofuran (30 mL), 1,1,3,3-tetramethylguanidine (0.07 mL, 0.5 mmol), and crystals of the target compound (1.0 mg) were added. After stirring at 25°C for 30 min, n-heptane (30 mL) was added and the mixture was stirred at 25°C overnight. The crystals were filtered from the resulting suspension and washed with tetrahydrofuran/n-heptane (1/1, 30 mL). The crystals were dried under reduced pressure to obtain the target compound as white crystals (3.7 g; yield: 73%).
¹H-NMR (500 MHz, CDCl₃) δ 7.99 (1H, s), 7.93 (1H, s), 7.42 (2H, d, J = 7.7 Hz), 7.33-7.29 (4H, m), 7.29-7.24 (3H, m), 7.23-7.19 (1H, m), 7.08-7.01 (1H, m), 6.83-6.78 (4H, m), 5.92 (1H, d, J = 5.2 Hz), 5.46 (1H, brs), 4.70-4.65 (1H, m), 4.64 (2H, d, J = 6.9 Hz), 4.50 (1H, dd, J = 5.4, 3.7 Hz), 4.19 (2H, t, J = 4.9 Hz) 4.18-4.12 (3H, m), 3.81-3.76 (8H, m), 3.73 (2H, d, J = 5.7 Hz), 3.48 (1H, dd, J = 10.9, 3.4 Hz), 3.26 (1H, dd, J = 10.9, 4.0 Hz), 3.16 (1H, d, J = 6.9 Hz), 0.99-0.93 (2H, m), 0.89 (9H, s), 0.09 (3H, s), 0.02 (9H, s), 0.01 (3H, s).

### B-3 Synthesis of compound (XV) (where X = I)

The raw material compound (XV) used in synthesis scheme B-4 below was synthesized according to the following scheme.

### [Synthesis Scheme B-3]

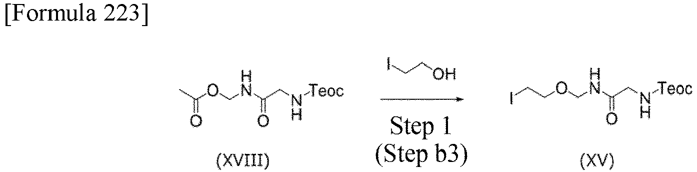

### Example B3-1:

### Synthesis of N-[(2-iodoethoxy)methyl]-N²-{[2-(trimethylsilyl)ethoxy]carbonyl}glycinamide (corresponding to a compound of formula (XV))

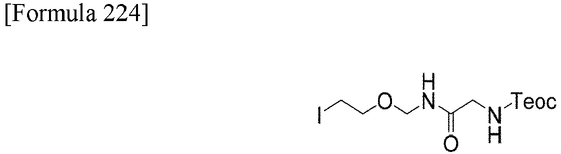

### (Step 1)

A solution prepared by adding 1,2-dimethoxyethane (225 mL) to [(N-{[2-(trimethylsilyl)ethoxy]carbonyl}glycyl)amino]methyl acetate (15.0 g, 51.7 mmol) and 1,2-iodoethane (8.1 mL, 103.3 mmol) was cooled to 0°C. Then, 10 mol/L aqueous sodium hydroxide solution (5.2 mL, 51.7 mmol) was added, and the mixture was stirred for 1 hour. Acetic acid (4.4 mL, 77.5 mol) was added to the reaction solution. Water (75 mL) was further added, seed crystals of the target compound (15 mg) were added, and water (300 mL) was then added. After additional seed crystals (15 mg) and water (45 mL) were added, seed crystals (15 mg) and water (75 mL) were further added. The crystals were filtered from the resulting suspension after 1 hour at 0°C and washed with 50% hydrous 1,2-dimethoxyethane (150 mL). The crystals were dried at 40°C overnight to obtain the target compound as white crystals (16.0 g; yield: 77%).
1H-NMR (500 MHz, CDCl₃) δ: 6.78 (1H, brs), 5.19 (1H, brs), 4.82 (2H, d, J = 6.9 Hz), 4.22-4.18 (2H, m), 3.88 (2H, d, J = 6.3 Hz), 3.78 (2H, t, J = 6.6 Hz), 3.25 (2H, t, J = 6.6 Hz), 1.03-0.98 (2H, m), 0.05 (9H, s).

### B-4. Synthesis 2 of compound (I)

The raw material compound (I) used in the above synthesis schemes A-1, A-5, A-8, and A-10 was synthesized according to the following scheme.

### [Synthesis Scheme B-4]

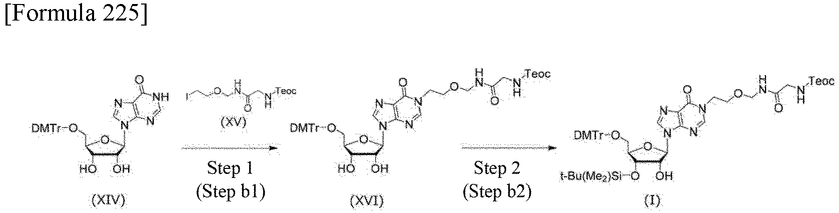

### Example B4-1:

### Synthesis of 5'-O-[bis(4-methoxyphenyl)(phenyl)methyl]-1-(2- { [(N- { [2-(trimethylsilyl)ethoxy]carbonyl}glycyl)amino]methoxy}ethyl)inosine (corresponding to a compound of (XVI))

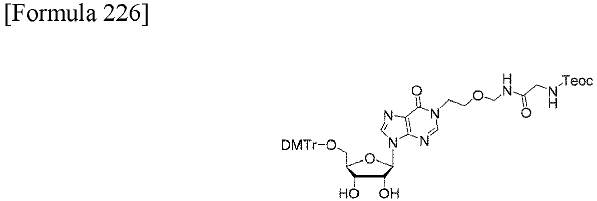

### (Step 1)

A solution containing 5'-O-[bis(4-methoxyphenyl)(phenyl)methyl]inosine (500 mg, 0.88 mmol), N-[(2-iodoethoxy)methyl]-N²-{[2-(trimethylsilyl)ethoxy]carbonyl}glycinamide (529 mg, 1.31 mmol), and dimethylformamide (2 mL) was stirred at room temperature for 25 hours. Toluene (10 mL) and 2.5% aqueous sodium bicarbonate solution (5 mL) were added, and the mixture was stirred for 15 minutes. The aqueous layer was discarded. The organic layer was then washed with 10% brine (5 mL). The organic layer was concentrated under reduced pressure to obtain a residue. Toluene (5 mL) was added thereto, and the mixture was again concentrated under reduced pressure to obtain a crude form of the target compound.

### Example B4-2:

### Synthesis of 5'-O-[bis(4-methoxyphenyl)(phenyl)methyl]-3'-O-(2,3,3-trimethylbutan-2-yl)-1-(2- f [(N-{ [2-(trimethylsilyl)ethoxy]carbonyl}glycyl)amino]methoxy}ethyl)inosine (corresponding to a compound of formula (I))

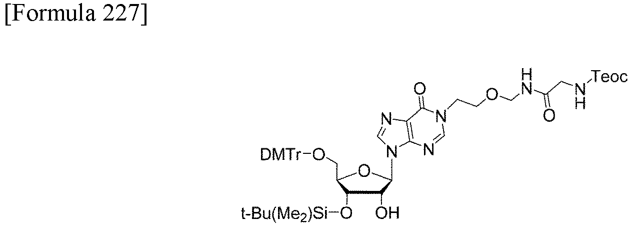

### (Step 2)

One-fifth of the compound obtained in Example B4-1 was added to 1,3-dimethyl-2-imidazolidinone (1 mL), 2,6-lutidine (41 µL, 0.35 mmol), and *tert-*butyldimethylsilyl trifluoromethanesulfonate (11 µL, 0.15 mmol), and the mixture was stirred for about 2 hours. Next, 2,6-lutidine (12 µL, 0.10 mmol) and *tert-*butyldimethylsilyl trifluoromethanesulfonate (11 µL, 0.05 mmol) were further added, and the mixture was stirred for about 19 hours. Then, 2,6-lutidine (3 µL, 0.02 mmol) and tert-butyldimethylsilyl trifluoromethanesulfonate (3 µL, 0.01 mmol) were further added, and the mixture was stirred for 2 hours. Toluene (2 mL) and 2.5% aqueous sodium bicarbonate solution (1 mL) were added, and the mixture was stirred. The aqueous layer was discarded. The resulting organic layer was washed with 20% brine (1 mL) to obtain an organic layer. The organic layer was concentrated under reduced pressure. The resulting residue was admixed with tetrahydrofuran (1 mL) and dissolved, and heptane (0.3 mL) was then added. Seed crystals of the target compound were added, and heptane (0.3 mL) was added twice. The crystals were filtered from the resulting suspension and washed with tetrahydrofuran/n-heptane (11/14, 0.5 mL). The crystals were dried under reduced pressure to obtain the target compound as white crystals (80 mg; yield: 48%).

### [C. Synthesis of raw material compound (VIII)]

### C-1 Synthesis of compound (XXIII)

The raw material compound (XXIII) used in synthesis scheme C-2 below was synthesized according to the following scheme.

### [Synthesis Scheme C-1]

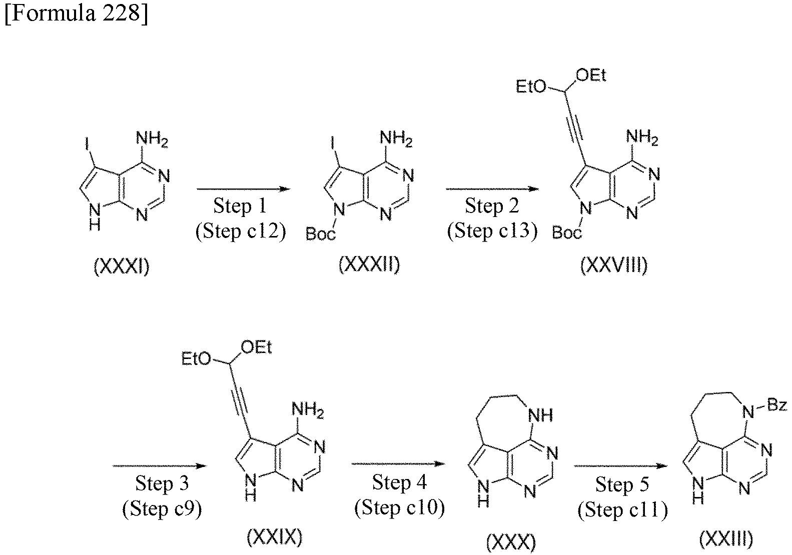

### Example 15:

### Synthesis of tert-butyl-4-amino-5-iodo-7H-pyrrolo[2,3-d]pyrimidin-7-carboxylate (corresponding to a compound of formula (XXXII))

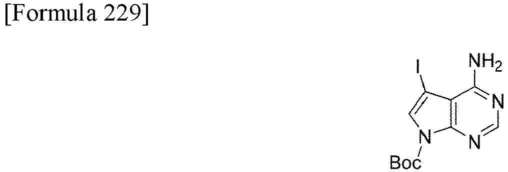

### (Step 1)

To a toluene solution (1750 mL) containing 5-iodo-7H-pyrrolo[2,3-d]pyrimidin-4-amine (318.5 g, 1.225 mol) were added di-tert-butyl dicarbonate (294.1 g, 1.347 mol) and 1-methylimidazole (50.28 g, 0.612 mol), and the mixture was stirred at room temperature for 6 hours. Heptane (7000 mL) was added dropwise to the reaction solution. The mixture was stirred at room temperature for 10 minutes, cooled to 0°C, and stirred for 1 hour. The precipitated crystals were filtered and washed with a mixture of toluene and heptane (280 mL/1120 mL), and then dried under reduced pressure at 40°C to obtain the target compound (415.7 g, 1.154 mol; yield: 94.2%).
MS (ESI) m/z: 361[M+H]⁺.
¹H-NMR (500 MHz, CDCl₃) δ 8.46 (1H, s), 7.55 (1H, s), 5.81 (2H, brs), 1.65 (9H, s).

### Example 16:

### Synthesis of tert-butyl-4-amino-5-(3,3-diethoxypropa-1-yn-1-yl)-7H-pyrrolo[2,3-d]pyrimidin-7-carboxylate (corresponding to a compound of formula (XXVIII))

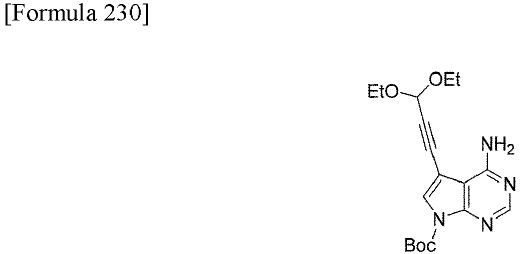

### (Step 2)

An N,N-dimethylformamide solution (1500 mL) containing the compound (285.4 g, 0.792 mol) obtained in Example 15 was degassed under reduced pressure. Next, copper(I) iodide (1.51 g, 0.0079 mol), bis(triphenylphosphine)palladium(II) dichloride (3.89 g, 0.0055 mol), propargylaldehyde diethyl acetal (182.8 g, 1.426 mol), and triethylamine (240.5 g, 2377 mol) were added. The mixture was stirred at room temperature for 18.5 hours. Toluene (6000 mL) and 10% aqueous ammonium chloride solution (1500 mL) were added to the reaction solution. The mixture was stirred and liquid-separated to remove the aqueous layer. The resulting organic layer was washed twice with 10% aqueous ammonium chloride solution (1500 mL) and then washed once with 20% aqueous sodium chloride solution (1500 mL). Activated carbon (30 g) was added, and the mixture was stirred at room temperature for about 2 hours. After the activated carbon was filtered out, the activated carbon was washed with toluene (600 mL). The filtrate was combined and concentrated under reduced pressure to 1500 mL. Heptane (6000 mL) was added dropwise. The mixture was stirred at room temperature for about 10 minutes, cooled to 0°C, and stirred for 1 hour. The precipitated crystals were filtered, washed with a toluene/heptane (240 mL/960 mL) mixture, and then dried under reduced pressure at 40°C to obtain the target compound (269.9 g, 0.749 mol; yield: 94.6%).
MS (ESI) m/z: 361[M+H]⁺.
¹H-NMR (500 MHz, CDCl₃) δ 8.47 (1H, s), 7.60 (1H, s), 5.71 (2H, brs), 5.49 (1H, s), 3.81 (2H, dq, J = 9.5, 7.5 Hz), 3.67 (2H, dq, J = 9.5, 7.5 Hz), 1.65 (9H, s), 1.28 (6H, t, J = 7.5 Hz).

### Example 17:

### Synthesis of 5-(3,3-diethoxypropa-1-yn-1-yl)-7H-pyrrolo[2,3-d]pyrimidin-4-amine (corresponding to a compound of formula (XXIX))

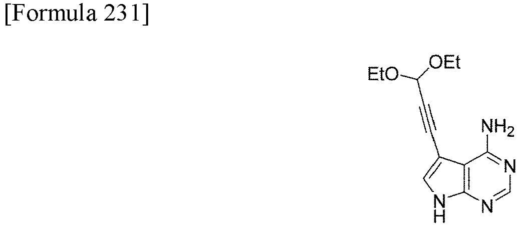

### (Step 3)

To an ethanol solution (1500 mL) containing the compound 250.0 g, 0.694 mol) obtained in Example 16 was added 4 mol/L sodium hydroxide solution (260.1 mL, 1.040 mol), and the mixture was stirred at room temperature for about 1.5 hours. Water (1500 mL) was added to the reaction solution. The pH was then adjusted to 7.5 with 2M hydrochloric acid, and the mixture was stirred at room temperature for about 45 minutes. Subsequently, water (3000 mL) was added dropwise, and the mixture was stirred at room temperature for 2 hours. The precipitated crystals were filtered and washed with an ethanol/water (250 mL/750 mL) mixture and acetonitrile (1000 mL) cooled to 0°C. The resulting crystals were dried under reduced pressure at 40°C to obtain the target compound (152.5 g, 0.586 mol; yield: 84.4%).
MS (ESI) m/z: 261[M+H]⁺.
¹H-NMR (500 MHz, DMSO-d₆) δ 12.01 (1H, brs), 8.09 (1H, s), 7.55 (1H, s), 6.50 (2H, brs), 5.58 (1H, s), 3.68 (2H, dq, J = 10.0, 7.5 Hz), 3.58 (2H, dq, J = 10.0, 7.5 Hz), 1.16 (6H, t, J = 7.5 Hz).

### Example 18:

### Synthesis of 6,7,8,9-tetrahydro-2H-2,3,5,6-tetraazabenzo[cd]azulene (corresponding to a compound of formula (XXX))

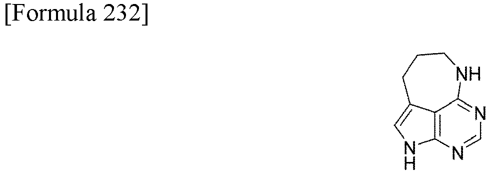

### (Step 4)

To a 1-methylpyrrolidone mixture (189 mL) containing the compound (63.0 g, 0.242 mol) obtained in Example 17 was added 5% palladium carbon (13.4 g, 53.2% moisture), and the mixture was stirred at 40°C for about 2 hours under a hydrogen atmosphere (300 kPa). The inside of the reaction system was replaced with nitrogen. Acetic acid (630 mL), water (32 mL), and 5% palladium carbon (13.4 g, 53.2% moisture) were added, and the mixture was stirred at 50°C for about 21 hours under a hydrogen atmosphere (300 kPa). The inside of the reaction system was replaced with nitrogen. Water (300 mL) was added, and the palladium carbon was then filtered out. The palladium carbon was washed with a mixture of acetic acid (150 mL) and water (150 mL), and the filtrate was concentrated under reduced pressure to 550 mL. Water (620 mL) and 20 mL of 48% aqueous potassium hydroxide solution (20 mL) were added dropwise at 50°C, and the mixture was cooled to 0-5°C and stirred for about 20 hours. The precipitate was filtered, and the filtered crystals were washed with a cooled 1-methylpyrrolidone/water (36 mL/144 mL) mixture and further washed with water (320 mL). The resulting crystals were dried under reduced pressure at 50°C to obtain the target compound (37.0 g, 0.212 mol; yield: 87.8%).
MS (ESI) m/z: 175[M+H]⁺.
¹H-NMR (500 MHz, DMSO-d₆) δ 11.24 (1H, s), 7.34 (1H, s), 6.86 (1H, s) 3.34-3.38 (2H, m), 2.80 (2H, brt, J = 6.0 Hz), 2.49-2.51 (2H, m), 1.85-1.91 (2H, m).

### Example 19:

### Synthesis of phenyl(2,7,8,9-tetrahydro-6H-2,3,5,6-tetraazabenzo[cd]azulen-6-yl)methanone (corresponding to a compound of formula (XXIII))

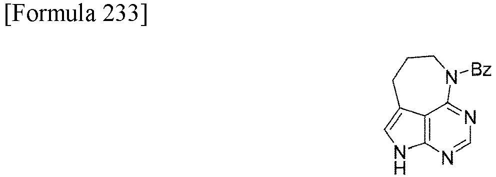

### (Step 5)

To a mixture containing the compound (4.0 g, 23.0 mmol) obtained in Example 18 in 1,3-dimethyl-2-imidazolidinone (40 mL) were added at room temperature triethylamine (10.6 mL, 75.9 mol) and 4-dimethylaminopyridine (280 mg, 2.3 mmol). The mixture was then cooled to 0-5°C, and benzoyl chloride (8.3 mL, 72.0 mmol) was added. After stirring for about 21 hours, methanol (20 mL) and triethylamine (12.0 mL) were added to the reaction solution. The mixture was stirred for about 23 hours. Acetic acid (6.7 mL) and water (102 mL) were added, and the mixture was stirred at 0-5°C for about 2 hours. The precipitate was filtered and the filtered powder was washed with an acetonitrile/water (2 mL/18 mL) mixture. The resulting powder was suspended in acetonitrile (40 mL) and stirred at 0-5°C for about 1 hour. The precipitate was filtered and the filtered powder was washed with cold acetonitrile (10 mL). The resulting powder was dried under reduced pressure at 50°C to obtain the target compound (5.13 g, 18.4 mmol; yield: 80.0%).
MS (ESI) m/z: 279[M+H]⁺.
¹H-NMR (500 MHz, DMSO-d₆) δ 11.99 (1H, s), 7.93 (1H, s), 7.32-7.39 (4H, m), 4.18 (2H, m), 2.98 (2H, t, J = 6.3 Hz), 2.12-2.18 (2H, m).

### C-2 Synthesis of compound (VIII)

The raw material compound (VIII) used in the above synthesis scheme A-2 was synthesized according to the following scheme.

### [Synthesis Scheme C-2]

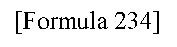

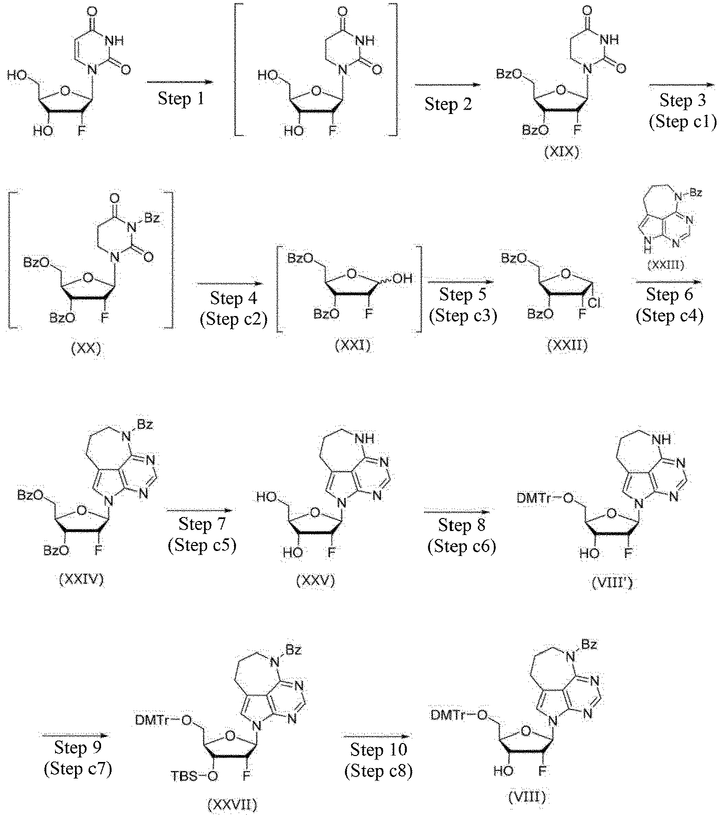

### Reference Example 3:

### Synthesis of 2'-deoxy-2'-fluoro-3,4,5,6-tetrahydrouridine

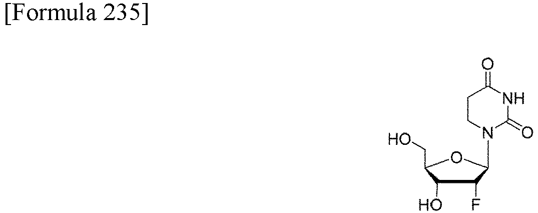

### (Step 1)

To a 1-L autoclave were added 2'-deoxy-2'-fluorouridine (70.0 g, 28.4 mmol) and methanol (420 mL). Next, 5% palladium carbon (13.57 g, 52.2% moisture) was added. The mixture was then stirred under a hydrogen atmosphere (300 kPa) at 50°C for 7 hours. The inside of the reaction system was replaced with nitrogen. After that, the palladium carbon was filtered and washed with a methanol/water (189 mL/21 mL) mixture. The filtrate was concentrated under reduced pressure to 210 mL or less, and N,N-dimethylacetamide (350 mL) and toluene (350 mL) were added. The mixture was concentrated under reduced pressure to 350 mL. Toluene (350 mL) was then added and concentrated again under reduced pressure to 350 mL. The same procedure was repeated two more times to obtain the target compound.

### Reference Example 4:

### Synthesis of 3',5'-di-O-benzoyl-2'-deoxy-2'-fluoro-3,4,5,6-tetrahydrouridine (corresponding to a compound of formula (XIX))

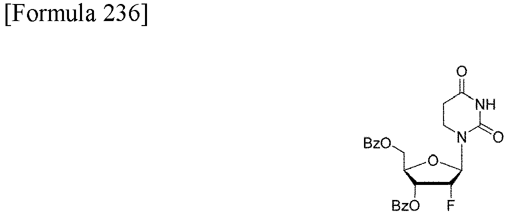

### (Step 2)

To the N,N-dimethylacetamide solution (340 mL) obtained in Reference Example 3 was added pyridine (76.5 g, 96.7 mmol). Benzoyl chloride (85.4 g, 60.5 mmol) was added dropwise under ice-cold conditions, and the mixture was stirred at room temperature for 2.5 hours. After water (6.8 mL) and 2-propanol (408 mL) were added to the reaction mixture, the temperature was raised to 50°C and water (102 mL) was added dropwise. After stirring at the same temperature for 30 minutes, water (136 mL) was added. The mixture was stirred for 30 minutes, cooled to room temperature, and then stirred for 22 hours. The mixture was cooled to 0°C and stirred for 1 hour. The precipitated crystals were then filtered, washed with a 2-propanol/water (204 mL/122 mL) mixture, and then dried under reduced pressure at 40°C to obtain the target compound (117.2 g, 25.7 mmol; yield: 93.0%) as a white solid.
MS (ESI) m/z: 457[M+H]⁺.
¹H-NMR (500 MHz, CDCl₃) δ 8.05 (4H, dd, J = 23.5, 8.0 Hz), 7.62-5.56 (2H, m), 7.48-7.41 (4H, m), 7.38-7.32 (1H, m), 5.85 (1H, dd, J = 22.3, 2.3 Hz), 5.54-5.37 (2H, m), 4.81 (1H, dd, J = 12.0, 2.9 Hz), 4.59-4.49 (2H, m), 3.53-3.44 (2H, m), 2.69-2.58 (2H, m).

### Example 20:

### Synthesis 1 of 3-benzoyl-3',5'-di-O-benzoyl-2'-deoxy-2'-fluoro-3,4,5,6-tetrahydrouridine (corresponding to a compound of formula (XX))

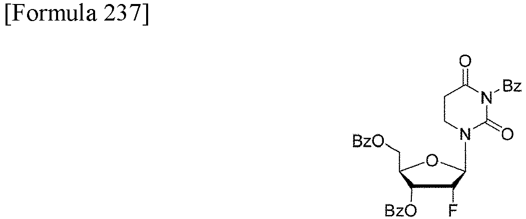

### (Step 3)

To a suspension containing the compound (67.0 g, 14.7 mmol) obtained in Reference Example 4 in acetonitrile (402 mL) were added 4-dimethylaminopyridine (18.0 g, 14.7 mmol) and triethylamine (26.8 g, 26.5 mmol), and the mixture was ice-cooled. Benzoyl chloride (33.1 g, 23.5 mmol) was added dropwise, the temperature was raised to room temperature, and the mixture was stirred for 1 hour. The reaction solution was ice-cooled, admixed and diluted with cyclopentyl methyl ether (670 mL), admixed with 10% brine (670 mL), stirred for 5 minutes, and then liquid-separated. The organic layer was washed with 0.5 M aqueous tosylate solution (670 mL) and further washed with water (335 mL). Cyclopentyl methyl ether (670 mL) was added to the resulting organic layer, and the mixture was concentrated under reduced pressure to 670 mL to obtain the target compound.

### Example 21:

### Synthesis 2 of 3-benzoyl-3',5'-di-O-benzoyl-2'-deoxy-2'-fluoro-3,4,5,6-tetrahydrouridine (corresponding to a compound of formula (XX))

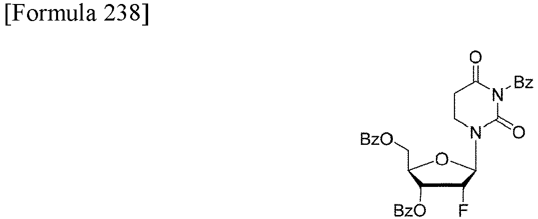

### (Step 3')

To a solution containing the compound (200 mg, 0.44 mmol) obtained in Reference Example 4 in N,N-dimethylacetamide (2 mL) were added 4-dimethylaminopyridine (5.35 mg, 0.04 mmol) and triethylamine (70.9 mg, 0.70 mmol) at room temperature. Benzoyl chloride (80.1 mg, 0.57 mmol) was added dropwise, and the mixture was then stirred for 3 hours. After 4-dimethylaminopyridine (5.35 mg, 0.04 mmol) and triethylamine (70.9 mg, 0.70 mmol) were added, benzoyl chloride (80.1 mg, 0.57 mmol) was further added. The mixture was stirred for another 17 hours. After 4-dimethylaminopyridine (5.35 mg, 0.04 mmol) and triethylamine (70.9 mg, 0.70 mmol) were added, benzoyl chloride (80.1 mg, 0.57 mmol) was further added. The mixture was then stirred for 2 hours. Ethyl acetate (4 mL) was added for dilution to the reaction solution. Then, 5% brine (4 mL) was added. The mixture was stirred for 5 min, and then liquid-separated. The organic layer was washed with 5% brine (4 mL), and concentrated under reduced pressure. The residue was purified by silica gel column chromatography (hexane/ethyl acetate = 70/30, 60/40) to obtain the target compound (226 mg, 0.40 mmol; yield: 91.6%) as a white foamy substance.
MS (ESI) m/z: 561[M+H]⁺.
¹H-NMR (500 MHz, CDCl₃) δ 8.02 (4H, dt, J = 16.6, 4.3 Hz), 7.89-7.87 (2H, m), 7.64-7.54 (3H, m), 7.49-7.38 (6H, m), 5.82 (1H, dd, J = 21.8, 2.3 Hz), 5.53-5.42 (2H, m), 4.78-4.76 (1H, m), 4.57-4.51 (2H, m), 3.67-3.62 (2H, m), 2.90-2.76 (2H, m).

### Example 22:

### Synthesis of 3,5-di-O-benzoyl-2-deoxy-2-fluoro-D-ribofuranose (corresponding to a compound of formula (XXI))

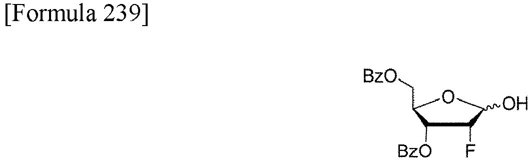

### (Step 4)

To the cyclopentyl methyl ether solution (670 mL) obtained in Example 20 was added an aqueous solution (134 mL) containing p-toluenesulfonic acid monohydrate (139.8 g, 73.5 mmol). The mixture was heated and stirred at 60°C for 6 hours. The reaction mixture was cooled to room temperature, and then liquid-separated. The aqueous layer was extracted with cyclopentyl methyl ether (335 mL). The organic layers were combined, and washed twice with 8% aqueous sodium bicarbonate solution (503 mL) and washed with water (335 mL). The organic layer was then concentrated under reduced pressure to 335 mL to obtain the target compound.

### Example 23:

### Synthesis of 3,5-di-O-benzoyl-2-deoxy-2-fluoro-α-D-ribofuranosyl chloride (corresponding to a compound of formula (XXII))

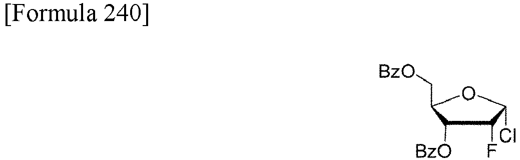

### (Step 5)

To the cyclopentyl methyl ether solution (50 mL) obtained in Example 22 was added dichloromethane (50 mL). Trichloroisocyanuric acid (1.55 g, 6.66 mmol) and tris(2,4-di-tert-butylphenyl)phosphite (8.64 g, 13.35 mmol) were added under ice cold conditions. The mixture was stirred at the same temperature for 1 hour. Trichloroisocyanuric acid (1.55 g, 6.66 mmol) and tris(2,4-di-tert-butylphenyl)phosphite (8.64 g, 13.35 mmol) were added, and the mixture was stirred for 1 hour. Cyclopentyl methyl ether (100 mL) was added to the reaction solution, and the mixture was concentrated under reduced pressure to 150 mL or less. Then, 2 M aqueous sodium hydroxide solution (50 mL) and 10% aqueous sodium thiosulfate solution (50 mL) were added, and the mixture was stirred for 20 minutes. The reaction mixture was liquid-separated. The organic layer was liquid-separated and washed twice with 2 M aqueous sodium hydroxide solution (100 mL), twice with 6% aqueous sodium bicarbonate solution (100 mL) and with water (100 mL). The organic layer was concentrated under reduced pressure to 30 mL or less, and heptane (100 mL) was added. The mixture was concentrated under reduced pressure to 30 mL or less. Heptane (100 mL) and acetonitrile (100 mL) were added and the mixture was liquid-separated. The acetonitrile layer was further liquid-separated and washed with heptane (100 mL). Activated carbon (0.5 g) was added to the acetonitrile layer. The mixture was stirred for 30 minutes, filtered, and then washed with cyclopentyl methyl ether (50 mL). The filtrate was concentrated under reduced pressure to 50 mL, and CPME (100 mL) was added. The mixture was concentrated under reduced pressure to 50 mL. The mixture was heated to 50°C, heptane (50 mL) was added, and the resulting mixture was concentrated under reduced pressure to 50 mL. Then, heptane (50 mL) was added dropwise. The mixture was stirred at the same temperature for 30 minutes, and then concentrated under reduced pressure to 50 mL. Subsequently, heptane (50 mL) was added. The same procedure was repeated two more times. Cyclopentyl methyl ether (10 mL) was then added. The mixture was cooled to 0°C. After that, the precipitated crystals were filtered, washed with a mixture of heptane and cyclopentyl methyl ether (45 mL/5 mL), and dried under reduced pressure at room temperature to obtain the target compound (6.2 g, 16.4 mmol; yield: 61.3%) as a white solid.
MS (ESI) m/z: 396[M+NH₃+H]⁺.
¹H-NMR (500 MHz, CDCl₃) δ 8.13 (2H, d, J = 7.4 Hz), 8.01 (2H, d, J = 8.0 Hz), 7.63-7.58 (2H, m), 7.49-7.43 (4H, m), 6.38 (1H, d, J = 4.0 Hz), 5.61-5.59 (1H, m), 5.26 (1H, ddd, J = 50.4, 6.9, 4.6 Hz), 4.86-4.84 (1H, m), 4.73 (1H, dd, J = 12.6, 2.9 Hz), 4.62 (1H, dd, J = 12.0, 4.0 Hz).

### Example 24-1:

### Synthesis 1 of 6-benzoyl-2-(3,5-di-O-benzoyl-2-deoxy-2-fluoro-β-D-ribofuranosyl)-6,7,8,9-tetrahydro-2H-2,3,5,6-tetraazabenzo[cd]azulene (corresponding to a compound of formula (XXIV))

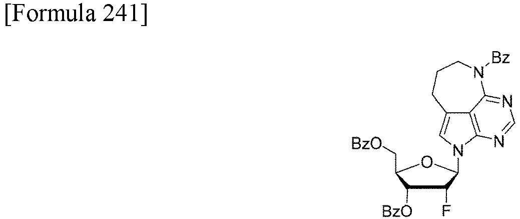

### (Step 6)

To a solution containing the compound (2.51 g, 9.02 mmol) obtained in Example 19 in dimethyl sulfoxide (25 mL) was added cesium carbonate (8.72 g, 26.78 mmol). After stirring for 10 minutes, the compound (6.82 g, 18.01 mmol) obtained in Example 23 was added. The mixture was stirred at room temperature for 2 hours. Toluene (25 mL), water (25 mL), and activated carbon (0.50 g) were added. The mixture was stirred for 30 minutes and filtered. The activated carbon was washed with toluene (12.5 mL). The filtrate was then liquid-separated, and 1 M hydrochloric acid (25 mL) and activated carbon (0.49 g) were added to the organic layer. The mixture was stirred for 30 minutes and filtered. The activated carbon was washed with toluene (12.5 mL). The filtrate was then liquid-separated. The organic layer was liquid-separated and washed twice with 10% aqueous dipotassium hydrogen phosphate solution (12.5 mL) and water (12.5 mL). The resulting organic layer was concentrated under reduced pressure at 40°C to a solution volume of 12.5 mL. The solution was stirred at 40°C, and the material of interest was found to be precipitated. The temperature was then raised to 50°C, and toluene (12.5 mL) and 2-propanol (50.0 mL) were added. The solution was gradually cooled to 0°C and then filtered. The filtrated material was washed with a mixture of toluene (2.0 mL)/2-propanol (8.0 mL) and dried under reduced pressure at 40°C to obtain the target compound (3.15 g, 5.08 mmol; yield: 56.4%) as a white solid.
MS (ESI) m/z: 621[M+H]⁺.
¹H-NMR (500 MHz, CDCl₃) δ 8.11-8.08 (3H, m), 8.03-8.00 (2H, m), 7.64-7.55 (2H, m), 7.50-7.44 (2H, m), 7.42-7.34 (5H, m), 7.27-7.23 (2H, m), 7.09 (1H, s), 6.47 (1H, dd, J = 19.5, 2.3 Hz), 5.93-5.74 (2H, m), 4.86 (2H, dd, J = 12.6, 3.4 Hz), 4.75 (1H, ddd, J = 7.4, 4.0, 3.4 Hz), 4.61 (1H, dd, J = 12.6, 4.0 Hz), 4.31-4.22 (2H, m), 2.88 (2H, dd, J = 6.9, 6.9 Hz), 2.24-2.17 (2H, m).

### Example 24-2:

### Synthesis 2 of 6-benzoyl-2-(3,5-di-O-benzoyl-2-deoxy-2-fluoro-β-D-ribofuranosyl)-6,7,8,9-tetrahydro-2H-2,3,5,6-tetraazabenzo[cd]azulene (corresponding to a compound of formula (XXIV))

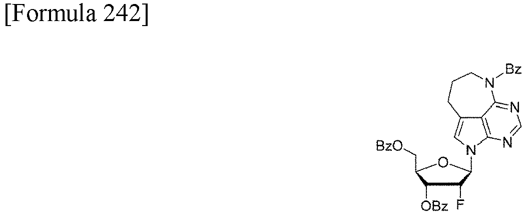

### (Step 6')

To a dimethyl sulfoxide solution (8 mL) containing the compound (1.0 g, 3.59 mmol) obtained in Example 19 was added cesium carbonate (3.51 g, 10.77 mmol). The mixture was stirred for 10 minutes. Next, the compound (2.72 g, 7.18 mmol) obtained in Example 23 was added as three portions. The tool used for the addition was washed with dimethyl sulfoxide (2 mL). The resulting mixture was stirred at room temperature for 3 hours. Acetic acid (1.19 g, 19.75 mmol), acetonitrile (15 mL), and methanol (5 mL) were added. The temperature was increased to 40°C, and water (5 mL) was added dropwise. Seed crystals of the target compound (0.5 mg) were added at about 40°C, and water (7 mL) was added dropwise over 1 hour. After dropping, the mixture was brought to room temperature and stirred overnight. The crystals were filtered from the resulting suspension, and washed with acetonitrile/water (7/3, 25 mL), followed by ethanol (5 mL). The crystals were admixed with ethanol (20 mL), and the mixture was stirred at room temperature for about 30 minutes. The crystals were filtered from the resulting suspension and washed with ethanol (5 mL). The crystals were dried at 40°C overnight to obtain the target compound as white crystals (1.4 g; yield: 63%).

### Example 25-1:

### Synthesis of 2-(2-deoxy-2-fluoro-β-D-ribofuranosyl)-6,7,8,9-tetrahydro-2H-2,3,5,6-tetraazabenzo[cd]azulene (corresponding to a compound of formula (XXV))

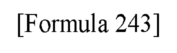

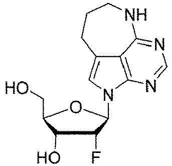

### (Step 7)

To a tetrahydrofuran solution (52 mL) containing the compound (6.50 g, 10.47 mmol) obtained in Example 24 were added water (26 mL) and 4 M aqueous sodium hydroxide solution (10.5 mL), and the mixture was stirred at room temperature for 21 hours. The pH of the reaction solution was adjusted to pH 9.6 using concentrated hydrochloric acid. Salt (8.15 g) and cyclopentyl methyl ether (26 mL) were then added, and the mixture was liquid-separated. The aqueous layer was extracted three times with a mixture of tetrahydrofuran (52 mL) and cyclopentyl methyl ether (26 mL). The organic layers were then mixed and washed with 20% brine (26 mL). The resulting organic layer was concentrated. Then, 1-propanol (130 mL) was added to the residue, and the mixture was concentrated. This procedure was repeated three times, and the solution volume was then adjusted to 33 mL. The solution was heated to 80°C, and heptane (130 mL) was added. The mixture was then cooled slowly to 5°C, and stirred for 2 hours. After that, the precipitated crystals were filtered, washed with a mixture of 1-propanol/heptane (6.5 mL/26 mL) and heptane (13 mL), and dried under reduced pressure at room temperature to obtain the target compound (2.88 g, 9.34 mmol; yield: 89.2%) as a white solid.
MS (ESI) m/z: 309[M+H]⁺.
¹H-NMR (500 MHz, CDCl₃) δ 8.04 (1H, s), 6.97 (1H, d, J = 12.0 Hz), 6.76 (1H, s), 5.97-5.83 (2H, m), 5.74 (1H, brs), 4.68 (1H, d, J = 4.6 Hz), 4.32 (1H, dd, J = 1.1, 1.1 Hz), 3.95 (1H, dd, J = 13.2, 1.1 Hz), 3.74 (1H, ddd, J = 13.2, 12.0, 1.1 Hz), 3.59-3.50 (2H, m), 2.95-2.84 (2H, m), 2.40 (1H, brs), 2.12-2.00 (2H, m).

### Example 25-2:

### Synthesis (alternative process) of 2-(2-deoxy-2-fluoro-β-D-ribofuranosyl)-6,7,8,9-tetrahydro-2H-2,3,5,6-tetraazabenzo[cd]azulene p-toluenesulfonate (corresponding to p-toluenesulfonate of compound of formula (XXV))

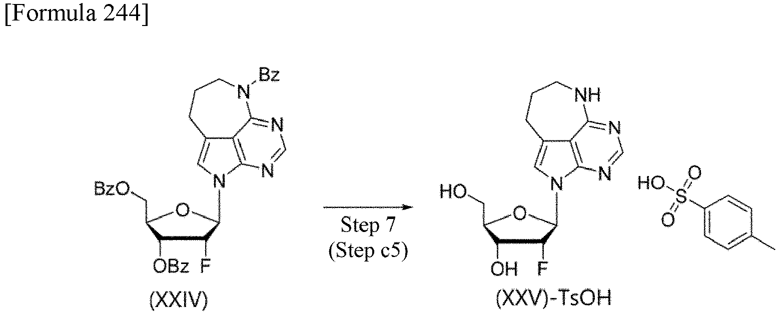

### (Step 7')

To an ethanol solution (1.5 mL) containing the compound 300 mg, 0.48 mmol) obtained in Example 24 was added a sodium ethoxide-containing ethanol solution (20%, 0.012 mL, 0.03 mmol), and the mixture was stirred at 65°C for 6 hours. The mixture was cooled to room temperature. Then, p-toluenesulfonic acid monohydrate (184 mg, 0.97 mmol) and ethanol (0.6 mL) were added, and the mixture was stirred at room temperature for 30 minutes. Toluene (6.3 mL) was then added. After stirred at room temperature overnight, the mixture was cooled to 0°C. The mixture was then stirred at 0°C for 4 hours. After that, the precipitated crystals were filtered and washed with an ethanol/toluene (0.3 mL/0.9 mL) mixture cooled to 0°C. The resulting crystals were dried under reduced pressure at 40°C to obtain the target compound (215.9 mg, 0.45 mmol; yield: 93.0%).
¹H-NMR (500 MHz, DMSO-d₆) δ 9.02 (1H, brs), 8.37 (1H, s), 7.56 (1H, brs), 7.48 (2H, dd, J = 10.3, 1.8 Hz), 7.12 (2H, d, J = 7.5 Hz), 6.40 (1H, dd, J = 16.1, 4.0 Hz), 5.24 (1H, m), 4.34 (1H, dt, J = 13.0, 5.2 Hz), 3.98 (1H, brs), 3.69-3.58 (4H, m), 2.87 (2H, t, J = 5.2 Hz), 2.29 (3H, s), 2.00-1.98 (2H, m).

### Example 26:

### Synthesis of 2-{5-O-[bis(4-methoxyphenyl)(phenyl)methyl]-2-deoxy-2-fluoro-β-D-ribofuranosyl}-6,7,8,9-tetrahydro-2H-2,3,5,6-tetraazabenzo[cd]azulene (corresponding to a compound of formula (VIII'))

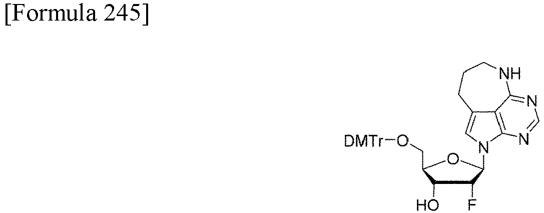

### (Step 8)

To a pyridine solution (8.4 mL) containing the compound (2.80 g, 9.08 mmol) obtained in Example 25 was added 4,4'-dimethoxytrityl chloride (3.98 g), and the mixture was stirred at room temperature for 7 hours. Toluene (56 mL) and water (15 mL) were added to the reaction solution, and the mixture was then liquid-separated. The organic layer was washed twice with water (15 mL). The organic layer was concentrated under reduced pressure. The residue was purified by silica gel column chromatography (hexane/ethyl acetate) to obtain the target compound (5.65 g, 9.25 mmol; yield: 101.9%).
MS (ESI) m/z: 611[M+H]⁺.
¹H-NMR (500 MHz, CDCl₃) δ 8.22 (1H, s), 7.45-7.42 (2H, m), 7.34-7.30 (4H, m), 7.29-7.25 (2H, m), 7.24-7.19 (1H, m), 6.97 (1H, s), 6.83-6.79 (2H, m), 6.46 (1H, dd, J = 18.3, 2.3 Hz), 5.65 (1H, dd, J = 4.0, 2.3 Hz), 5.35 (1H, ddd, J = 53.3, 4.6, 2.3 Hz), 4.73 (1H, ddd, J = 17.8, 6.9, 5.2 Hz), 4.14-4.10 (1H, m), 3.79 (3H, s), 3.78 (3H, s), 3.57 (1H, dd, J = 10.9, 2.9 Hz), 3.52-3.49 (1H, m), 3.42 (1H, dd, J = 10.9, 3.4 Hz), 2.76-2.66 (2H, m), 2.04-1.96 (2H, m).

### Example 27:

### Synthesis of 6-benzoyl-2-{5-O-[bis(4-methoxyphenyl)(phenyl)methyl]-3-O-[tert-butyl(dimethyl)silyl]-2-deoxy-2-fluoro-β-D-ribofuranosyl}-6,7,8,9-tetrahydro-2H-2,3,5,6-tetraazabenzo[cd]azulene (corresponding to a compound of formula (XXVII))

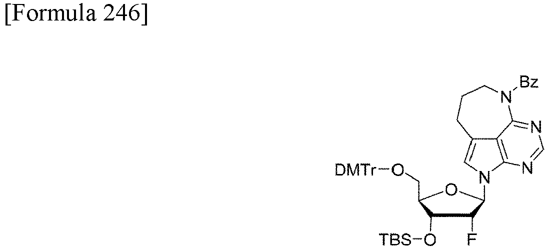

### (Step 9)

To a pyridine solution (0.15 mL) containing the compound (50.0 mg, 0.0819 mmol) obtained in Example 26 was added tert-butyldimethylsilyl trifluoromethanesulfonate (0.038 mL, 0.165 mmol). The mixture was stirred for 30 minutes. Benzoyl chloride (0.019 gmL, 0.165 mmol) was then added and the mixture was stirred for 2 hours. Toluene (0.5 mL) and water (0.5 mL) were added, and the mixture was then liquid-separated. The resulting organic layer was washed twice with water (0.5 mL) and concentrated under reduced pressure. The residue was purified by silica gel column chromatography (hexane/ethyl acetate) to obtain the target compound (56.4 mg, 0.0680 mmol; yield: 83.1%).
¹H-NMR (500 MHz, CDCl₃) δ 8.11 (1H, s), 7.42-7.39 (2H, m), 7.35 (1H, s), 7.32-7.20 (10H, m), 7.16-7.12 (2H, m), 6.82-6.77 (4H, m), 6.46 (1H, dd, J = 16.0, 2.9 Hz), 5.34 (1H, ddd, J = 52.7, 4.6, 2.9 Hz), 4.77 (1H, ddd, J = 16.0, 6.3, 4.6 Hz), 4.28-4.24 (2H, m), 4.18-4.14 (1H, m), 3.78 (3H, s), 3.78 (3H, s), 3.59 (1H, dd, J = 10.9, 2.9 Hz), 3.24 (1H, dd, J = 10.9, 3.4 Hz), 2.88-2.75 (2H, m), 2.21-2.15 (2H, m).

### Example 28:

### Synthesis of 6-benzoyl-2-{5-O-[bis(4-methoxyphenyl)(phenyl)methyl]-2-deoxy-2-fluoro-β-D-ribofuranosyl}-6,7,8,9-tetrahydro-2H-2,3,5,6-tetraazabenzo[cd]azulene (corresponding to a compound of formula (VIII))

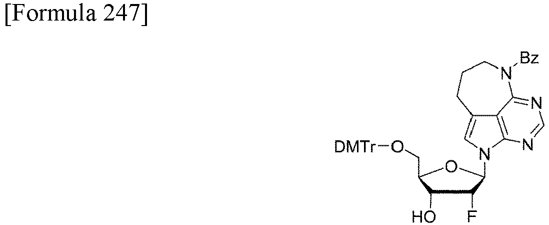

### (Step 10)

To a tetrahydrofuran solution (0.075 mL) containing the compound (15.0 mg, 0.0181 mmol) obtained in Example 27 was added a tetrabutylammonium fluoride-containing tetrahydrofuran solution (1 M, 0.0090 mL, 0.009 mmol), and the mixture was stirred at room temperature for 1.5 hours. Toluene (0.3 mL) and saturated aqueous ammonium chloride solution (0.3 mL) were added, and the mixture was liquid-separated. The resulting organic layer was washed with saturated aqueous ammonium chloride solution (0.3 mL), and the organic layer was concentrated under reduced pressure to obtain the target compound (12.2 mg, 0.0171 mmol; yield: 94.5%).
¹H-NMR (500 MHz, CDCl₃) δ 8.09 (1H, s), 7.44-7.41 (2H, m), 7.35-7.18 (13H, m), 6.84-6.80 (4H, m), 6.52 (1H, dd, J = 17.2, 2.3 Hz), 5.40 (1H, ddd, J = 53.3, 4.0, 2.3 Hz), 4.77 (1H, ddd, J = 16.6, 6.3, 4.6 Hz), 4.30-4.22 (2H, m), 4.18-4.14 (1H, m), 3.79 (3H, s), 3.78 (3H, s), 3.59 (1H, dd, J = 10.9, 2.9 Hz), 3.24 (1H, dd, J = 10.9, 3.4 Hz), 2.85-2.75 (2H, m), 2.21-2.14 (2H, m).

### C-3 Synthesis of compound (XXIV)

The compound (XXIV) used in the above synthesis scheme C-2 can be synthesized according to the following scheme.

### [Synthesis Scheme C-3]

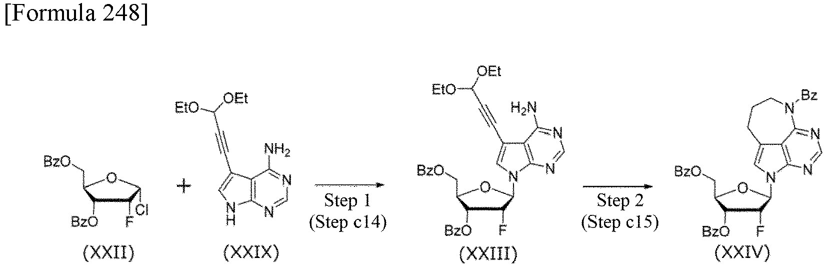

### Example 29:

### Synthesis of 7-(3,5-di-O-benzoyl-2-deoxy-2-fluoro-β-D-ribofuranosyl)-5-(3,3-diethoxypropa-1-yn-1-yl)-7H-pyrrolo[2,3-d]pyrimidin-4-amine (corresponding to a compound of formula (XXXIII))

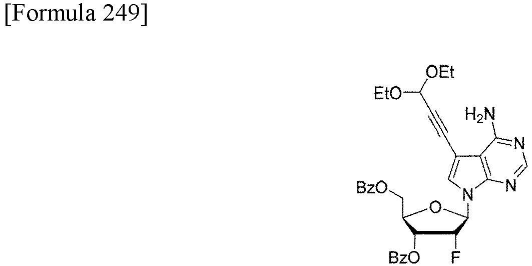

### (Step 1)

To an acetonitrile solution (2 mL) containing the compound (132.2 mg, 0.508 mmol) obtained in Example 17 was added cesium carbonate (210.1 mg, 0.595 mmol), and the mixture was stirred for 15 minutes. The compound (100.0 mg, 0.264 mmol) obtained in Example 23 was then added, and the mixture was stirred for 17 hours. Acetic acid (50 µL) was added to the reaction solution, and ethyl acetate (1 mL) and water (1 mL) were then added. The mixture was liquid-separated. The aqueous layer was extracted with ethyl acetate (1 mL), and the resulting organic layer was combined and concentrated under reduced pressure. The residue was purified by silica gel column chromatography (hexane/ethyl acetate) to obtain the target compound (78.7 mg, 0.131 mmol; yield: 49.5%).
¹H-NMR (500 MHz, CDCl₃) δ 8.28 (1H, s), 8.10-8.07 (2H, m), 8.01-7.97 (2H, m), 7.64-7.59 (1H, m), 7.58-7.53 (1H, m), 7.49-7.44 (2H, m), 7.44-7.39 (2H, m), 7.31 (1H, s), 6.31 (1H, dd, J = 20.0, 1.7 Hz), 5.97-5.79 (3H, m), 5.60 (2H, brs), 5.48 (1H, s), 4.79 (1H, dd, J = 12.0, 3.4 Hz), 4.76-4.72 (1H, m), 4.64 (1H, dd, J = 12.0, 4.0 Hz), 3.83-3.75 (2H, m), 3.69-3.62 (2H, m), 1.30-1.25 (6H, m).

### Example 30:

### Synthesis of 6-benzoyl-2-(3,5-di-O-benzoyl-2-deoxy-2-fluoro-β-D-ribofuranosyl)-6,7,8,9-tetrahydro-2H-2,3,5,6-tetraazabenzo[cd]azulene (corresponding to a compound of formula (XXIV))

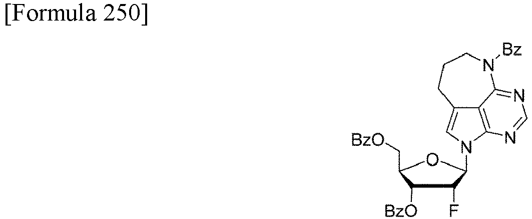

### (Step 2)

To an ethanol solution (3.5 mL) containing the compound (78.7 mg, 0.131 mmol) obtained in Example 29 was added 10% palladium carbon (34.4 mg, 48% moisture). The mixture was then stirred under a hydrogen atmosphere (300 kPa) at room temperature for 23 hours. The reaction solution was then stirred under a hydrogen atmosphere (500 kPa) at 40°C for 10 hours. The reaction system was replaced with nitrogen. After that, the palladium carbon was filtered and washed with ethanol (1 mL). The filtrate was concentrated under reduced pressure. Subsequently, acetic acid (1.4 mL), water (70 µL), and 10% palladium carbon (34.4 mg, 48% moisture) were added. The mixture was then stirred under a hydrogen atmosphere (500 kPa) at 50°C for 14 hours. The reaction system was replaced with nitrogen. After that, the palladium carbon was filtered and washed with ethyl acetate (1 mL). The filtrate was concentrated under reduced pressure. Ethyl acetate (1.5 mL), water (1.5 mL), and potassium phosphate (0.15 g) were then added, the mixture was liquid-separated, and the organic layer was concentrated under reduced pressure. To the residue were added pyridine (1.5 mL), benzoyl chloride (60 µL, 0.51 mmol), and 4-dimethylaminopyridine (3.0 mg, 0.025 mmol). The mixture was stirred at 50°C for 2 hours. Benzoyl chloride (30 µL, 0.26 mmol) and 4-dimethylaminopyridine (8.5 mg, 0.070 mmol) were then added, and the mixture was stirred at 70°C for 1 hour. The reaction solution was cooled to room temperature and stirred for 16 hours. Then, ethyl acetate (2 mL) and water (2 mL) were added, and the mixture was liquid-separated. The organic layer was washed twice with 10% aqueous potassium phosphate solution (1 mL). The organic layer was concentrated under reduced pressure. The residue was purified by silica gel column chromatography (hexane/ethyl acetate) to obtain the target compound (50.8 mg, 0.0819 mmol; yield: 62.5%).

### Reference Example 5: To produce anti-CD70 antibody 1

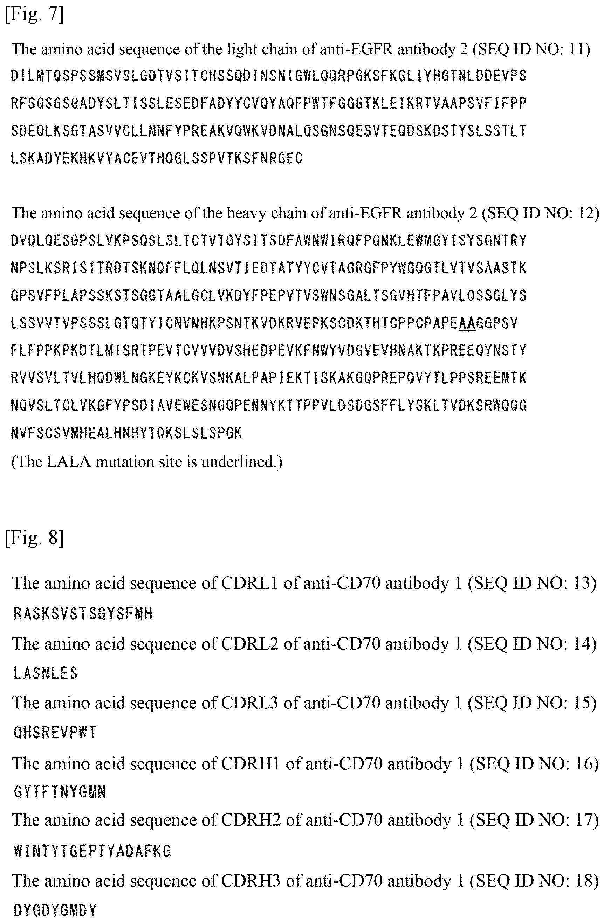

Anti-CD70 antibody 1 was produced with reference to WO2004/073656. The anti-CD70 antibody 1 used in the Examples has an isotype of IgG1 and a LALA mutation. Fig. 2 shows the light chain amino acid sequence (SEQ ID NO: 1) or the heavy chain amino acid sequence (SEQ ID NO: 2) of anti-CD70 antibody 1 used in the Examples. In the light chain sequence set forth in SEQ ID NO: 1 (Fig. 2), the amino acid sequence consisting of amino acid residues 1 to 112 is the light chain variable region, and in the heavy chain sequence set forth in SEQ ID NO: 2 (Fig. 2), the amino acid sequence consisting of amino acid residues 1 to 118 is the heavy chain variable region. Fig. 8 shows the amino acid sequence of CDRL1 (SEQ ID NO: 13), the amino acid sequence of CDRL2 (SEQ ID NO: 14), the amino acid sequence of CDRL3 (SEQ ID NO: 15), the amino acid sequence of CDRH1 (SEQ ID NO: 16), the amino acid sequence of CDRH2 (SEQ ID NO: 17), and the amino acid sequence of CDRH3 (SEQ ID NO: 18) of this antibody.

### Reference Example 6: To produce anti-CD70 antibody 2

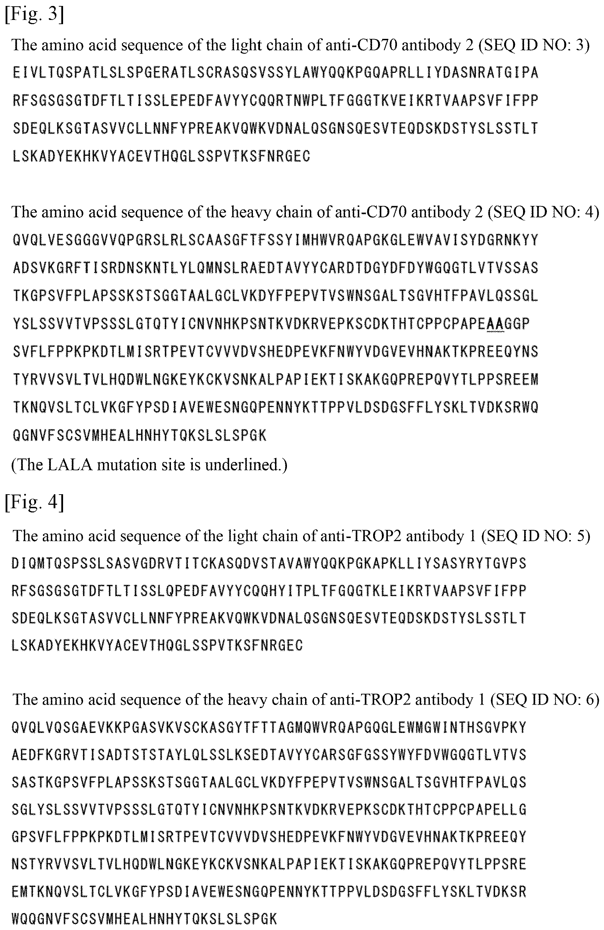

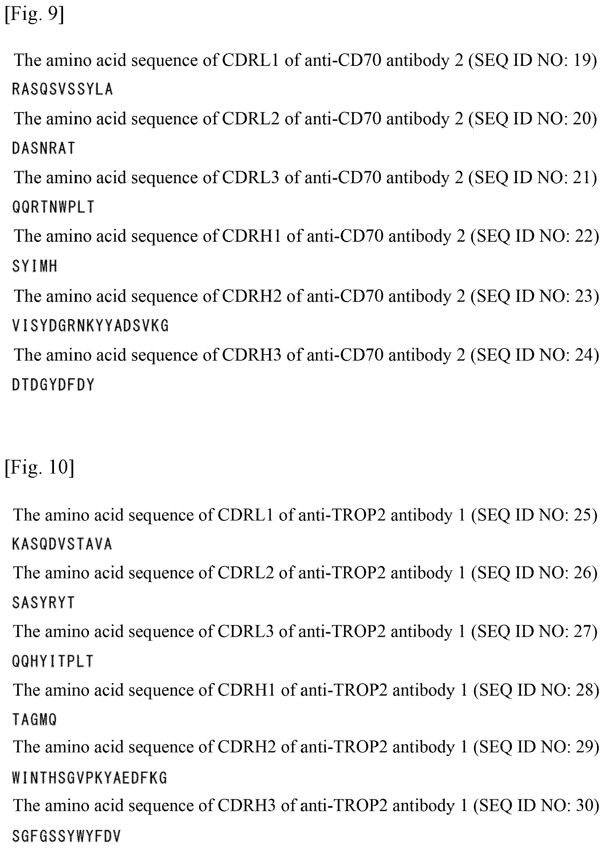

Anti-CD70 antibody 2 was produced with reference to WO2007/038637. The anti-CD70 antibody 2 used in the Examples has an isotype of IgG1 and a LALA mutation. Fig. 3 shows the light chain amino acid sequence (SEQ ID NO: 3) or the heavy chain amino acid sequence (SEQ ID NO: 4) of anti-CD70 antibody 2 used in the Examples. In the light chain sequence set forth in SEQ ID NO: 3 (Fig. 3), the amino acid sequence consisting of amino acid residues 1 to 108 is the light chain variable region, and in the heavy chain sequence set forth in SEQ ID NO: 4 (Fig. 3), the amino acid sequence consisting of amino acid residues 1 to 118 is the heavy chain variable region. Fig. 9 shows the amino acid sequence of CDRL1 (SEQ ID NO: 19), the amino acid sequence of CDRL2 (SEQ ID NO: 20), the amino acid sequence of CDRL3 (SEQ ID NO: 21), the amino acid sequence of CDRH1 (SEQ ID NO: 22), the amino acid sequence of CDRH2 (SEQ ID NO: 23), and the amino acid sequence of CDRH3 (SEQ ID NO: 24) of this antibody.

### Reference Example 7: To produce anti-TROP2 antibody 1

Anti-TROP2 antibody 1 was produced with reference to WO2015/098099. The anti-TROP2 antibody 1 used in the Examples has an isotype of IgG1. Fig. 4 shows the light chain amino acid sequence (SEQ ID NO: 5) and the heavy chain amino acid sequence (SEQ ID NO: 6) of anti-TROP2 antibody 1 used in the Examples. In the light chain sequence set forth in SEQ ID NO: 5 (Fig. 4), the amino acid sequence consisting of amino acid residues 1 to 108 is the light chain variable region, and in the heavy chain sequence set forth in SEQ ID NO: 6 (Fig. 4), the amino acid sequence consisting of amino acid residues 1 to 121 is the heavy chain variable region. Fig. 10 shows the amino acid sequence of CDRL1 (SEQ ID NO: 25), the amino acid sequence of CDRL2 (SEQ ID NO: 26), the amino acid sequence of CDRL3 (SEQ ID NO: 27), the amino acid sequence of CDRH1 (SEQ ID NO: 28), the amino acid sequence of CDRH2 (SEQ ID NO: 29), and the amino acid sequence of CDRH3 (SEQ ID NO: 30) of this antibody.

### Reference Example 8: To produce anti-TROP2 antibody 2

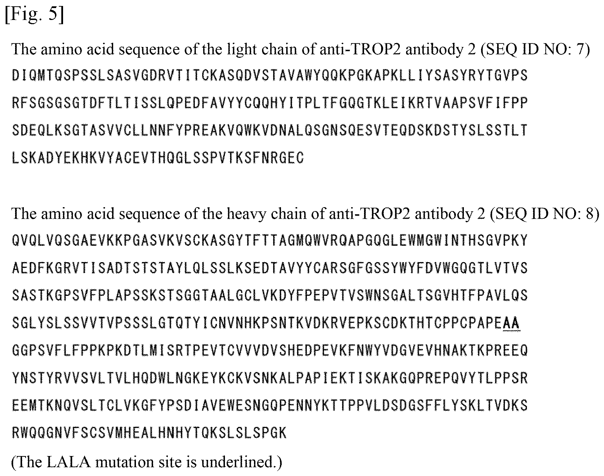

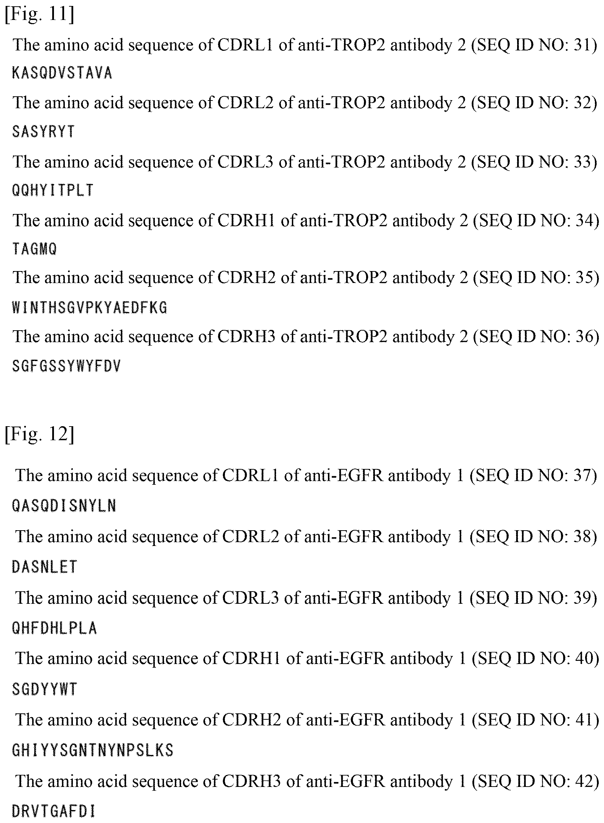

Anti-TROP2 antibody 1 was produced with reference to WO2015/098099. The anti-TROP2 antibody 1 used in the Examples has an isotype of IgG1. Anti-TROP2 antibody 2 was created by introducing a LALA mutation into the anti-TROP2 antibody 1. Fig. 5 shows the light chain amino acid sequence (SEQ ID NO: 7) or the heavy chain amino acid sequence (SEQ ID NO: 8) of anti-TROP2 antibody 2 used in the Examples. In the light chain sequence set forth in SEQ ID NO: 7 (Fig. 5), the amino acid sequence consisting of amino acid residues 1 to 108 is the light chain variable region, and in the heavy chain sequence set forth in SEQ ID NO: 8 (Fig. 5), the amino acid sequence consisting of amino acid residues 1 to 121 is the heavy chain variable region. Fig. 11 shows the amino acid sequence of CDRL1 (SEQ ID NO: 31), the amino acid sequence of CDRL2 (SEQ ID NO: 32), the amino acid sequence of CDRL3 (SEQ ID NO: 33), the amino acid sequence of CDRH1 (SEQ ID NO: 34), the amino acid sequence of CDRH2 (SEQ ID NO: 35), and the amino acid sequence of CDRH3 (SEQ ID NO: 36) of this antibody.

### Reference Example 9: To produce anti-EGFR antibody 1

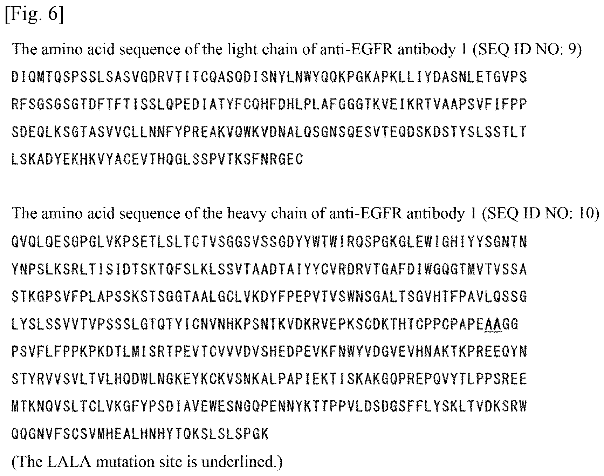

Anti-EGFR antibody 1 was produced by referring to the Vectibix 100 mg Intravenous Infusion Review Results Report (March 5, 2010, Review and Administration Division, Pharmaceutical and Food Safety Bureau). The anti-EGFR antibody 1 used in the Examples has an isotype of IgG1 and a LALA mutation. Fig. 6 shows the light chain amino acid sequence (SEQ ID NO: 9) or the heavy chain amino acid sequence (SEQ ID NO: 10) of anti-EGFR antibody 1 used in the Examples. In the light chain sequence set forth in SEQ ID NO: 9 (Fig. 6), the amino acid sequence consisting of amino acid residues 1 to 108 is the light chain variable region, and in the heavy chain sequence set forth in SEQ ID NO: 10 (Fig. 6), the amino acid sequence consisting of amino acid residues 1 to 119 is the heavy chain variable region. Fig. 12 shows the amino acid sequence of CDRL1 (SEQ ID NO: 37), the amino acid sequence of CDRL2 (SEQ ID NO: 38), the amino acid sequence of CDRL3 (SEQ ID NO: 39), the amino acid sequence of CDRH1 (SEQ ID NO: 40), the amino acid sequence of CDRH2 (SEQ ID NO: 41), and the amino acid sequence of CDRH3 (SEQ ID NO: 42) of this antibody. Each CDR sequence was referred to WO1998/050433.

### Reference Example 10: To produce anti-EGFR antibody 2

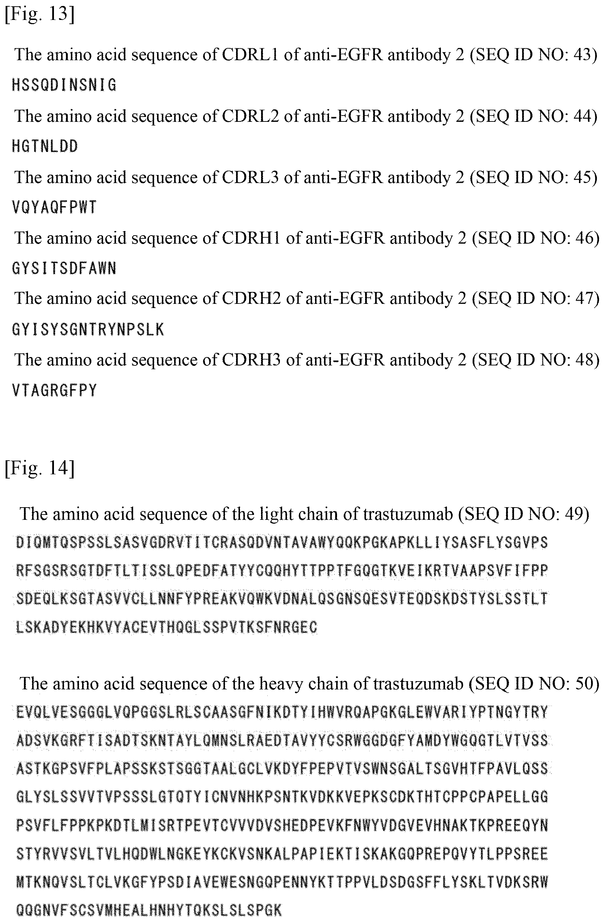

Anti-EGFR antibody 2 was produced with reference to WO2002/092771. The anti-EGFR antibody 2 used in the Examples has an isotype of IgG1 and a LALA mutation. Fig. 7 shows the light chain amino acid sequence (SEQ ID NO: 11) and the heavy chain amino acid sequence (SEQ ID NO: 12) of anti-EGFR antibody 2 used in the Examples. In the light chain sequence set forth in SEQ ID NO: 11 (Fig. 7), the amino acid sequence consisting of amino acid residues 1 to 108 is the light chain variable region, and in the heavy chain sequence set forth in SEQ ID NO: 12 (Fig. 7), the amino acid sequence consisting of amino acid residues 1 to 116 is the heavy chain variable region. Fig. 13 shows the amino acid sequence of CDRL1 (SEQ ID NO: 43), the amino acid sequence of CDRL2 (SEQ ID NO: 44), the amino acid sequence of CDRL3 (SEQ ID NO: 45), the amino acid sequence of CDRH1 (SEQ ID NO: 46), the amino acid sequence of CDRH2 (SEQ ID NO: 47), and the amino acid sequence of CDRH3 (SEQ ID NO: 48) of this antibody.

### Reference Example 11: To produce anti-HER2 antibody

As used herein, "trastuzumab" is sometimes referred to as HERCEPTIN (registered trademark), huMAb4D5-8, or rhuMAb4D5-8. The "trastuzumab" is a humanized IgG1 antibody comprising a light chain consisting of the amino acid sequence set forth in SEQ ID NO: 1 and a heavy chain consisting of the amino acid sequence set forth in SEQ ID NO: 2. The amino acid sequence was with reference to US5821337. Fig. 14 shows the light chain amino acid sequence (SEQ ID NO: 49) or the heavy chain amino acid sequence (SEQ ID NO: 50) of trastuzumab.

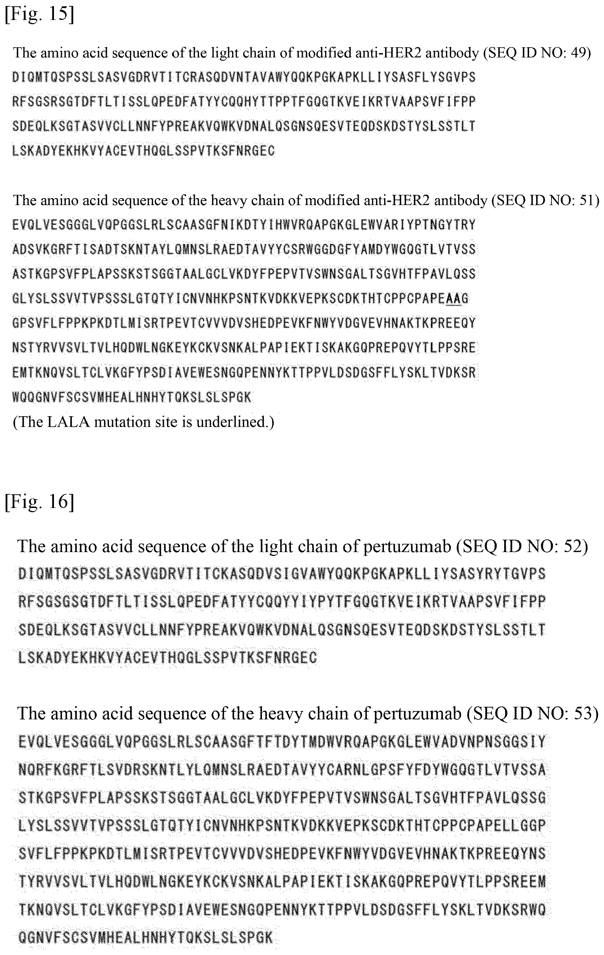

In the anti-HER2 antibody, the leucine residues (L) at the 234- and 235-positions according to the EU index of the heavy chain amino acid sequence of trastuzumab were each mutated to alanine (A) (herein sometimes referred to as LALA mutation). This trastuzumab constant region-modified IgG1 antibody (herein also referred to as modified anti-HER2 antibody) can be designed and produced. Fig. 15 shows the light chain amino acid sequence (SEQ ID NO: 49) and the heavy chain amino acid sequence (SEQ ID NO: 51) of the modified anti-HER2 antibody.

### Reference Example 12: To produce anti-HER2 antibody 2

"Pertuzumab" is sometimes referred to as PERJETA (registered trademark). The "pertuzumab" is a humanized IgG1 antibody comprising a light chain consisting of the amino acid sequence set forth in SEQ ID NO: 28 and a heavy chain consisting of the amino acid sequence set forth in SEQ ID NO: 53. The amino acid sequence was with reference to WO2004/008099. This antibody was herein referred to as anti-HER2 antibody 2. Fig. 16 shows the light chain amino acid sequence (SEQ ID NO: 52) or the heavy chain amino acid sequence (SEQ ID NO: 53) of pertuzumab.

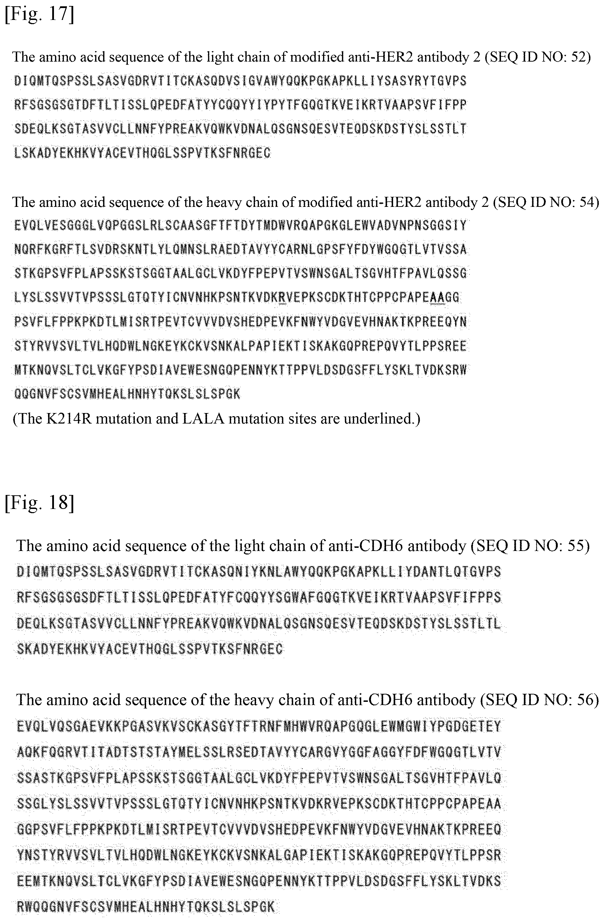

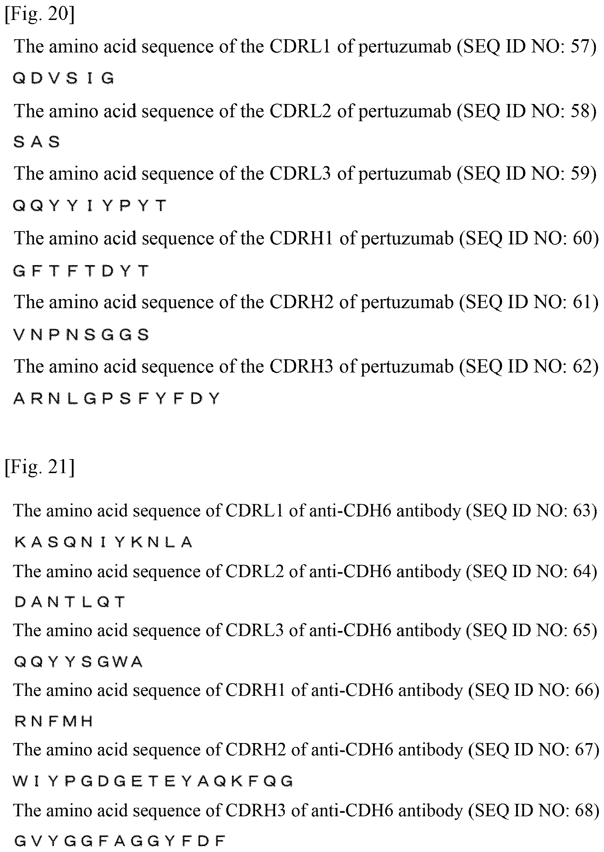

The anti-HER2 antibody 2 has a G1m3-allotype constant region in which in addition to the LALA mutation, the lysine (K) at EU index 214 of the heavy chain amino acid sequence is mutated to arginine (R). This anti-HER2 antibody 2 (herein also referred to as modified anti-HER2 antibody 2) can be designed and produced. Fig. 17 shows the light chain amino acid sequence (SEQ ID NO: 52) and the heavy chain amino acid sequence (SEQ ID NO: 54) of the modified anti-HER2 antibody 2 used in the Examples. Fig. 21 shows the amino acid sequence of CDRL 1 (SEQ ID NO: 57), the amino acid sequence of CDRL2 (SEQ ID NO: 58), the amino acid sequence of CDRL3 (SEQ ID NO: 59), the amino acid sequence of CDRH1 (SEQ ID NO: 60), the amino acid sequence of CDRH2 (SEQ ID NO: 61), and the amino acid sequence of CDRH3 (SEQ ID NO: 62) of this antibody.

### Reference Example 13: To produce anti-CDH6 antibody

Anti-CDH6 antibody can be produced with reference to WO2018/212136. The anti-CDH6 antibody used in the Examples has an isotype ofIgG 1 and has, in addition to the LALA mutation, a mutation in which the proline (P) at 329-position according to the EU index of the heavy chain amino acid sequence is mutated to glycine (G). Fig. 18 shows the light chain amino acid sequence (SEQ ID NO: 55) and the heavy chain amino acid sequence (SEQ ID NO: 56) of anti-CDH6 antibody used in the Examples. In the light chain sequence set forth in SEQ ID NO: 55 (Fig. 18), the amino acid sequence consisting of amino acid residues 1 to 107 is the light chain variable region, and in the heavy chain sequence set forth in SEQ ID NO: 56 (Fig. 18), the amino acid sequence consisting of amino acid residues 1 to 122 is the heavy chain variable region. Fig. 21 shows the amino acid sequence of CDRL1 (SEQ ID NO: 63), the amino acid sequence of CDRL2 (SEQ ID NO: 64), the amino acid sequence of CDRL3 (SEQ ID NO: 65), the amino acid sequence of CDRH1 (SEQ ID NO: 66), the amino acid sequence of CDRH2 (SEQ ID NO: 67), and the amino acid sequence of CDRH3 (SEQ ID NO: 68) of this antibody.

## Claims

1. A method for preparing a compound represented by formula (Rp-VII): 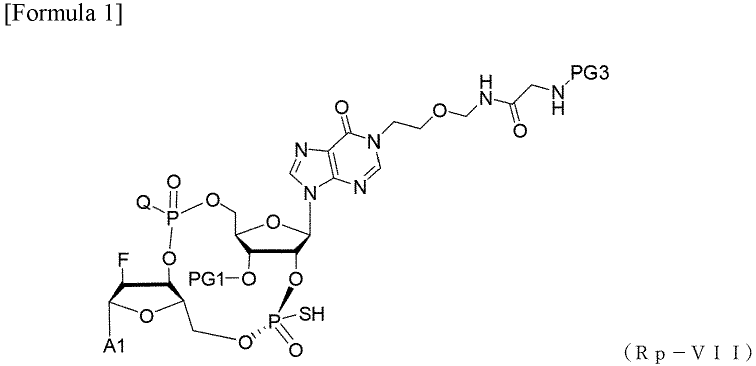 or a salt thereof, wherein
A1 is 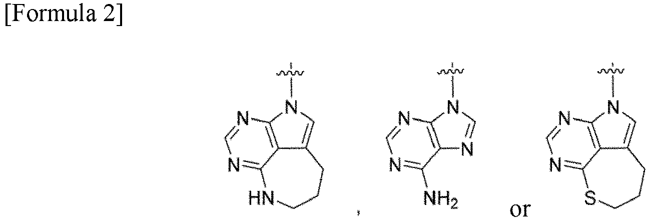
Q is a thiol group or a hydroxy group,
PG1 is a protecting group for a hydroxy group, and
PG3 is a protecting group for an amino group, the method comprising the steps of:
(step a1) reacting a compound represented by formula (I): 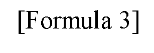 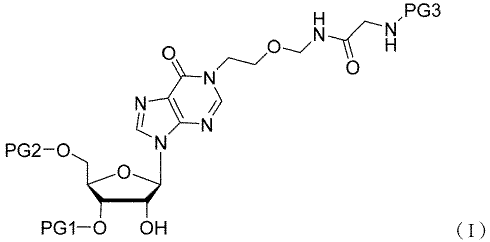 wherein
PG1 and PG3 are as defined above, and
PG2 is a protecting group for a hydroxy group,
with an optically active phosphitylating agent (Rc-II) selected from the group consisting of the following formulas (Rc-II-1) and (Rc-II-2): 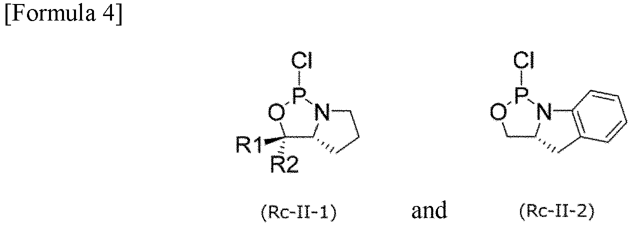 wherein
R1 is hydrogen or methyl, and
R2 is hydrogen, C₁₋₃ alkyl, or phenyl,
wherein the alkyl is unsubstituted or substituted with one or more phenyl, tosyl, or diphenylmethylsilyl, and
the phenyl is unsubstituted or substituted with nitro or methoxy, to obtain a compound represented by formula (Rc-III): 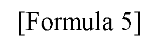 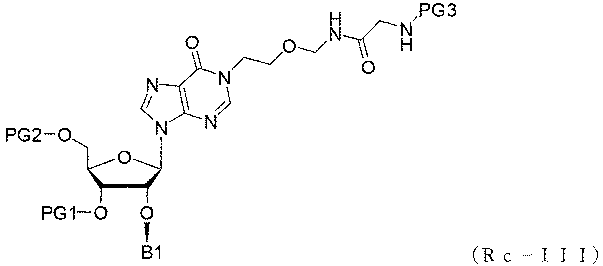 wherein
PG1, PG2, and PG3 are as defined above, and
B1 is 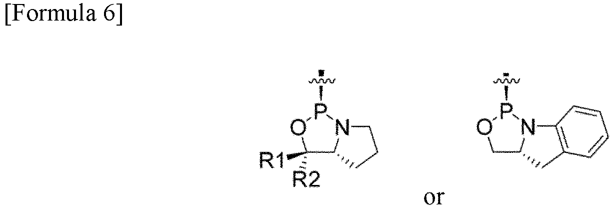 wherein R1 and R2 are as defined above;
(step a2) reacting, in the presence of an activator, the resulting compound of formula (Rc-III) with a compound represented by formula (IV): 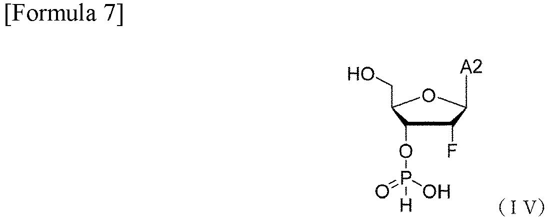 or a salt thereof, wherein
A2 is 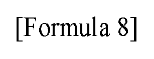 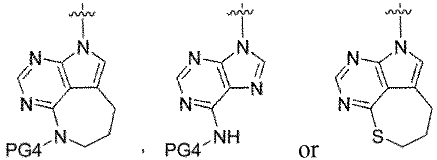 wherein PG4 is a protecting group for an amino group,
followed by treatment with an acylating agent or alkoxycarbonylating agent, a further reaction with a sulfurizing agent, and then removal of PG2 by deprotection to obtain a compound represented by (Rc-V): 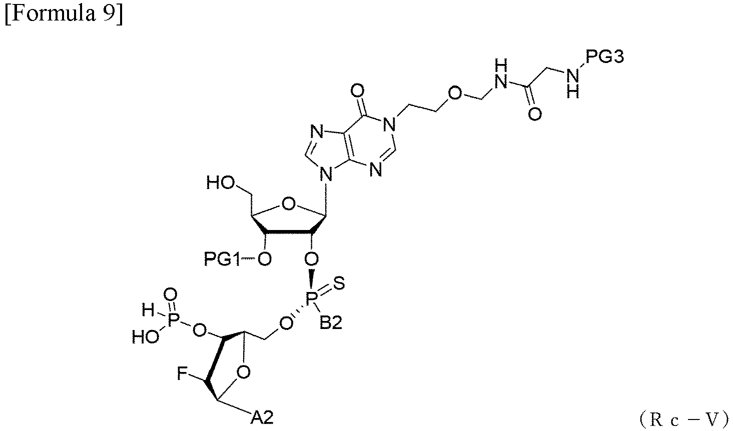
or a salt thereof, wherein
A2, PG1, and PG3 are as defined above,
B2 is 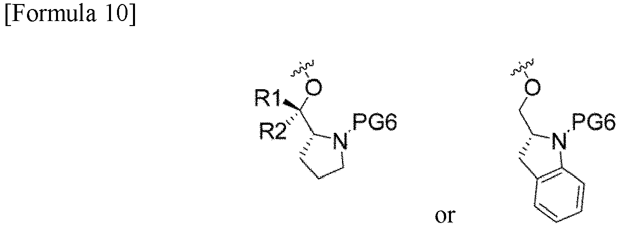 wherein PG6 is a protecting group for an amino group;
(step a3) cyclizing the resulting compound of formula (Rc-V) or a salt thereof in the presence of a condensing agent, followed by a reaction with a sulfurizing or oxidizing agent to obtain a compound represented by formula (Rc-VI): 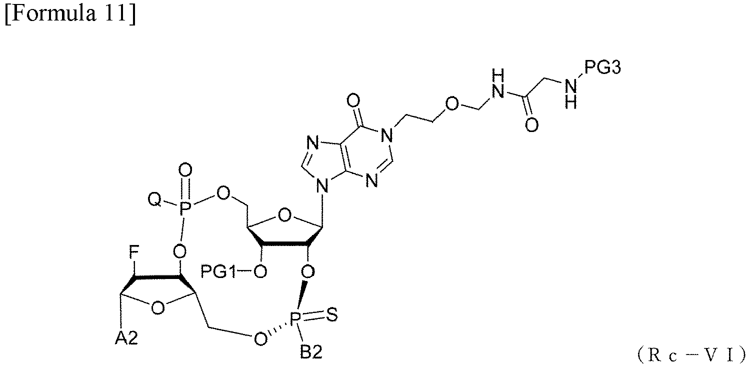 or a salt thereof, wherein
A2, B2, Q, PG1, and PG3 are as defined above; and
(step a4) removing B2, which is a protecting group for a thiophosphoric acid moiety in the resulting compound of formula (Rc-VI), and PG4, which is a protecting group in A2, by deprotection to obtain a compound of formula (Rp-VII) or a salt thereof.

2. The method according to claim 1, wherein in step a2, an intermediate obtained after the reaction with the sulfurizing agent but before the removal of PG2 by deprotection is a compound represented by formula (Rc-V₀): 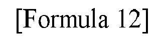 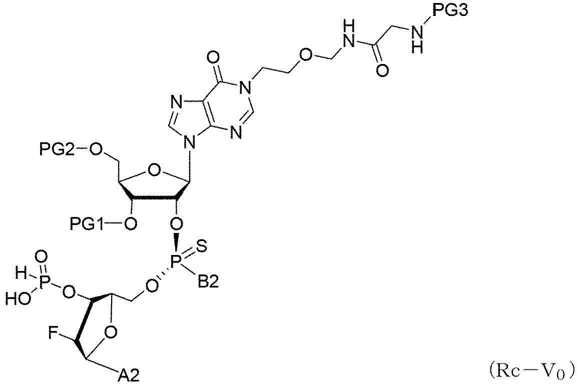 or a salt thereof, wherein
A2, B2, PG1, PG2, and PG3 are as defined in claim 1.

3. A method for preparing a compound represented by formula (Sp-VII): 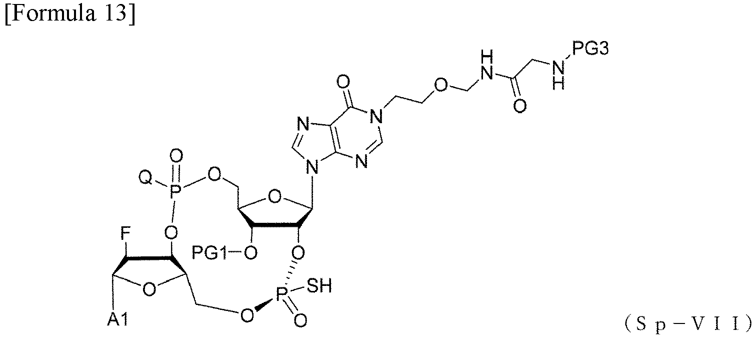 or a salt thereof, wherein
A1 is 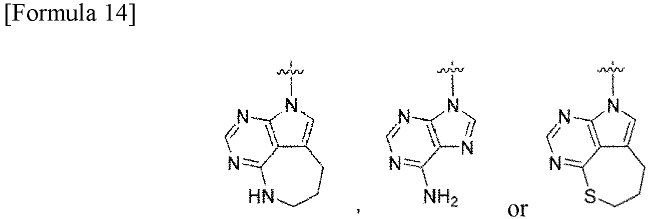
Q is a thiol group or a hydroxy group,
PG1 is a protecting group for a hydroxy group, and
PG3 is a protecting group for an amino group, the method comprising the steps of:
(step a1) reacting a compound represented by formula (I): 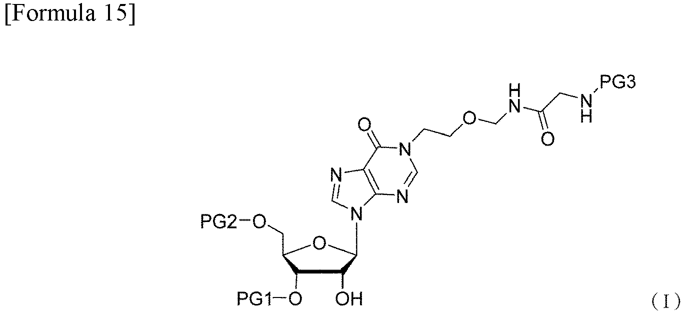 wherein
PG1 and PG3 are as defined above, and
PG2 is a protecting group for a hydroxy group,
with an optically active phosphitylating agent (Sc-II) selected from the group consisting of the following formulas (Sc-II-1) and (Sc-II-2): 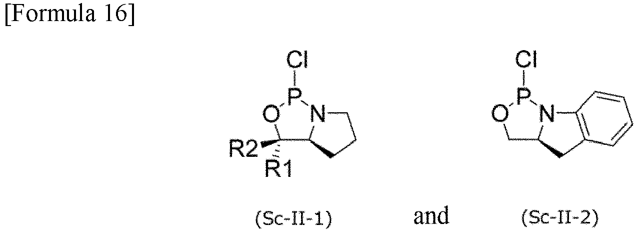 wherein
R1 is hydrogen or methyl, and
R2 is hydrogen, C₁₋₃ alkyl, or phenyl,
wherein the alkyl is unsubstituted or substituted with one or more phenyl, tosyl, or diphenylmethylsilyl, and
the phenyl is unsubstituted or substituted with nitro or methoxy, to obtain a compound represented by formula (Sc-III): 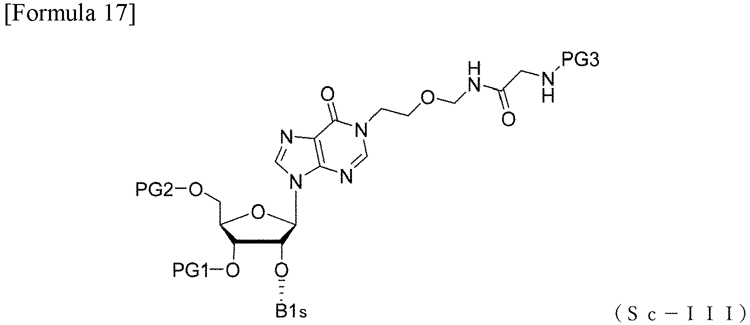 wherein
PG1, PG2, and PG3 are as defined above, and
B1_{S} is 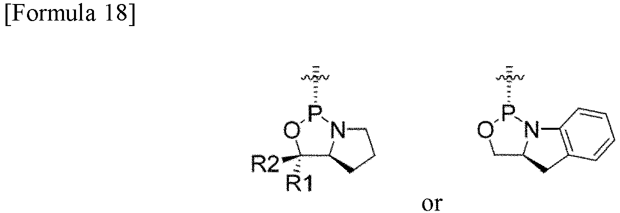 wherein R1 and R2 are as defined above;
(step a2) reacting, in the presence of an activator, the resulting compound of formula (Sc-III) with a compound represented by formula (IV): 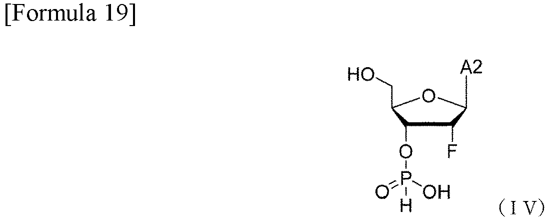 or a salt thereof, wherein
A2 is 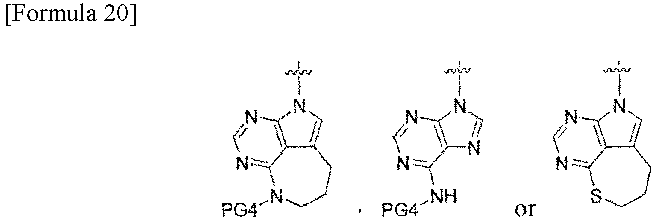 wherein PG4 is a protecting group for an amino group,
followed by treatment with an acylating agent or alkoxycarbonylating agent, a further reaction with a sulfurizing agent, and then removal of PG2 by deprotection to obtain a compound represented by (Sc-V): 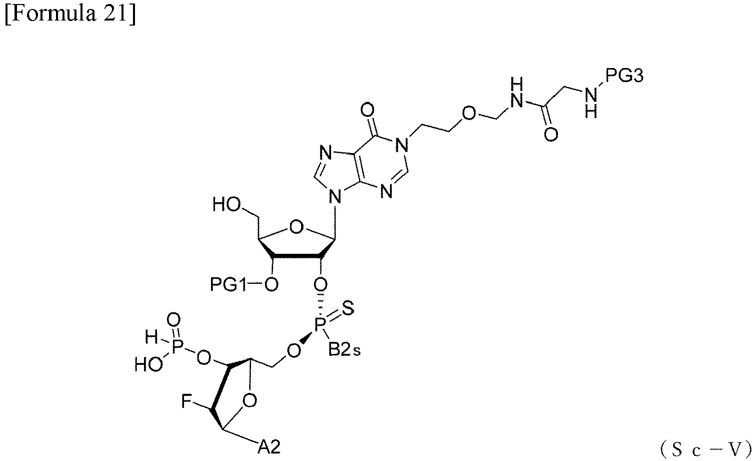
or a salt thereof, wherein
A2, PG1, and PG3 are as defined above, and
B2s is 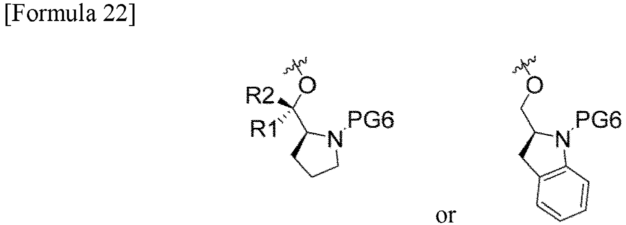 wherein PG6 is a protecting group for an amino group;
(step a3) cyclizing the resulting compound of formula (Sc-V) or a salt thereof in the presence of a condensing agent, followed by a reaction with a sulfurizing or oxidizing agent to obtain a compound represented by formula (Sc-VI): 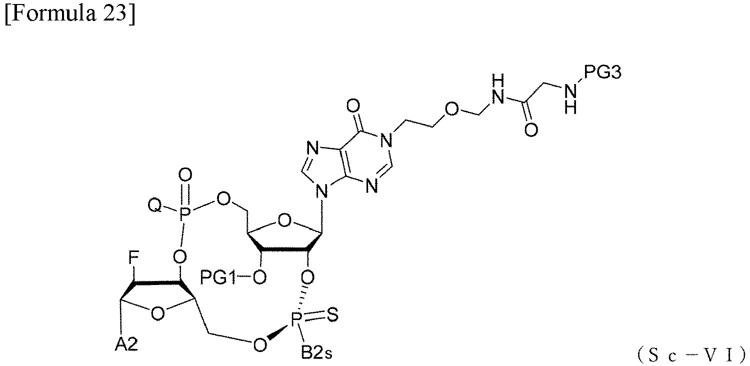 or a salt thereof, wherein A2, B2ₛ, Q, PG1, and PG3 are as defined above; and
(step a4) removing B2ₛ, which is a protecting group for a thiophosphoric acid moiety in the resulting compound of formula (Sc-VI), and PG4, which is a protecting group in A2, by deprotection to obtain a compound of formula (Sp-VII) or a salt thereof.

4. The method according to claim 2, wherein in step a2, the intermediate obtained after the reaction with the sulfurizing agent but before the removal of PG2 by deprotection is a compound represented by formula (Sc-V₀): 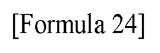 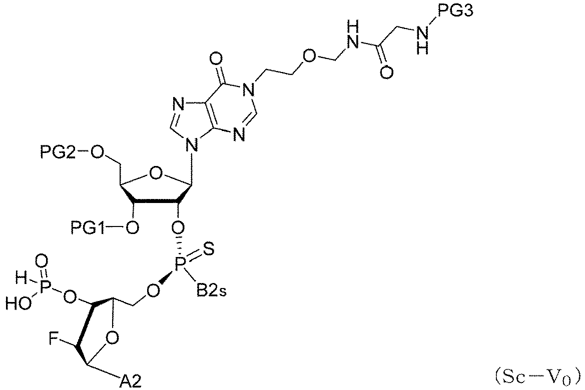 or a salt thereof, wherein
A2, B2ₛ, PG1, PG2, and PG3 are as defined in claim 2.

5. The method according to any one of claims 1 to 4, wherein the activator in step a2 is at least one selected from the group consisting of 1-phenylimidazole, benzimidazole, 1-methylbenzimidazole, 1-cyanomethylpiperidine, 1-pyrrolidineacetonitrile, 1-(cyanomethyl)imidazole, and salts thereof.

6. The method according to any one of claims 1 to 5, wherein the acylating or alkoxycarbonylating agent in step a2 is at least one selected from the group consisting of acetic anhydride, N-succinimidyl acetate, pentafluorophenyl acetate, ethyl trifluoroacetate, methyl trifluoroacetate, pentafluorophenyl trifluoroacetate, trifluoroacetyl benzotriazole, 1-trifluoroacetylimidazole, benzoic anhydride, pentafluorophenylbenzoate, pentafluorophenylbenzoate, 1-*tert*-butoxycarbonyl-1,2,4-triazole, N-tert-butoxycarbonyl imidazole, di-tert-butyl dicarbonate, 9-fluorenyl methyl pentafluorophenyl carbonate, 1-[(9H-fluoren-9-ylmethoxy)carbonyloxy]benzotriazole, N-[(9H-fluoren-9-ylmethoxy)carbonyloxy]succinimide, N-(2,2,2-(trichloroethoxycarbonyloxy)succinimide, N-carbobenzyloxysuccinimide, dibenzyl dicarbonate, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, and methylimidazole-1 -carboxylate.

7. The method according to any one of claims 1 to 6, wherein the sulfurizing agent in step a2 is at least one selected from the group consisting of xanthane hydride, bis(phenylacetyl)disulfide, 3H-1,2-benzodithiol-3-one-1,1-dioxide, 5-phenyl-3H-1,2,4-dithiazol-3-one, and [(N,N-dimethylaminomethylidene)amino]-3H-1,2,4-dithiazolin-3 -thione.

8. The method according to any one of claims 1 to 7, wherein the condensing agent in step a3 is at least one selected from the group consisting of 2-chloro-5,5-dimethyl-1,3,2-dioxaphosphorinane 2-oxide, dimethylchlorophosphate, diethylchlorophosphate, 2-chloro-2-oxo-1,3,2-dioxaphosphorane, 1H-benzotriazol-1-yloxytripyrrolidino phosphonium hexafluorophosphate, chlorotripyrrolidino hexafluorophosphate, bromotripyrrolidino hexafluorophosphate, and propylphosphonic anhydride.

9. The method according to any one of claims 1 to 8, wherein the sulfurizing agent in step a3 is at least one selected from the group consisting of xanthane hydride, bis(phenylacetyl)disulfide, 3H-1,2-benzodithiol-3-one-1,1-dioxide, 5-phenyl-3H-1,2,4-dithiazol-3-one, and [(N,N-dimethylaminomethylidene)amino]-3H-1,2,4-dithiazolin-3 -thione.

10. The method according to any one of claims 1 to 8, wherein the oxidizing agent in step a3 is at least one selected from the group consisting of iodine, *tert-*butylhydroperoxide, 3-chloroperbenzoic acid, hydrogen peroxide, periodic acid, potassium permanganate, and oxygen.

11. The method according to any one of claims 1 to 10, wherein PG4 is benzyl, benzoyl, tert-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, or ethoxycarbonyl.

12. The method according to any one of claims 1 to 11, wherein PG6 is acetyl, trifluoroacetyl, benzoyl, tert-butoxycarbonyl, 9-fluorenylmethyloxycarbonyl, allyloxycarbonyl, 2,2,2-trichloroethoxycarbonyl, benzyloxycarbonyl, 2-(trimethylsilyl)ethoxycarbonyl, methoxycarbonyl, or ethoxycarbonyl.

13. The method according to any one of claims 1 to 12, wherein the compound of formula (IV) or a salt thereof is prepared by the following steps of:
(step a5) reacting a compound represented by formula (VIII): 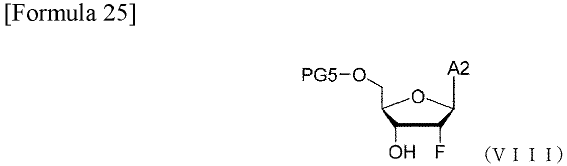 or a salt thereof, wherein
A2 is as defined in claim 1, and
PG5 is a protecting group for a hydroxy group,
with a phosphite ester to obtain a compound represented by formula (IX): 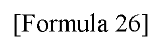 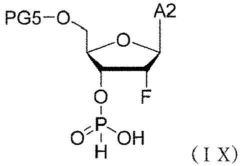 or a salt thereof, wherein A2 and PG5 are as defined above,
or
(step a5') reacting a compound represented by formula (VIII'): 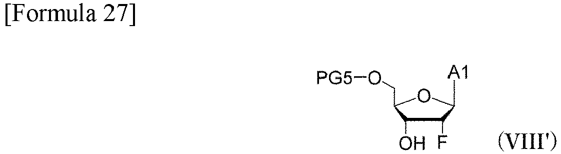 or a salt thereof, wherein
A1 is as defined in claim 1, and
PG5 is as defined above,
with a phosphite ester to obtain a compound represented by formula (IX'): 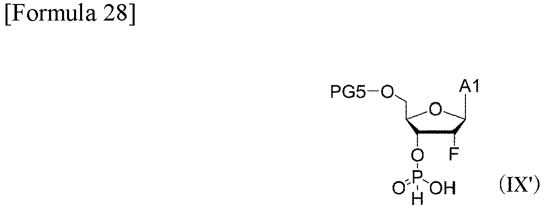 or a salt thereof, wherein A1 and PG5 are as defined above, and
(step a5") reacting the resulting compound of formula (IX') or a salt thereof with an acylating or alkoxycarbonylating agent to obtain a compound represented by formula (IX): 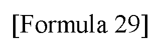 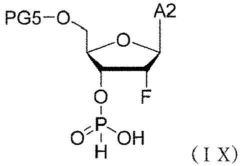 or a salt thereof, wherein A2 and PG5 are as defined above; and
(step a6) removing PG5, which is a protecting group for a 5' hydroxyl group in the resulting compound of formula (IX) obtained in step a5 or a5", by deprotection to obtain a compound of formula (IV) or a salt thereof.

14. The method according to claim 13, wherein the phosphite ester in steps a5 and a5' is at least one selected from the group consisting of diphenyl phosphite, methyl phosphite, diethyl phosphite, and dibutyl phosphite.

15. The method according to claim 13 or 14, wherein the acylating or alkoxycarbonylating agent in step a5" is at least one selected from the group consisting of acetic anhydride, acetyl chloride, N-succinimidyl acetate, pentafluorophenyl acetate, 1-acetyl-1H-1,2,3-triazolo[4,5-b]pyridine, N-methoxydiacetamide, N-acetylimidazole, trifluoroacetic anhydride, bistrifluoroacetamide, ethyl trifluoroacetate, methyl trifluoroacetate, pentafluorophenyl trifluoroacetate, trifluoroacetyl benzotriazole, S-ethyl trifluorothioacetate, N-methylbistrifluoroacetamide, trifluoroacetyltriflate, 1-trifluoroacetylimidazole, benzoic anhydride, benzoyl chloride, pentafluorophenylbenzoate, pentafluorophenylbenzoate, benzoyl trifluoromethanesulfonate, 3-benzoylthiazolidine-2-thione, *tert-*butylphenylcarbonate, N-(tert-butoxycarbonyloxy)phthalimide, 2-(*tert-*butoxycarbonylthio)-4,6-dimethylpyridine, N-tert-butoxycarbonyl imidazole, *tert-*butylcarbazate, 2-(tert-butoxycarbonyloxyimino)-2-phenylacetonitrile, 1*-tert-*butoxycarbonyl-1,2,4-triazole, N-tert-butoxycarbonyl imidazole, di*-tert-*butyldicarbonate, 9-fluorenylmethylpentafluorophenylcarbonate, 9-fluorenylmethyl chloroformate, 9-fluorenylmethylcarbazate, 19-fluorenylmethylcarbamate, 1-[(9H-fluoren-9-ylmethoxy)carbonyloxy]benzotriazole, N-[(9H-fluoren-9-ylmethoxy)carbonyloxy]succinimide, allyl chloroformate, diallyl dicarbonate, N-(allyloxycarbonyloxy)succinimide, allyl phenyl carbonate, N-(2,2,2-trichloroethoxycarbonyloxy)succinimide, 2,2,2-trichloroethyl chloroformate, benzyl chloroformate, benzyl carbazate, benzyl phenyl carbonate, N-carbobenzyloxysuccinimide, dibenzyl dicarbonate, 4-[2-(trimethylsilyl)ethoxycarbonyloxy]nitrobenzene, 2-(trimethylsilyl)ethyl-3-nitro-1H-1,2,4-triazole-1-carboxylate, N-[2-(trimethylsilyl)ethoxycarbonyloxy]succinimide, N-ethoxycarbonylphthalimide, ethyl chloroformate, diethyldicarbonate, ethyl imidazole-1-carboxylate, 2-ethoxy-1-(ethoxycarbonyl)-1,2-dihydroquinoline, methyl chloroformate, dimethyl carbonate, dimethyldicarbonate, and methyl imidazole-1-carboxylate.

16. The method according to any one of claims 1, 2, 5 to 9, and 11 to 12, wherein in step a3 described in claim 1, a compound of formula (Rp-VII), where Q is a thiol group, or a salt thereof is obtained by using the sulfurizing agent.

17. The method according to claim 16, wherein in step a4 described in claim 1, the resulting compound of formula (Rp-VII), where Q is a thiol group, is subjected to silica gel column chromatography prior to conversion to a salt thereof and/or crystallization after conversion to a salt thereof to obtain a compound of formula (Rp,Rp-VII'): 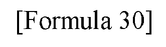 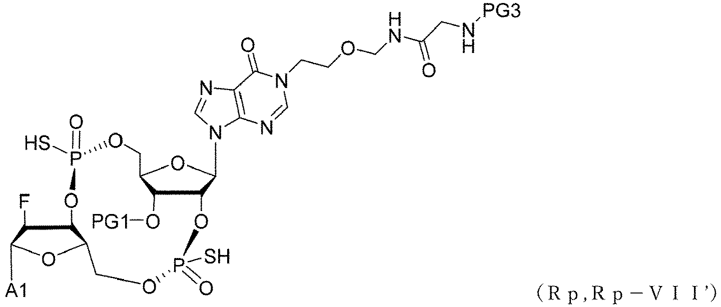 or a salt thereof.

18. The method according to claim 17, further comprising the steps of:
(step a7) removing PG1 and PG3, which are protecting groups in the resulting compound of formula (Rp,Rp-VII'), by deprotection to obtain a compound represented by formula (Rp,Rp-X): 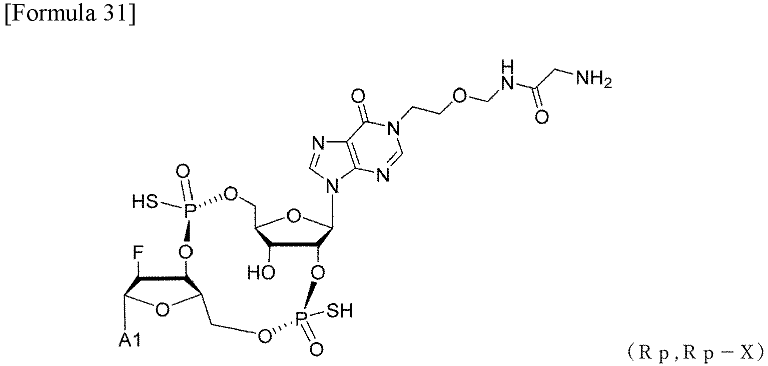 or a salt thereof, wherein
A1 is as defined in claim 1; and
(step a8) condensing the resulting compound of formula (Rp,Rp-X) or a salt thereof with a compound represented by formula (XI): 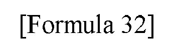 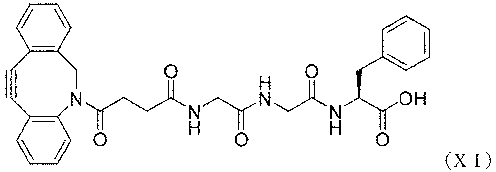 or an active ester thereof to obtain a compound represented by formula (Rp,Rp-XII): 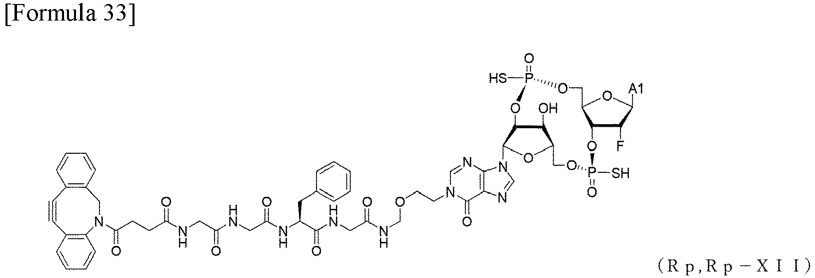 or a salt thereof, wherein A1 is as defined above.

19. The method according to claim 18, further comprising the step of:
(step a9) conjugating the resulting compound of formula (Rp,Rp-XII) or a salt thereof with an antibody or a functional fragment of the antibody (hereinafter, referred to as "Ab") to obtain an antibody-immunostimulant conjugate represented by formula (Rp,Rp-XIII): 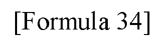 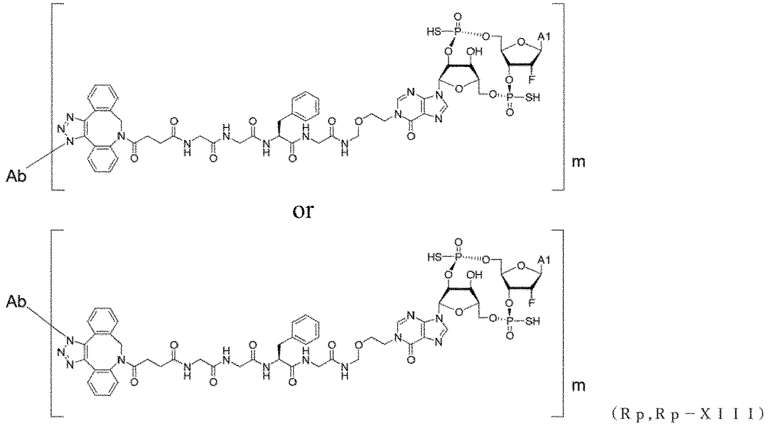
or a mixture thereof, wherein
m ranges from 1 to 10,
a glycan of Ab is optionally remodeled,
Ab is conjugated to a compound of formula (Rp,Rp-XII) either directly from a side chain of an optionally modified amino acid residue or from a glycan or a remodeled glycan of Ab, and
A1 is

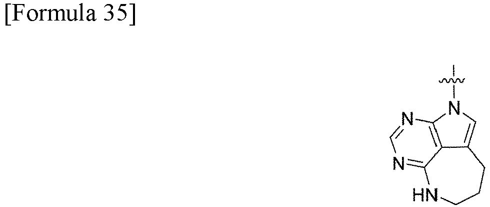

20. The method according to claim 19, wherein in step a9, the compound of formula (Rp,Rp-XII) or a salt thereof is conjugated to Ab by a strain-promoted azide-alkyne cycloaddition reaction.

21. The method according to claim 19 or 20, wherein the antibody is an antibody selected from the group consisting of anti-HER2 antibody, anti-HER3 antibody, anti-DLL3 antibody, anti-FAP antibody, anti-CDH11 antibody, anti-CDH6 antibody, anti-A33 antibody, anti-CanAg antibody, anti-CD 19 antibody, anti-CD20 antibody, anti-CD22 antibody, anti-CD30 antibody, anti-CD33 antibody, anti-CD56 antibody, anti-CD70 antibody, anti-CD98 antibody, anti-TROP2 antibody, anti-CEA antibody, anti-Cripto antibody, anti-EphA2 antibody, anti-G250 antibody, anti-MUC1 antibody, anti-GPNMB antibody, anti-Integrin antibody, anti-PSMA antibody, anti-Tenascin-C antibody, anti-SLC44A4 antibody, anti-Mesothelin antibody, anti-ENPP3 antibody, anti-CD47 antibody, anti-EGFR antibody, anti-GPR20 antibody, and anti-DR5 antibody.

22. A compound represented by formula (Rc-III): 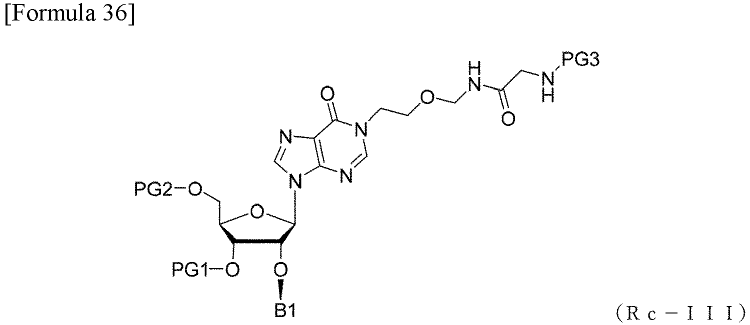 wherein
B1 is as defined in claim 1,
PG1 is tert-butyldimethylsilyl, trimethylsilyl, triethylsilyl, triisopropylsilyl, or tert-butyldiphenylsilyl,
PG2 is 4,4'-dimethoxytrityl, 4-methoxytrityl, 2-chlorotrityl, or trityl, and
PG3 is 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl.

23. A compound represented by formula (Rc-V₀): 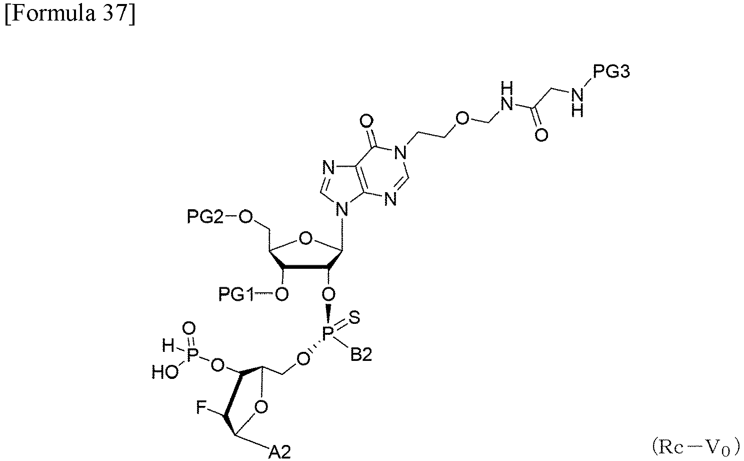 or a salt thereof, wherein
A2 and B2 are as defined in claim 1,
PG1 is tert-butyldimethylsilyl, trimethylsilyl, triethylsilyl, triisopropylsilyl, or tert-butyldiphenylsilyl,
PG2 is 4,4'-dimethoxytrityl, 4-methoxytrityl, 2-chlorotrityl, or trityl, and
PG3 is 2-(trimethylsilyl)ethoxycarbonyl, *tert*-butoxycarbonyl, 9-fluorenylmethyloxycarbonyl, allyloxycarbonyl, 2,2,2-trichloroethoxycarbonyl, or benzyloxycarbonyl.

24. A compound represented by formula (Rc-V): 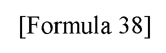 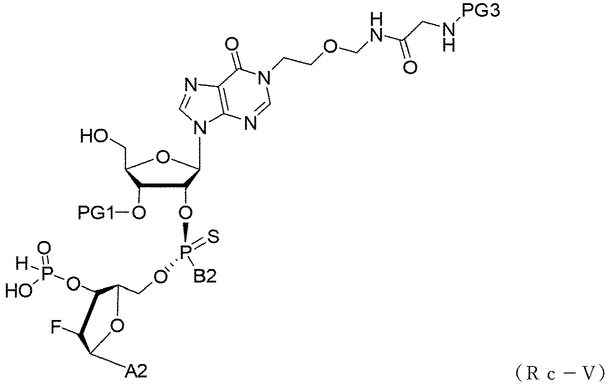 or a salt thereof, wherein
A2 and B2 are as defined in claim 1,
PG1 is tert-butyldimethylsilyl, trimethylsilyl, triethylsilyl, triisopropylsilyl, or tert-butyldiphenylsilyl, and
PG3 is 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl.

25. The method according to any one of claims 1 to 21, wherein the compound of formula (I) where PG1 is tert-butyldimethylsilyl is prepared through the following steps of:
(step b1) reacting a compound represented by formula (XIV): 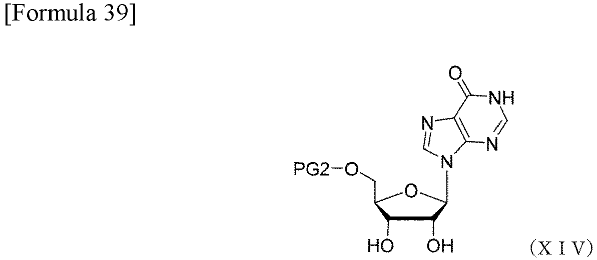 wherein PG2 is as defined in claim 1, with a compound represented by formula (XV): 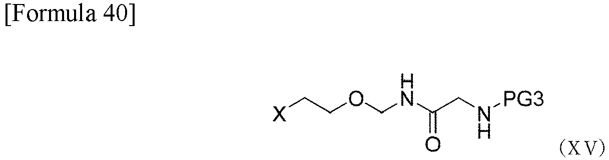 wherein
PG3 is as defined in claim 1, and
X is Cl, Br, or I,
to obtain a compound represented by formula (XVI): 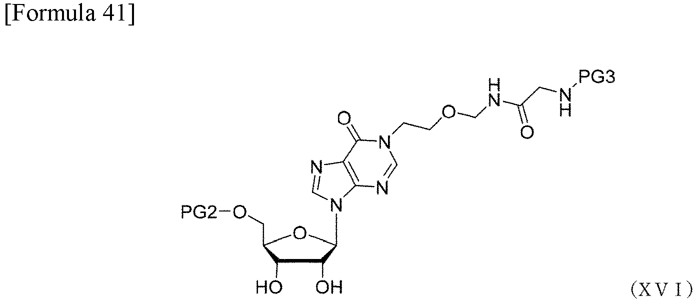 wherein PG2 and PG3 are as defined above; and
(step b2) reacting the resulting compound of formula (XVI) with a silylating agent to obtain a mixture of the following compounds of formula (I') and formula (XVII): 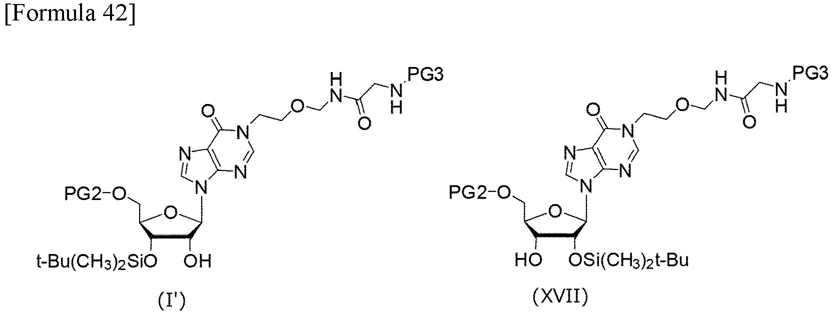 wherein PG2 and PG3 are as defined above; and then converting the compound of formula (XVII) in the mixture to the compound of formula (I') in the presence of a base to obtain the compound of formula (I').

26. The method according to claim 25, wherein the reaction in step b1 is carried out in the presence of a base and the base is 1,1,3,3-tetramethylguanidine, triethylamine, diisopropylethylamine, or 1,8-diazabicyclo[5.4.0]-7-undecene.

27. The method according to claim 25 or 26, wherein in step b2, the reaction of the compound of formula (XVI) with the silylating agent is carried out in the presence of a first base to obtain the mixture of the compound of formula (I') and the compound of formula (XVII), and then the compound of formula (XVII) in the mixture is converted in the presence of a second base to the compound of formula (I') to obtain the compound of formula (I').

28. The method according to claim 27, wherein the first base is at least one selected from the group consisting of 1,1,3,3-tetramethylguanidine, triethylamine, diisopropylethylamine, and 1,8-diazabicyclo[5.4.0]-7-undecene.

29. The method according to claim 27 or 28, wherein the second base is at least one selected from the group consisting of 1,1,3,3-tetramethylguanidine, triethylamine, diisopropylethylamine, and 1,8-diazabicyclo[5.4.0]-7-undecene.

30. The method according to any one of claims 25 to 29, wherein the silylating agent in step b2 is tert-butyldimethylchlorosilane or *tert*-butyldimethylsilyl triflate.

31. The method according to any one of claims 25 to 30, wherein the compound of formula (XV) is prepared by the following step of:
(step b3) reacting a compound represented by formula (XVIII): 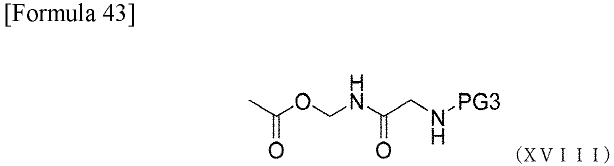 wherein PG3 is as defined in claim 1,
with 2-haloethanol in the presence of an acid or a base to obtain a compound of formula (XV).

32. The method according to claim 31, wherein the base in step b3 is at least one selected from the group consisting of sodium hydroxide, potassium hydroxide, lithium hydroxide, triethylamine, diisopropylethylamine, and 1,8-diazabicyclo[5.4.0]-7-undecene.

33. A compound represented by formula (XV): 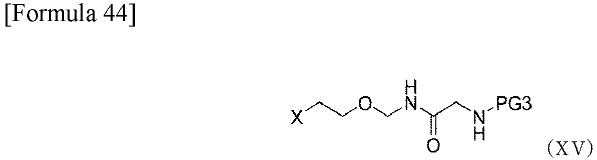 wherein
PG3 is 2-(trimethylsilyl)ethoxycarbonyl, and
X is Cl, Br, or I.

34. The method according to any one of claims 13 to 21 and claims 25 to 32, wherein the compound of formula (VIII') wherein
A1 is 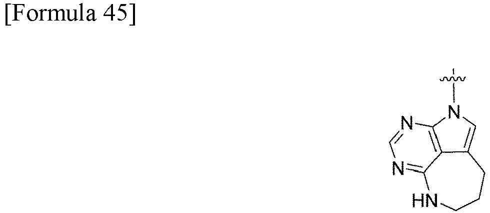 and
PG5 is 4,4'-dimethoxytrityl, 4-methoxytrityl, 2-chlorotrityl, or trityl, is prepared by the following steps of:
(step c1) reacting a compound represented by formula (XIX): 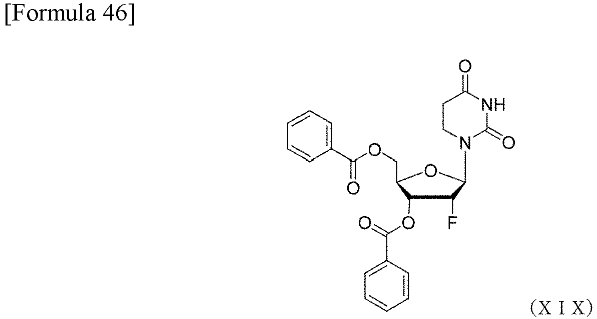 with a benzoylating agent to obtain a compound represented by formula (XX): 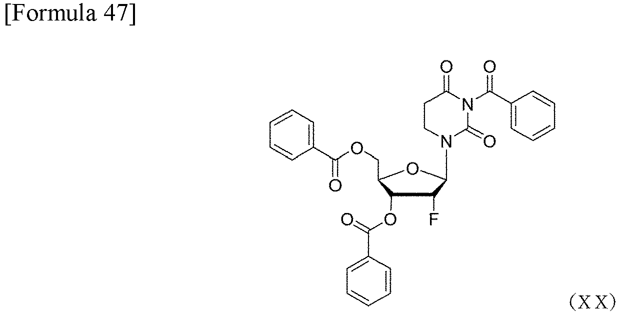
(step c2) hydrolyzing the resulting compound of formula (XX) to obtain a compound represented by formula (XXI): 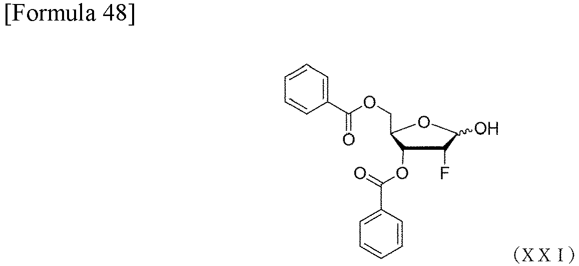
(step c3) reacting the resulting compound of formula (XXI) with a chlorinating agent to obtain a compound represented by formula (XXII): 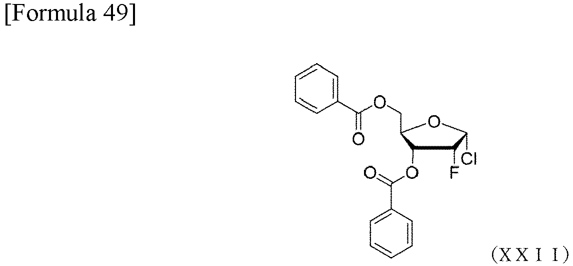
(step c4) reacting the resulting compound of formula (XXII) with a compound represented by formula (XXIII): 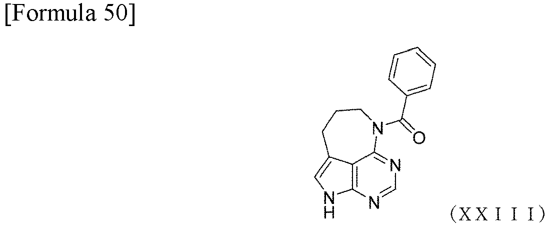 to obtain a compound represented by formula (XVIV): 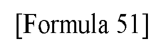 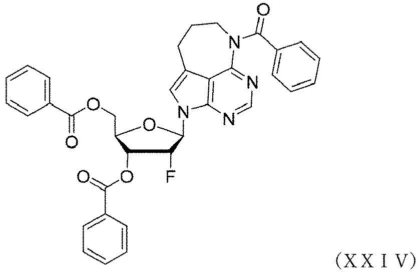
(step c5) deprotecting the resulting compound of formula (XXIV) to remove a benzoyl group, to obtain a compound represented by formula (XXV): 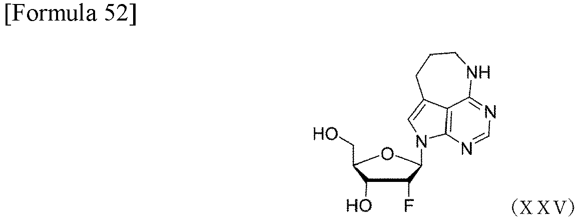 or a salt thereof; and
(step c6) reacting the resulting compound of formula (XXV) or a salt thereof with a tritylating agent to obtain the compound of formula (VIII'), wherein A1 and PG5 are as defined above, or a salt thereof.

35. The method according to claim 34, further comprising the step of:
(step c7) reacting the resulting compound of formula (VIII'), wherein A1 and PG5 are as defined in claim 34, or a salt thereof with a silylating agent, followed by a reaction with an acylating or alkoxycarbonylating agent to obtain a compound represented by formula (XXVII): 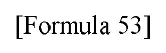 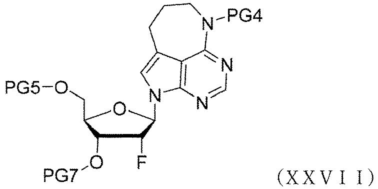 wherein
PG4 is benzoyl, 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl,
PG5 is as defined above, and
PG7 is a protecting group for a hydroxy group; and
(step c8) removing PG7, which is a protecting group in the resulting compound of formula (XXVII), by deprotection to obtain a compound represented by formula (VIII): 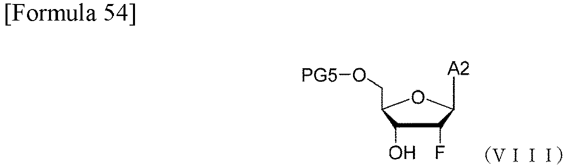 or a salt thereof, wherein
A2 is 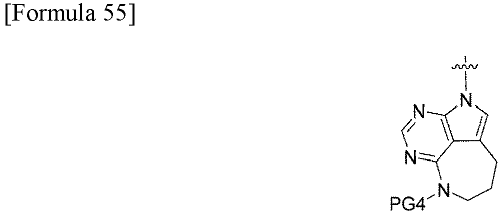 and
PG4 and PG5 are as defined above.

36. The method according to claim 34 or 35, wherein the chlorinating agent in step c3 is at least one selected from the group consisting of trichloroisocyanuric acid, chloroisocyanuric acid, dichloroisocyanuric acid, N-chlorosuccinimide, 1,3-dichloro-5,5-dimethylhydantoin, and carbon tetrachloride, and
the reaction in step c3 is carried out in the presence of at least one selected from the group consisting of tris(2,4-di-*tert*-butylphenyl)phosphite, tri-o-tolyl phosphite, triphenyl phosphite, triethylphosphite, and triphenylphosphine.

37. The method according to any one of claims 34 to 36, wherein step c4 is carried out in the presence of at least one selected from the group consisting of cesium carbonate, potassium carbonate, sodium carbonate, lithium carbonate, sodium hydroxide, potassium hydroxide, lithium hydroxide, and 1,8-diazabicyclo[5.4.0]-7-undecene.

38. The method according to any one of claims 34 to 37, wherein the tritylating agent in step c6 is at least one selected from the group consisting of 4,4-dimethoxy trityl chloride, 4-methoxy trityl chloride, 2-chlorotrityl chloride, and trityl chloride.

39. The method according to any one of claims 35 to 38, wherein the acylating or alkoxycarbonylating agent in step c7 is at least one selected from the group consisting of benzoylating agents, 2-(trimethylsilyl)ethoxycarbonylating agents, *tert-*butoxycarbonylating agents, 9-fluorenylmethyloxycarbonylating agents, allyloxycarbonylating agents, 2,2,2-trichloroethoxycarbonylating agents, and benzyloxycarbonylating agents.

40. The method according to any one of claims 34 to 39, wherein the compound of formula (XXIII) is prepared by the following steps of:
(step c9) deprotecting a compound represented by formula (XXVIII): 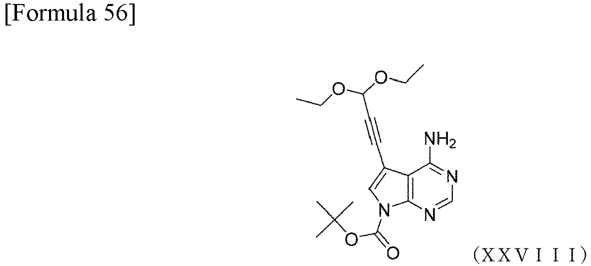 to remove a tert-butoxycarbonyl group, to obtain a compound represented by formula (XXIX): 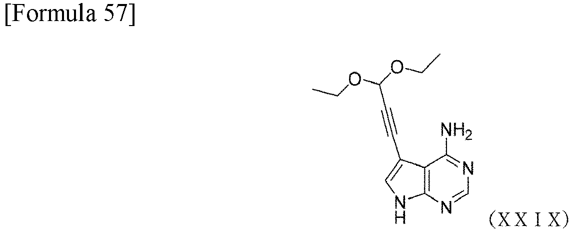
(step c 10) subjecting the resulting compound of formula (XXIX) to alkyne reduction reaction in the presence of a catalyst, followed by reductive amination reaction to obtain a compound represented by formula (XXX): 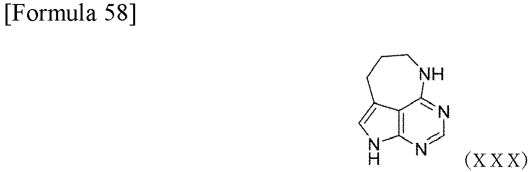 ; and
(step c11) reacting the resulting compound of formula (XXX) with a benzoylating agent to obtain a compound of formula (XXIII).

41. The method according to claim 40, wherein the compound of formula (XXVIII) is prepared by the following steps of:
(step c12) reacting a compound represented by formula (XXXI): 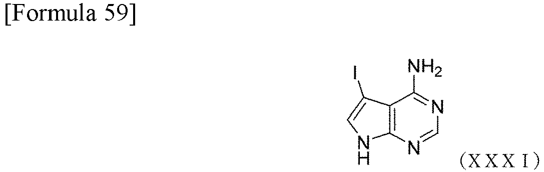 with a tert-butoxycarbonylating agent in the presence of 1-methylimidazole to obtain a compound represented by formula (XXXII): 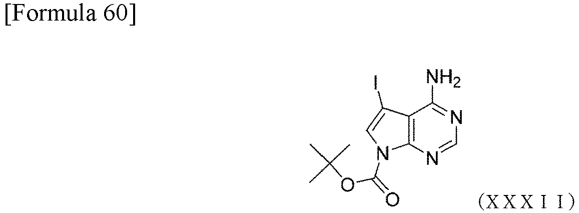 ; and
(step c13) reacting the resulting compound of formula (XXXII) with propargylaldehyde diethyl acetal to obtain a compound of formula (XXVIII).

42. The method according to any one of claims 34 to 39 and 41, wherein the compound of formula (XXIV) is prepared by, instead of step c4, the following steps:
(step c14) reacting the compound of formula (XXII) with the compound of formula (XXIX) to obtain a compound represented by formula (XXXIII): 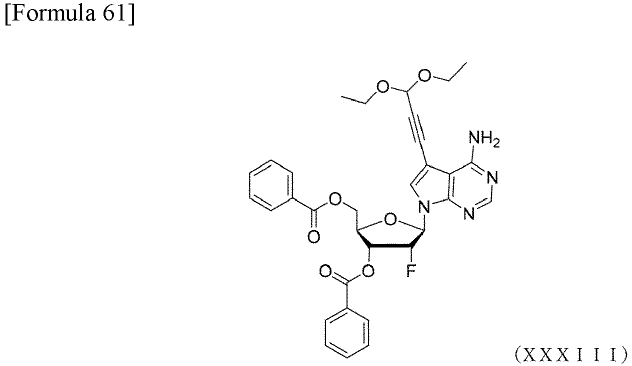 ; and
(step c 15) subjecting the resulting compound of formula (XXXIII) to alkyne reduction reaction in the presence of a catalyst, followed by reductive amination reaction and a further reaction with a benzoylating agent to obtain a compound of formula (XXIV).

43. A compound represented by formula (XX):

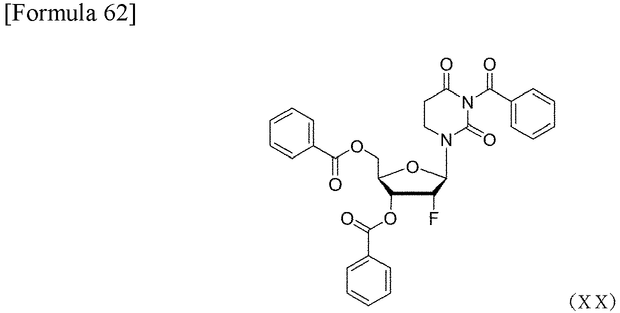

44. A compound represented by formula (XXII):

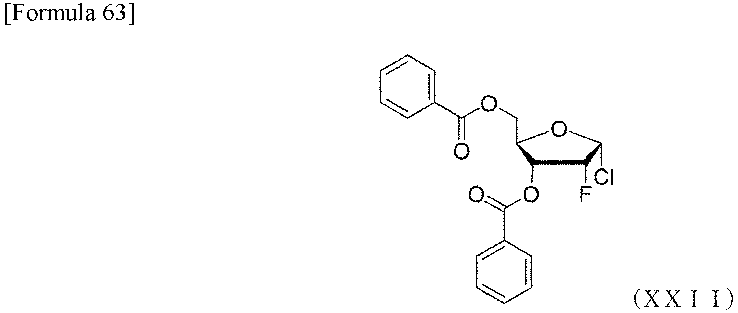

45. A compound represented by formula (XXIV):

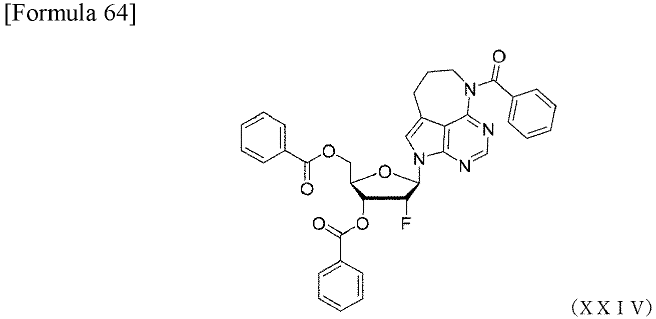

46. A compound represented by formula (XXV): 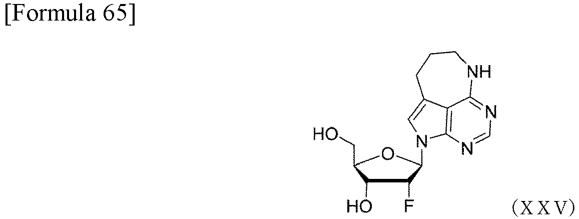 or a salt thereof.

47. A compound represented by formula (VIII'-1): 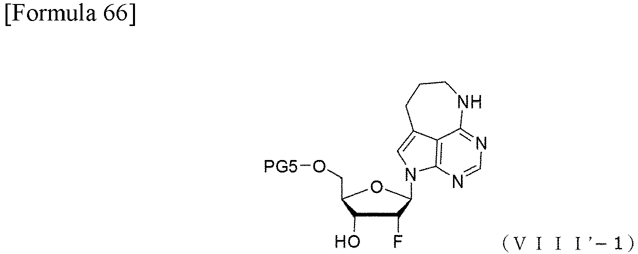 or a salt thereof, wherein PG5 is 4,4'-dimethoxytrityl, 4-methoxytrityl, 2-chlorotrityl, or trityl.

48. A compound represented by formula (XXVII): 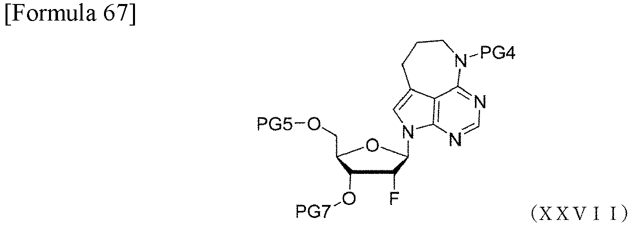 wherein
PG4 is benzoyl, 2-(trimethylsilyl)ethoxycarbonyl, tert-butoxycarbonyl, 9-fluorenylmethyloxycarbonyl, allyloxycarbonyl, 2,2,2-trichloroethoxycarbonyl, or benzyloxycarbonyl,
PG5 is 4,4'-dimethoxytrityl, 4-methoxytrityl, 2-chlorotrityl, or trityl, and
PG7 is trimethylsilyl, triethylsilyl, triisopropylsilyl, tert-butyldimethylsilyl, tert-butyldiphenylsilyl, or triphenylsilyl.

49. A compound represented by formula (XXVIII):
